(19) Europäisches Patentamt
European Patent Office
Office européen des brevets

(11) **EP 1 688 416 A1**

(12) **DEMANDE DE BREVET EUROPEEN**

(43) Date de publication:
**09.08.2006 Bulletin 2006/32**

(21) Numéro de dépôt: **06005775.9**

(22) Date de dépôt: **30.01.1996**

(51) Int Cl.:
***C07D 413/06** (2006.01)* ***C07D 401/06** (2006.01)*
***C07D 413/12** (2006.01)* ***C07D 265/30** (2006.01)*
***C07D 265/32** (2006.01)* ***C07D 453/02** (2006.01)*
***C07D 309/12** (2006.01)* ***C07D 263/38** (2006.01)*
***C07D 405/12** (2006.01)* ***C07C 219/22** (2006.01)*
***C07C 255/36** (2006.01)* ***C07C 233/18** (2006.01)*
***C07C 255/42** (2006.01)* ***C07C 271/22** (2006.01)*
***C07C 215/30** (2006.01)* ***A61K 31/445** (2006.01)*

(84) Etats contractants désignés:
**AT BE CH DE DK ES FR GB GR IE IT LI LU MC NL PT SE**
Etats d'extension désignés:
**LT LV SI**

(30) Priorité: **30.01.1995 FR 9501016**
**04.07.1995 FR 9508046**
**03.11.1995 FR 9513005**

(62) Numéro(s) de document de la (des) demande(s) initiale(s) en application de l'article 76 CBE:
**03012771.6 / 1 340 754**
**01119949.4 / 1 156 049**
**96902305.0 / 0 807 111**

(71) Demandeur: **Sanofi-Aventis**
**75013 Paris (FR)**

(72) Inventeurs:
- **Edmonds-Alts, Xavier**
**34980 Combaillaux (FR)**
- **Franceschini, Isabelle**
**75014 Paris (FR)**
- **Gueule, Patrick**
**34820 Teyran (FR)**
- **Proietto, Vincenzo**
**34680 Saint Georges D'Orques (FR)**
- **Van Broeck, Didier**
**34570 Murviel Les Montpellier (FR)**

(74) Mandataire: **Gillard, Marie-Louise et al**
**Cabinet Beau de Loménie**
**158, rue de l'Université**
**75340 Paris Cédex 07 (FR)**

Remarques:
Cette demande a été déposée le 21 - 03 - 2006 comme demande divisionnaire de la demande mentionnée sous le code INID 62.

# (54) Composés hétérocycliques substitués, procédé pour leur préparation et compositions pharmaceutiques les contenant

(57) L'invention a pour objet des composés de formule :

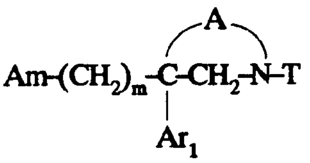

(I)

dans laquelle :

- A représente un radical bivalent choisi parmi :

$A_1$) *-O-CO-
$A_2$) $-CH_2-O-CO-$
$A_3$) $-O-CH_2-CO-$
$A_4$) $-O-CH_2-CH_2-$
$A_5$) $-N(R_1)-CO-$
$A_6$) $-N(R_1)-CO-CO-$
$A_7$) $-N(R_1)-CH_2-CH_2-$
$A_8$) $-O-CH_2-$

dans lesquels:

- $R_1$ représente un hydrogène ou un $(C_1-C_4)$alkyle;
- Am représente un hétérocycle azoté.

APPLICATION : Antagonistes des récepteurs des neurokinines

EP 1 688 416 A1

## Description

La présente invention a pour objet de nouveaux composés hétérocycliques substitués, un procédé pour leur préparation et les compositions pharmaceutiques en contenant en tant que principe actif.

Plus particulièrement, la présente invention concerne une nouvelle classe de composés hétérocycliques substitués à usage thérapeutique, dans les phénomènes pathologiques qui impliquent le système des tachykinines comme par exemple de manière non limitative et exclusive : la douleur (D. Regoli et aL, Life Sciences, 1987, 40, 109-117), l'allérgie et l'inflammation (J.E. Morlay et al., Life Sciences, 1987, 41, 527-544), l'insuffisance circulatoire (J. Losay et al., 1977, Substance P, Von Euler, I.S. and Pernow ed., 287-293, Raven Press, New York), les troubles gastro-intestinaux (D. Regoli et al., Trends Pharmacol. Sci., 1985, 6, 481-484), les troubles respiratoires (J. Mizrahi et aL, Pharmacology, 1982, 25, 39-50) les troubles neurologiques, les troubles neuropsychiatriques (C.A. Maggi et al., J. Autonomic. Pharmacol., 1993,13, 23-93).

Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$- La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.

Les récepteurs $NK_1$, $NK_2$, $NK_3$ ont été mis en évidence chez différentes espèces. Une revue de C.A. Maggi et aL fait le point sur les récepteurs aux tachykinines et leurs antagonistes et expose les études pharmacologiques et les applications en thérapeutique humaine (J. Autonomic Pharmacol., 1993, 13, 23-93).

Parmi les antagonistes spécifiques du récepteur $NK_1$ on peut citer les composés non peptidiques suivants : CP-96345 (J. Med. Chem., 1992, 35, 2591-2600), RP-68651 (Proc. Natl. Acad. Sci. USA, 1991, 88, 10208-10212), SR 140333 (Curr. J. Pharmacol., 1993, 250, 403-413).

Pour le récepteur $NK_2$, un antagoniste sélectif non peptidique, le SR 48968 a été décrit en détail (Life Sci., 1992, 50, PL101-PL106).

En ce qui concerne le récepteur $NK_3$, certains composés non peptidiques, ont été décrits, comme ayant une affinité pour le récepteur $NK_3$ du cerveau de rat et de cobaye (FASEB J., 1993, 7 (4), A710-4104) ; un antagoniste peptidique [$Trp^7$, $\beta Ala^8$]$NK_A$, faiblement spécifique du récepteur NK3 du rat a également été décrit (J. Autonomic. Pharmacol., 1993, 13, 23-93).

La demande de brevet EP-A-336230 décrit des dérivés peptidiques antagonistes de la substance P et de la neurokinine A utiles pour le traitement et la prévention de l'asthme.

Les demandes de brevet internationales WO 90/05525, WO 90/05729, WO 91/09844, WO 91/18899 et européennes EP-A-0436334, EP-A-0429466 et EP-A-0430771 décrivent des antagonistes de la Substance P.

Les demandes de brevet européennes EP-A-0474561, EP-A-512901, EP-A-515240, EP-A-559538 et EP-A-591040 concernent également des antagonistes des récepteurs des neurokinines.

On a maintenant trouvé des nouveaux composés hétérocycliques substitués qui sont des antagonistes des récepteurs des neurokinines.

Ainsi selon un de ses aspects, la présente invention a pour objet des composés de formule :

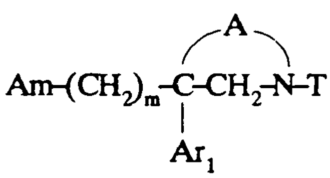

(I)

dans laquelle :

- A représente un radical bivalent choisi parmi :

  $A_1$)-O-CO-
  $A_2$) $-CH_2-O-CO-$
  $A_3$) $-O-CH_2-CO-$
  $A_4$) $-O-CH_2-CH_2-$
  $A_5$) $-N(R_1)-CO-$
  $A_6$) $-N(R_1)-CO-CO-$
  $A_7$) $-N(R_1)-CH_2-CH_2-$

$A_8$)-O-$CH_2$-

dans lesquels $R_1$ représente un hydrogène ou un ($C_1$-$C_4$)alkyle ;

- m est 2 ou 3;
- $Ar_1$ représente un phényle non substitué ou substitué une ou plusieurs fois par un substituant choisi parmi : un atome d'halogène, un hydroxy, un ($C_1$-$C_4$)alcoxy, un ($C_1$-$C_4$)alkyle, un trifluorométhyle, un méthylènedioxy, lesdits substituants étant identiques ou différents ; un thiényle non substitué ou substitué par un atome d'halogène ; un benzothiényle non substitué ou substitué par un atome d'halogène ; un naphtyle non substitué ou substitué par un atome d'halogène ; un indolyle non substitué ou N-substitué par un ($C_1$-$C_4$)alkyle ou un benzyle ; un imidazolyle non substitué ou substitué par un atome d'halogène ; un pyridyle non substitué ou substitué par un atome d'halogène ; un biphényle ;
- T représente un groupe choisi parmi : $CH_2$-Z, -CH($C_6H_5$)$_2$, -C($C_6H_5$)$_3$ ; T peut de plus représenter le groupe -CO-B-Z lorsque A représente un radical bivalent choisi parmi : -O-$CH_2$-$CH_2$- ou -N($R_1$)-$CH_2$-$CH_2$ - ou -O-$CH_2$- ;
- B représente une liaison directe ou un méthylène ;
- Z représente un groupe aromatique ou hétéroaromatique mono-, di- ou tricyclique éventuellement substitué ;
- Am représente:

i- soit un groupe $Am_1$ de formule :

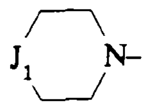

dans laquelle $J_1$ représente :

il - soit un groupe

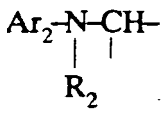

dans lequel :

- $Ar_2$ représente un pyridyle ; un phényle non substitué ou substitué une ou plusieurs fois par un substituant choisi parmi : un atome d'halogène, un hydroxy, un ($C_1$-$C_4$)alcoxy, un ($C_1$-$C_4$)alkyle, un trifluorométhyle, un nitro, un méthylènedioxy, lesdits substituants étant identiques ou différents ; un thiényle ; un pyrimidyle ; un imidazolyle non substitué ou substitué par un ($C_1$-$C_4$)alkyle ;
- $R_2$ représente un hydrogène ; un ($C_1$-$C_7$)alkyle ; un benzyle ; un formyle ; un ($C_1$-$C_7$)alkylcarbonyle ;

$i_2$- soit un groupe

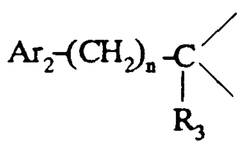

dans lequel :

- $Ar_2$ est tel que défini ci-dessus ;
- n est 0 ou 1 ;
- $R_3$ représente un groupe choisi parmi :

(1) hydrogène ;

(2) $(C_1-C_7)$alkyle ;
(3) formyle ;
(4) $(C_1-C_7)$alkylcarbonyle ;
(5) cyano ;
(6) $-(CH_2)_q-OH$ ;
(7) $-(CH_2)_q-O-(C_1-C_7)$alkyle;
(8) $-(CH_2)_q-OCHO$ ;
(9) $-(CH_2)_q-OCOR_{17}$ ;
(10) $-(CH_2)_q-OCONH-(C_1-C_7)$alkyle ;
(11) $-NR_4R_5$ ;
(12) $-(CH_2)_q-NR_6C(=W_1)R_7$ ;
(13) $-(CH_2)_q-NR_6COOR_8$;
(14) $-(CH_2)_q-NR_6SO_2R_9$ ;
(15) $-(CH_2)_q-NR_6C(=W_1)NR_{10}R_{11}$;
(16) $-CH_2-NR_{12}R_{13}$ ;
(17) $-CH_2-CH_2-NR_{12}R_{13}$ ;
(18) -COOH ;
(19) $(C_1-C_7)$alcoxycarbonyle ;
(20) $-C(=W_1)NR_{10}R_{11}$ ;
(21) $-CH_2-COOH$ ;
(22) $(C_1-C_7)$alcoxycarbonylméthyle ;
(23) $-CH_2-C(=W_1)NR_{10}R_{11}$ ;
(24) $-O-CH_2CH_2-OR_{18}$ ;
(25) $-NR_6COCOR_{19}$ ;
(26) $-CO-NR_{20}-NR_{21}R_{22}$ ;
(27)

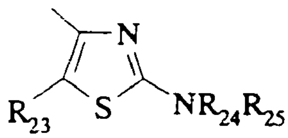 ;

(28)

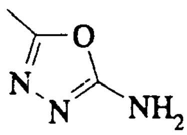 ;

- ou $R_3$ constitue une double liaison entre l'atome de carbone auquel il est lié et l'atome de carbone voisin du cycle pipéridine ;
- q est 0,1 ou 2 ;
- $W_1$ représente un atome d'oxygène ou un atome de soufre ;
- $R_4$ et $R_5$ représentent chacun indépendamment un hydrogène ou un $(C_1-C_7)$alkyle ; $R_5$ peut de plus représenter un $(C_3-C_7)$cycloalkylméthyle, un benzyle ou un phényle ; ou $R_4$ et $R_5$ ensemble avec l'atome d'azote auquel ils sont liés constituent un hétérocycle choisi parmi l'azétidine, la pyrrolidine, la pipéridine, la morpholine, la thiomorpholine, la perhydroazépine ou la pipérazine non substituée ou substituée en position 4 par un $(C_1-C_4)$alkyle ;
- $R_6$ représente un hydrogène ou un $(C_1-C_7)$alkyle ;
- $R_7$ représente un hydrogène ; un $(C_1-C_7)$alkyle ; un vinyle ; un phényle ; un benzyle ; un pyridyle ; un $(C_3-C_7)$cycloalkyle non substitué ou substitué par un ou plusieurs méthyles ; un furyle ; un thiényle ; un pyrolyle ; un imidazolyle ;
- ou $R_6$ et $R_7$ ensemble représentent un groupe $-(CH_2)_p-$
- p est 3 ou 4 ;

- $R_8$ représente un $(C_1-C_7)$alkyle ou un phényle ;
- $R_9$ représente un $(C_1-C_7)$alkyle ; un amino libre ou substitué par un ou deux $(C_1-C_7)$alkyles ; un phényle non substitué ou substitué une ou plusieurs fois par un substituant choisi parmi : un atome d'halogène, un $(C_1-C_7)$alkyle, un trifluorométhyle, un hydroxy, un $(C_1-C_7)$alcoxy, un carboxy, un $(C_1-C_7)$alcoxycarbonyle, un $(C_1-C_7)$alkylcarbonyloxy, un cyano, un nitro, un amino libre ou substitué par un ou deux $(C_1-C_7)$alkyles, lesdits substituants étant identiques ou différents ;
- $R_{10}$ et $R_{11}$ représentent chacun indépendamment un hydrogène ou un $(C_1-C_7)$alkyle ; $R_{11}$ peut de plus représenter un $(C_3-C_7)$cycloalkyle, un $(C_3-C_7)$cycloalkylméthyle, un hydroxy, un $(C_1-C_4)$alcoxy, un benzyle ou un phényle ; ou $R_{10}$ et $R_{11}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;
- $R_{12}$ et $R_{13}$ représentent chacun indépendamment un hydrogène ou un $(C_1-C_7)$alkyle ; $R_{13}$ peut de plus représenter un $(C_3-C_7)$cycloalkylméthyle ou un benzyle ;
- $R_{17}$ représente un $(C_1-C_7)$alkyle ; un $(C_3-C_7)$cycloalkyle non substitué ou substitué par un ou plusieurs méthyles ; un phényle ; un pyridyle ;
- $R_{18}$ représente un hydrogène ; un $(C_1-C_7)$alkyle ; un formyle ; un $(C_1-C_7)$alkylcarbonyle ;
- $R_{19}$ représente un $(C_1-C_4)$alcoxy ;
- $R_{20}$ représente un hydrogène ou un $(C_1-C_7)$alkyle ;
- $R_{21}$ et $R_{22}$ représentent chacun indépendamment un hydrogène ou un $(C_1-C_7)$alkyle ;
- ou bien $R_{21}$ et $R_{22}$ ensemble avec l'atome d'azote auquel ils sont liés constituent un hétérocycle choisi parmi la pyrrolidine, la pipéridine ou la morpholine ;
- $R_{23}$ représente un hydrogène ou un $(C_1-C_7)$alkyle ;
- $R_{24}$ et $R_{25}$ représentent chacun indépendamment un hydrogène ou un $(C_1-C_7)$alkyle ; $R_{25}$ peut de plus représenter un formyle ou un $(C_1-C_7)$alkylcarbonyle ;

$i_3$ - soit un groupe

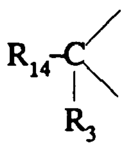

dans lequel :

- $R_3$ est tel que défini ci-dessus ;
- $R_{14}$ représente un $(C_1-C_7)$alkyle ; un $(C_3-C_7)$cycloalkyle ; $R_{14}$ peut de plus représenter soit un groupe $-CONR_{15}R_{16}$ lorsque $R_3$ représente l'hydrogène, soit un groupe $-NR_{15}R_{16}$ lorsque $R_3$ représente l'hydrogène, un cyano, un carboxy, un $(C_1-C_7)$alcoxycarbonyle ou un groupe $-C(=W_1)NR_{10}R_{11}$;
- $R_{15}$ et $R_{16}$ représentent chacun indépendamment un $(C_1-C_7)$alkyle ; ou $R_{15}$ et $R_{16}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;

ii- soit un groupe $Am_2$ de formule :

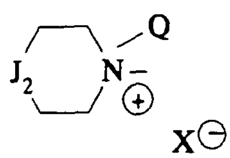

dans laquelle $J_2$ représente :

$ii_1$- soit un groupe

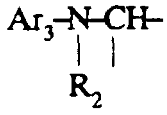

dans lequel :

- $Ar_3$ représente un phényle non substitué ou substitué une ou plusieurs fois par un substituant choisi parmi : un atome d'halogène, un hydroxy, un ($C_1$-$C_4$)alcoxy, un ($C_1$-$C_4$)alkyle, un trifluorométhyle, lesdits substituants étant identiques ou différents ;
- $R_2$ est tel que défini ci-dessus pour $J_1$ ;

$ii_2$ soit un groupe

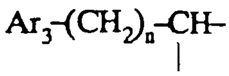

dans lequel :

- $Ar_3$ est tel que défini ci-dessus ;
- n est 0 ou 1 ;
- Q représente un ($C_1$-$C_6$)alkyle ou un benzyle ; ledit substituant étant soit en position axiale soit en position équatoriale ;
- $X^{\ominus}$ est un anion ;

iii- soit un groupe $Am_3$ de formule :

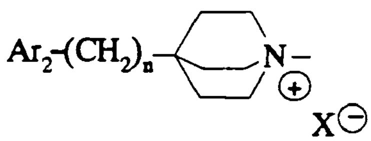

dans laquelle :

- $Ar_2$ est tel que défini ci-dessus ;
- n est 0 ou 1 ;
- $X^c$ représente un anion ;

iv- soit un groupe $Am_4$ de formule :

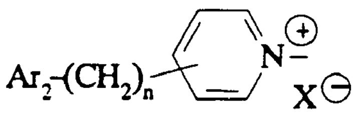

dans laquelle :

- $Ar_2$ est tel que défini ci-dessus ;
- n est 0 ou 1;
- $X^{\ominus}$ représente un anion ;

ainsi que leurs sels éventuels avec des acides minéraux ou organiques.

Les composés de formule (I) selon l'invention comprennent aussi bien les racémiques, les isoméres optiquement purs, ainsi que les isomères axiaux et équatoriaux lorsque dans le composé de formule (I), Am représente $Am_2$.

Plus particulièrement, le radical Z peut être un groupe phényle, qui peut être non substitué ou éventuellement contenir un ou plusieurs substituants.

Lorsque Z est un groupe phényle, celui-ci peut être mono substitué ou disubstitué notamment en position 2,4 mais aussi par exemple en position 2,3 ou 4,5 ou 3,4 ou 3,5 ; il peut aussi être trisubstitué, notamment en position 2,4,6 mais aussi par exemple en 2,3,4 ou 2,3,5 ou 2,4,5 ou 3,4,5 ; tétrasubstitué, par exemple en 2,3,4,5 ; ou pentasubstitué.

Le radical Z peut également représenter un groupe aromatique bicyclique tel que le 1- ou 2-naphtyle ; 1-, 2-, 3-, 4-, 5-, 6-, 7-indényle, dont une ou plusieurs liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, oxo, alkylcarbonylamino, alcoxycarbonyle, thioalkyle, halogène, alcoxy et trifluorométhyle, dans lesquels les alkyles sont en $C_1$-$C_4$.

Le radical Z peut être aussi un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino, alcoxycarbonyle, thioalkyle dans lesquels les alkyles sont en $C_1$-$C_4$.

Particulièrement, l'invention concerne des composés de formule (I) dans laquelle ; - Z est Z' et représente :

- un phényle non substitué ou substitué une ou plusieurs fois par un substituant choisi parmi : un atome d'halogène ; un trifluorométhyle ; un cyano ; un hydroxy ; un nitro ; un phényle non substitué ou substitué une ou plusieurs fois par un halogène, un trifluorométhyle, un ($C_1$-$C_4$)alkyle, un hydroxy, un ($C_1$-$C_4$)alcoxy, lesdits substituants étant identiques ou différents ; un amino non substitué ou substitué une ou plusieurs fois par un ($C_1$-$C_4$)alkyle ; un benzylamino ; un carboxy ; un ($C_1$-$C_{10}$)alkyle ; un ($C_3$-$C_8$)cycloalkyle non substitué ou substitué une ou plusieurs fois par un méthyle ; un ($C_1$-$C_{10}$)alcoxy ; un ($C_3$-$C_8$)cycloalkyloxy non substitué ou substitué une ou plusieurs fois par un méthyle ; un mercapto ; un ($C_1$-$C_{10}$)alkylthio ; un formyloxy ; un ($C_1$-$C_6$)alkylcarbonyloxy; un formylamino ; un ($C_1$-$C_6$)alkylcarbonylamino ; un benzoylamino ; un ($C_1$-$C_4$)alcoxycarbonyle ; un ($C_3$-$C_7$)cycloalkyloxycarbonyle ; un ($C_3$-$C_7$)cycloalkylcarbonyle ; un carbamoyle non substitué ou substitué une ou deux fois par un ($C_1$-$C_4$)alkyle ; un uréido non substitué ou substitué une ou deux fois en position 3 par un ($C_1$-$C_4$)alkyle ou un ($C_3$-$C_7$)cycloalkyle ; un (pyrrolidin-1-yl)carbonylamino, lesdits substituants étant identiques ou différents ;
- un naphtyle non substitué ou substitué une ou plusieurs fois par un halogène, un trifluorométhyle, un ($C_1$-$C_4$)alkyle, un hydroxy, un ($C_1$-$C_4$)alcoxy ;
- un pyridyle ; un thiényle ; un indolyle ; un quinolyle ; un benzothiényle ; un imidazolyle ;

Outre les sels d'ammonium quaternaire, on peut former des sels des composés de formule (I). Ces sels comprennent aussi bien ceux avec des acides minéraux et organiques qui permettent une séparation ou une cristallisation convenable des composés de formule (I), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le benzènesulfonate, le gluconate, le citrate, l'iséthionate, le *p*-toluènesulfonate.

Les anions $X^{\ominus}$ sont ceux normalement utilisés pour salifier les ions d'ammonium quaternaire, de préférence les ions chlorure, bromure, iodure, acétate, hydrogènosulfate, méthanesulfonate, paratoluènesulfonate, benzènesulfonate.

Préférentiellement, on utilise les anions pharmaceutiquement acceptables par exemple le chlorure, le méthanesulfonate ou benzènesulfonate.

Dans la présente description les groupes alkyles ou les groupes alcoxy sont droits ou ramifiés ; par atome d'halogène on entend un atome de chlore, de brome, de fluor ou d'iode.

Dans les substituants du groupe Z = phényle, par ($C_1$-$C_{10}$)alkyle on entend par exemple un méthyle, un éthyle, un n-propyle, un isopropyle, un n-butyle, un isobutyle, un *sec*-butyle, un *tert*-butyle, un pentyle ou n-pentyle, un hexyle ou n-hexyle, un heptyle ou un n-heptyle, un octyle ou n-octyle, un nonyle ou n-nonyle, un décyle ou n-décyle ; par ($C_3$-$C_8$) cycloalkyle éventuellement substitué par un méthyle on entend par exemple un cyclopropyle, un cyclobutyle, un cyclopentyle, un 1-, 2- ou 3-méthylcyclopentyle, un cyclohexyle, un 1-, 2-, 3- ou 4-méthyl-cyclohexyle, un cycloheptyle, ou un cyclooctyle ; par ($C_1$-$C_{10}$)alcoxy on entend par exemple un méthoxy, un éthoxy, un n-propoxy, un isopropoxy, un n-butoxy, un isobutoxy, un *sec*-butoxy, un *tert*-butoxy, un pentyloxy, un hexyloxy, un heptyloxy, un nonyloxy ou un décyloxy ;

par $(C_3-C_8)$cycloalkyloxy éventuellement substitué par un méthyle on entend par exemple un cyclopropyloxy, un cyclohexyloxy, un 1-, 2-, 3- ou 4-méthylcyclohexyloxy, un cycloheptyloxy ou un cyclooctyloxy ; par $(C_1-C_{10})$alkylthio on entend par exemple un méthylthio, un éthylthio, un n-propylthio, un isopropylthio, un n-butylthio, un isobutylthio, un *sec*-butylthio, un *tert*-butylthio, un pentylthio, un hexylthio, un heptylthio, un octylthio, un nonylthio ou un décylthio ; par $(C_1-C_6)$alkylcarbonyloxy on entend par exemple un acétyloxy, un propionyloxy, un butyryloxy, un valéryloxy, un caproyloxy, un heptanoyloxy ; par un $(C_1-C_6)$alkylcarbonylamino on entend par exemple un acétylamino, un propionylamino, un butyrylamino, un isobutyrylamino, un valérylamino, un caproylamino ou un heptanoylamino ; par $(C_1-C_4)$alcoxycarbonyle on entend par exemple un méthoxycarbonyle, un éthoxycarbonyle, un n-propoxycarbonyle, un isopropoxycarbonyle, un n-butoxycarbonyle, un isobutoxycarbonyle, un *sec*-butoxycarbonyle ou un *tert*-butoxycarbonyle ; par $(C_3-C_7)$cycloalkyloxycarbonyle on entend par exemple un cyclopropyloxycarbonyle, un cyclobutyloxycarbonyle, un cyclopentyloxycarbonyle, un cyclohexyloxycarbonyle ou un cycloheptyloxycarbonyle.

De manière avantageuse, le radical Z représente un phényle non substitué ou substitué une ou plusieurs fois par un atome d'halogène, plus particulièrement un atome de chlore, de fluor ou d'iode, un trifluorométhyle, un $(C_1-C_4)$ alkyle, un hydroxy, un $(C_1-C_4)$alcoxy ; un naphtyle non substitué ou substitué une ou plusieurs fois par un halogène, un trifluorométhyle, un $(C_1-C_4)$alkyle, un hydroxy, un $(C_1-C_4)$alcoxy ; un pyridyle ; un thiényle ; un indolyle ; un quinolyle ; un benzothiényle ; un imidazolyle.

Selon la présente invention, on préfère les composés de formule (I) dans laquelle :

- A représente un radical bivalent choisi parmi :

  $A_1$) -O-CO-
  $A_2$) $-CH_2-O-CO-$
  $A_3$) $-O-CH_2-CO-$
  $A_4$) $-O-CH_2-CH_2-$
  $A_5$) $-NR_1)-CO-$
  $A_6$) $-N(R_1)-CO-CO-$
  $A_7$) $-N(R_1)-CH_2-CH_2-$

  dans lesquels :

  - $R_1$ représente un hydrogène ou un $(C_1-C_4)$alkyle ;

  - Am représente :

    i- soit un groupe $Am_1$ de formule :

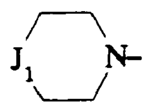

dans laquelle $J_1$ représente :
$i_1$ - soit un groupe

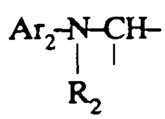

dans lequel :

- $Ar_2$ représente un pyridyle ; un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- $R_2$ représente un hydrogène ; un $(C_1-C_7)$alkyle ; un benzyle ; un formyle ; un $(C_1-C_7)$alkylcarbonyle ;

$i_2$- soit un groupe

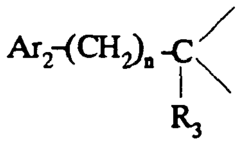

dans lequel :

- $Ar_2$ est tel que défini ci-dessus ;
- n est 0 ou 1 ;
- $R_3$ représente un hydrogène ; un ($C_1$-$C_7$)alkyle ; un formyle ; un ($C_1$-$C_7$)alkylcarbonyle ; un cyano ; un groupe -$(CH_2)_q$-OH ; un groupe ($C_1$-$C_7$)alkyl-O-$(CH_2)$q- ; un groupe HCOO-$(CH_2)_q$- ; un groupe ($C_1$-$C_7$) alkyl-COO-$(CH_2)_q$- ; un groupe ($C_1$-$C_7$)alkyl-NHCOO-$(CH_2)_q$- ; un groupe -$NR_4R_5$ ; un groupe -$(CH_2)_q$-$NR_6COR_7$ ; un groupe -$(CH_2)_q$-$NR_6COOR_8$ ; un groupe -$(CH_2)_q$-$NR_6SO_2R_9$ ; un groupe -$(CH_2)_q$-$NR_6CONR_{10}R_{11}$ ; un groupe -$CH_2$-$NR_{12}R_{13}$ ; un groupe -$CH_2$-$CH_2$-$NR_{12}R_{13}$ ; un carboxy ; un ($C_1$-$C_7$)alcoxycarbonyle ; un groupe -$CONR_{10}R_{11}$ ; un carboxyméthyle ; un ($C_1$-$C_7$) alcoxycarbonylméthyle ; un groupe -$CH_2$-$CONR_{10}R_{11}$ ; un 2-aminothiazol-4-yle dans lequel l'amino est libre ou substitué par un ou deux ($C_1$-$C_7$)alkyles ;
- ou $R_3$ constitue une double liaison entre l'atome de carbone auquel il est lié et l'atome de carbone voisin du cycle pipéridine ;
- q est 0, 1 ou 2 ;
- $R_4$ et $R_5$ représentent chacun indépendamment un hydrogène ou un ($C_1$-$C_7$)alkyle ; $R_5$ peut de plus représenter un ($C_3$-$C_7$)cycloalkylméthyle, un benzyle ou un phényle ; ou $R_4$ et $R_5$ ensemble avec l'atome d'azote auquel ils sont liés constituent un hétérocycle choisi parmi l'azétidine, la pyrrolidine, la pipéridine, la morpholiné, la thiomorpholine ou la perhydroazépine ;
- $R_6$ représente un hydrogène ou un ($C_1$-$C_4$)alkyle ;
- $R_7$ représente un hydrogène, un ($C_1$-$C_7$)alkyle, un vinyle, un phényle, un benzyle, un pyridyle ou un ($C_3$-$C_7$)cycloalkyle non substitué ou substitué par un ou plusieurs méthyles ;
- ou $R_6$ et $R_7$ ensemble représentent un groupe -$(CH_2)_p$- ;
- p est 3 ou 4 ;
- $R_8$ représente un ($C_1$-$C_7$)alkyle ou un phényle ;
- $R_9$ représente un ($C_1$-$C_7$)alkyle ; un amino libre ou substitué par un ou deux ($C_1$-$C_7$)alkyles ; un phényle non substitué ou substitué une ou plusieurs fois par un substituant choisi parmi : un atome d'halogène, un ($C_1$-$C_7$)alkyle, un trifluorométhyle, un hydroxy, un ($C_1$-$C_7$)alcoxy, un carboxy, un ($C_1$-$C_7$)alcoxycarbonyle, un ($C_1$-$C_7$)alkylcarbonyloxy, un cyano, un nitro, un amino libre ou substitué par un ou deux ($C_1$-$C_7$)alkyles, lesdits substituants étant identiques ou différents ;
- $R_{10}$ et $R_{11}$ représentent chacun indépendamment un hydrogène ou un ($C_1$-$C_7$)alkyle ; $R_{11}$ peut de plus représenter un ($C_3$-$C_7$)cycloalkyle, un ($C_3$-$C_7$)cycloalkylméthyle, un hydroxy, un ($C_1$-$C_4$)alcoxy, un benzyle ou un phényle ; ou $R_{10}$ et $R_{11}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;
- $R_{12}$ et $R_{13}$ représentent chacun indépendamment un hydrogène ou un ($C_1$-$C_7$)alkyle ; $R_{13}$ peut de plus représenter un ($C_3$-$C_7$)cycloalkylméthyle ou un benzyle ;

$i_3$ - soit un groupe

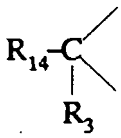

dans lequel :

- $R_3$ est tel que défini ci-dessus ;
- $R_{14}$ représente un ($C_1$-$C_7$)alkyle ; un ($C_3$-$C_7$)cycloalkyle ; $R_{14}$ peut de plus représenter soit un groupe

-$CONR_{15}R_{16}$ lorsque $R_3$ représente l'hydrogène, soit un groupe -$NR_{15}R_{16}$ lorsque $R_3$ représente un cyano, un carboxy, un ($C_{1-}C_7$)alcoxycarbonyle ou un groupe -$CONR_{10}R_{11}$.

- $R_{15}$ et $R_{16}$ représentent chacun indépendamment un ($C_1$-$C_7$)alkyle ; ou $R_{15}$ et $R_{16}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;

ii- soit un groupe $Am_2$ de formule :

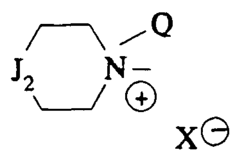

dans laquelle $J_2$ représente :
$ii_1$- soit un groupe

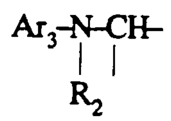

dans lequel :

- $Ar_3$ représente un phényle non substitué ou substitué une ou plusieurs fois par un substituant choisi parmi : un atome d'halogène, un hydroxy, un ($C_1$-$C_4$)alcoxy, un ($C_1$-$C_4$)alkyle, un trifluorométhyle, lesdits substituants étant identiques ou différents ;

- $R_2$ est tel que défini ci-dessus pour $J_1$ ;

$ii_2$- soit un groupe

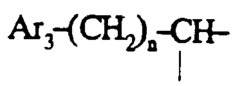

dans lequel :

- $Ar_3$ est tel que défini ci-dessus ;
- n est 0 ou 1 ;
- Q représente un ($C_1$-$C_6$)alkyle ou un benzyle ; ledit substituant étant soit en position axiale soit en position équatoriale ;
- $X^{\ominus}$ est un anion ;

iii- soit un groupe $Am_3$ de formule :

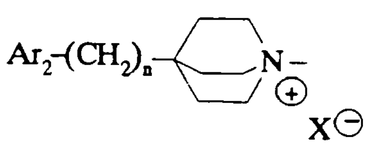

dans laquelle :

- $Ar_2$ est tel que défini ci-dessus ;
- n est 0 ou 1 ;
- $X^{\ominus}$ représente un anion ;

iv- soit un groupe $Am_4$ de formule :

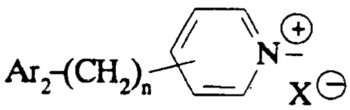

dans laquelle :

- $Ar_2$ est tel que défini ci-dessus ;
- n est 0 ou 1 ;
- $X^{\ominus}$ représente un anion ;
- m est 2 ou 3 ;
- $Ar_1$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ; un thiényle ; un benzothiényle ; un naphtyle ; un indolyle non substitué ou N-substitué par un $(C_1-C_4)$alkyle ou un benzyle ;
- T représente un groupe choisi parmi : $-CH_2-Z$, $-CH(C_6H_5)_2$, $-C(C_6H_5)_3$ ; T peut de plus représenter le groupe -CO-B-Z lorsque A représente un radical bivalent choisi parmi : $-O-CH_2-CH_2-$ ou $-N(R_1)-CH_2-CH_2-$ ;
- B représente une liaison directe ou un méthylène ;
- Z représente :
  - un phényle non substitué ou substitué une ou plusieurs fois par un substituant choisi parmi : un atome d'halogène ; un trifluorométhyle ; un cyano ; un hydroxy ; un nitro ; un phényle non substitué ou substitué une ou plusieurs fois par un halogène, un trifluorométhyle, un $(C_1-C_4)$alkyle, un hydroxy, un $(C_1-C_4)$alkoxy, lesdits substituants étant identiques ou différents ; un amino non substitué ou substitué une ou plusieurs fois par un $(C_1-C_4)$alkyle ; un benzylamino ; un carboxy ; un $(C_1-C_{10})$alkyle ; un $(C_3-C_7)$cycloalkyle non substitué ou substitué une ou plusieurs fois par un méthyle ; un $(C_1-C_{10})$alcoxy ; un $(C_3-C_7)$cycloalkyloxy non substitué ou substitué une ou plusieurs fois par un méthyle ; un mercapto ; un $(C_1-C_{10})$alkylthio ; un $(C_1-C_6)$alkylcarbonyloxy ; un $(C_1-C_6)$alkylcarbonylamino ; un benzoylamino ; un $(C_1-C_4)$alcoxycarbonyle ; un $(C_3-C_7)$cycloalkylcarbonyle ; un carbamoyle non substitué ou substitué une ou deux fois par un $(C_1-C_4)$alkyle ; un uréido non substitué ou substitué une ou deux fois en position 3 par un $(C_1-C_4)$alkyle ou un $(C_3-C_7)$cycloalkyle ; un (pyrrolidin-1-yl)-carbonylamino, lesdits substituants étant identiques ou différents ;
  - un naphtyle non substitué ou substitué une ou plusieurs fois par un halogène, un trifluorométhyle, un $(C_1-C_4)$alkyle, un hydroxy, un $(C_1-C_4)$alcoxy ;
  - un pyridyle ; un thiényle ; un indolyle ; un quinolyle ; un benzothiényle ; .un imidazolyle ;

ainsi que leurs sels éventuels avec des acides minéraux ou organiques.

On préfère également les composés de formule (I) dans laquelle :

- A représente un radical bivalent choisi parmi :

  $A_1$) -O-CO-
  $A_2$) $-CH_2-O-CO-$
  $A_3$) $-O-CH_2-CO-$
  $A_4$) $-O-CH_2-CH_2-$
  $A_5$) $-N(R_1)-CO-$
  $A_6$) $-N(R_1)-CO-CO-$
  $A_7$) $-N(R_1)-CH_2-CH_2-$

dans lesquels $R_1$ représente un hydrogène ou un $(C_1-C_4)$alkyle ;

- m est 2 ou 3;
- $Ar_1$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, un méthylènedioxy, lesdits substituants étant identiques ou différents ; un thiényle non substitué ou substitué par un atome d'halogène ; un benzothiényle non substitué ou substitué par un atome d'halogène ; un naphtyle non substitué ou substitué par un atome d'halogène ; un indolyle non substitué ou N-substitué par un $(C_1-C_4)$alkyle ou un benzyle ; un imidazolyle non substitué ou substitué par un atome d'halogène ; un pyridyle non substitué ou substitué par un atome d'halogène ; un biphényle ;
- T représente un groupe choisi parmi : $CH_2$-Z, $-CH(C_6H_5)_2$, $-C(C_6H_5)_3$ ; T peut de plus représenter le groupe -CO-B-Z lorsque A représente un radical bivalent choisi parmi : $-O-CH_2-CH_2-$ ou $-N(R_1)-CH_2-CH_2-$;
- B représente une liaison directe ou un méthylène ;
- Z représente un groupe aromatique ou hétéroaromatique mono-, di- ou tricyclique éventuellement substitué;
- Am représente :

i- soit un groupe $Am_1$ de formule :

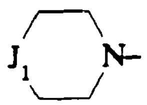

dans laquelle $J_1$ représente :
il - soit un groupe

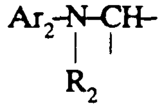

dans lequel :

- $Ar_2$ représente un pyridyle ; un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, un nitro, un méthylènedioxy, lesdits substituants étant identiques ou différents ; un thiényle ; un pyrimidyle ; un imidazolyle non substitué ou substitué par un $(C_1-C_4)$alkyle ;
- $R_2$ représente un hydrogène ; un $(C_1-C_7)$alkyle ; un benzyle ; un formyle ; un $(C_1-C_7)$alkylcarbonyle ;

$i_2$ soit un groupe

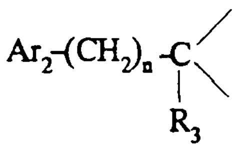

dans lequel :

- $Ar_2$ est tel que défini ci-dessus ;
- n est 0 ou 1;
- $R_3$ représente un groupe choisi parmi:

(1) hydrogène ;
(2) $(C_1-C_7)$alkyle ;
(3) formyle ;

(4) $(C_1-C_7)$alkylcarbonyle
(5) cyano ;
(6) $-(CH_2)_q-OH$;
(7) $-(CH_2)_q-O-(C_1-C_7)$alkyle;
(8) $-(CH_2)q-OCHO$;
(9) $-(CH_2)q-OCOR_{17}$;
(10) $-(CH_2)_q-OCONH-(C_1-C_7)$alkyle ;
(11) $-NR_4R_5$;
(12) $-(CH_2)_q-NR_6C(=W_1)R_7$ ;
(13) $-(CH_2)_q-NR_6COOR_8$;
(14) $-(CH_2)_q-NR_6SO_2R_9$;
(15) $-(CH_2)_q-NR_6C(=W_1)NR_{10}R_{11}$ ;
(16) $-CH_2-NR_{12}R_{13}$ ;
(17) $-CH_2-CH_2-NR_{12}R_{13}$ ;
(18) -COOH ;
(19) $(C_1-C_7)$alcoxycarbonyle ;
(20) $-C(=W_1)NR_{10}R_{11}$ ;
(21) $-CH_2-COOH$ ;
(22) $(C_1-C_7)$alcoxycarbonylméthyle ;
(23) $-CH_2-C(=W_1)NR_{10}R_{11}$ ;
(24) $-O-CH_2CH_2-OR_{18}$ ;
(25) $-NR_6COCOR_{19}$ ;
(26) $-CO-NR_{20}-NR_{21}R_{22}$ ;
(27)

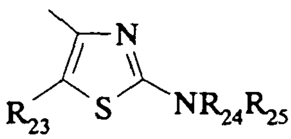 ;

(28)

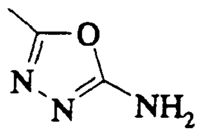 ;

- ou $R_3$ constitue une double liaison entre l'atome de carbone auquel il est lié et l'atome de carbone voisin du cycle pipéridine ;
- q est 0,1 ou 2;
- $W_1$ représente un atome d'oxygène ou un atome de soufre ;
- $R_4$ et $R_5$ représentent chacun indépendamment un hydrogène ou un $(C_1-C_7)$alkyle ; $R_5$ peut de plus représenter un $(C_3-C_7)$cycloalkylméthyle, un benzyle ou un phényle ; ou $R_4$ et $R_5$ ensemble avec l'atome d'azote auquel ils sont liés constituent un hétérocycle choisi parmi l'azétidine, la pyrrolidine, la pipéridine, la morpholine, la thiomorpholine, la perhydroazépine ou la pipérazine non substituée ou substituée en position 4 par un $(C_1-C_4)$alkyle ;
- $R_6$ représente un hydrogène ou un $(C_1-C_4)$alkyle ;
- $R_7$ représente un hydrogène ; un $(C_1-C_7)$alkyle ; un vinyle ; un phényle ; un benzyle ; un pyridyle ; un $(C_3-C_7)$cycloalkyle non substitué ou substitué par un ou plusieurs méthyles ; un furyle ; un thiényle ; un pyrrolyle ; un imidazolyle ;
- ou $R_6$ et $R_7$ ensemble représentent un groupe $-(CH_2)_p-$;
- p est 3 ou 4 ;
- $R_8$ représente un $(C_1-C_7)$alkyle ou un phényle ;

- $R_9$ représente un ($C_1$-$C_7$)alkyle ; un amino libre ou substitué par un ou deux ($C_1$-$C_7$)alkyles ; un phényle non substitué ou substitué une ou plusieurs fois par un substituant choisi parmi : un atome d'halogène, un ($C_1$-$C_7$)alkyle, un trifluorométhyle, un hydroxy, un ($C_1$-$C_7$)alcoxy, un carboxy, un ($C_1$-$C_7$)alcoxycarbonyle, un ($C_1$-$C_7$)alkylcarbonyloxy, un cyano, un nitro, un amino libre ou substitué par un ou deux ($C_1$-$C_7$)alkyles, lesdits substituants étant identiques ou différents ;
- $R_{10}$ et $R_{11}$ représentent chacun indépendamment un hydrogène ou un ($C_1$-$C_7$)alkyle ; $R_{11}$ peut de plus représenter un ($C_3$-$C_7$)cycloalkyle, un ($C_3$-$C_7$)cycloalkylméthyle, un hydroxy, un ($C_1$-$C_4$) alcoxy, un benzyle ou un phényle ; ou $R_{10}$ et $R_{11}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;
- $R_{12}$ et $R_{13}$ représentent chacun indépendamment un hydrogène ou un ($C_1$-$C_7$)alkyle ; $R_{13}$ peut de plus représenter un ($C_3$-$C_7$)cycloalkylméthyle ou un benzyle ;
- $R_{17}$ représente un ($C_1$-$C_7$)alkyle ; un ($C_3$-$C_7$)cycloalkyle non substitué ou substitué par un ou plusieurs méthyles ; un phényle ; un pyridyle ;
- $R_{18}$ représente un hydrogène ; un ($C_1$-$C_7$)alkyle ; un formyle ; un ($C_1$-$C_7$)alkylcarbonyle ;
- $R_{19}$ représente un ($C_1$-$C_4$)alcoxy ;
- $R_{20}$ représente un hydrogène ou un ($C_1$-$C_7$)alkyle
- $R_{21}$ et $R_{22}$ représentent chacun indépendamment un hydrogène ou un ($C_1$-$C_7$)alkyle ;
- ou bien $R_{21}$ et $R_{22}$ ensemble avec l'atome d'azote auquel ils sont liés constituent un hétérocycle choisi parmi la pyrrolidine, la pipéridine ou la morpholine ;
- $R_{23}$ représente un hydrogène ou un ($C_1$-$C_7$)alkyle ;
- $R_{24}$ et $R_{25}$ représentent chacun indépendamment un hydrogène ou un ($C_1$-$C_7$)alkyle ; $R_{25}$ peut de plus représenter un formyle ou un ($C_1$-$C_7$)alkylcarbonyle ;

$i_3$ - soit un groupe

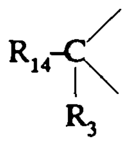

dans lequel :

- $R_3$ est tel que défini ci-dessus ;
- $R_{14}$ représente un ($C_1$-$C_7$)alkyle ; un ($C_3$-$C_7$)cycloalkyle ; $R_{14}$ peut de plus représenter soit un groupe $-CONR_{15}R_{16}$ lorsque $R_3$ représente l'hydrogène, soit un groupe $-NR_{15}R_{16}$ lorsque $R_3$ représente un cyano, un carboxy, un ($C_1$-$C_7$)alcoxycarbonyle ou un groupe $-C(=W_1)NR_{10}R_{11}$ ;
- $R_{15}$ et $R_{16}$ représentent chacun indépendamment un ($C_1$-$C_7$)alkyle ; ou $R_{15}$ et $R_{16}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;

ii- soit un groupe $Am_2$ de formule :

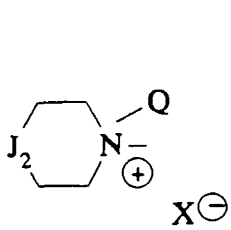

dans laquelle $J_2$ représente :

$ii_1$- soit un groupe

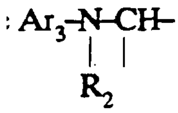

dans lequel :

- $Ar_3$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- $R_2$ est tel que défini ci-dessus pour $J_1$;

$ii_2$- soit un groupe

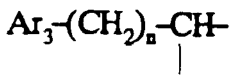

dans lequel :

- $Ar_3$ est tel que défini ci-dessus ;
- n est 0 ou 1 ;
- Q représente un $(C_1-C_6)$alkyle ou un benzyle ; ledit substituant étant soit en position axiale soit en position équatoriale ;
- $X^{\ominus}$ est un anion ;

iii- soit un groupe $Am_3$ de fonnule :

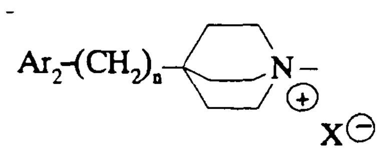

dans laquelle :

- $Ar_2$ est tel que défini ci-dessus ;
- n est 0 ou 1 ;
- $X^{\ominus}$ représente un anion ;

iv- soit un groupe $Am_4$ de formule :

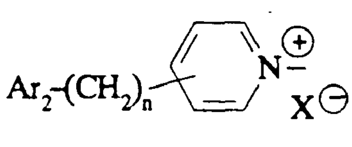

dans laquelle :

- $Ar_2$ est tel que défini ci-dessus ;
- n est 0 ou 1 ;
- $X^{\ominus}$ représente un anion ;

ainsi que leurs sels éventuels avec des acides minéraux ou organiques.

Un autre groupe de composés préférés selon la présente invention sont ceux de formule :

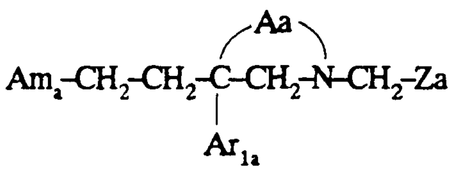

(Ia)

dans laquelle :

- Aa représente un radical bivalent choisi parmi : -O-CO- ; $-CH_2$-O-CO- ; -O-$CH_2$-CO- ; -N($R_1$)-CO- ou -N($R_1$)-CO-CO- ;
  dans lesquels $R_1$ représente un hydrogène ou un ($C_1$-$C_4$)alkyle ;
- $Am_a$ représente :

  * soit un groupe $Am_{2a}$ de formule :

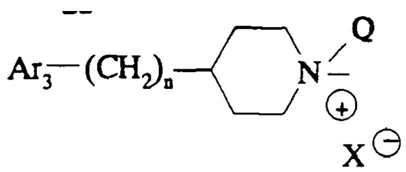

  * soit un groupe $Am_3$ de formule :

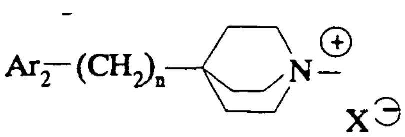

- n est 0 ou 1;
- Q est tel que défini précédemment pour un composé de formule (I) et est en position axiale ;
- $X^{\ominus}$ représente un anion pharmaceutiquement acceptable ;
- $Ar_2$ et $Ar_3$ sont tels que définis précédemment pour un composé de formule (I) ;
- $Ar_{1a}$ représente un phényle non substitué ou substitué une ou plusieurs fois par un substituant choisi parmi : un atome d'halogène, un hydroxy, un ($C_1$-$C_4$)alcoxy, un ($C_1$-$C_4$)alkyle, un trifluorométhyle, lesdits substituants étant identiques ou différents ;
- Za représente un phényle non substitué ou substitué une ou plusieurs fois par un substituant choisi parmi : un atome d'halogène, un trifluorométhyle, un ($C_1$-$C_{10}$)alkyle, un ($C_1$-$C_{10}$)alcoxy, un hydroxy, lesdits substituants étant identiques ou différents.

Parmi ces composés, ceux de formule :

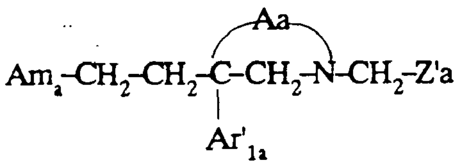

(I'a)

dans laquelle :

- Aa et $Am_a$ sont tels que définis ci-dessus pour un composé de formule (Ia) ;
- $Ar'_{1a}$ représente un 3,4-dichlorophényle ou un 3,4-difluorophényle ;

- Z'a représente un 3,5-bis(trifluorométhyl)phényle, un 3,5-diméthylphényle ou un 2,4-bis(trifluorométhyl)phényle ;

sont particulièrement préférés.

Un autre groupe de composés préférés selon l'invention sont ceux de formule :

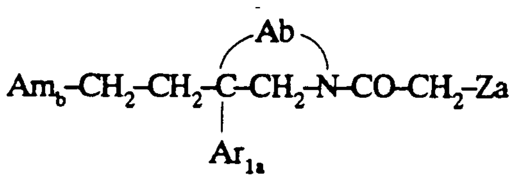

(Ib)

dans laquelle :

- Ab représente le radical bivalent $-O-CH_2-CH_2-$ ; $-N(R_1)-CH_2-CH_2-$ ou $-O-CH_2-$; dans lequel $R_1$ représente un hydrogène ou un $(C_1-C_4)$alkyle ;
- $Am_b$ représente :

  * soit un groupe $Am_{2a}$ de formule :

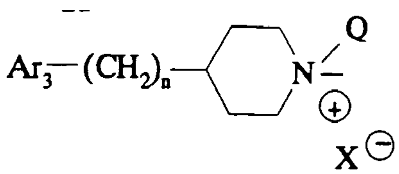

  * soit un groupe $Am_3$ de formule :

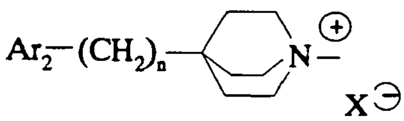

  * soit un groupe $Am_{1a}$ de formule :

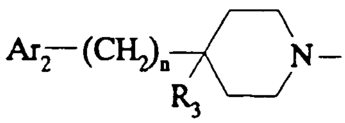

- n est 0 ou 1;
- Q est tel que défini précédemment pour un composé de formule (I) et est en position axiale ;
- $X^{\ominus}$ représente un anion pharmaceutiquement acceptable ;
- $Ar_2$, $Ar_3$ et $R_3$ sont tels que définis précédemment pour un composé de formule (I) ;
- $Ar_{1a}$ et Za sont tels que définis précédemment ;

et leurs sels avec des acides minéraux ou organiques.

Parmi ces composés, ceux de formule :

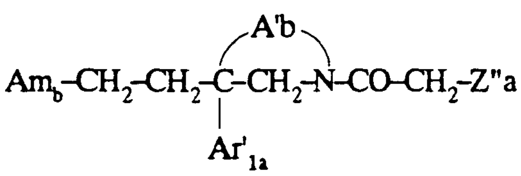

(I'b)

dans laquelle :

- $Am_b$ est tel que défini ci-dessus pour un composé de formule (Ib) ;
- A'b représente le radical bivalent $-O-CH_2-CH_2-$ ou $-N(R_1)-CH_2-CH_2$ ;
- $Ar'_{1a}$ est tel que défini ci-dessus pour un composé de formule (I'a) ;
- Z"a représente un phényle substitué en position 3 par un halogène ou un groupe $(C_1-C_{10})$alcoxy ;

et leurs sels avec des acides minéraux ou organiques, sont particulièrement préférés.

Un autre groupe de composés préférés selon l'invention sont ceux de formule :

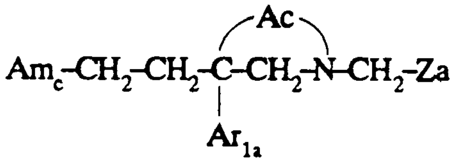

(Ic)

dans laquelle :

- Ac représente un radical bivalent choisi parmi : $-O-CH_2-CO-$ ; $-CH_2-O-CO-$ ou $-O-CO-$;

- $Am_c$ représente un groupe $Am_{1a}$ de formule :

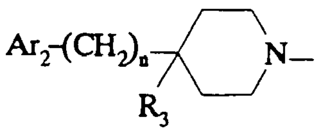

- n est 0 ou 1 ;

- $Ar_2$ et $R_3$ sont tels que définis précédemment pour un composé de formule (I) ;

- $Ar_{1a}$ et Za sont tels que définis précédemment ;

et leurs sels avec des acides minéraux ou organiques.

Parmi ces composés, ceux de formule :

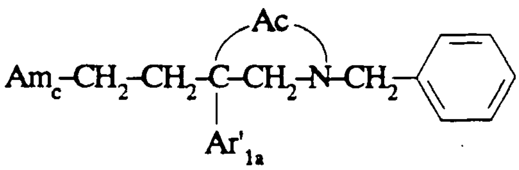

(I'c)

dans laquelle :

- Ac, $Am_c$, $Ar'_{1a}$ sont tels que définis précédemment ;

et leurs sels avec des acides minéraux ou organiques, sont particulièrement préférés.

Un autre groupe de composés préférés selon l'invention sont ceux de formule :

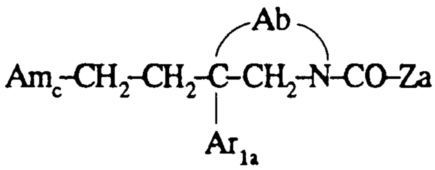

(Id)

dans laquelle :

- Ab, $Am_c$, $Ar_{1a}$, et Za sont tels que définis précédemment ;

et leurs sels avec des acides minéraux ou organiques.

Parmi ces composés, ceux de formule :

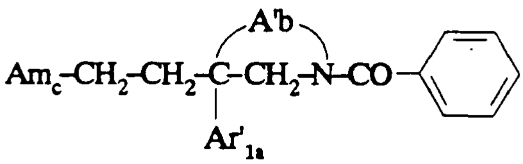

(I'd)

dans laquelle :

- A'b, $Am_c$ et $Ar'_{1a}$ sont tels que définis précédemment ;

et leurs sels avec des acides minéraux ou organiques, sont particulièrement préférés.

Parmi les composés de formule (Ia), (I'a) ci-dessus on préfère tout particulièrement ceux dans lesquels $Ar_2$ représente un pyridyle ; un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;

et leurs sels avec des acides minéraux ou organiques

Parmi les composés de formule (I'b), (Ic), (I'c) et (I'd) ci-dessus, on préfère tout particulièrement ceux dans lesquels :

- $Ar_2$ représente un pyridyle ; un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- $R_3$ représente un hydrogène ; un $(C_1-C_7)$alkyle ; un formyle ; un $(C_1-C_7)$alkylcarbonyle ; un cyano ; un groupe $-(CH_2)_q-OH$ ; un groupe $(C_1-C_7)$alkyl-O-$(CH_2)_q$- ; un groupe HCOO-$(CH_2)$q- ; un groupe $(C_1-C_7)$alkyl-COO-$(CH_2)$ q- ; un groupe $(C_1-C_7)$alkyl-NHCOO-$(CH_2)_{q-}$ ; un groupe $-NR_4R_5$ ; un groupe $-(CH_2)q-NR_6COR_7$ ; un groupe $-(CH_2)$ q-$NR_6COOR_8$ ; un groupe $-(CH_2)_q-NR_6SO_2R_9$ ; un groupe $-(CH_2)_q-NR_6CONR_{10}R_{11}$ ; un groupe $-CH_2-NR_{12}P_{13}$; un groupe $-CH_2-CH_2-NR_{12}R_{13}$ ; un carboxy ; un $(C_1-C_7)$alcoxycarbonyle ; un groupe $-CONR_{10}R_{11}$ ; un carboxyméthyle ; un $(C_1-C_7)$alcoxycarbonylméthyle ; un groupe $-CH_2-CONR_{10}R_{11}$ ; un 2-aminothiazol-4-yle dans lequel l'amino est libre ou substitué par un ou deux $(C_1-C_7)$alkyles;
- ou $R_3$ constitue une double liaison entre l'atome de carbone auquel il est lié et l'atome de carbone voisin du cycle pipéridine ;
- q est 0, 1 ou 2;

et leurs sels avec des acides minéraux ou organiques.

Parmi les composés de formules (Ib) et (Id), on préfère tout particulièrement ceux dans lesquels Ab représente le radical bivalent $-O-CH_2-CH_2-$ et $-N(R_1)-CH_2-CH_2-$ ;

- $Ar_2$ représente un pyridyle ; un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- $R_3$ représente un hydrogène ; un $(C_1-C_7)$alkyle ; un formyle ; un $(C_1-C_7)$alkylcarbonyle ; un cyano ; un groupe

$-(CH_2)_q-OH$; un groupe $(C_1-C_7)alkyl-O-(CH_2)_q-$ ; un groupe $HCOO-(CH_2)q-$ ; un groupe $(C_1-C_7)alkyl-COO-(CH_2)q-$; un groupe $(C_1-C_7)alkyl-NHCOO-(CH_2)_q-$ ; un groupe $-NR_4R_5$ ; un groupe $-(CH_2)_q-NR_6COR_7$ ; un groupe $-(CH_2)_q-NR_6COOR_8$; un groupe $-(CH_2)_q-NR_6SO_2R_9$; un groupe $-(CH_2)q-NR_6CONR_{10}R_{11}$; un groupe $-CH_2-NR_{12}R_{13}$ ; un groupe $-CH_2-CH_2-NR_{12}R_{13}$ ; un carboxy; un $(C_1-C_7)$alcoxycarbonyle ; un groupe $-CONR_{10}R_1$ ; un carboxyméthyle ; un $(C_1-C_7)$alcoxycarbonylméthyle ; un groupe $-CH_2-CONR_{10}R_{11}$; un 2-aminothiazol-4-yle dans lequel l'amino est libre ou substitué par un ou deux $(C_1-C_7)$alkyles ;

- ou $R_3$ constitue une double liaison entre l'atome de carbone auquel il est lié et l'atome de carbone voisin du cycle pipéridine ;
- q est 0,1 ou 2;

et leurs sels avec des acides minéraux ou organiques.

Un autre groupe de composés préférés selon l'invention sont ceux de formule :

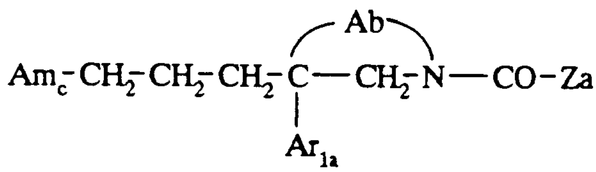

(Ie)

dans laquelle :

- Ab, $Am_c$, $Ar_{1a}$, et Za sont tels que définis précédemment ;

et leurs sels avec des acides minéraux ou organiques.

Un autre groupe de composés préférés selon l'invention sont ceux de formule :

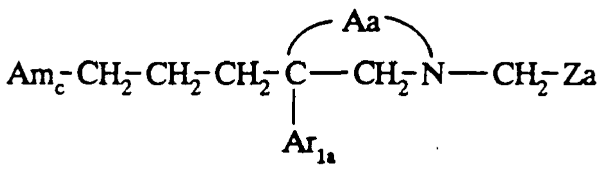

(If)

dans laquelle :

- Aa, $Am_c$, $Ar_{1a}$, et Za sont tels que définis précédemment ;

et leurs sels avec des acides minéraux ou organiques.

Le 4-benzoyl-2-(3,4-difluorophényl)-2-[2-[4-(N',N'-diméthyluréido)-4-phényl pipérid-1-yl]éthyl]morpholine, sous forme optiquement pure, préférentiellement sous forme de l'isomère (+) et ses sels avec des acides minéraux ou organiques sont tout particulièrement préférés.

Le 5-(3,4-difluorophényl)-5-[2-[4-phényl-1-azoniabicyclo[2.2.2]oct-1-yl]éthyl]-3-[3,5-bis(trifluorométhyl)bcnzyl] oxazolidin-2-one, avec un anion pharmaceutiquement acceptable, sous forme optiquement pure, préférentiellement sous forme de l'isomère (+), est tout particulièrement préféré.

Selon un autre de ses aspects, la présente invention concerne un procédé pour la préparation des composés de formule (I) et de leurs sels, caractérisé en ce que :

1) on traite un composé de formule :

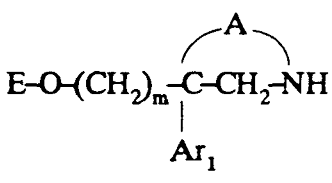

(II)

dans laquelle m, $Ar_1$ et A sont tels que définis précédemment pour un composé de formule (I) et E représente l'hydrogène ou un groupe O-protecteur,

- soit avec un dérivé fonctionnel d'un acide de formule :

HOCO-B-Z (III)

dans laquelle B et Z sont tels que définis précédemment pour (I), lorsqu'on doit préparer un composé de formule (I) où T est -CO-B-Z,

- soit avec un dérivé halogéné de formule :

Hal-$CH_2$-Z (IV)

dans laquelle Z est tel que défini précédemment, et Hal représente un halogène, préférentiellement le brome ou le chlore, lorsqu'on doit préparer un composé de formule (I) où T est -$CH_2$-Z,

- soit avec un dérivé halogéné de formule :

Hal-$CH(C_6H_5)_2$ (V)

lorsqu'on doit préparer un composé de formule (I) où T est un groupe -$CH(C_6H_5)_2$,

- soit avec un dérivé halogéné de formule :

Hal-C-$(C_6H_5)_3$ (VI)

lorsqu'on doit préparer un composé de formule (I) où T est un groupe -$C(C_6H_5)_3$, pour obtenir un composé de formule :

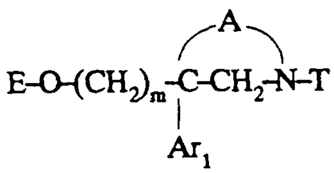

(VII)

2) on élimine éventuellement le groupe O-protecteur par action d'un acide ou d'une base, pour obtenir l'alcool de formule :

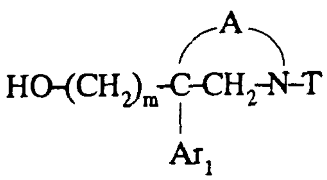

(VIII)

3) on traite l'alcool (VIII) avec un composé de formule :

Y-$SO_2$-Cl (IX)

dans laquelle Y représente un groupe méthyle, phényle, tolyle, trifluorométhyle, pour obtenir un composé de formule :

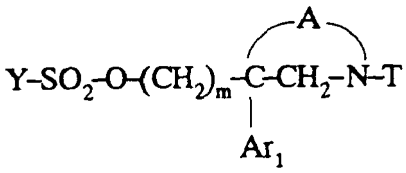 (X)

4) on fait réagir le composé (X) :

- soit avec une amine secondaire cyclique de formule :

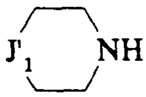 (XI)

dans laquelle $J'_1$ représente :

* soit un groupe

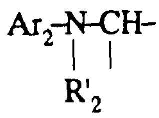

dans lequel $Ar_2$ est tel que défini pour (I) et $R'_2$ représente soit $R_2$ tel que défini pour (I) soit un précurseur de $R_2$ ;

* soit un groupe,

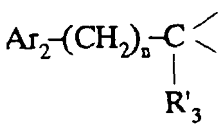

dans lequel $Ar_2$ et n sont tels que définis pour (I) et $R'_3$ représente soit $R_3$ tel que défini pour (I), soit un précurseur de $R_3$, étant entendu que lorsque $R'_3$ représente un hydroxyle ou un amino, ces groupes peuvent être protégés ;

* soit un groupe

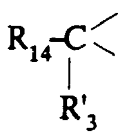

dans lequel $R_{14}$ est tel que défini pour (I) et $R'_3$ est tel que défini ci-dessus ;

- soit avec une amine tertiaire de formule :

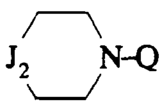 (XII)

dans laquelle $J_2$ et Q sont tels que définis pour (I) ;

- soit avec une amine tertiaire cyclique de formule :

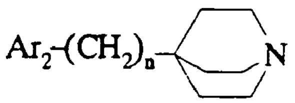 (XIII)

dans laquelle $Ar_2$ et n sont tels que définis pour (I) ;

- soit avec un composé de formule :

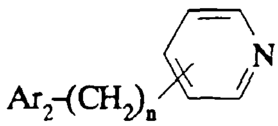 (XIV)

dans laquelle $Ar_2$ et n sont tels que définis pour (I) ;
et

5) - soit, lorsqu'on utilise une amine secondaire cyclique de formule (XI), et après déprotection éventuelle du groupe hydroxy ou du groupe amino représenté par $R'_3$ ou transformation éventuelle de $R'_2$ en $R_2$ ou de $R'_3$ en $R_3$, on transforme éventuellement le produit ainsi obtenu en un de ses sels ;

- soit, lorsqu'on utilise une amine tertiaire de formule (XII) ou une amine tertiaire cyclique de formule (XIII) ou un composé de formule (XIV), on isole le produit ainsi obtenu sous forme d'un sulfonate et éventuellement d'un sel d'acide sulfonique ou bien, éventuellement on échange l'anion et éventuellement le sel d'acide ainsi obtenu avec un autre anion et éventuellement un autre sel d'acide minéral ou organique pharmaceutiquement acceptables.

Selon une variante du procédé, lorsque Am représente un groupe $Am_1$,

1') on oxyde un composé de formule (VIII) tel que défini précédemment pour obtenir un composé de formule :

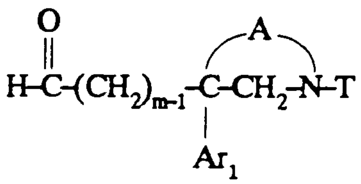 (XXXVIII)

dans laquelle m, $Ar_1$, A et T sont tels que définis pour un composé de formule (I),
2') on fait réagir le composé de formule (XXXVIII) avec un composé de formule (XI) tel que défini précédemment en présence d'un acide, puis on réduit le sel d'iminium formé intermédiairement au moyen d'un agent réducteur ; et
3') après déprotection éventuelle des groupes hydroxyles ou des groupes aminés ou transformation éventuelle de $R'_2$ en $R_2$ ou de $R'_3$ en $R_3$, on transforme éventuellement le produit ainsi obtenu en l'un de ses sels.

Les composés de formule :

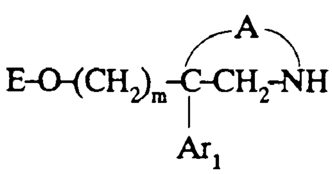 (II)

sous forme énantiomériquement pure ou sous forme racémique sont nouveaux et font partie de l'invention.

Les composés de formule :

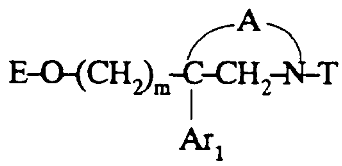

(VII)

sous forme énantiomériquement pure ou sous forme racémique sont nouveaux et font partie de l'invention.

Les composés de formule (II) et (VII) dans lesquels E représente l'hydrogène sont particulièrement préférés.

Les composés de formule :

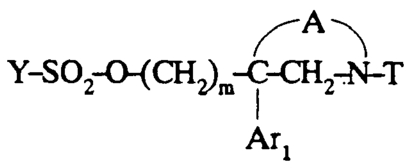

(X)

sous forme énantiomériquement pure ou sous forme racémique sont nouveaux et font partie de l'invention.

Les composés de formule :

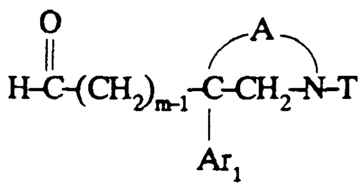

(XXXVIII)

sous forme énantiomériquement pure ou sous forme racémique sont nouveaux et font partie de l'invention.

Dans les formules (II), (VII), (X) et (XXXVIII), m et les groupes E, A, $Ar_1$ T et Y ont les définitions données ci-dessus.

Ainsi, selon un autre de ses aspects, la présente invention a pour objet des composés de formule :

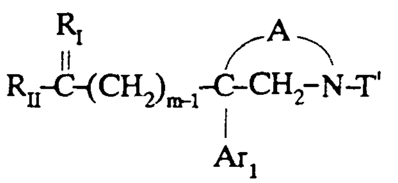

(XXXIX)

dans laquelle :

- m, $Ar_1$, A sont tels que définis pour un composé de formule (I) ;
- $R_I$ représente deux atomes d'hydrogène et $R_{II}$ représente :
    - soit un groupe -O-E dans lequel E représente un atome d'hydrogène ou un groupe O-protecteur,
    - soit un groupe $-O-SO_2-Y$ dans lequel Y représente un groupe méthyle, phényle, tolyle ou trifluorométhyle ;
- ou bien $R_I$ représente un atome d'oxygène et $R_{II}$ représente un atome d'hydrogène;
- T représente T tel que défini pour un composé de formule (I), T peut de plus représenter l'hydrogène lorsque à la fois $R_I$ représente 2 atomes d'hydrogène et $R_{II}$ représente un groupe -O-E ;

sous forme énantiomèriquement pure ou sous forme de racémique.

Ainsi, lorsque E représente un groupe O-protecteur, celui-ci est choisi parmi les groupes O-protecteurs classiques bien connus de l'homme de l'art, tels que, par exemple, le tétrahydropyran-2-yle, le benzoyle ou un ($C_1$-$C_4$) alkylcarbonyle.

Les groupes O-protecteurs éventuellement utilisés pour obtenir un composé de formule (I) dans laquelle $R_3$ représente un hydroxy sont les groupes 0-protecteurs classiques bien connus de l'homme de l'art tels que définis ci-dessus pour E.

Les groupes N-protecteurs éventuellement utilisés pour obtenir un composé de formule (I) dans laquelle $R_3$ représente un amino sont les groupes N-protecteurs classiques bien connus de l'homme de l'art tels que, par exemple, le groupe trityle, méthoxytrityle, *tert*-butoxycarbonyle ou benzyloxycarbonyle.

De façon particulière, lorsqu'on utilise comme groupe O-protecteur un groupe acétyle, le composé de formule (I) obtenu représente le produit final dans lequel $R_3$ représente un acétoxy ou lorsqu'on utilise comme groupe N-protecteur un groupe *tert*-butoxycarbonyle, le composé de formule (I) obtenu représente le produit final dans lequel $R_3$ représente un *tert*-butoxycarbonylamino.

Dans l'étape 1), comme dérivé fonctionnel de l'acide (III), on utilise l'acide lui-même, ou bien un des dérivés fonctionnels qui réagissent avec les amines, par exemple un anhydride, un anhydride mixte, le chlorure d'acide, ou un ester activé, comme l'ester de paranitrophényle.

Lorsqu'on met en oeuvre l'acide de formule (III) lui-même, on opère en présence d'un agent de couplage utilisé en chimie peptidique tel que le 1,3-dicyclo-hexylcarbodiimide ou l'hexafluorophosphate de benzotriazol-1-yloxytris (diméthylamino)phosphonium en présence d'une base telle que la triéthylamine ou la N,N-diisopropyléthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante.

Lorsqu'on utilise un dérivé halogéné de formule (IV), (V) ou (VI), la réaction s'effectue dans un solvant inerte tel que le tétrahydrofuranc, le N,N-diméthylformamide ou le diméthylsulfoxyde en présence d'une base telle que le *tert*-butylate de potassium, l'hydrure de sodium ou le diisopropylamidure de lithium et à une température comprise entre 0°C et 80°C.

Le composé de formule (VII) ainsi obtenu est éventuellement déprotégé à l'étape 2) selon les méthodes connues de l'homme de l'art. Par exemple, lorsque E représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe ($C_1$-$C_4$)alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, l'éthanol, le dioxane ou un mélange de ces solvants, à une température comprise entre 0°C et la température de reflux du solvant.

A l'étape 3) la réaction de l'alcool de formule (VIII) avec un chlorure de sulfonyle de formule (IX) s'effectue en présence d'une base telle que la triéthylamine, la pyridine, la N,N-diisopropyléthylamine ou la N-méthylmorpholine, dans un solvant inerte tel que le dichlorométhane, le benzène ou le toluène, à une température comprise entre -20°C et la température de reflux du solvant.

Le composé de formule (X) ainsi obtenu est mis en réaction à l'étape 4), avec un composé de formule (XI), (XII), (XIII) ou (XIV) selon des modes opératoires différents.

Lorsqu'on fait réagir un composé de formule (X) avec un composé de formule (XI), la réaction s'effectue dans un solvant inerte tel que le N,N-diméthylformamide, l'acétonitrile, le chlorure de méthylène, le toluène ou l'isopropanol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (XI) et en présence d'un iodure de métal alcalin tel que l'iodure de potassium ou l'iodure de sodium. Lorsque dans le composé de formule (X), -A- représente le radical bivalent -O-CO- ou $-CH_2$-O-CO-, la réaction s'effectue à une température comprise entre la température ambiante et 80°C. Lorsque dans le composé de formule (X) -A- représente le radical bivalent -O-$CH_2$-CO-, -O-$CH_2$-$CH_2$-, -N($R_1$)-CO-CO-, -N($R_1$)-$CH_2$-$CH_2$-, -N($R_1$)-CO- ou -O-$CH_2$-, la réaction s'effectue à une température comprise entre la température ambiante et 100°C.

Lorsqu'on fait réagir un composé de formule (X) avec un composé de formule (XII) ou (XIII), la réaction s'effectue dans un solvant aprotique polaire tel que l'acétonitrile, le N,N-diméthylformamide, ou le N,N-diméthylphénylacétamide ou dans un éther tel que le tétrahydrofurane, le dioxane ou le méthyl-*tert*-butyléther ou dans une cétone telle que la méthyléthylcétone. Lorsque dans le composé de formule (X), -A-représente le radical bivalent -O-CO- ou $-CH_2$-O-CO-,

la réaction s'effectue à une température comprise entre la température ambiante et 60°C. Lorsque dans le composé de formule (X) -A- représente le radical bivalent -O-$CH_2$-CO-, -O-$CH_2$-$CH_2$-,-N($R_1$)-CO-CO-,-N($R_1$)-$CH_2$-$CH_2$-,-N($R_1$)-CO- ou -O-$CH_2$, la réaction s'effectue à une température comprise entre la température ambiante et 100°C.

Lorsqu'on fait réagir un composé de formule (X) avec un composé de formule (XIV), la réaction s'effectue dans un solvant aprotique polaire tel que l'acétonitrile, le N,N-diméthylformamide, le N,N-diméthylphénylacétamide ou dans un éther tel que le tétrahydrofurane, le dioxane ou le méthyl-*tert*-butyléther ou dans une cétone telle que la méthyléthylcétone. La réaction s'effectue à une température comprise entre la température ambiante et 100°C.

Selon la variante du procédé, à l'étape 1') on soumet un alcool de formule (VIII) à une oxydation pour obtenir un aldéhyde de formule (XXXVIII). La réaction d'oxydation s'effectue en utilisant par exemple le chlorure d'oxalyle, le diméthylsulfoxyde et la triéthylamine dans un solvant tel que de dichlorométhane et à une température comprise entre -78°C et la température ambiante.

Puis à l'étape 2'), on fait réagir le composé de formule (XI) avec un aldéhyde de formule (XXXVIII) en présence d'un acide tel que l'acide acétique, dans un solvant alcoolique tel que le méthanol, pour former *in-situ* une imine intermédiaire qui est réduite chimiquement en utilisant par exemple le cyanoborohydrure de sodium ou catalytiquement en utilisant l'hydrogène et un catalyseur tel que le palladium sur charbon ou le nickel de Raney® .

On obtient finalement après déprotection éventuelle des groupes hydroxyles ou des groupes aminés, ou transformation éventuelle de $R'_2$ en $R_2$ et $R'_3$ en $R_3$, les composés de formule (I) selon l'invention.

Les produits de formule (I) ainsi obtenus sont :

- soit isolés sous forme de base libre ou de sel, selon les techniques classiques, lorsque Am représente $Am_1$,
- soit, lorsque Am représente $Am_2$, $Am_3$ ou $Am_4$, isolés sous forme de sulfonate ($YSO_3^{\ominus}$) ou bien l'anion sulfonate du sel quaternaire ainsi obtenu est éventuellement échangé avec un autre anion pharmaceutiquement acceptable.

Lorsque le composé de formule (I) dans laquelle Am représente $Am_1$ est obtenu sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, avec une solution de l'acide choisi dans le même solvant, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le naphtalène-2-sulfonate, le benzènesulfonate.

A la fin de la réaction, les composés de formule (I) dans laquelle Am représente $Am_1$ peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

L'anion sulfonate $YSO_3^{\ominus}$ issu de la réaction entre le composé de formule (XII), (XIII) ou (XIV) et le composé de formule (X) peut être échangé, in situ ou après isolement du composé de formule (I) dans laquelle Am représente un groupe $Am_2$, $Am_3$ ou $Am_4$ dans lequel $X^{\ominus}$ est l'ion $YSO_3^{\ominus}$, par un autre anion $X^{\ominus}$, selon les méthodes conventionnelles, par exemple par échange en solution avec une solution saturée de chlorure de sodium ou avec une solution d'acide chlorhydrique lorsque $X^{\ominus}$ représente un anion chlorure ou par échange de l'anion par élution du composé (I) sur une résine échangeuse d'ion, par exemple l'Amberlite IRA68® ou Duolite A375® .

A la fin de la réaction, les composés de formule (I) dans laquelle Am représente $Am_2$ sont obtenus sous forme d'un mélange des isomères axiaux et équatoriaux. Les isomères sont séparés selon les méthodes habituelles, par exemple, par chromatographie ou par recristallisation.

Les composés de formule (II) se préparent selon différents modes opératoires.

Les composés de formule (II) dans laquelle -A- représente le radical bivalent -$CH_2$-O-CO- et E représente l'hydrogène ou un groupe O-protecteur se préparent selon le SCHEMA 1 ci-après dans lequel m et $Ar_1$ sont tels que définis pour un composé de formule (I) et Pr désigne un groupe O-protecteur tel que défini ci-dessus pour E.

SCHEMA 1

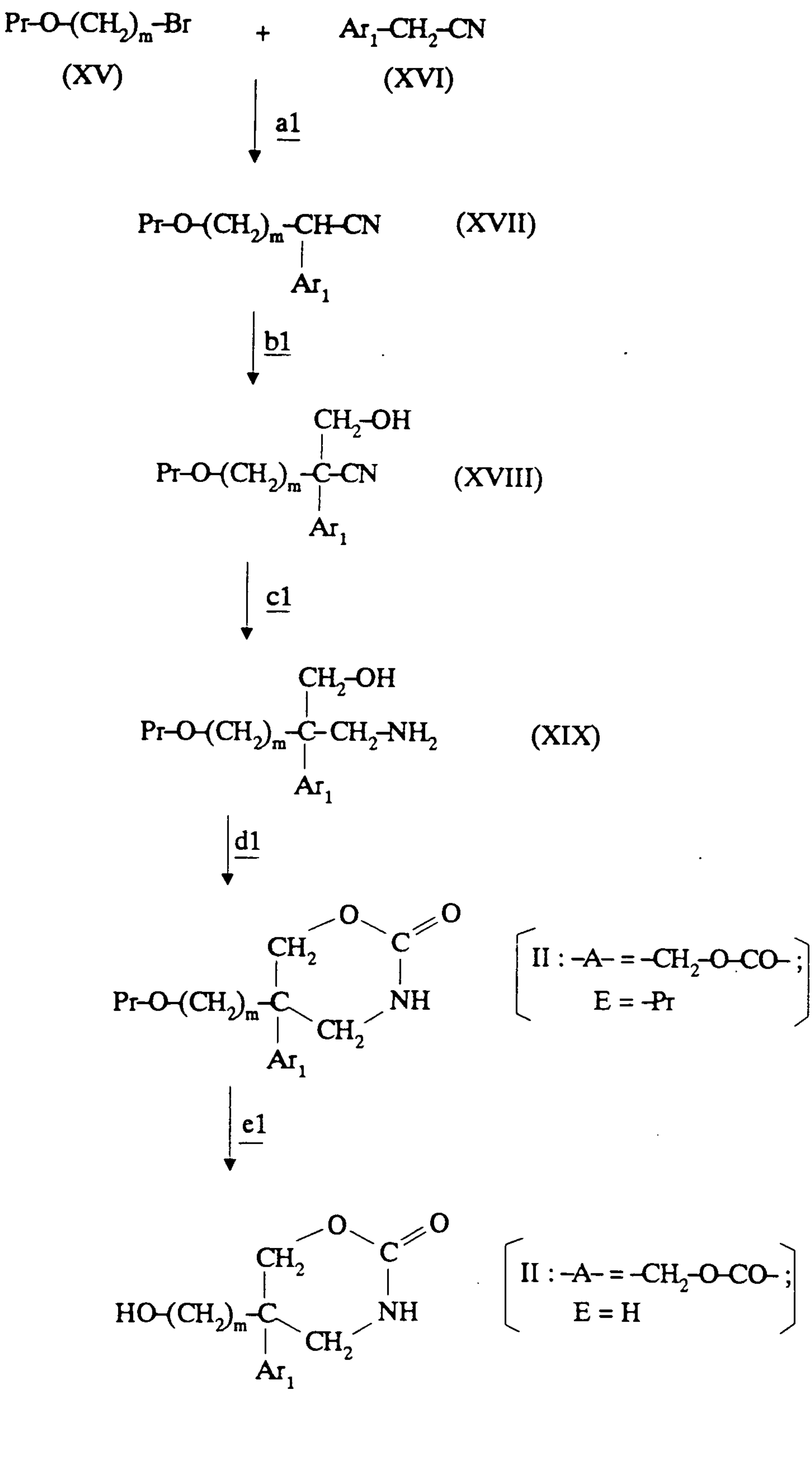


A l'étape a1 du SCHEMA 1, on fait réagir un composé de formule (XV) avec un composé de formule (XVI)

selon la méthode décrite dans les demandes de brevet EP-A-0428434 et EP-A-0474561.

Le composé (XVII) ainsi obtenu est mis en réaction à l'étape b1 avec une solution aqueuse de formaldéhyde, en présence d'une base telle que le 1,8-diazabicyclo[5.4.0]undec-7-ène, dans un solvant tel que le 1,2-diméthoxyéthane, et à une température comprise entre la température ambiante et la température de reflux du solvant.

Le dérivé nitrile de formule (XVIII) est réduit à l'étape c1 pour obtenir l'amine primaire de formule (XIX). Cette réduction peut s'effectuer au moyen d'hydrogène, en présence d'un catalyseur tel que le nickel de Raney® , l'oxyde de platine ou le palladium sur charbon, dans un solvant inerte tel qu'un alcool, l'éthanol par exemple, seul ou en mélange avec de l'ammoniaque, ou au moyen d'un agent réducteur tel que l'hydrure d'aluminium lithium, l'hydrure de diisobutylaluminium, le borane dans le THF, dans un solvant tel que le toluène, l'hexane, l'éther de pétrole, le xylène ou le tétrahydrofurane. La réaction s'effectue à une température comprise entre 0°C et 70°C.

Le composé (XIX) est mis en réaction à l'étape d1 avec un dérivé réactif de l'acide carbonique tel que le phosgène en solution dans le toluène, le 1,1'-carbonyldiimidazole, en présence d'une base telle que la triéthylamine, la N,N-diisopropyléthylamine ou la N-méthylmorpholine, dans un solvant chloré tel que le dichlorométhane, le 1,2-dichloroéthane ou un éther tel que le tétrahydrofurane, et à une température comprise entre -70°C et la température ambiante pour obtenir un composé de formule (II) attendue dans laquelle E représente un groupe O-protecteur.

Par hydrolyse, selon les méthodes décrites précédemment, on élimine le groupe O-protecteur (étape e1) et obtient le composé de formule (II) dans laquelle E représente l'hydrogène.

Les composés de formule (II) dans laquelle -A- représente le radical bivalent -O-$CH_2$-CO- et E représente l'hydrogène ou un groupe O-protecteur se préparent selon le SCHEMA 2 ci-après dans lequel m et $Ar_1$ sont tels que définis pour un composé de formule (I). $Pr_1$ et $Pr_2$ représentent le groupe O-protecteur Pr tel que défini ci-dessus pour E, plus particulièrement $Pr_1$ représente un groupe O-protecteur hydrolysable en milieu acide, $Pr_2$ représente un groupe O-protecteur hydrolysable en milieu basique.

## SCHEMA 2

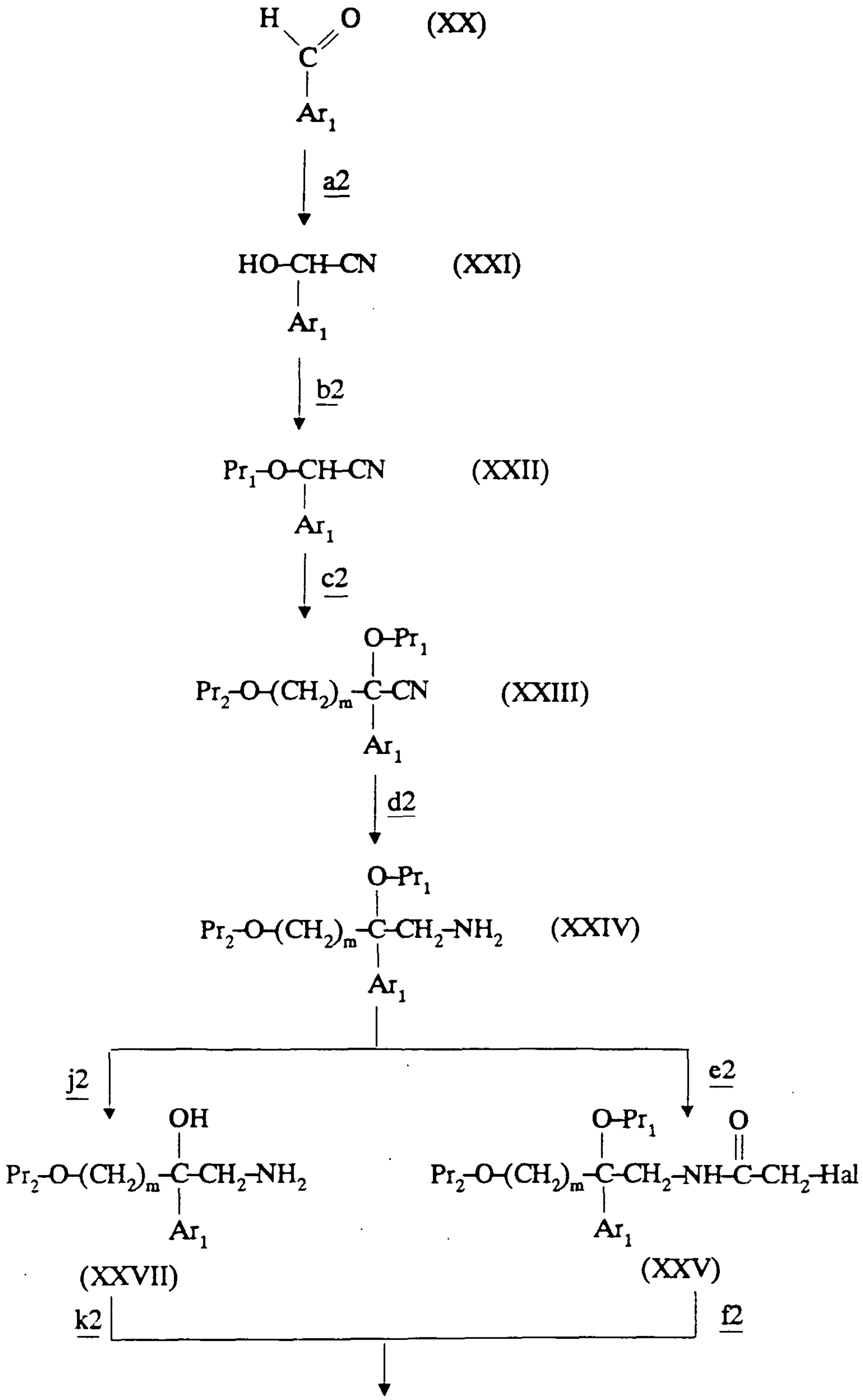


(XX) (XXI) (XXII) (XXIII) (XXIV) (XXVII) (XXV)

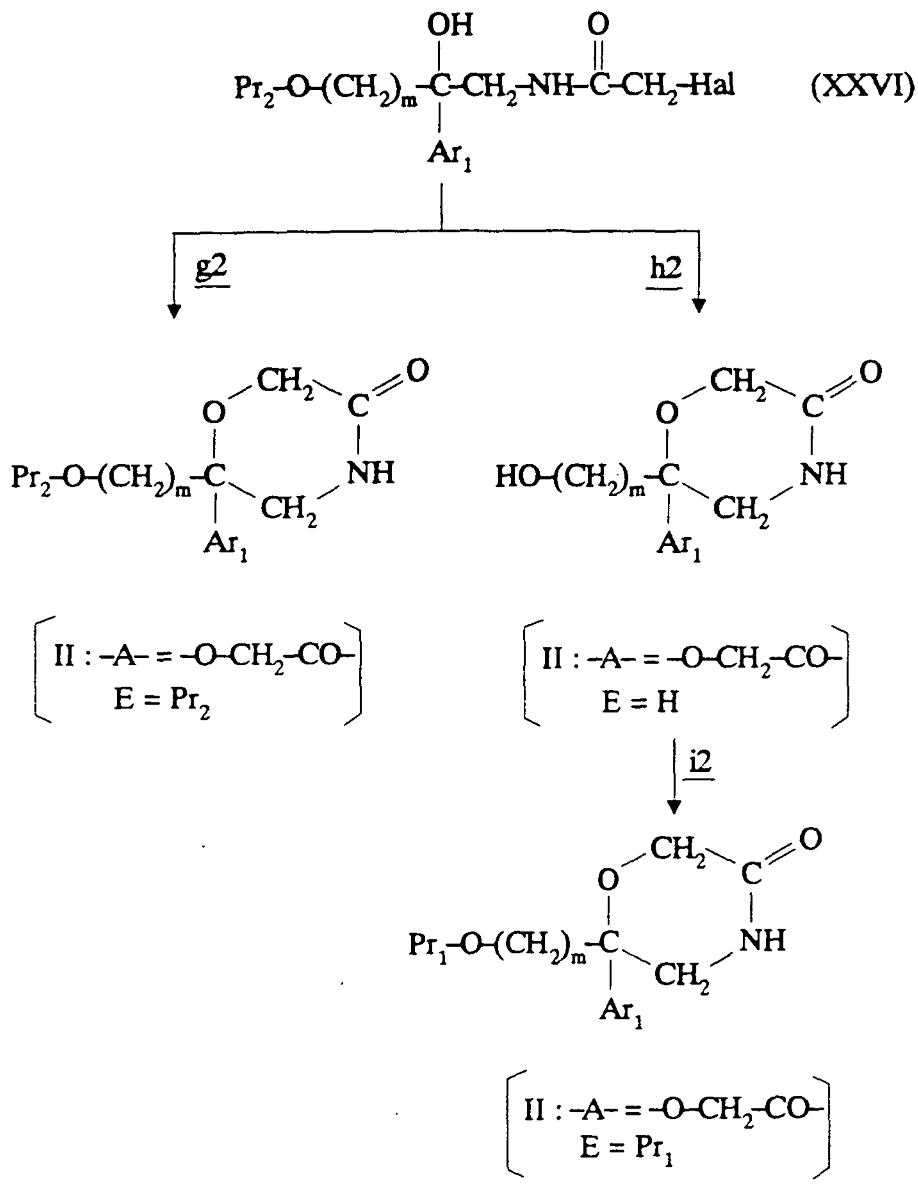


A l'étape a2 du SCHEMA 2, la synthèse d'une cyanhydrine de formule (XXI) à partir d'un aldéhyde de formule (XX) s'effectue selon les méthodes bien connues de l'homme de l'art telle que par exemple celle décrite dans Organic Syntheses ; Wiley, New-York, 1932 ; Collect. vol. 1, p. 336, ou par adaptation de cette méthode en utilisant l'action du métabisulfite de sodium et du cyanure de potassium en solution aqueuse.

A l'étape b2, le groupe hydroxy du composé de formule (XXI) est protégé selon les méthodes connues de l'homme de l'art.

Le composé de formule (XXII) ainsi obtenu est traité à l'étape c2 par une base forte telle que le diisopropylamidure de lithium, le *tert*-butylate de potassium ou l'hydrure de sodium pour fournir un carbanion qui est mis en réaction avec un composé de formule Hal-$(CH_2)_m$-O-$Pr_2$, dans laquelle Hal représente un halogène de préférence le brome ou le chlore, pour obtenir le composé de formule (XXIII). La réaction s'effectue dans un solvant inerte tel qu'un éther (tétrahydrofurane, éther diéthylique, 1,2-diméthoxyéthane par exemple) ou un amide (N,N-diméthylformamide par exemple) ou un hydrocarbure aromatique (toluène, xylène par exemple) à une température comprise entre -70°C et +60°C.

Le dérivé nitrile de formule (XXIII) est réduit à l'étape d2. selon les méthodes précédemment décrites, pour obtenir l'amine primaire de formule (XXIV).

A l'étape e2, le composé de formule (XXIV) est mis en réaction, avec un composé de formule Hal-CO-$CH_2$-Hal dans laquelle Hal représente un halogène, de préférence le chlore ou le brome, en présence d'une base telle qu'une amine tertiaire (triéthylamine, N-méthylmorpholine, pyridine par exemple) pour obtenir un composé de formule (XXV).

La réaction s'effectue dans un solvant inerte tel qu'un solvant chloré (dichlorométhane, dichloroéthane, chloroforme par exemple), un éther (tétrahydrofurane, dioxane par exemple) ou un amide (N,N-diméthylformamide par exemple) à une température comprise entre -70°C et la température ambiante.

On élimine à l'étape f2 le groupe O-protecteur $Pr_1$ du composé de formule (XXV), par hydrolyse acide selon les méthodes précédemment décrites.

Alternativement, on élimine à l'étape j2 le groupe O-protecteur $Pr_1$ du composé de formule (XXIV) par hydrolyse acide, puis on met en réaction, à l'étape k2, le composé (XXVII) ainsi obtenu avec un composé de formule Hal-CO-$CH_2$-Hal selon les méthodes décrites ci-dessus à l'étape e2.

Le composé de formule (XXVI) ainsi obtenu est cyclisé en présence d'une base pour obtenir le composé de formule (II) attendue. Lorsqu'on veut obtenir un composé de formule (II) dans laquelle E représente un groupe protecteur $Pr_2$, on utilise une base telle qu'un carbonate de métal alcalin (le carbonate de potassium par exemple) ou un hydrure de métal alcalin (l'hydrure de sodium par exemple) ou le *tert*-butylate de potassium, dans un solvant inerte tel qu'un hydrocarbure aromatique (xylène, toluène par exemple) ou un amide (N,N-diméthylfonnamide par exemple) ou un éther (tétrahydrofurane par exemple), à une température comprise entre -30°C et la température de reflux du solvant (étape g2). Lorsqu'on veut obtenir un composé de formule (II) dans laquelle E représente l'hydrogène, on utilise une base telle qu'un hydroxyde de métal alcalin (l'hydroxyde de sodium, l'hydroxyde de potassium par exemple) en solution aqueuse concentrée dans un solvant tel qu'un alcanol (le propan-2-ol par exemple) ou un amide (le N,N-diméthylformamide par exemple) ou un mélange de ces solvants à une température comprise entre la température ambiante et la température de reflux du solvant (étape h2).

Eventuellement, on prépare à l'étape i2 un composé de formule (II) dans laquelle E représente un groupe O-protecteur $Pr_1$ selon les méthodes connues de l'homme de l'art.

Les composés de formule (II) dans laquelle -A- représente le radical bivalent -O-$CH_2$-$CH_2$- et E représente l'hydrogène ou un groupe O-protecteur, se préparent selon le SCHEMA 3 ci-après dans lequel m et $Ar_1$ sont tels que définis pour un composé de formule (I) et $Pr_1$ et $Pr_2$ sont tels que définis dans le SCHEMA 2 ci-dessus.

SCHEMA 3

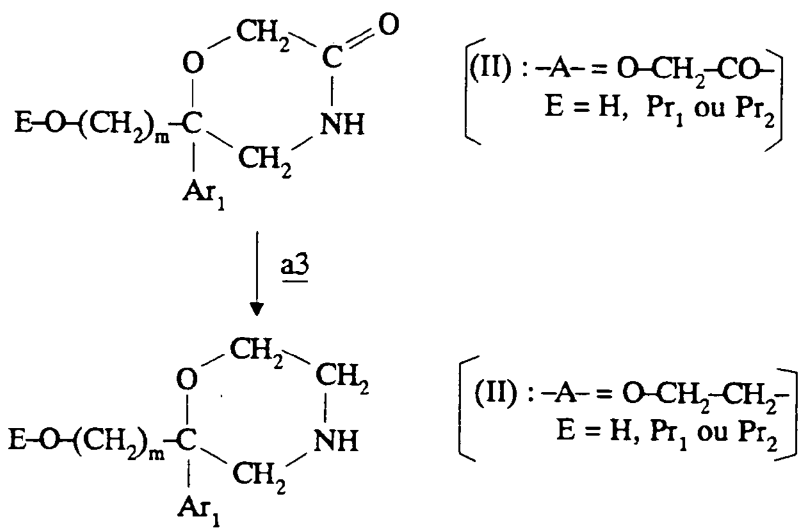


A l'étape a3 du SCHEMA 3, on réduit un composé de formule (II) dans laquelle -A- représente le radical bivalent -O-$CH_2$-CO- et E représente l'hydrogène ou un groupe O-protecteur, obtenu selon le SCHEMA 2. La réduction s'effectue au moyen d'un agent réducteur tel que l'hydrure aluminium et de lithium, l'hydrure de diisobutylaluminium, le borohydrure de sodium, le borane dans le THF, dans un solvant inerte tel que le tétrahydrofurane, l'éther diéthylique, le 1,2-diméthoxyéthane ou le toluène à une température comprise entre la température ambiante et la température de reflux du solvant.

Les composés de formule (II) dans laquelle -A- représente le radical bivalent -O-CO- et E représente l'hydrogène ou un groupe O-protecteur, se préparent selon le SCHEMA 4 ci-après dans lequel m et $Ar_1$ sont tels que définis pour un composé de formule (I) et $Pr_1$ et $Pr_2$ sont tels que définis dans le SCHEMA 2 ci-dessus.

SCHEMA 4

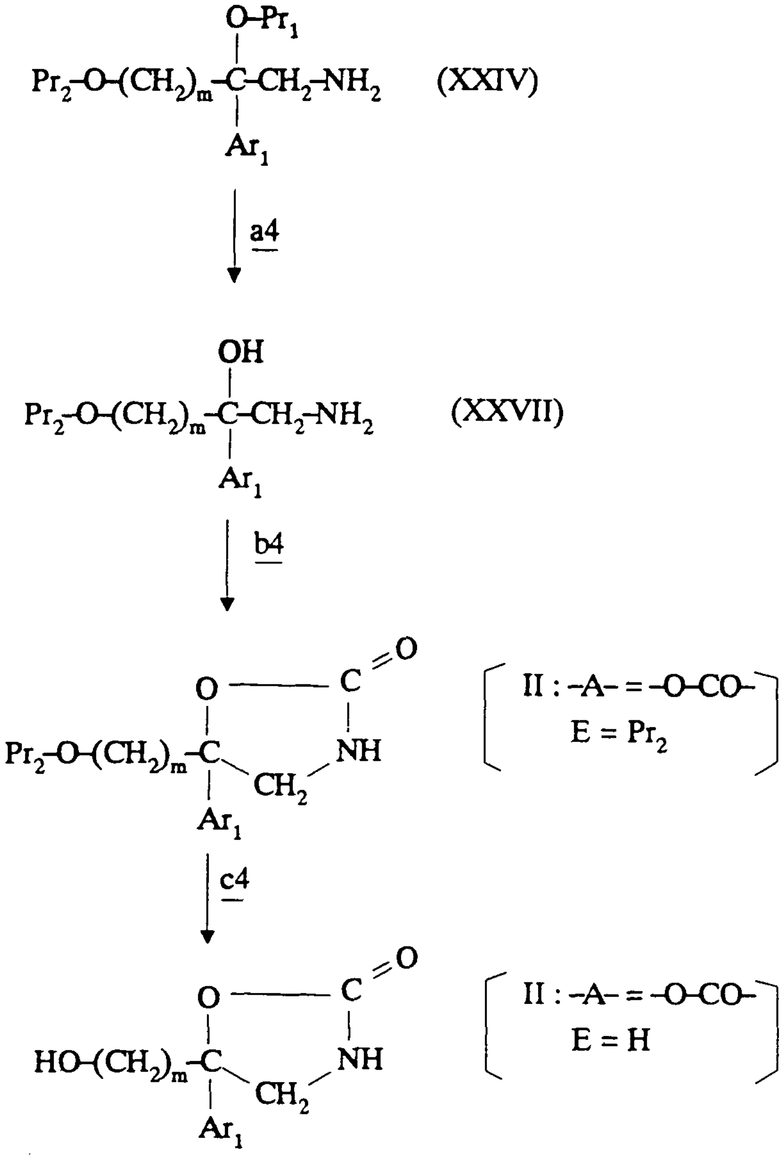


A l'étape a4 du SCHEMA 4, on élimine le groupe O-protecteur $Pr_1$ du composé de formule (XXIV), obtenu à l'étape d2 du SCHEMA 2, par hydrolyse acide selon les méthodes précédemment décrites.

Le composé de formule (XXVII) ainsi obtenu est mis en réaction à l'étape b4 avec un dérivé réactif de l'acide carbonique tel que le 1,1'-carbonyldiimidazole, le phosgène dans le toluène, le chloroformiate de *p*-nitrophényle, et en présence d'une base telle que la triéthylamine, la N,N-diisopropyléthylamine, la N-méthylmorpholine, pour obtenir un composé de formule (II) attendue dans laquelle E représente un groupe O-protecteur. La réaction s'effectue dans un solvant inerte tel qu'un solvant chloré (le 1,2-dichloroéthane, le dichlorométhane par exemple) ou un éther tel que le tétrahydrofurane ou un amide tel que le N,N-diméthylformamide ou un solvant aromatique tel que le toluène à une température comprise entre -60°C et la température ambiante.

Par hydrolyse basique, selon les méthodes décrites précédemment, on élimine le groupe O-protecteur $Pr_2$ (étape c4) pour obtenir le composé de formule (II) dans laquelle E représente l'hydrogène.

Les composés de formule (II) dans laquelle -A- représente le radical bivalent -N($R_1$)-CO-CO- et E représente l'hydrogène ou un groupe O-protecteur se préparent selon le SCHEMA 5 ci-après dans lequel m, $Ar_1$ et $R_1$ sont tels que définis pour un composé de formule (I) et $Pr_1$ est tel que défini ci-dessus.

SCHEMA 5

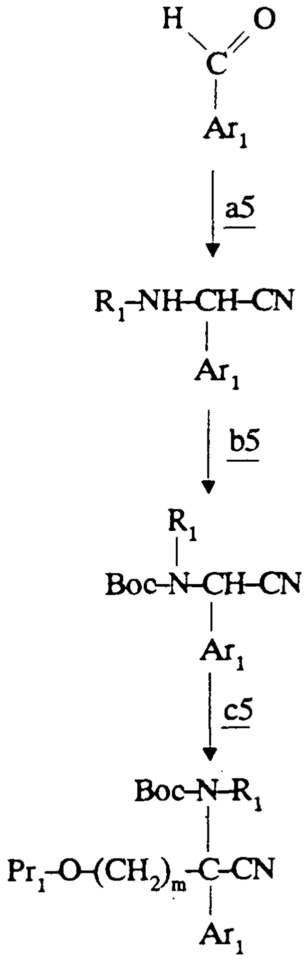


(XX)

(XXVIII)

(XXIX)

(XXX)

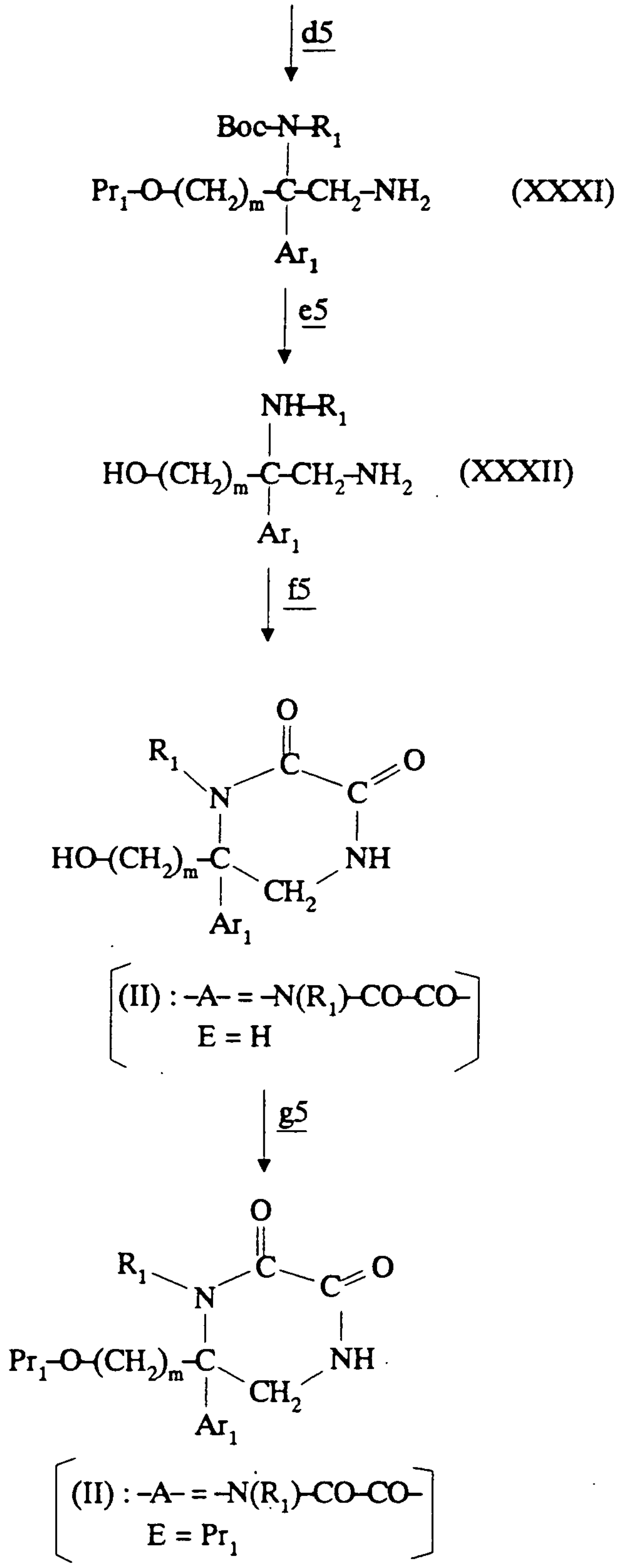


(XXXI)

(XXXII)

(II) : $-A- = -N(R_1)-CO-CO-$
E = H

(II) : $-A- = -N(R_1)-CO-CO-$
E = $Pr_1$

A l'étape a5 du SCHEMA 5, la préparation d'un composé α-aminonitrile de formule (XXVIII) s'effectue à partir d'un aldéhyde de formule (XX) selon la méthode décrite dans Tetrahedron Letters, 1984, 25 (41), 4583-4586 et en utilisant une amine de formule $H_2N-R_1$.

Le groupe amino du composé de formule (XXVIII) est protégé à l'étape b5 par un groupe N-protecteur tel que

le *tert*-butoxycarbonyle (Boc), le benzyloxycarbonyle par exemple selon les méthodes connues de l'homme de l'art. Le groupe *tert*-butoxycarbonyle est illustré dans le SCHEMA 5 ci-dessus.

Le composé de formule (XXIX) ainsi obtenu est traité à l'étape c5 par une base forte pour former un carbanion qui est mis en réaction avec un composé de formule Hal-$(CH_2)_m$-O-$Pr_1$ pour obtenir un composé de formule (XXX). La réaction s'effectue selon la méthode décrite à l'étape c2 du SCHEMA 2.

Le dérivé nitrile de formule (XXX) est réduit à l'étape d5 selon les méthodes précédemment décrites pour obtenir l'amine primaire de formule (XXXI).

On élimine à l'étape e5 le groupe O-protecteur et le groupe N-protecteur du composé de formule (XXXI) par hydrolyse acide au moyen d'acide chlorhydrique ou d'acide trifluoroacétique par exemple dans un solvant tel qu'un alcool (méthanol par exemple) ou un éther (éther diéthylique, dioxanc, tétrahydrofurane par exemple) ou un solvant chloré (dichlorométhane par exemple) à une température comprise entre 0°C et la température de reflux du mélange réactionnel.

A l'étape f5, la préparation du composé de formule (II) attendue s'effectue par application ou adaptation de la méthode décrite par R. Granger, H. Orzalesi et Y. Robbe dans Trav. Soc. Pharm. Montpellier, 1965, 25, Fasc. 4, 313-317, en utilisant la réaction d'un composé de formule (XXXII) avec l'oxalate de diéthyle dans un solvant alcoolique tel que l'éthanol ou un solvant aromatique tel que le toluène ou un mélange de ces solvants, à une température comprise entre la température ambiante et la température de reflux du mélange réactionnel.

Eventuellement, on prépare à l'étape g5 un composé de formule (II) dans laquelle E représente un groupe O-protecteur $Pr_1$ selon les méthodes connues de l'homme de l'art.

Les composés de formule (II) dans laquelle -A- représente le radical bivalent -N($R_1$)-$CH_2$-$CH_2$- et E représente l'hydrogène ou un groupe O-protecteur se préparent selon le SCHEMA 6 ci-après dans lequel m, $R_1$ et $Ar_1$ sont tels que définis pour un composé de formule (I) et $Pr_1$ désigne un groupé O-protecteur tel que défini ci-dessus pour E.

SCHEMA 6

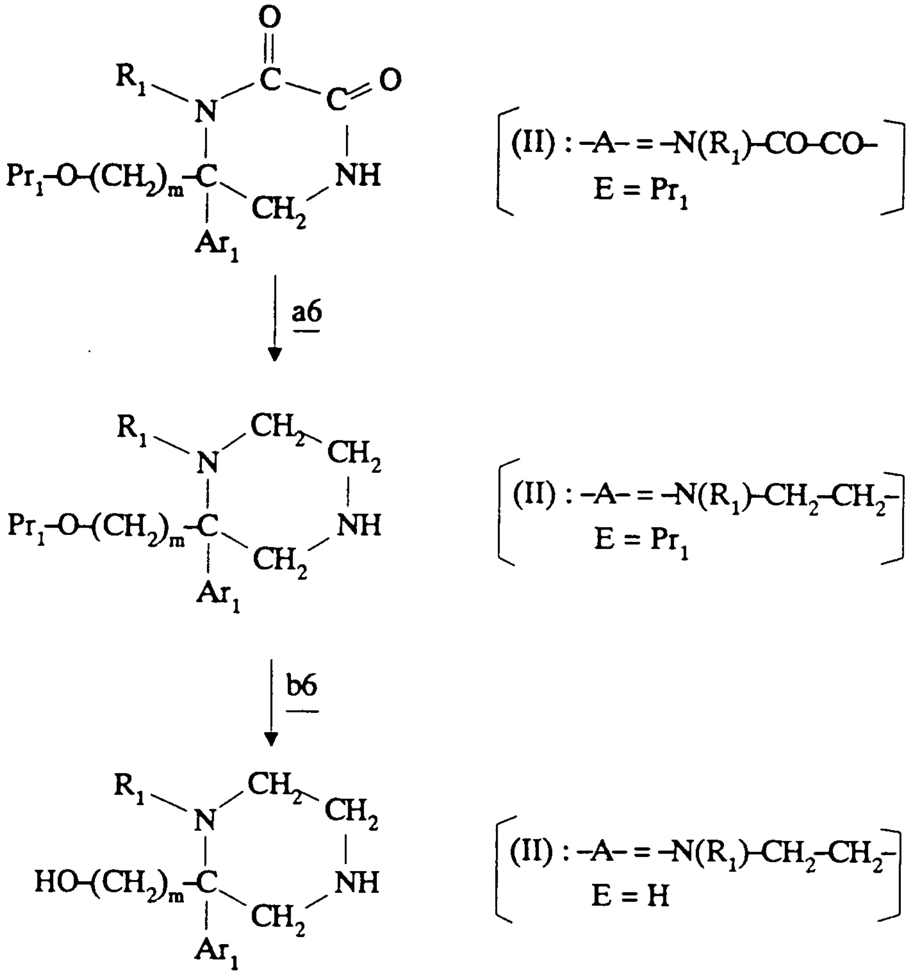


A l'étape a6 du SCHEMA 6, on réduit un composé de formule (II) dans laquelle -A- représente le radical bivalent $-N(R_1)$-CO-CO- et E représente un groupe O-protecteur, obtenu à l'étape g5 du SCHEMA 5. La réduction s'effectue au moyen d'un agent réducteur tel que l'hydrure d'aluminium et de lithium, dans un solvant inerte tel qu'un éther (tétrahydrofurane, 1,2-diméthoxyéthane ou éther diéthylique par exemple) ou un solvant aromatique tel que le toluène à une température comprise entre la température ambiante et la température de reflux du solvant.

Eventuellement, on élimine à l'étape b6 le groupe O-protecteur par hydrolyse acide selon les méthodes décrites précédemment, pour obtenir le composé de formule (II) dans laquelle E représente l'hydrogène.

Les composés de formule (II) dans laquelle -A- représente le radical bivalent $-N(R_1)$-CO- et E représente l'hydrogène ou un groupe O-protecteur se préparent selon le SCHEMA 7 ci-après dans lequel m, $R_1$ et $Ar_1$ sont tels que définis pour un composé de formule (I) et $Pr_1$ est tel que défini dans le SCHEMA 2 ci-dessus.

## SCHEMA 7

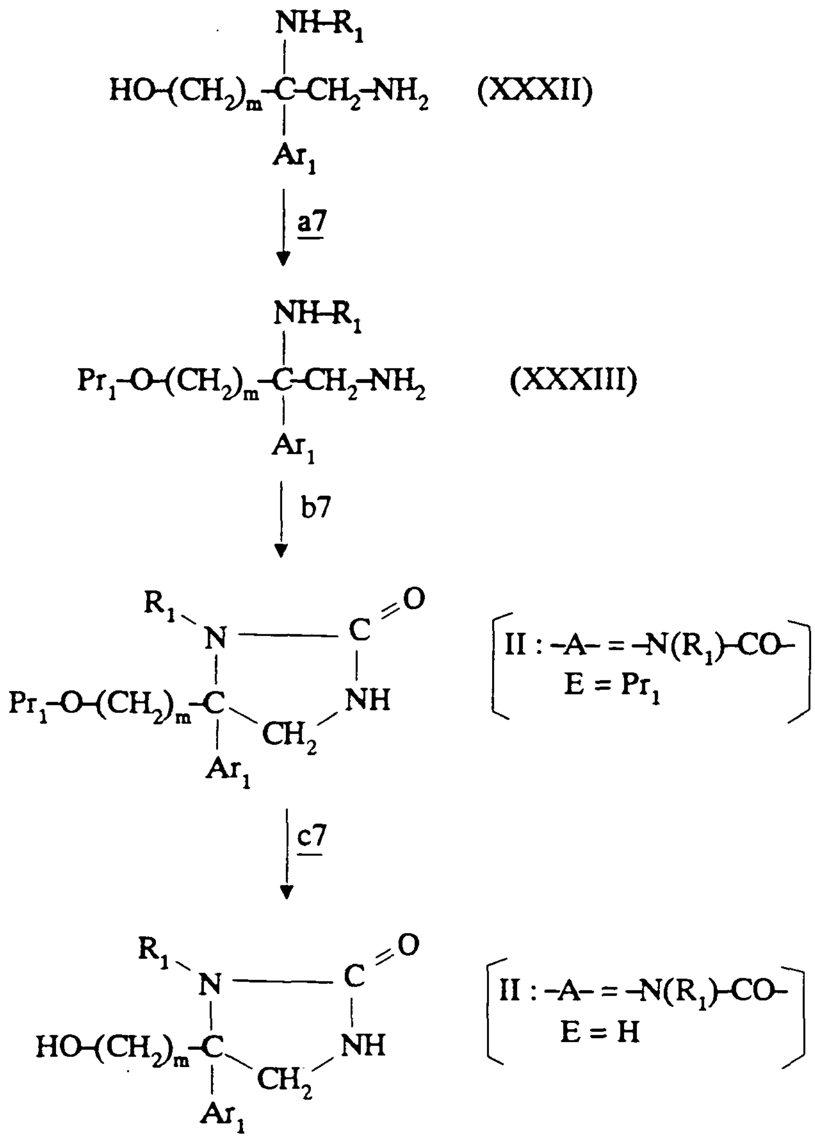


(XXXII)

(XXXIII)

A l'étape a7 , l'hydroxyle du composé de formule (XXXII), obtenu à l'étape e5 du SCHEMA 5, est protégé selon les méthodes connues de l'homme de l'art.

A l'étape b7, le composé de formule (XXXIII) ainsi obtenu est mis en réaction avec un dérivé réactif de l'acide carbonique tel que le 1,1'-carbonyldiimidazole, le phosgène dans le toluène, le chloroformiate de *p*-nitrophényle, en présence d'une base telle que la triéthylamine, la N,N-diisopropyléthylamine, la N-méthylmorpholine, pour obtenir un composé de formule (II) attendu dans laquelle E représente un groupe O-protecteur. La réaction s'effectue dans un solvant inerte tel qu'un solvant chloré (le 1,2-dichloroéthane, le dichlorométhane par exemple) ou un éther (le tétrahydrofurane par exemple) ou un amide (le N,N-diméthylformamide par exemple) ou un solvant aromatique (le toluène par exemple) à une température comprise entre -60°C et 60°C.

Eventuellement, on prépare à l'étape c7 un composé de formule (II) dans laquelle E représente l'hydrogène selon les méthodes connues de l'homme de l'art.

Les composés de formule (II) dans laquelle -A- représente le radical bivalent $-O-CH_2-$ et E représente l'hydrogène ou un groupe O-protecteur, se préparent selon le SCHEMA 8 ci-après dans lequel m et $Ar_1$ sont tels que définis pour un composé de formule (I) et $Pr_2$ est tel que défini dans le SCHEMA 2 ci-dessus.

## SCHEMA 8

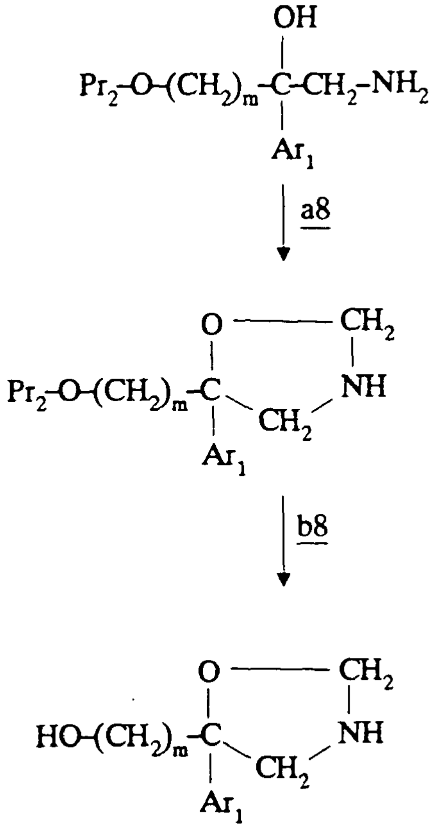


(XXVII)

[II : -A- = -O-$CH_2$- E = $Pr_2$]

[II : -A- = -O-$CH_2$- E = H]

A l'étape a8 du SCHEMA 8, on fait réagir un composé de formule (XXVII) avec une solution aqueuse de formaldéhyde dans un solvant inerte tel que le tétrahydrofurane et à une température comprise entre la température ambiante et la température de reflux du solvant pour obtenir un composé de formule (II) attendue dans laquelle E représente un groupe O-protecteur.

Par hydrolyse basique, selon les méthodes décrites précédemment, on élimine le groupe O-protecteur $Pr_2$ (étape b8) pour obtenir le composé de formule (II) dans laquelle E représente l'hydrogène.

Les composés de formule (XVIII), (XIX), (XXIII), (XXIV), (XXVII), (XXXI), (XXX), (XXXII), (XXXIII) ainsi que les composés de formule (XXXIV) définis ultérieurement sont des produits nouveaux qui constituent les intermédiaires clés pour la préparation des composés de formule (I).

Ainsi, les composés de formule :

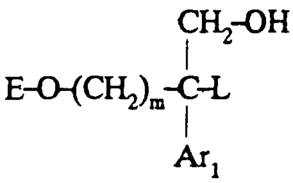

(XXXV)

dans laquelle :

- m et $Ar_1$ sont tels que définis pour un composé de formule (I) ;
- E est tel que défini pour un composé de formule (II) ;
- L représente un groupe cyano ou un groupe aminométhyle ; sous forme énantiomériquement pure ou sous forme racémique lorsque L est un groupe aminométhyle sont nouveaux et font partie de l'invention.

Les composés de formule :

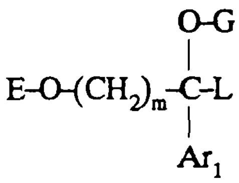 (XXXVI)

dans laquelle :

- m, $Ar_1$, E et L sont tels que définis précédemment ;
- G représente un hydrogène ou un groupe O-protecteur ; sous forme énantiomériquement pure ou sous forme racémique lorsque L est un groupe aminométhyle sont nouveaux et font partie de l'invention.

Les composés de formule :

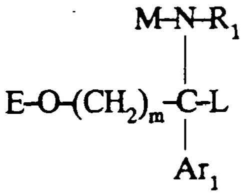 (XXXVII)

dans laquelle :

- m, $Ar_1$, E et L sont tels que définis précédemment ;
- $R_1$ est tel que défini pour un composé de formule (I) ;
- M représente un hydrogène ou un groupe N-protecteur ; sous forme énantiomériquement pure ou sous forme racémique lorsque L est un groupe aminométhyle sont nouveaux et font partie de l'invention.

Les composés de formule (XXXV), (XXXVI) et (XXXVII) sont obtenus selon les procédés décrits précédemment. Plus particulièrement les composés déprotégés (E=G=M=H) s'obtiennent après élimination des groupes O-protecteurs ou N-protecteurs selon les méthodes bien connues de l'homme de l'art.

Ainsi, selon un autre de ses aspects, la présente invention a pour objet des composés de formule :

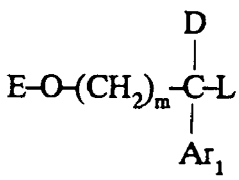 (XXXX)

dans laquelle :

- m et $Ar_1$ sont tels que définis pour un composé de formule (I) ;
- E représente un hydrogène ou un groupe O-protecteur ;
- L représente un groupe cyano ou un groupe aminométhyle ;
- D représente un groupe choisi parmi :
  $-CH_2-OH$, -O-G,

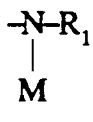 ;

- G représente un hydrogène ou un groupe O-protecteur ;

- M représente un hydrogène ou un groupe N-protecteur ;
- $R_1$ est tel que défini pour un composé de formule (I);

sous forme énantiomériquement pure ou sous forme racémique lorsque L est un groupe aminométhyle.

Les pipéridines de formule (XI) sont connues ou préparées par des méthodes connues, telles que celles décrites dans EP-A-0428434, EP-A-0474561, EP-A-0512901 et EP-A-0515240.

On peut également préparer les pipéridines de formule (XI) par des méthodes bien connues de l'homme de l'art, telles que celles décrites dans les publications suivantes ;

J. Heterocyclic. Chem., 1986, 23, 73-75
J. Chem. Soc., 1950, 1469
J. Chem. Soc., 1945, 917
J. Pharmaceutical. Sci., 1972, 61, 1316-1317
J. Org. Chem. 1957, 22, 1484-1489
Chem. Ber., 1975, 108, 3475-3482.

Les composés de formule (XI) sont généralement préparés sous forme protégée sur l'azote de la pipéridine ; après une étape de déprotection, on obtient les composés de formule (XI) eux-mêmes.

Plus particulièrement, pour préparer par exemple un composé de formule (XI), dans laquelle $J'_1$ représente un groupe

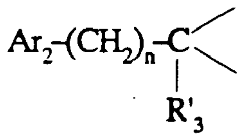

dans lequel $Ar_2$ représente un radical pyrid-2-yle,
n est zéro et $R'_3$ représente un hydroxyle, on fait réagir la 2-bromopyridine sur la 1-benzylpipérid-4-one en présence d'une base telle que le butyllithium. Après élimination du groupe N-protecteur, on obtient la 4-hydroxy-4-(pyrid-2-yl) pipéridine attendue.

Pour préparer un composé de formule (XI) dans laquelle $R'_3$ représente un groupe $-NR_4R_5$ dans lequel $R_4$ et $R_5$ représentent chacun l'hydrogène, on effectue une hydrolyse en milieu acide d'un composé de formule (XI) dans laquelle $R'_3$ représente un groupe acétamido.

On prépare un composé de formule (XI) dans laquelle $R'_3$ représente un groupe $-NR_4R_5$ dans lequel $R_4$ et $R_5$ ensemble avec l'atome d'azote auquel ils sont liés constituent un hétérocycle, par application ou adaptation de la réaction de Bruylants (Bull. Soc. Chim. Belges, 1924, 33, 467 et Tetrahedron Letters, 1988, 29 (52), 6827-6830).

Pour préparer un composé de formule (XI) dans laquelle $R'_3$ représente un groupe $-CH_2-NR_{12}R_{13}$ dans lequel $R_{12}$ et $R_{13}$ représentent chacun l'hydrogène, on effectue la réduction d'un composé de formule (XI) dans laquelle $R'_3$ représente un cyano. Cette réduction s'effectue selon les méthodes bien connues de l'homme de l'art.

On prépare un composé de formule (XI) dans laquelle $R'_3$ représente un groupe $-CH_2-CH_2-NR_{12}R_{13}$ dans lequel $R_{12}$ et $R_{13}$ représentent chacun un hydrogène, à partir d'un composé de formule (XI) dans laquelle $R'_3$ représente un groupe $-CH_2-CH_2-OH$, par application ou adaptation de la méthode décrite dans J. Med. Chem., 1989, 32, 391-396.

Pour préparer un composé de formule (XI) dans laquelle $R'_3$ représente un groupe $-NR_4R_5$ dans lequel $R_4$ représente un hydrogène et $R_5$ représente un $(C_1-C_7)$alkyl.e, ou respectivement un $(C_3-C_7)$cycloalkylméthyle ou un benzyle on peut effectuer une réduction d'un composé de formule (XI) dans laquelle $R'_3$ représente un groupe $-(CH_2)_q-NR_6COR_7$ dans lequel q est zéro, $R_6$ représente l'hydrogène et $R_7$ représente un hydrogène ou un $(C_1-C_6)$ alkyle, ou respectivement un $(C_3-C_7)$cycloalkyle ou un phényle. La réaction s'effectue au moyen d'un agent réducteur tel que l'hydrure d'aluminium et de lithium dans un solvant tel que le tétrahydrofurane à la température de reflux du solvant.

Par une réaction identique on peut préparer les composés de formule (XI) dans laquelle $R'_3$ représente un groupe $-NR_4R_5$ dans lequel $R_4$ représente un $(C_1-C_7)$ alkyle et $R_5$ représente un $(C_1-C_7)$alkyle, ou respectivement un $(C_3-C_7)$cycloalkyl-méthyle ou un benzyle à partir d'un composé de formule (XI) dans laquelle $R'_3$ représente un groupe $-(CH_2)_q-NR_6COR_7$ dans lequel q est zéro, $R_6$ représente un $(C_1-C_7)$alkyle et $R_7$ représente un hydrogène ou un $(C_1-C_6)$ alkyle, ou respectivement un $(C_3-C_7)$cycloalkyle ou un phényle.

De même on peut préparer les composés de formule (XI) dans laquelle $R'_3$ représente un groupe $-CH_2-NR_{12}R_{13}$ ou respectivement $-CH_2CH_2NR_{12}R_{13}$ dans lesquels $R_{12}$ représente un hydrogène ou un $(C_1-C_7)$alkyle et $R_{13}$ représente un $(C_1-C_7)$alkyle, un $(C_3-C_7)$cycloalkylméthyle ou un benzyle à partir d'un composé de formule (XI) dans laquelle $R'_3$ représente un groupe $-(CH_2)_q-NR_6COR_7$ dans lequel q est respectivement 1 ou 2, $R_6$ représente un hydrogène ou un

$(C_1-C_7)$alkyle et $R_7$ représente un hydrogène, un $(C_1-C_6)$alkyle, un $(C_3-C_7)$cycloalkyle ou un phényle.

On prépare un composé de formule (XI) dans laquelle $R'_3$ représente un groupe $-(CH_2)_q-NR_6COR_7$ dans lequel $R_6$ et $R_7$ ensemble représentent un groupe $-(CH_2)_3-$ ou $-(CH_2)_4-$ par application ou adaptation de la méthode décrite dans J. Med. Chem., 1985, 28, 46-50.

Les composés de formule (XI) dans laquelle $R'_3$ représente un groupe $-(CH_2)_q-NR_6C(=W_1)R_7$ dans lequel $W_1$ représente un atome d'oxygène, q est 0, 1 ou 2, $R_6$ représente un hydrogène ou un $(C_1-C_7)$alkyle et $R_7$ représente un hydrogène ou respectivement, un $(C_1-C_7)$alkyle, un phényle, un benzyle, un pyridyle, un $(C_3-C_7)$cycloalkyle éventuellement substitué, un furyle, un thiényle, un pyrrolyle ou un imidazolyle, s'obtiennent par action, de l'acide formique dans l'anhydride acétique ou respectivement de l'anhydride approprié $(R_7CO)_2O$ ou d'un chlorure d'acide approprié $R_7COCl$ en présence d'une base telle que la triéthylamine, sur un composé de formule (XI) dans laquelle $R'_3$ représente un groupe $-NHR_6$, $-CH_2-NHR_6$ ou $-CH_2-CH_2-NHR_6$.

De la même façon, il est clair pour l'homme du métier que par action du chlorure d'acryloyle on peut préparer les composés de formule (XI) dans laquelle $R'_3$ représente le groupe $-CH_2)_q-NR_6\ C(=W_1)R_7$ dans lequel q, $R_6$ et $W_1$ sont tels que définis ci-dessus et $R_7$ est un groupe vinyle.

De même, par action d'un chloroformiate de formule $ClCOOR_8$ en présence d'une base telle que la triéthylamine, sur un composé de formule (XI) dans laquelle $R'_3$ représente un groupe $-NHR_6$, $-CH_2-NHR_6$ ou $-CH_2-CH_2-NHR_6$, on prépare les composés de formule (XI) dans laquelle $R'_3$ représente un groupe $-(CH_2)_q-NR_6COOR_8$.

Par action d'un chlorure de sulfonyle de formule $ClSO_2R_9$, en présence d'une base telle que la triéthylamine, sur un composé de formule (XI) dans laquelle $R'_3$ représente un groupe $-NHR_6$, $-CH_2-NHR_6$ ou $-CH_2-CH_2-NHR_6$, on prépare les composés de formule (XI) dans laquelle $R'_3$ représente un groupe $-(CH_2)_q-NR_6SO_2R_9$.

Par action d'un isocyanate de formule $R_{11}N=C=O$, en présence d'une base telle que la triéthylamine, sur un composé de formule (XI) dans laquelle $R'_3$ représente un groupe $-NHR_6$, $-CH_2-NHR_6$ ou $-CH_2-CH_2-NHR_6$, on prépare les composés de formule (XI) dans laquelle $R'_3$ représente un groupe $-(CH_2)_q-NR_6C(=W_1)NR_{10}R_{11}$ dans lequel $R_{10}$ représente un hydrogène et $W_1$ représente un atome d'oxygène.

Par action d'un chlorure de carbamoyle de formule $ClCONR_{10}R_{11}$, en présence d'une base telle que la triéthylamine, sur un composé de formule (XI) dans laquelle $R'_3$ représente un groupe $-NHR_6$, $-CH_2-NHR_6$ ou $-CH_2-CH_2-NHR_6$, on prépare les composés de formule (XI) dans laquelle $R'_3$ représente un groupe $-(CH_2)_q-NR_6C(=W_1)NR_{10}R_{11}$ dans lequel $R_{10}$ représente un $(C_1-C_7)$alkyle et $W_1$ représente un atome d'oxygène.

On peut également obtenir un composé de formule (XI) dans laquelle $R'_3$ représente un groupe $-(CH_2)_q-NR_6CONR_{10}R_{11}$ par action d'un composé $HNR_{10}R_{11}$ avec un composé de formule (XI) dans laquelle $R'_3$ représente un groupe $-(CH_2)_q-NR_6COOR_8$ dans lequel $R_8$ représente un phényle.

On peut également préparer un composé de formule (XI) dans laquelle $R'_3$ représente un groupe $-(CH_2)_q-NR_6COOR_8$ dans lequel q = 0 et $R_6$ représente l'hydrogène par action d'un composé $R_8OH$ avec un composé de formule (XI) dans laquelle $R'_3$ représente un groupe isocyanato, -N = C = O.

On peut aussi préparer un composé de formule (XI) dans laquelle $R'_3$ représente un groupe $-(CH_2)_q-NR_6CONR_{10}R_{11}$ dans lequel q = 0 et $R_6$ représente l'hydrogène par action d'un composé $NHR_{10}R_{11}$ avec un composé de formule (XI) dans laquelle $R'_3$ représente un groupe isocyanato.

On prépare un composé de formule (XI) dans laquelle $R'_3$ représente un groupe isocyanato à partir d'un composé de formule (XI) dans laquelle $R'_3$ représente un carboxy selon la méthode décrite dans Organic Synthesis, 51, 48-52.

On obtient un composé de formule (XI) dans laquelle $R'_3$ représente un groupe $-(CH_2)_q\ NR_6C(=W_1)R_7$ dans lequel $W_1$ représente un atome de soufre à partir d'un composé de formule (XI) correspondant, protégé sur l'azote de la pipéridine, et dans lequel $W_1$ représente un atome d'oxygène par réaction avec du pentasulfure de phosphore ou avec le réactif de Lawesson, le 2,4-bis(4-méthoxyphényl)-1,3-dithia-2,4-diphosphétane-2,4-disulfure, suivi de la déprotection de l'azote de la pipéridine.

Par réaction d'un composé de formule (XI), protégé sur l'azote de la pipéridine, dans laquelle $R'_3$ représente un groupe $-(CH_2)_q-NR_6CONR_{10}R_{11}$ avec du pentasulfure de phosphore ou avec le réactif de Lawesson, on prépare un composé de formule (XI) dans laquelle $R'_3$ représente un groupe $-(CH_2)_q-NR_6C(=W_1)NR_{10}R_{11}$ dans lequel $W_1$ est un atome de soufre.

On prépare un composé de formule (XI) dans laquelle $R'_3$ représente un groupe $-(CH_2)_q-OH$ dans lequel q est un ou respectivement deux, par réduction d'un composé de formule (XI) dans laquelle $R'_3$ représente un méthoxycarbonyle ou respectivement un méthoxycarbonylméthyle selon la méthode décrite dans Chem. Ber., 1975, 108 ,3475-3482.

Par action d'un chlorure d'acide $R_{17}COCl$ sur les composés de formule (XI) dans laquelle $R'_3$ représente un groupe $-(CH_2)_q-OH$ et en présence d'une base telle que la triéthylamine, on obtient les composés de formule (XI) dans laquelle $R'_3$ représente un groupe $-(CH_2)_q-OCOR_{17}$ ; par action de l'acide formique on obtient les composés de formule (XI) dans laquelle $R'_3$ représente un groupe $HCOO-(CH_2)_q-$.

Par action d'un chlorure de carbamoyle $(C_1-C_7)$alkyl-NHCOCl sur les composés de formule (XI) dans laquelle $R'_3$ représente un groupe $-(CH_2)_q-OH$, on obtient les composés de formule (XI) dans laquelle $R'_3$ représente un groupe

$(C_1-C_7)$alkyl-NHCOO-$(CH_2)_q$-.

On prépare les mêmes composés par action d'un isocyanate $(C_1-C_7)$alkyl-N=C=O sur les composés de formule (XI) dans laquelle R'$_3$ représente un groupe -$(CH_2)_q$-OH.

On peut préparer un composé de formule (XI) dans laquelle R'$_3$ représente un carboxy par hydrolyse d'un composé de formule (XI) dans laquelle R'$_3$ représente un cyano selon les méthodes connues de l'homme de l'art.

On peut préparer un composé de formule (XI) dans laquelle R'$_3$ représente un carboxyméthyle selon la méthode décrite dans Chem. Ber., 1975, 108, 3475-3482.

On peut préparer un composé de formule (XI) dans laquelle R'$_3$ représente un $(C_1-C_7)$alcoxycarbonyle ou respectivement un $(C_1-C_7)$alcoxycarbonylméthyle à partir d'un composé de formule (XI) dans laquelle R'$_3$ représente un carboxy ou respectivement un carboxyméthyle, par réaction d'estérification selon les méthodes bien connues de l'homme de l'art.

Pour préparer un composé de formule (XI) dans laquelle J'$_1$ représente un groupe

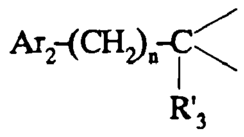

dans lequel $Ar_2$ représente un radical phényle éventuellement substitué, n est un et R'$_3$ représente un $(C_1-C_7)$alcoxycarbonyle, on fait réagir un 4-$(C_1-C_7)$alcoxycarbonylpipéridine protégé avec un halogénure de benzyle éventuellement substitué en présence d'une base telle que l'hydrure de sodium, le *tert*-butylate de potassium ou le diisopropylamidure de sodium dans un solvant tel que le tétrahydrofurane, le N,N-diméthylformamide ou le diméthylsulfoxyde, à une température comprise entre -78°C et la température ambiante. Après une étape de déprotection, on obtient le composé de formule (XI) attendue.

Pour préparer un composé de formule (XI) dans laquelle R'$_3$ représente un groupe $-CONR_{10}R_{11}$ ou respectivement un groupe $-CH_2CONR_{10}R_{11}$, on fait réagir un composé de formule (XI) dans laquelle R'$_3$ représente un carboxy ou respectivement un carboxyméthyle avec un composé de formule $HNR_{10}R_{11}$ selon les méthodes bien connues de l'homme de l'art.

Selon les méthodes précédemment citées, on prépare un composé de formule (XI) dans laquelle R'$_3$ représente un groupe $-C(=W_1)NR_{10}R_{11}$ ou un groupe $-CH_2-C(=W_1)NR_{10}R_{11}$ dans lesquels $W_1$ représente un atome de soufre à partir d'un composé de formule (XI) correspondante dans laquelle $W_1$ représente un atome d'oxygène.

Pour préparer un composé de formule (XI) dans laquelle R'$_3$ représente un groupe

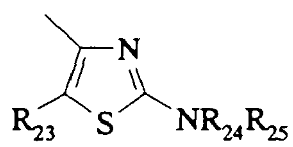

dans lequel $R_{24}$ et $R_{25}$ représentent chacun indépendamment un hydrogène ou un $(C_1-C_7)$alkyle, on fait réagir un composé de formule (XI) dans laquelle R'$_3$ représente un groupe :

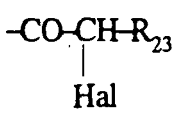

dans lequel Hal représente un atome d'halogène de préférence le brome, avec une thiourée dans laquelle un des groupes amino est libre ou substitué par un ou deux $(C_1-C_7)$alkyles.

On prépare un composé de formule (XI) dans laquelle R'$_3$ représente un groupe :

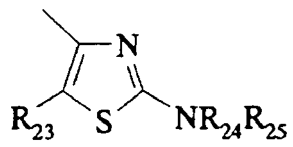

dans lequel $R_{25}$ représente un formyle ou respectivement un $(C_1\text{-}C_7)$alkylcarbonyle par réaction de l'acide formique dans l'anhydride acétique ou respectivement d'un chlorure d'acide $(C_1\text{-}C_7)$alkyl-COCl en présence d'une base telle que la triéthylamine, sur le composé de formule (XI) ci-dessus protégé sur l'azote de la pipéridine et dans laquelle $R_{25}$ représente l'hydrogène. Après une étape de déprotection on obtient le composé attendu.

Le composé de formule (XI) dans laquelle $R'_3$ représente un groupe :

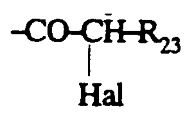

dans lequel Hal représente un atome de brome s'obtient par bromation selon les méthodes classiques d'un composé de formule (XI) dans laquelle $R'_3$ représente un groupe $-CO-CH_2-R_{23}$.

On peut préparer un composé de formule (XI) dans laquelle $J'_1$ représente un groupe

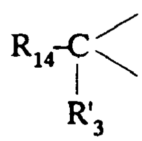

dans lequel $R_{14}$ représente un groupe $-CONR_{15}R_{16}$
et $R'_3$ représente l'hydrogène, par réaction d'une 4-carboxypipéridine protégée avec un composé de formule $HNR_{15}R_{16}$ selon les méthodes bien connues de l'homme de l'art. Après une étape de déprotection on obtient le composé de formule (XI) attendue.

Pour préparer un composé de formule (XI) dans laquelle $J'_1$ représente un groupe

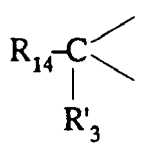

dans lequel $R_{14}$ représente un groupe $-NR_{15}R_{16}$ et $R'_3$ représente
un cyano, on effectue une réaction de Strecker en faisant réagir une 1-benzylpipérid-4-one avec un composé de formule $HNR_{15}R_{16}$ en présence de cyanure de sodium ou en présence de cyanure de potassium et de métabisulfite de sodium. Après une étape de déprotection, on obtient le composé de formule (XI) attendue.

On peut préparer un composé de formule (XI) dans laquelle $R'_3$ représente un groupe $-O-CH_2-CH_2-OR_{18}$ dans lequel $R_{18}$ représente l'hydrogène par réaction d'un composé de formule (XI) dans laquelle $R'_3$ représente un benzoyloxy avec l'éthylèneglycol en présence d'un acide tel que l'acide sulfurique.

Par une réaction identique et en utilisant un 2-$(C_1\text{-}C_7)$alcoxyéthanol, on prépare les composés de formule (XI) dans laquelle $R'_3$ représente un groupe $-O-CH_2CH_2-OR_{18}$ dans lequel $R_{18}$ représente un $(C_1\text{-}C_7)$alkyle.

Par action de l'acide formique sur un composé de formule (XI) dans laquelle $R'_3$ représente un groupe $-O-CH_2CH_2-OH$ on prépare les composés de formule (XI) dans laquelle $R'_3$ représente un groupe $-O-CH_2CH_2-OR_{18}$ dans lequel $R_{18}$ représente un formyle. Par action d'un chlorure d'acide en $C_2\text{-}C_8$ et en présence d'une base telle que la triéthylamine, on prépare les composés de formule (XI) dans laquelle $R'_3$ représente un groupe $-O-CH_2CH_2-OR_{18}$ dans lequel $R_{18}$ représente un $(C_1\text{-}C_7)$alkylcarbonyle.

On prépare un composé de formule (XI) dans laquelle $R'_3$ représente un groupe $-NR_6COCOR_{19}$ dans lequel $R_{19}$ représente un $(C_1\text{-}C_4)$alcoxy par réaction d'un composé de formule $Cl-COCOR_{19}$ avec un composé de formule (XI) dans laquelle $R'_3$ représente un groupe $-NHR_6$.

On prépare un composé de formule (XI) dans laquelle $R'_3$ représente un groupe $-CO-NR_{20}-NR_{21}R_{22}$ par

réaction d'un composé $HNR_2p-NR_{21}R_{22}$ avec un composé de formule (XI) dans laquelle $R'_3$ représente un chloroformyle.

On peut préparer un composé de formule (XI) dans laquelle $R'_3$ représente un groupe :

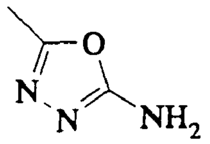

par réaction d'un composé de formule (XI) protégé dans laquelle $R'_3$ représente un groupe carbazoyle ($-CONH-NH_2$) avec le bromure de cyanogène selon la méthode décrite dans J. Org. Chem., 1961, 26, 88-95. Le composé de formule (XI) dans laquelle $R'_3$ représente un groupe carbazoyle s'obtient par réaction de l'hydrazine avec un composé de formule (XI) dans laquelle $R'_3$ représente un chloroformyle, lui-même obtenu par réaction du chlorure de thionyle avec un composé de formule (XI) dans laquelle $R'_3$ représente un carboxy.

Les pipéridines de formule (XII) sont connues ou préparées par des méthodes connues.

Les quinuclidines de formule (XIII) sont connues ou préparées par des méthodes connues telles que celles décrites par T. Perrine, J. Org. Chem., 1957, 22, 1484-1489.

Les pyridines de formule (XIV) sont connues ou préparées par des méthodes connues.

Les énantiomères des composés selon l'invention, de formule :

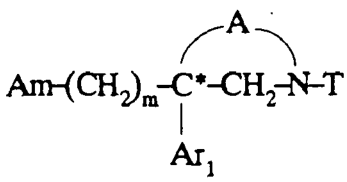 (I*)

dans laquelle :

- "*" signifie que l'atome de carbone ainsi marqué a la configuration absolue (+) ou (-) déterminée ;
- Am, m, $Ar_1$, A et T sont tels que définis pour les composés de formule (I) ;

  ainsi que leurs sels éventuels avec des acides minéraux ou organiques ;

  sont des composés nouveaux qui font partie de l'invention.

  La résolution des mélanges racémiques des composés de formule (I) permet d'isoler les énantiomères de formule (I*). Il est cependant préférable d'effectuer le dédoublement des mélanges racémiques à partir d'un composé de formule (II) ou d'un composé intermédiaire utile pour la préparation d'un composé de formule (II).

  Ainsi, lorsqu'on veut préparer les énantiomères (I*) des composés de formule (I)

dans laquelle -A- représente le radical bivalent $-CH_2-O-CO-$, on effectue le dédoublement du mélange racémique d'un composé intermédiaire de formule :

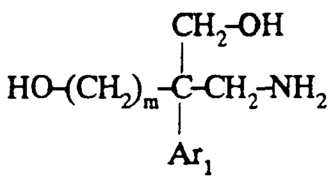 (XXXIV)

dans laquelle m et $Ar_1$ sont tels que définis pour un composé de formule (I), obtenu par élimination du groupe O-protecteur Pr, selon les méthodes précédemment décrites, d'un composé de formule (XIX).

Lorsqu'on veut préparer les énantiomères (I*) des composés de formule (I) dans laquelle -A- représente le radical bivalent -O-CO- ou $-O-CH_2-CO$ ou $-O-CH_2-CH_2-$ ou $-O-CH_2-$, on effectue le dédoublement du mélange racémique d'un composé intermédiaire de formule :

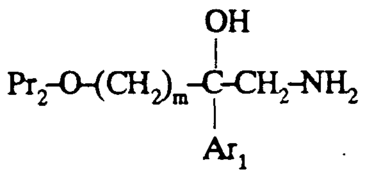

(XXVII)

dans laquelle m et $Ar_1$ sont tels que définis pour un composé de formule (I) et $Pr_2$ est tel que défini précédemment dans le SCHEMA 2 ci-dessus.

Lorsqu'on veut préparer les énantiomères (I*) des composés de formule (I) dans laquelle -A- représente le radical bivalent $-O-CH_2-CH_2-$, on peut également effectuer le dédoublement du mélange racémique d'un composé de formule :

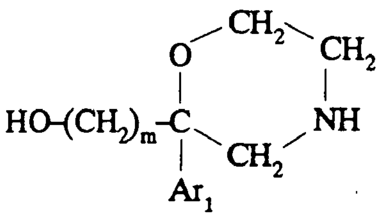

[(II) : $-A- = -O-CH_2-CH_2-$
E = H]

dans laquelle m et $Ar_1$ sont tels que définis pour un composé de formule (I).

Lorsqu'on veut préparer les énantiomères (I*) des composés de formule (I) dans laquelle -A- représente le radical bivalent $-N(R_1)-CO-$ ou $-N(R_1)-CO-CO-$ ou $-N(R_1)-CH_2-CH_2-$, on effectue le dédoublement du mélange racémique d'un composé intermédiaire de formule :

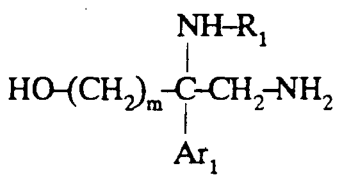

(XXXII)

dans laquelle m, $Ar_1$ et $R_1$ sont tels que définis pour un composé de formule (I).

Lorsque le dédoublement des racémiques est effectué sur les composés intermédiaires de formule (XXXIV), (XXVII), (XXXII) ou (II) [$-A- = -O-CH_2-CH_2-$ et E = H] celui-ci peut s'effectuer selon des méthodes connues par formation d'un sel avec des acides optiquement actifs, par exemple avec l'acide (+) ou (-) tartrique. Les diastéréoisomères sont alors séparés par des méthodes classiques telles que la cristallisation ou la chromatographie puis par hydrolyse on obtient les énantiomères optiquement purs.

Les composés de formule (I) ci-dessus comprennent également ceux dans lesquels un ou plusieurs atomes d'hydrogène, de carbone ou d'iode ont été remplacés par leur isotope radioactif par exemple le tritium, le carbone-14 ou l'iode-125. De tels composés marqués sont utiles dans des travaux de recherche, de métabolisme ou de pharmacocinétique, dans des essais biochimiques en tant que ligands de récepteurs.

L'affinité des composés pour les récepteurs aux tachykinines a été évaluée in vitro par plusieurs essais biochimiques utilisant des radioligands :

1) La liaison de [$^{125}$I] BH-SP (Substance P marquée à l'iode-125 à l'aide du réactif de Bolton-Hunter) aux récepteurs $NK_1$ des cellules lymphoblastiques humaines.
2) La liaison [$^{125}$I] His-$NK_A$ aux récepteurs $NK_2$ du duodénum ou de la vessie de rat.
3) La liaison [$^{125}$I] His [$Mephe^7$] $NK_B$ aux récepteurs $NK_3$ du cortex cérébral de rat, du cortex cérébral de cobaye et du cortex cérébral de gerbille ainsi qu'aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol., 1993, 250, 403-413).

Les composés selon l'invention présentent généralement une affinité pour les récepteurs aux tachykinines cités ci-dessus, avec une constante d'inhibition Ki inférieure à $10^{-8}$M.

Les composés de la présente invention sont notamment des principes actifs de compositions pharmaceutiques, dont la toxicité est compatible avec leur utilisation en tant que médicaments.

Les composés de la présente invention sont généralement administrés en unités de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Les composés de formule (I) ci-dessus et leurs sels pharmaceutiquement acceptables peuvent être utilisés à des doses journalières de 0,01. à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la silice, la gélatine, l'amidon, le lactose, le stéarate de magnésium, le talc, la gomme arabique ou analogues. On peut enrober les comprimés de saccharose, de divers polyméres ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être formulé également sous forme de microcapsules, éventuellement avec un ou plusieurs supports ou additifs.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les compositions susdites peuvent également renfermer d'autres produits actifs tels que, par exemple, des bronchodilatateurs, des antitussifs, des antihistaminiques, des antiinflammatoires, des antiémétiques, des agents de chimiothérapie.

Selon un autre de ses aspects, la présente invention concerne l'utilisation des produits de formule (I) pour la préparation de médicaments destinés à traiter des troubles physiologiques associés à un excès de tachykinines et toutes les pathologies neurokinine-dépendantes du système respiratoire, gastro-intestinal, urinaire, immunitaire, cardiovasculaire et du système nerveux central ainsi que la douleur et la migraine.

Par exemple et de manière non limitative :

- douleurs aigües et chroniques liées par exemple à la migraine, aux douleurs du cancéreux et de l'angineux, aux processus inflammatoires chroniques tels que l'ostéoarthrite et l'arthrite rhumatoïde,
- les inflammations telles que les inflammations neurogéniques, les maladies inflammatoires chroniques, par exemple, les maladies respiratoires chroniques obstructives, l'asthme, les allergies, les rhinites, les toux, les bronchites, l'hypersensibilité par exemple aux pollens et aux acariens, les arthrites rhumatoïdes, les fibrosites, les ostéoarthrites, les psoriasis, les colites ulcératives, la maladie de Crohn, l'inflammation des intestins (colon irritable), la prostatite, la vessie neurologique, l'incontinence, la cystite, l'urétrite, la néphrite, les maladies ophtalmiques telle que la conjonctivite, la vitréorétinopathie, les maladies cutanées telles que les dermatites de contact, les dermatites atopiques, l'urticaire, l'eczéma, le prurit, les brûlures, notamment les coups de soleil,
- les maladies du système immunitaire liées à la suppression ou à la stimulation des fonctions des cellules immunes par exemple l'arthrite rhumatoïde, le psoriasis, la maladie de Crohn, le diabète, le lupus, les réactions de rejet après transplantation,
- les cancers du poumon à petite cellule, les maladies de demyélination telles que la sclérose multiple ou la sclérose latérale amyotrophique,
- les maladies du système nerveux central du type neuropsychiatrique ou neurologique telles que l'anxiété, les troubles de la vigilance, de l'humeur, la dépression, la psychose, la schizophrénie, la manie, la démence, l'épilepsie, la maladie de Parkinson, la maladie d'Alzheimer, la drogue-dépendance, l'alcoolisme, le syndrôme de Down et la chorée d'Huntington ainsi que les maladies neurodégénératives, les désordres somatiques liés au stress,
- les maladies du système gastro-intestinal telles que nausées, vomissements de toutes origines, colon irritable, ulcères gastriques et duodénaux, ulcères oesophagiques, diarrhées, hypersécrétions,

- les maladies du système cardiovasculaire telles que l'hypertension, les aspects vasculaires de la migraine, les oedèmes, la thrombose, l'angine de poitrine, les spasmes vasculaires, les maladies circulatoires dues à une vasodilatation, les maladies de Reynauld, les fibroses, les maladies du collagène,
- les troubles de la fréquence et du rythme cardiaque, en particulier ceux qui sont occasionnés par la douleur ou le stress.

La présente invention inclut aussi une méthode pour traiter lesdites affections aux doses indiquées ci-dessus.

Dans les Préparations et dans les Exemples on utilise les abréviations suivantes :

Me, OMe : méthyle, méthoxy
Et, OEt : éthyle, éthoxy
EtOH : éthanol
MeOH : méthanol
Ether : éther diéthylique
Ether iso : éther diisopropylique
DMF : diméthylformamide
DMSO : diméthylsulfoxyde
DCM : dichlorométhane
THF : tétrahydrofurane
AcOEt : acétate d'éthyle
$Na_2CO_3$ : carbonate de sodium
$NaHCO_3$ : hydrogénocarbonate de sodium
NaCl : chlorure de sodium
$Na_2SO_4$ : sulfate de sodium
$MgSO_4$ : sulfate de magnésium
NaOH : soude
HCl : acide chlorhydrique
TFA : acide trifluoroacétique
KCN : cyanure de potassium
$Na_2S_2O_5$ : métabisulfite de sodium
DBU : 1,8-diazabicyclo[5.4.0]undec-7-ène
$NH_4Cl$ : chlorure d'ammonium
F : point de fusion
TA : température ambiante
silice H : gel de silice 60H commercialisé par Merck (DARMSTADT)
RMN : résonnance magnétique nucléaire
$\delta$ : déplacement chimique
s : singulet
se : singulet élargi

d : doublet
t : triplet
qd : quadruplet
mt : multiplet
m : massif

PREPARATIONS

Préparation 1.1.

5-(3,4-Dichlorophényl)-5-[2-(tétrahydropyran-2-yloxy)éthyl]-tétrahydro-2H-1,3-oxazin-2-one.

A) 2-(3,4-dichlorophényl)-4-(tétrahydropyran-2-yloxy)butanenitrile.

On refroidit au bain de glace une suspension de 17,75 g d'hydrure de sodium (à 80 % dans l'huile) dans 750 ml de THF, ajoute goutte à goutte une solution de 100 g de 3,4-dichlorophénylacétonitrile dans 250 ml de THF et laisse deux heures sous agitation à TA. On refroidit à -20°C le mélange réactionnel, ajoute goutte à goutte une solution de 112,36 g de 1-bromo-2-(tétrahydropyran-2-yloxy)éthane dans 120 ml de THF et laisse 2 heures sous agitation à TA. On concentre sous vide le mélange réactionnel, reprend le résidu à l'eau, extrait à l'éther, lave deux fois la phase organique par une solution tampon pH = 4, par une solution tampon pH = 7, deux fois par une solution saturée de NaCl, sèche sur $Na_2SO_4$ et évapore sous vide le solvant. On chromatographie le résidu sur silice en éluant au toluène puis par le mélange toluène/AcOEt (100/3 ; v/v). On obtient 113,5 g du produit attendu que l'on utilise tel quel.

B) 2-(3,4-Dichlorophényl)-2-(hydroxyméthyl)-4-(tétrahydropyran-2-yloxy) butanenitrile.

On chauffe à reflux pendant 1 heure un mélange de 12,56 g du composé obtenu à l'étape précédente, 9,6 g d'une solution à 37 % de formaldéhyde dans l'eau, 0,3 g de DBU dans 25 ml de 1,2-diméthoxyéthane. On concentre sous vide le mélange réactionnel, extrait le résidu à l'éther, lave deux fois la phase organique à l'eau, deux fois par une solution tampon pH = 4, deux fois à l'eau, deux fois par une solution saturée de NaCl, sèche sur $Na_2SO_4$ et évapore sous vide le solvant. On obtient 17 g du produit attendu que l'on utilise tel quel.

C) 2-(3,4-Dichlorophényl)-2-(hydroxyméthyl)-4-(tétrahydropyran-2-yloxy) butylamine.

On hydrogène pendant 5 heures à 40°C et à pression atmosphérique un mélange de 17 g du composé obtenu à l'étape précédente, 6 g de nickel de Raney® dans 300 ml d'EtOH et 40 ml d'une solution à 20% d'ammoniac dans l'eau. On filtre le catalyseur et concentre sous vide le filtrat. On reprend le résidu au DCM, lave la phase organique à l'eau, par une solution saturée de NaCl, sèche sur $MgSO_4$ et évapore sous vide le solvant. On obtient 16,5 g du produit attendu sous forme d'huile et que l'on utilise tel quel.

D) 5-(3,4-Dichlorophényl)-5-[2-(tétrahydropyran-2-yloxy)éthyl]-tétrahydro-2*H*-1,3-oxazin-2-one.

On refroidit à -70°C 24,6 g d'une solution à 20% de phosgène dans le toluène, dilué dans 150 ml de DCM, ajoute goutte à goutte une solution de 16,5 g du composé obtenu à l'étape précédente, 5,7 g de triéthylamine dans 100 ml de DCM et laisse sous agitation en laissant remonter la température à TA. On concentre sous vide le mélange réactionnel, reprend le résidu par un mélange eau/AcOEt, essore le produit qui cristallise à l'interphase et obtient un premier jet du produit attendu. Après décantation du filtrat, on lave la phase organique à l'eau, par une solution tampon pH = 4, par une solution saturée de NaCl, sèche sur $MgSO_4$ et évapore sous vide le solvant. On reprend le résidu à l'AcOEt, essore le produit cristallisé formé et obtient le deuxième jet du produit. On obtient au total 4,5 g du produit attendu.

Préparation 1.2

5-(3,4-dichlorophényl)-5-[3-(tétrahydropyran-2-yloxy)propyl]-tétrahydro-2*H*-1,3-oxazin-2-one.

A) 2-(3,4-dichlorophényl)-5-(tétrahydropyran-2-yloxy)pentanenitrile.

A une suspension de 12 g d'hydrure de sodium (à 55 % dans l'huile) dans 175 ml de THF, on ajoute à une température inférieure à 20°C et goutte à goutte une solution de 50,8 g de 3,4-dichlorophénylacétonitrile dans 250 ml de THF et laisse 2 heures sous agitation à TA. On refroidit à -20°C le mélange réactionnel, ajoute goutte à goutte une

solution de 62,5 g de 1-bromo-3-(tétrahydropyran-2-yloxy)propane dans 60 ml de THF et laisse sous agitation en laissant remonter la température à TA. On verse le mélange réactionnel sur une solution de 31 g de chlorure d'ammonium dans 1,4 litre d'eau, extrait à l'éther, lave les phases organiques jointes par une solution saturée de NaCl, sèche sur $MgSO_4$ et évapore sous vide les solvants. On chromatographie le résidu sur silice en éluant au toluène puis par le mélange toluène/AcOEt (95/5 ; v/v). On obtient 64 g du produit attendu que l'on utilise tel quel.

B) 2-(3,4-Dichlorophényl)-2-(hydroxyméthyl)-5-(tétrahydropyran-2-yloxy) pentanenitrile.

On chauffe à reflux pendant 1 heure un mélange de 15 g du composé obtenu à l'étape précédente, 11,2 g d'une solution à 37 % de formaldéhyde dans l'eau, 0,35 g de DBU dans 30 ml de 1,2-diméthoxyéthane. On concentre sous vide le mélange réactionnel, extrait le résidu à l'éther, lave la phase organique à l'eau, deux fois par une solution tampon pH = 4, deux fois à l'eau, deux fois par une solution saturée de NaCl, sèche sur $Na_2SO_4$ et évapore sous vide le solvant. On chromatographie le résidu sur silice en éluant au toluène puis par le mélange toluène/AcOEt (80/20 ; v/v). On obtient 15,5 g du produit attendu que l'on utilise tel quel.

C) 2-(3,4-Dichlorophényl)-2-(hydroxyméthyl)-5-(tétrahydropyran-2-yloxy) pentylamine.

On hydrogène pendant 5 heures à 30°C et à pression atmosphérique un mélange de 15,5 g du composé obtenu à l'étape précédente, 5 g de nickel de Raney® dans 200 ml d'EtOH et 40 ml d'une solution à 20 % d'ammoniac dans l'eau. On filtre le catalyseur et concentre sous vide le filtrat. On reprend le résidu au DCM, lave la phase organique à l'eau, par une solution saturée de NaCl, sèche sur $MgSO_4$ et évapore sous vide le solvant. On obtient 14,9 g du produit attendu sous forme d'huile et que l'on utilise tel quel.

D) 5-(3,4-dichlorophényl)-5-[3-(tétrahydropyran-2-yloxy)propyl]-tétrahydro-2*H*-1,3-oxazin-2-one.

On refroidit à -70°C 21,4 g d'une solution à 20 % de phosgène dans le toluène dilué dans 120 ml de DCM, ajoute goutte à goutte une solution de 14,9 g du composé obtenu à l'étape précédente, 4,98 g de triéthylamine dans 80 ml de DCM et laisse sous agitation en laissant remonter la température à TA. On concentre sous vide le mélange réactionnel, reprend le résidu à l'eau, extrait à l'éther, lave la phase organique à l'eau, par une solution saturée de NaCl, sèche sur $MgSO_4$ et évapore sous vide le solvant. On obtient 12,5 g du produit attendu que l'on utilise tel quel.

Préparation 1.3

6-(3,4-Dichlorophényl)-6-[2-(tétrahydropyran-2-yloxy)éthyl]morpholin-3-one.

A) 2-(3,4-dichlorophényl)-2-hydroxyacétonitrile.

On laisse une nuit sous agitation à TA un mélange de 70 g de 3,4-dichlorobenzaldéhyde, 90 g de $Na_2S_2O_5$ dans 300 ml d'eau. On refroidit à 0°C le mélange réactionnel, ajoute goutte à goutte une solution de 52 g de KCN dans 100 ml d'eau et laisse sous agitation en laissant remonter la température à TA. On extrait le mélange réactionnel à l'éther, lave la phase organique à l'eau, sèche sur $Na_2SO_4$ et évapore sous vide le solvant. On obtient 76 g du produit attendu que l'on utilise tel quel.

B) 2-(3,4-dichlorophényl)-2-(tétrahydropyran-2-yloxy)acétonitrile.

On refroidit à 0°C une solution de 76 g du composé obtenu à l'étape précédente, 0,25 g d'acide *p*-toluènesulfonique monohydrate dans 300 ml de DCM, ajoute goutte à goutte une solution de 39 g de 3,4-dihydro-2H-pyrane dans 50 ml de DCM et laisse sous agitation en laissant remonter la température à TA. On lave le mélange réactionnel par une solution saturée de $NaHCO_3$, à l'eau, sèche la phase organique sur $Na_2SO_4$ et évapore sous vide le solvant. On obtient 33 g du produit attendu après cristallisation à 0°C dans le pentane, F = 61°C.

C) 4-(Benzoyloxy)-2-(3,4-dichlorophényl)-2-(tétrahydropyran-2-yloxy)butanenitrile.

On refroidit à -60°C 56 ml d'une solution 2M de diisopropylamidure de lithium dans le THF, ajoute goutte à goutte une solution de 32 g du composé obtenu à l'étape précédente dans 50 ml de THF et laisse 1 heure sous agitation à -60°C. On ajoute ensuite à -60°C, goutte à goutte, une solution de 25,4 g de benzoate de 2-bromoéthyle dans 50 ml de THF et laisse sous agitation en laissant remonter la température à TA. On concentre sous vide le mélange réactionnel, extrait le résidu à l'éther, lave la phase organique à l'eau, par une solution tampon pH = 4, sèche sur $Na_2SO_4$ et évapore

sous vide le solvant. On chromatographie le résidu sur silice en éluant par le mélange toluène/AcOEt (100/5 ; v/v). On obtient 34 g du produit attendu que l'on utilise tel quel.

D) 4-(Benzoyloxy)-2-(3,4-dichlorophényl)-2-(tétrahydropyran-2-yloxy)butylamine.

On hydrogène à TA et à pression atmosphérique un mélange de 34 g du composé obtenu à l'étape précédente, 10 g de nickel de Raney® dans 400 ml d'EtOH et 40 ml d'une solution concentrée d'ammoniaque. On filtre le catalyseur et concentre sous vide le filtrat. On reprend le résidu à l'eau, extrait à l'éther, lave la phase organique par une solution saturée de NaCl, sèche sur $Na_2SO_4$ et évapore sous vide le solvant. On chromatographie le résidu sur silice H en éluant par le gradient du mélange DCM/MeOH de (100/1 ; v/v) à (100/3 ; v/v). On obtient 16 g du produit attendu que l'on utilise tel quel.

E) N-(2-bromoacétyl)-4-(benzoyloxy)-2-(3,4-dichlorophényl)-2-hydroxybutylamine.

On refroidit à -60°C une solution de 16 g du composé obtenu à l'étape précédente, 4,8 g de triéthylamine dans 100 ml de DCM, ajoute goutte à goutte une solution de 5,68 g de chlorure de bromoacétyle dans 20 ml de DCM et laisse 30 minutes sous agitation. On concentre sous vide le mélange réactionnel, extrait le résidu à l'éther, lave la phase organique à l'eau, par une solution tampon pH = 4, sèche sur $Na_2SO_4$ et évapore sous vide le solvant. On dissout le produit obtenu dans un minimum de MeOH, acidifie à pH = 1 par ajout d'une solution saturée d'HCl gaz dans l'éther et évapore sous vide les solvants. On reprend le résidu à l'eau, extrait à l'AcOEt, lave la phase organique par une solution saturée de $NaHCO_3$, à l'eau, sèche sur $Na_2SO_4$ et évapore sous vide le solvant. On obtient 16 g du produit attendu que l'on utilise tel quel.

F) 6-(3,4-dichlorophényl)-6-(2-hydroxyéthyl)morpholin-3-one.

On laisse 4 heures sous agitation un mélange de 16 g du composé obtenu à l'étape précédente, 50 ml de propan-2-ol, 15 ml d'une solution de NaOH 10N et 10 ml de DMF. On concentre sous vide le mélange réactionnel, extrait le résidu à l'AcOEt, lave la phase organique à l'eau, sèche sur $Na_2SO_4$ et évapore sous vide le solvant. On chromatographie le résidu sur silice H en éluant par le gradient du mélange DCM/MeOH de (100/3 ; v/v) à (100/5 ; v/v). On obtient 6,1 g du produit attendu.

Spectre de RMN du proton à 200 MHz dans DMSO-$d_6$

$\delta$ : 2,05 ppm : mt : 2H

3,0 à 4,4 ppm : m : 6H
4,5 ppm : t : 1H
7,3 à 8,3 ppm : m : 4H

G) 6-(3,4-Dichlorophényl)-6-[2-(tétrahydropyran-2-yloxy)éthyl]morpholin-3-one.

On refroidit à 0°C une solution de 1,7 g du composé obtenu à l'étape précédente, 0,003 g d'acide *p*-toluènesulfonique monohydrate dans 50 ml de DCM, ajoute goutte à goutte 0,588 g de 3,4-dihydro-2*H*-pyrane dans 10 ml de DCM et laisse 1 heure sous agitation à TA. On concentre sous vide le mélange réactionnel, extrait le résidu à l'éther, lave la phase organique par une solution saturée de $NaHCO_3$, à l'eau, sèche sur $Na_2SO_4$ et évapore sous vide le solvant. On chromatographie le résidu sur silice H en éluant par le mélange DCM/MeOH (100/2 ; v/v). On obtient 1,8 g du produit attendu que l'on utilise tel quel.

Préparation 1.4

6-[2-(Benzoyloxy)éthyl]-6-(3,4-dichlorophényl)morpholin-3-one.

On chauffe à 130°C pendant une nuit un mélange de 4,8 g du composé obtenu à l'étape E de la Préparation 1.3, 1,4 g de $K_2CO_3$ dans 100 ml de xylène. Après refroidissement à TA, on filtre le mélange réactionnel et concentre sous vide le filtrat. On extrait le résidu à l'éther, lave la phase organique par une solution tampon pH = 2, à l'eau, sèche sur $MgSO_4$ et évapore sous vide le solvant. On chromatographie le résidu sur silice H en éluant par le mélange DCM/MeOH (100/1 ; v/v). On obtient 1,4 g du produit attendu.

Spectre de RMN du proton à 200 MHz dans DMSO-$d_6$

$\delta$ : 2,45 ppm : mt : 2H

3,75 ppm : système AB : 2H
3,9 à 4,5 ppm : m : 4H
7,4 à 7,9 ppm : m : 8H
8,25 ppm : se : 1 H

On peut également obtenir ce composé en suivant les trois étapes du procédé décrit ci-après.

A') Chlorhydrate de 4-(benzoyloxy)-2-(3,4-dichlorophényl)-2-hydroxybutylamine.

Ce composé est décrit à l'étape A de la Préparation 1.7.

B') N-(2-Chloroacétyl)-4-(benzoyloxy)-2-(3,4-dichlorophényl)-2-hydroxybutylamine.

On refroidit à 0°C une solution de 20 g du composé obtenu à l'étape précédente, 10,3 g de triéthylamine dans 100 ml de DCM, ajoute goutte à goutte 5,8 g de chlorure de chloroacétyle et laisse 30 minutes sous agitation. On concentre sous vide le mélange réactionnel, extrait le résidu à l'AcOEt, lave la phase organique à l'eau, par une solution tampon pH = 2, à l'eau, sèche sur $MgSO_4$ et évapore sous vide le solvant. On obtient 22 g du produit attendu que l'on utilise tel quel.

C') 6-[2-(Benzoyloxy)éthyl]-6-(3,4-dichlorophényl)morpholin-3-one.

On refroidit à -10°C une solution de 22 g du composé obtenu à l'étape précédente dans 600 ml de THF, ajoute 11,42 g de *tert*-butylate de potassium et laisse sous agitation jusqu'à dissolution complète. On concentre sous vide le mélange réactionnel, extrait le résidu à l'AcOEt, lave la phase organique par une solution tampon pH = 2, à l'eau, sèche sur $MgSO_4$ et évapore sous vide le solvant. On obtient 12,8 g du produit attendu après cristallisation dans l'éther.

Préparation 1.5

2-(3,4-dichlorophényl)-2-(2-hydroxyéthyl)morpholine.

On chauffe à 60°C une suspension de 1,6 g d'hydrure d'aluminium et de lithium dans 25 ml de THF, ajoute goutte à goutte une solution de 4 g du composé obtenu à l'étape F de la Préparation 1.3 dans 20 ml de THF et laisse 30 minutes sous agitation à reflux. Après refroidissement, on ajoute 1,5 ml d'eau, 1,5 ml de NaOH 4N puis 4,5 ml d'eau. On filtre sur Célite® les sels minéraux, décante le filtrat, et évapore sous vide la phase organique. On reprend le résidu à l'éther, sèche sur $Na_2SO_4$ et évapore sous vide le solvant. On obtient 3,6 g du produit attendu.

Préparation 1.6

2-(3,4-Dichlorophényl)-2-(3-hydroxypropyl)morpholine.

A) 5-(Benzoyloxy)-2-(3,4-dichlorophényl)-2-(tétrahydropyran-2-yloxy) pentanenitrile.

On refroidit à -60°C 47 ml d'une solution 1,5M de diisopropylamidure de lithium dans le THF, ajoute goutte à goutte une solution de 19,3 g du composé obtenu à l'étape B de la Préparation 1.3 dans 100 ml de THF et laisse 30 minutes sous agitation à -60°C. On ajoute ensuite à -60°C, goutte à goutte, 17 g de benzoate de 3-bromopropyle et laisse sous agitation en laissant remonter la température à TA. On concentre sous vide le mélange réactionnel, extrait le résidu à l'éther, lave la phase organique à l'eau, par une solution saturée de NaCl, sèche sur $Na_2SO_4$ et évapore sous vide le solvant. On obtient 21 g du produit attendu après cristallisation dans l'hexane.

B) 5-(Benzoyloxy)-2-(3,4-dichlorophényl)-2-(tétrahydropyran-2-yloxy)pentylamine.

On hydrogène à TA et à pression atmosphérique un mélange de 20 g du composé obtenu à l'étape précédente, 7 g de nickel de Raney® dans 300 ml de MeOH. On filtre le catalyseur et concentre sous vide le filtrat. On reprend le résidu à l'eau, extrait à l'éther, lave la phase organique à l'eau, sèche sur $Na_2SO_4$ et évapore sous vide le solvant. On obtient 20 g du produit attendu que l'on utilise tel quel.

C) N-(2-Chloroacétyl)-5-(benzoyloxy)-2-(3,4-dichlorophényl)-2-(tétrahydropyran-2-yloxy)pentylamine.

On refroidit à 0°C une solution de 9 g du composé obtenu à l'étape précédente, 2,4 g de triéthylamine dans

100 ml de DCM, ajoute goutte à goutte une solution de 2,23 g de chlorure de chloroacétyle dans 20 ml de DCM et laisse 30 minutes sous agitation. On concentre sous vide le mélange réactionnel, extrait le résidu à l'éther, lave la phase organique à l'eau, sèche sur $Na_2SO_4$ et évapore sous vide le solvant. On obtient 9,5 g du produit attendu que l'on utilise tel quel.

D) N-(2-Chloroacétyl)-5-(benzoyloxy)-2-(3,4-dichlorophényl)-2-hydroxy pentylamine.

A une solution de 9 g du composé obtenu à l'étape précédente dans 50 ml de DCM et 50 ml de MeOH on ajoute jusqu'à pH = 1 une solution saturée d'HCl gaz dans l'éther et laisse 30 minutes sous agitation à TA. On concentre sous vide, extrait le résidu à l'éther, lave la phase organique à l'eau, par une solution saturée de $NaHCO_3$, sèche sur $Na_2SO_4$ et évapore sous vide le solvant. On chromatographie le résidu sur silice H en éluant par le mélange DCM/ MeOH (100/3 ; v/v). On obtient 4,7 g du produit attendu que l'on utilise tel quel.

E) 6-(3,4-Dichlorophényl)-6-(3-hydroxypropyl)morpholin-3-one.

On laisse 2 heures sous agitation à TA un mélange de 2,95 g du composé obtenu à l'étape précédente, 40 ml de propan-2-ol, 3 ml d'une solution de NaOH 10N. On concentre sous vide le mélange réactionnel, extrait le résidu au DCM, lave la phase organique à l'eau, sèche sur $Na_2SO_4$ et évapore sous vide le solvant. On chromatographie le résidu sur silice H en éluant par le gradient du mélange DCM/MeOH (100/2 ; v/v) à (100/5 ; v/v). On obtient 0,5 g du produit attendu, F = 130-132°C.

F) 2-(3,4-Dichlorophényl)-2-(3-hydroxypropyl)morpholine.

On chauffe à 60°C une suspension de 0,82 g d'hydrure d'aluminium et de lithium dans 10 ml de THF, ajoute goutte à goutte une solution de 2 g du composé obtenu à l'étape précédente dans 20 ml de THF et laisse 30 minutes sous agitation à reflux. Après refroidissement, on ajoute 1 ml d'eau, 1 ml de NaOH 4N puis 3 ml d'eau. On filtre sur Célite® les sels minéraux, décante le filtrat, et évapore sous vide la phase organique. On reprend le résidu à l'éther, sèche sur $Na_2SO_4$ et évapore sous vide le solvant. On obtient 2 g du produit attendu.
Spectre de RMN du proton à 200 MHz dans DMSO-$d_6$
$\delta$ : 0,8 à 2,2 ppm : m : 4H

2,5 à 3,7 ppm : m : 8H
4,3 ppm : t : 1H
7,2 à 7,7 ppm : m : 3H

Préparation 1.7

5-[2-(Benzoyloxy)éthyl]-5-(3,4-dichlorophényl)oxazolidin-2-one.

A) Chlorhydrate de 4-(benzoyloxy)-2-(3,4-dichlorophényl)-2-hydroxybutylamine.

A une solution de 12 g du composé obtenu à l'étape D de la Préparation 1.3 dans 50 ml de MeOH on ajoute à TA jusqu'à pH = 1 une solution saturée d'HCl gaz dans l'éther et laisse 1 heure sous agitation à TA. On concentre sous vide le mélange réactionnel, reprend le résidu au DCM, essore le précipité formé et le lave à l'éther. On obtient 3,4 g du produit attendu après recristallisation dans le propan-2-ol, F = 200-204°C.

B) 5-[2-(Benzoyloxy)éthyl]-5-(3,4-dichlorophényl)oxazolidin-2-one.

A une solution de 3 g du composé obtenu à l'étape précédente, 0,85 g de triéthylamine dans 30 ml de 1,2-dichloroéthane, on ajoute à TA 1,4 g de 1,1'-carbonyldiimidazole, laisse 30 minutes sous agitation à TA puis chauffe à 50°C pendant 2 heures. On concentre sous vide le mélange réactionnel, reprend le résidu à l'eau, extrait au DCM, lave la phase organique par une solution tampon pH = 2, à l'eau, sèche sur $Na_2SO_4$ et évapore sous vide le solvant. On obtient 3 g du produit attendu.
Spectre de RMN du proton à 200 MHz dans DMSO-$d_6$
$\delta$ : 2,6 ppm : mt : 2H

3,75 ppm : système AB : 2H
4,35 ppm : mt : 2H

7,4 à 7,8 ppm : m : 8H
7,9 ppm : s : 1H

Préparation 1.8

6-(3,4-Dichlorophényl)-1-méthyl-6-[2-(tétrahydropyran-2-yloxy)éthyl]pipérazin-2,3-dione.

A) Chlorhydrate de 2-(3,4-dichlorophényl)-2-(méthylamino)acétonitrile.

On refroidit au bain de glace un mélange de 10 g de 3,4-dichlorobenzaldéhyde et 9,5 ml de cyanotriméthylsilane, ajoute 10 mg d'iodure de zinc et laisse 30 minutes sous agitation à TA. Puis on ajoute 20 ml d'une solution à 33 % de méthylamine dans l'EtOH et chauffe à 40°C pendant 2 heures. On concentre sous vide le solvant, extrait le résidu à l'éther, sèche la phase organique sur $MgSO_4$ et filtre. On ajoute au filtrat une solution saturée d'HCl gaz dans l'éther jusqu'à pH = 1, puis de l'acétone jusqu'à précipitation du produit. On essore le précipité formé, le lave à l'éther et sèche. On obtient 12,8 g du produit attendu, F = 172°C.

B) 2-(*tert*-Butoxycarbonyl-N-méthylamino)-2-(3,4-dichlorophényl)acétonitrile.

A une suspension de 12,8 g du composé obtenu à l'étape précédente dans l'eau, on ajoute jusqu'à pH = 13 une solution de NaOH concentrée, extrait à l'éther, sèche la phase organique sur $MgSO_4$ et évapore sous vide le solvant. On reprend le résidu dans 20 ml de 1,4-dioxane, ajoute 12,5 g de di-*tert*-butyl dicarbonate et chauffe à 60°C pendant 2 heures. On concentre sous vide le mélange réactionnel, extrait le résidu à l'éther, lave la phase organique par une solution tampon pH = 2, par une solution saturée de NaCl, par une solution à 10 % de $Na_2CO_3$, sèche sur $MgSO_4$ et évapore sous vide le solvant. On chromatographie le résidu sur silice en éluant à l'heptane, puis par le mélange heptane/AcOEt (96/4 ; v/v). On obtient 12,7 g du produit attendu que l'on utilise tel quel.

C) 2-(*tert*-Butoxycarbonyl-N-méthylamino)-2-(3,4-dichlorophényl)-4-(tétrahydro pyran-2-yloxy)butanenitrile.

A une suspension de 1,5 g d'hydrure de sodium (à 60 % dans l'huile) dans 50 ml de DMF, on ajoute, goutte à goutte et en maintenant la température à 25°C, une solution de 11,1 g du composé obtenu à l'étape précédente dans 60 ml de DMF et laisse 1 heure sous agitation à TA. Puis on ajoute une solution de 8,1 g de 1-bromo-2-(tétrahydropyran-2-yloxy)éthane dans 20 ml de DMF et chauffe à 60°C pendant 4 heures. Après refroidissement à TA, on verse le mélange réactionnel sur un mélange glace/tampon pH = 2, extrait à l'éther, lave deux fois la phase organique à l'eau, sèche sur $MgSO_4$ et évapore sous vide le solvant. On chromatographie le résidu sur silice en éluant à l'heptane puis par le mélange heptane/AcOEt (75/25 ; v/v). On obtient 13,2 g du produit attendu que l'on utilise tel quel.

D) 2-(*tert*-Butoxycarbonyl-N-méthylamino)-2-(3,4-dichlorophényl)-4-(tétrahydro pyran-2-yloxy)butylamine.

On hydrogène à 30°C et à pression atmosphérique un mélange de 13,2 g du composé obtenu à l'étape précédente, 4 g de nickel de Raney® , dans 150 ml d'EtOH et 50 ml d'une solution à 20 % d'ammoniaque. Après 5 heures, on filtre le catalyseur et concentre sous vide le filtrat. On extrait le résidu à l'éther, lave deux fois la phase organique à l'eau, sèche sur $MgSO_4$ et évapore sous vide le solvant. On obtient 12,6 g du produit attendu que l'on utilise tel quel.

E) Chlorhydrate de 2-(3,4-dichlorophényl)-4-hydroxy-2-(méthylamino)butylamine.

On chauffe à 70°C pendant 1 heure un mélange de 4,6 g du composé obtenu à l'étape précédente, 10 ml d'une solution d'HCl concentré dans 40 ml de MeOH. Puis on concentre sous vide le solvant, reprend le résidu dans l'acétone, essore le précipité formé, le lave à l'éther et sèche. On obtient 2,79 g du produit attendu, F = 240°C (déc).

F) 6-(3,4-dichlorophényl)-6-(2-hydroxyéthyl)-1-méthylpipérazin-2,3-dione.

A une suspension de 5,3 g du composé obtenu à l'étape précédente dans l'eau, on ajoute jusqu'à pH = 13 une solution de NaOH concentrée, extrait à l'éther, sèche la phase organique sur $MgSO_4$ et évapore sous vide le solvant. On reprend le produit obtenu (4 g) dans 50 ml d'EtOH, ajouté 2,57 g d'oxalate de diéthyle et laisse 1 heure sous agitation à TA. On concentre sous vide le mélange réactionnel, reprend le résidu dans 60 ml de toluène et chauffe à reflux pendant 70 heures. On concentre sous vide et obtient 2,8 g du produit attendu après cristallisation dans le DCM, F = 260°C.

G) 6-(3,4-dichlorophényl)-1-méthyl-6-[2-(tétrahydropyran-2-yloxy)éthyl] pipérazin-2,3-dione.

A une suspension de 2,8 g du composé obtenu à l'étape précédente dans 50 ml de DCM, on ajoute 0,1 g d'acide *p*-toluènesulfonique monohydrate puis 1,26 ml de 3,4-dihydro-2*H*-pyrane et laisse une nuit sous agitation à TA. On lave le mélange réactionnel par une solution à 10 % de $Na_2CO_3$, à l'eau, sèche la phase organique sur $MgSO_4$ et évapore sous vide le solvant. On chromatographie le résidu sur silice en éluant à l'AcOEt, puis par le mélange AcOEt/MeOH (93/7 ; v/v). On obtient 3,1 g du produit attendu que l'on utilise tel quel.

Préparation 1.9

2-(3,4-dichlorophényl)-1-méthyl-2-[2-(tétrahydropyran-2-yloxy)éthyl]pipérazine

A une suspension de 1,2 g d'hydrure d'aluminium et de lithium dans 20 ml de THF, on ajoute goutte à goutte une solution de 2 g du composé obtenu à la Préparation 1.8 dans 20 ml de THF et chauffe à reflux pendant 1 heure. Après refroidissement, on ajoute 5 ml d'eau, filtre les sels minéraux, concentre sous vide le filtrat, reprend le résidu à l'éther, sèche la phase organique sur $MgSO_4$ et évapore sous vide le solvant. On obtient 1,9 g du produit attendu sous forme d'huile.

Préparation 1.10

6-[2-(Benzoyloxy)éthyl]-6-(3,4-difluorophényl)morpholin-3-one.

A) 2-(3,4-Difluorophényl)-2-hydroxyacétonitrile.

On chauffe à 50°C une solution de 80,2 g de $Na_2S_2O_5$ dans 250 ml d'eau, ajoute 50 g de 3,4-difluorobenzaldéhyde, laisse 1 heure sous agitation à 50°C et une nuit à TA. On refroidit à 0°C le mélange réactionnel, ajoute goutte à goutte une solution de 77,7 g du KCN dans 100 ml d'eau et laisse sous agitation en laissant remonter la température à TA puis poursuit l'agitation 1 heure à TA. On extrait le mélange réactionnel à l'éther, lave la phase organique à l'eau, sèche sur $MgSO_4$ et évapore sous vide le solvant. On obtient 48 g du produit attendu que l'on utilise tel quel.

B) 2-(3,4-Difluorophényl)-2-(tétrahydropyran-2-yloxy)acétonitrile.

On refroidit à 0°C une solution de 48 g du composé obtenu à l'étape précédente, 0,2 g d'acide *p*-toluènesulfonique monohydrate dans 500 ml de DCM, ajoute goutte à goutte une solution de 28,6 g de 3,4-dihydro-2*H*-pyrane dans 50 ml de DCM et laisse sous agitation en laissant remonter la température à TA puis poursuit l'agitation une nuit à TA. On concentre sous vide le mélange réactionnel, extrait le résidu au DCM, lave la phase organique par une solution à 10 % de $Na_2CO_3$, à l'eau, sèche la phase organique sur $Na_2SO_4$ et évapore sous vide le solvant. On chromatographie le résidu sur silice en éluant par le mélange toluène/AcOEt (100/15 ; v/v). On obtient 43 g du produit attendu que l'on utilise tel quel.

C) 4-(Benzoyloxy)-2-(3,4-difluorophényl)-2-(tétrahydropyran-2-yloxy) butanenitrile.

On refroidit à -60°C 133 ml d'une solution 1,5M de diisopropylamidure de lithium dans le THF, ajoute goutte à goutte une solution de 43 g du composé obtenu à l'étape précédente dans 250 ml de THF et laisse 30 minutes sous agitation à -60°C. On ajoute ensuite à -60°C, goutte à goutte, une solution de 45,8 g de benzoate de 2-bromoéthyle dans 100 ml de THF et laisse sous agitation en laissant remonter la température à TA. On concentre sous vide le mélange réactionnel, extrait le résidu à l'éther, lave la phase organique à l'eau, par une solution tampon pH = 4, sèche sur $Na_2SO_4$ et évapore sous vide le solvant. On chromatographie le résidu sur silice en éluant par le mélange toluène/AcOEt (100/5 ; v/v). On obtient 47 g du produit attendu que l'on utilise tel quel.

D) 4-(Benzoyloxy)-2-(3,4-difluorophényl)-2-(tétrahydropyran-2-yloxy)butylamine.

On hydrogène à TA et à pression atmosphérique un mélange de 47 g du composé obtenu à l'étape précédente, 10 g de nickel de Raney® dans 400 ml d'EtOH. On filtre le catalyseur et concentre sous vide le filtrat. On reprend le résidu à l'eau, extrait à l'éther, lave la phase organique à l'eau, sèche sur $Na_2SO_4$ et évapore sous vide le solvant. On obtient 45 g du produit attendu que l'on utilise tel quel.

E) Chlorhydrate de 4-(benzoyloxy)-2-(3,4-difluorophényl)-2-hydroxybutylamine.

A une solution de 45 g du composé obtenu à l'étape précédente dans 250 ml de MeOH on ajoute à TA jusqu'à pH = 1 une solution saturée d'HCl gaz dans l'éther et laisse 30 minutes sous agitation à TA. On concentre sous vide le mélange réactionnel, reprend le résidu à l'éther, essore le précipité formé et le lave à l'éther. On obtient 15 g du produit attendu après recristallisation dans le propan-2-ol, F = 202-204°C.

F) N-(2-Chloroacétyl)-4-(benzoyloxy)-2-(3,4-difluorophényl)-2-hydroxybutylamine.

On refroidit à 0°C une solution de 12,2 g du composé obtenu à l'étape précédente, 7,88 g de triéthylamine dans 100 ml de DCM, ajoute goutte à goutte une solution de 3,85 g de chlorure de chloroacétyle dans 100 ml de DCM et laisse 30 minutes sous agitation. On concentre sous vide le mélange réactionnel, extrait le résidu par le mélange éther/AcOEt (50/50 ; v/v), lave la phase organique à l'eau, par une solution tampon pH = 4, à l'eau, sèche sur $Na_2SO_4$ et évapore sous vide le solvant. On obtient 13,5 g du produit attendu que l'on utilise tel quel.

G) 6-[2-(Benzoyloxy)éthyl]-6-(3,4-difluorophényl)morpholin-3-one.

On chauffe à reflux pendant une nuit un mélange de 13,5 g du composé obtenu à l'étape précédente, 20,7 g de $K_2CO_3$ dans 100 ml de toluène. On concentre sous vide le mélange réactionnel, reprend le résidu à l'éther et filtre. On lave le filtrat à l'eau, par une solution tampon pH = 2, à l'eau, sèche sur $MgSO_4$ et évapore sous vide le solvant. On chromatographie le résidu sur silice H en éluant par le mélange DCM/MeOH (100/1 ; v/v). On obtient 4,9 g du produit attendu.

Spectre de RMN du proton à 200 MHz dans DMSO-$d_6$

$\delta$ : 2,35 ppm : mt : 2H

3,65 ppm : système AB : 2H
3,8 à 4,35 ppm : m : 4H
7,1 à 7,8 ppm : m : 8H
8,2 ppm : se : 1H

Préparation 1.11

2-(3,4-Difluorophényl)-2-(2-hydroxyéthyl)morpholine.

A une solution de 2,8 g du composé obtenu à la Préparation 1.10 dans 20 ml de THF, on ajoute goutte à goutte à TA, une suspension de 1,8 g d'hydrure d'aluminium et de lithium dans 20 ml de THF, puis on chauffe à reflux pendant 5 heures. Après refroidissement, on ajoute 2 ml d'eau, 2 ml de NaOH 4N puis 6 ml d'eau. On filtre les sels minéraux et concentre sous vide le filtrat. On dissout le résidu dans du DCM, acidifie jusqu'à pH = 1 par ajout d'une solution saturée d'HCl gaz dans l'éther, extrait à l'eau, lave la phase aqueuse à l'éther, alcalinise la phase aqueuse jusqu'à pH = 8 par ajout d'une solution concentrée de NaOH, extrait au DCM, lave la phase organique à l'eau, sèche sur $MgSO_4$ et évapore sous vide le solvant. On obtient 0,77 g du produit attendu que l'on utilise tel quel à l'EXEMPLE 27 étape A.

Préparation 1.12

5-[2-(Benzoyloxy)éthyl]-5-(3,4-difluorophényl)oxazolidin-2-one.

A une solution de 2,1 g du composé obtenu à l'étape E de la Préparation 1.10, 0,63 g de triéthylamine dans 50 ml de 1,2-dichloroéthane, on ajoute à TA 1 g de 1,1'-carbonyldiimidazole, laisse ensuite 1 heure sous agitation à TA puis chauffe à 50°C pendant 2 heures. On concentre sous vide le mélange réactionnel, extrait le résidu au DCM, lave la phase organique à l'eau, par une solution tampon pH = 2, à l'eau, sèche sur $MgSO_4$ et évapore sous vide le solvant. On obtient 1,5 g du produit attendu.

Préparation 1.13

5-(3,4-Dichlorophényl)-1-méthyl-5-[2-(tétrahydropyran-2-yloxy)éthyl] imidazolidin-2-one.

A) 2-(3,4-Dichlorophényl)-2-(méthylamino)-4-(tétrahydropyran-2-yloxy) butylamine.

On chauffe à 60°C pendant 45 minutes un mélange de 3,4 g du composé obtenu à l'étape E de la Préparation 1.8, 0,05 g d'acide *p*-toluènesulfonique monohydrate, 2,1 ml de 3,4-dihydro-2*H*-pyrane dans 30 ml de DMF. On verse le mélange réactionnel sur de la glace, alcalinise par ajout de NaOH concentré, extrait à l'éther, lave la phase organique à l'eau, sèche sur $MgSO_4$ et évapore sous vide le solvant. On obtient 4,3 g du produit attendu que l'on utilise tel quel.

B) 5-(3,4-Dichlorophényl)-1-méthyl-5-[2-(tétrahydropyran-2-yloxy)éthyl] imidazolidin-2-one.

A une solution de 3,2 g du composé obtenu à l'étape précédente dans 100 ml de 1,2-dichloroéthane on ajoute 1,6 g de 1,1'-carbonyldiimidazole et laisse 30 minutes sous agitation à TA. Puis on chauffe à 60°C pendant 2 heures et concentre sous vide le mélange réactionnel. On extrait le résidu à l'AcOEt, lave la phase organique par une solution tampon pH = 2, par une solution saturée de NaCl, par une solution à 10 % de $Na_2CO_3$, sèche sur $MgSO_4$ et évapore sous vide le solvant. On chromatographie le résidu sur silice H en éluant au DCM puis par le mélange DCM/MeOH (98/2 ; v/v). On obtient 2,4 g du produit attendu sous forme d'huile.

Préparation 1.14

6-[3-(Benzoyloxy)propyl]-6-(3,4-dichlorophényl)morpholin-3-one.

On chauffe pendant une nuit à reflux un mélange de 7,5 g du composé obtenu à l'étape D de la Préparation 1.6, 9,3 g de $K_2CO_3$, 3,75 g d'iodure de sodium dans 100 ml de méthyléthylcétone. Après refroidissement, on concentre sous vide le mélange réactionnel, reprend le résidu à l'éther, filtre les sels minéraux, lave le filtrat par une solution tampon pH = 2, à l'eau, sèche la phase organique sur $MgSO_4$ et évapore sous vide le solvant. On chromatographie le résidu sur silice H en éluant au DCM puis par le gradient du mélange DCM/MeOH de (99/1 ; v/v) à (98/2 ; v/v). On obtient 1,3 g du produit attendu.

Spectre de RMN du proton à 200 MHz dans DMSO-$d_6$

δ: 1,1 à 2,3 ppm:m:4H

3,65 ppm : système AB : 2H
3,8 à 4,4 ppm : m : 4H
7,2 à 8,05 ppm: m : 8H
8,2 ppm : s : 1H

Préparation 1.15

6-[2-(Benzoyloxy)éthyl]-6-(3,4-difluorophényl)morpholin-3-one, isomère (-).

A) 4-(Benzoyloxy)-2-(3,4-difluorophényl)-2-hydroxybutylamine, isomère (+).

On chauffe à reflux une solution de 41 g d'acide L-(+)-tartrique dans 1200 ml de MeOH, puis ajoute en une seule fois une solution de 81,4 g du composé obtenu à l'étape E de la Préparation 1.10, sous forme de base libre, dans 200 ml de MeOH et laisse 48 heures en cristallisation en laissant revenir la température à TA. On essore les cristaux formés et les lave à l'éther. On obtient 42,5 g du sel d'acide tartrique.

$$\alpha_D^{20} = +36{,}2° \text{ (c = 1 ; DMF)}$$

On recristallise le sel ainsi obtenu dans 1450 ml d'EtOH 70, et obtient, après essorage et lavage à l'éther des cristaux formés, 35 g du sel d'acide tartrique.

$$\alpha_D^{20} = +38{,}9° \text{ (c = 1 ; DMF)}$$

On reprend le sel ainsi obtenu dans 2000 ml d'AcOEt, ajoute 40 ml d'une solution de $Na_2CO_3$ à 10 %, puis, après agitation et décantation, lave la phase organique à l'eau, sèche sur $MgSO_4$ et évapore sous vide le solvant. On obtient 23,5 g du produit attendu après cristallisation dans le mélange éther iso/pentane, F = 100,5-100,6°C.

$$\alpha_D^{20} = +42,5° \ (c = 1 ; MeOH)$$

B) N-(2-Chloroacétyl)-4-(benzoyloxy)-2-(3,4-difluorophényl)-2-hydroxybutylamine, isomère (+).

On refroidit à 0°C une solution de 23,5 g du composé obtenu à l'étape précédente, 8,1 g de triéthylamine dans 300 ml de DCM, ajoute goutte à goutte une solution de 8,3 g de chlorure de chloroacétyle dans 50 ml de DCM et laisse 30 minutes sous agitation à 0°C. On concentre sous vide le mélange réactionnel, extrait le résidu par le mélange AcOEt/éther (50/50 ; v/v), lave la phase organique par une solution tampon pH = 2, sèche sur $MgSO_4$ et évapore sous vide le solvant. On obtient 24,8 g du produit attendu après cristallisation dans le mélange éther iso/pentane.

$$\alpha_D^{20} = +26,1° \ (c = 1 ; MeOH)$$

C) 6-[2-(Benzoyloxy)éthyl]-6-(3,4-difluorophényl)morpholin-3-one, isomère (-).

On refroidit à 0°C une solution de 23,6 g du composé obtenu à l'étape précédente dans 750 ml de THF, ajoute 13,7 g de *tert*-butylate de potassium et laisse 15 minutes sous agitation. On concentre sous vide le mélange réactionnel, extrait le résidu à l'AcOEt, lave la phase organique par une solution tampon pH = 2, à l'eau, sèche sur $MgSO_4$ et évapore sous vide le solvant. On chromatographie le résidu sur silice H en éluant par le mélange DCM/MeOH de (100/1 ; v/v) à (100/1,5 ; v/v). On obtient 14,5 g du produit attendu après cristallisation dans le pentane.

$$\alpha_D^{20} = -7,1° \ (c = 1 ; MeOH)$$

Préparation 1.16

6-[2-(Benzoyloxy)éthyl]-6-(3,4-difluorophényl)morpholin-3-one, isomère (+).

A) 4-(Benzoyloxy)-2-(3,4-difluorophényl)-2-hydroxybutylamine, isomère (-).

On concentre sous vide les jus d'essorage et de lavage obtenus à la cristallisation puis à la recristallisation du sel d'acide tartrique préparé à l'étape A de la Préparation 1.15. On traite le résidu par une solution de $Na_2CO_3$ à 10 %, extrait à l'AcOEt, sèche la phase organique sur $MgSO_4$, évapore sous vide le solvant et obtient 49 g d'amino-alcool sous forme d'un mélange d'isomères. On dissout l'amino-alcool dans 120 ml de MeOH et ajoute en une seule fois cette solution à une solution, chauffée à reflux, de 24,1 g d'acide D-(-)-tartrique dans 730 ml de MeOH. On laisse 48 heures en cristallisation en laissant revenir la température à TA. On essore les cristaux formés et les lave à l'éther. On obtient 40,7 g du sel d'acide tartrique.

On recristallise le sel ainsi obtenu dans 1445 ml d'EtOH 70, et obtient, après essorage et lavage à l'éther des cristaux formés, 35 g du sel d'acide tartrique. On reprend le sel ainsi obtenu dans 2000 ml d'AcOEt, ajoute une solution de $Na_2CO_3$ à 10 %, puis, après agitation et décantation, lave la phase organique à l'eau, sèche sur $MgSO_4$ et évapore sous vide le solvant. On obtient 27 g du produit attendu, F = 102°C.

$$\alpha_D^{20} = -40,5° \ (c = 1 ; MeOH)$$

B) N-(2-Chloroacétyl)-4-(benzoyloxy)-2-(3,4-difluorophényl)-2-hydroxybutylamine, isomère (-).

On refroidit à -30°C une solution de 27 g du composé obtenu à l'étape précédente, 13 ml de triéthylamine dans 500 ml de DCM, ajoute goutte à goutte 5,8 g de chlorure de chloroacétyle et laisse 15 minutes sous agitation. Puis on lave le mélange réactionnel à l'eau, par une solution d'HCl 1N, par une solution de $Na_2CO_3$ à 10 %, sèche la phase organique sur $MgSO_4$ et évapore sous vide le solvant. On obtient 28,6 g du produit attendu après cristallisation dans le mélange éther iso/pentane, F = 63°C.

$$\alpha_D^{20} = -31,5° \ (c = 1 \ ; MeOH)$$

C) 6-[2-(Benzoyloxy)éthyl]-6-(3,4-difluorophényl)morpholin-3-one, isomère (+).

On refroidit à -30°C une solution de 28,5 g du composé obtenu à l'étape précédente dans 250 ml de THF, ajoute en une fois 18,5 g de *tert*-butylate de potassium et laisse 45 minutes sous agitation. On verse le mélange réactionnel dans 1000 ml d'une solution tampon pH = 2, extrait à l'éther, sèche la phase organique sur $MgSO_4$ et évapore sous vide le solvant. On obtient 20,5 g du produit attendu après cristallisation dans le mélange éther/éther iso, F = 92°C.

$$\alpha_D^{20} = +8,2° \ (c = 1 \ ; MeOH)$$

Préparation 1.17

2-[2-(Benzoyloxy)éthyl]-2-(3,4-difluorophényl)morpholine, isomère (-).

A 100 ml d'une solution 1M de borane dans le THF, on ajoute à TA et goutte à goutte une solution de 12 g du composé obtenu à la Préparation 1.15 (isomère (-)) dans 75 ml de THF et laisse 30 minutes sous agitation à TA. Puis on chauffe 3 heures à reflux le mélange réactionnel, ajoute 40 ml d'une solution 1M de borane dans le THF et poursuit le reflux pendant 30 minutes. On ajoute 80 ml de MeOH bouillant et continue le reflux pendant 30 minutes. On refroidit le mélange réactionnel au bain de glace, ajoute 30 ml d'une solution saturée d'HCl gaz dans l'éther et laisse une nuit sous agitation à TA. On concentre sous vide le mélange réactionnel, reprend le résidu par une solution de $Na_2CO_3$ à 10 %, extrait à l'éther, lave la phase organique à l'eau, par une solution saturée de NaCl, sèche sur $MgSO_4$ et évapore sous vide le solvant. On obtient 11,2 g du produit attendu sous forme d'huile.

Préparation 1.18

2-[2-(Benzoyloxy)éthyl]-2-(3,4-difluorophényl)morpholine, isomère (+).

On chauffe à 60°C pendant 2 heures un mélange de 19,9 g du composé obtenu à la Préparation 1.16 (isomére (+)) et 300 ml d'une solution 1M de borane dans le THF. On ajoute 60 ml de MeOH bouillant et poursuit le reflux pendant 30 minutes. On refroidit à 10°C le mélange réactionnel, ajoute 50 ml d'une solution d'HCl gaz dans l'éther et laisse une nuit à TA. On concentre sous vide le mélange réactionnel, reprend le résidu par 300 ml d'une solution de $Na_2CO_3$ à 10 %, ajoute 300 ml d'éther et laisse 30 minutes sous agitation. Après décantation, on sèche la phase organique sur $MgSO_4$ et évapore sous vide le solvant. On obtient 20 g du produit attendu sous forme d'huile.

Préparation 1.19

2-[2-(Benzoyloxy)éthyl]-2-(3,4-dichlorophényl)morpholine.

A 76 ml d'une solution 1M de borane dans le THF, on ajoute à TA et goutte à goutte une solution de 6 g du composé obtenu à la Préparation 1.4 dans 30 ml de THF, puis chauffe à reflux pendant 4 heures. On ajoute goutte à goutte 30 ml de MeOH et poursuit le reflux pendant 30 minutes. On refroidit à 0°C le mélange réactionnel, ajoute 30 ml d'une solution saturée d'HCl gaz dans l'éther et laisse une nuit sous agitation à TA. On concentre sous vide le mélange réactionnel, reprend le résidu par une solution de $Na_2CO_3$ à 10 %, extrait à l'éther, lave la phase organique par une solution de $Na_2CO_3$ à 10 %, à l'eau, sèche sur $MgSO_4$ et évapore sous vide le solvant. On obtient 5 g du produit attendu.

Préparation 1.20

5-[2-(Benzoyloxy)éthyl]-5-(3,4-difluorophényl)oxazolidin-2-one,isomère (-).

On laisse 30 minutes sous agitation à TA un mélange de 4 g du composé obtenu à l'étape A de la Préparation 1.16 (isomère (-)), 2,2 g de 1,1'-carbonyldiimidazole dans 40 ml de DCM, puis chauffe à reflux pendant 1 heure. Après refroidissement à TA, on lave deux fois le mélange réactionnel par une solution d'HCl 1N, sèche la phase organique sur $MgSO_4$ et évapore sous vide le solvant. On obtient 4,18 g de produit attendu après cristallisation dans l'éther iso, F = 147°C.

$$\alpha_D^{20} = -62{,}2° \ (c = 1\ ;\ DMF)$$

Préparation 1.21

2-[2-(Benzoyioxy)éthyl]-2-(3,4-difluorophényl)morpholine.

A 700 ml d'une solution 1 M de borane dans le THF, on ajoute à TA et par portions 48 g du composé obtenu à la Préparation 1.10 puis chauffe à 60°C pendant 2 heures. On ajoute goutte à goutte 150 ml de MeOH et poursuit le chauffage pendant 30 minutes. On refroidit le mélange réactionnel à 10°C, ajoute 120 ml d'éther chlorhydrique et abandonne une nuit à TA. On concentre sous vide le mélange réactionnel, reprend le résidu dans 600 ml d'une solution saturée de $Na_2CO_3$ et 500 ml d'éther et laisse 1 heure sous agitation à TA. Après décantation on lave la phase organique à l'eau, sèche sur $MgSO_4$ et évapore sous vide le solvant. On reprend le résidu dans 400 ml de propan-2-ol, ajoute 15 g d'acide fumarique, laisse 30 minutes sous agitation et essore le précipité formé. On reprend le précipité dans 400 ml d'une solution à 10 % de $Na_2CO_3$, extrait à l'éther, sèche la phase organique sur $MgSO_4$ et évapore sous vide le solvant. On obtient 22 g du produit attendu sous forme d'huile.

Préparation 1.22

5-[2-(Benzoyloxy)éthyl]-5-(3,4-dichlorophényl)oxazolidine.

A une solution de 9 g du composé obtenu à l'étape A de la Préparation 1.7 (sous forme de base libre) dans 100 ml de THF on ajoute 4 g d'une solution à 37 % de formaldéhyde dans l'eau puis chauffe à reflux pendant 30 minutes et laisse une nuit sous agitation à TA. On concentre sous vide, extrait le résidu à l'éther, lave la phase organique à l'eau, sèche sur $Na_2SO_4$ et évapore sous vide le solvant. On obtient 9 g du produit attendu.
Spectre de RMN du proton à 200 MHz dans DMSO-$d_6$ :
$\delta$ : 2,4 ppm : mt : 2 H ; 3,2 ppm : mt : 2H ; 3,8 à 5,0 ppm : m : 4H ; 7,0 à 8,0 ppm : m : 8H.

Préparation 1.23

5-[2-(Benzoyloxy)éthyl]-5-(3,4-difluorophényl)oxazolidin-2-one, isomère (+).

On prépare ce composé selon le mode opératoire décrit à la Préparation 1.20 à partir du composé obtenu à l'étape A de la Préparation 1.15 (isomère (+)).

$$\alpha_D^{20} = +61° \ (c = 1\ ;\ DMF).$$

Préparation 2.1

4-Phényl-4-(pyrrolidin-1-ylcarbonyl)pipéridine, hémihydrate.

A) 1-*tert*-Butoxycarbonyl-4-carboxy-4-phénylpipéridine.

A un mélange de 30 g de *p*-toluènesulfonate de 4-carboxy-4-phénylpipéridine dans 300 ml de dioxane, on ajoute 30 ml d'eau, 32,9 g de $K_2CO_3$, puis chauffe à 60°C et ajoute goutte à goutte 18,2 g de di-*tert*-butyldicarbonate. On chauffe ensuite 2 heures à 60°C puis 30 minutes à reflux. Après refroidissement à TA, on concentre sous vide le mélange réactionnel, extrait le résidu au DCM, lave la phase organique par une solution tampon pH = 2, acidifie à pH

= 4 par ajout d'HCl 2N, lave par une solution tampon pH = 2, à l'eau, par une solution saturée de NaCl, sèche sur $MgSO_4$ et évapore sous vide le solvant. On obtient 23,7 g du produit attendu.

B) 1-*tert*-Butoxycarbonyl-4-(pyrrolidin-1-ylcarbonyl)-4-phénylpipéridine.

A une solution de 14 g du composé obtenu à l'étape précédente dans 200 ml de DCM, on ajoute 9,29 g de triéthylamine puis 3,27 g de pyrrolidine. On refroidit au bain de glace, ajoute 22,4 g de BOP et laisse sous agitation en laissant remonter la température à TA. On concentre sous vide le mélange réactionnel, extrait le résidu au DCM, lave la phase organique à l'eau, trois fois par une solution de NaOH à 10 %, à l'eau, trois fois par une solution saturée de NaCl, sèche sur $MgSO_4$ et évapore sous vide le solvant. On obtient 16,4 g du produit attendu.

C) 4-Phényl-4-(pyrrolidin-1-ylcarbonyl)pipéridine, hémihydrate.

A une solution de 16,4 g du composé obtenu à l'étape précédente dans 200 ml de MeOH, on ajoute jusqu'à pH = 1 une solution d'HCl concentrée et laisse 5 heures sous agitation à TA. On concentre sous vide le mélange réactionnel, reprend le résidu à l'acétone et évapore sous vide le solvant. On obtient un solide blanc que l'on recristallise dans le propan-2-ol. On reprend le produit obtenu par une solution de NaOH à 10 %, extrait au DCM, lave la phase organique par une solution de NaOH à 10 %, par une solution saturée de NaCl, sèche sur $MgSO_4$ et évapore sous vide le solvant. On obtient 7 g du produit attendu après cristallisation dans l'éther, F = 126°C.

Préparation 2.2

*p*-Toluènesulfonate de 4-(N',N'-diméthyluréido)-4-phénylpipéridine, hémihydrate

A) 4-Acétamido-1-benzyl-4-phénylpipéridine.

Ce composé est préparé par action de l'acétonitrile sur le 1-benzyl-4-hydroxy-4-phénylpipéridine selon le procédé décrit dans EP-A-474561.

B) Dichlorhydrate de 4-amino-1-benzyl-4-phénylpipéridine.

On chauffe à reflux pendant 48 heures un mélange de 30 g du composé obtenu à l'étape précédente, 58 ml d'une solution d'HCl concentrée dans 135 ml d'eau. On concentre sous vide le mélange réactionnel, reprend le résidu dans un mélange EtOH/toluène et évapore sous vide les solvants. On dissout le résidu dans 50 ml de MeOH et cristallise par addition de 250 ml d'acétone. On obtient 30,5 g du produit attendu après essorage et séchage.

C) 1-Benzyl-4-(N',N'-diméthyluréido)-4-phénylpipéridine.

A une solution de 6 g du composé obtenu à l'étape précédente, 7,14 g de triéthylamine dans 50 ml de 1,2-dichloroéthane, on ajoute à TA, goutte à goutte, une solution de 1,9 g de chlorure de N,N-diméthylcarbamoyle dans 10 ml de 1,2-dichloroéthane et chauffe à reflux pendant 8 heures. On rajoute quelques gouttes de chlorure de N,N-diméthylcarbamoyle et poursuit le reflux pendant 3 heures. On concentre sous vide le mélange réactionnel, extrait le résidu au DCM, lave la phase organique à l'eau, par une solution de NaOH à 10 %, à l'eau, par une solution saturée de NaCl, sèche sur $MgSO_4$ et évapore sous vide le solvant. On chromatographie le résidu sur silice en éluant par le gradient du mélange DCM/MeOH de (99/1 ; v/v) à (96/4 ; v/v). On obtient 1,8 g du produit attendu.

D) *p*-Toluènesulfonate de 4-(N',N'-diméthyluréido)-4-phénylpipéridine, hémihydrate.

On hydrogène à 40°C et à pression atmosphérique un mélange de 1,8 g du composé obtenu à l'étape précédente, 1,11 g d'acide *p*-toluènesulfonique monohydrate, 0,2 g de palladium sur charbon à 10 % dans 150 ml d'EtOH 95. On filtre le catalyseur sur Célite® et évapore sous vide le filtrat. On reprend le résidu à l'acétone et évapore sous vide le solvant. On dissout le produit obtenu dans 25 ml d'acétone, ajoute lentement cette solution dans 200 ml d'éther et essore le produit cristallisé formé. On obtient 1,86 g du produit attendu, F = 120-122°C.

Préparation 2.3

*p*-Toluènesulfonate de 4-(acétyl-N-méthylamino)-4-phénylpipéridine.

A) 1-Benzyl-4-(formylamino)-4-phénylpipéridine.

A une solution de 48,9 g du composé obtenu à l'étape B de la Préparation 2.2, 25 g de formiate de sodium dans 340 ml d'acide formique, on ajoute goutte à goutte 110 ml d'anhydride acétique puis laisse une nuit sous agitation à TA. On concentre sous vide le mélange réactionnel, reprend le résidu à l'eau, alcalinise par ajout d'une solution de NaOH concentrée, extrait au DCM, sèche la phase organique sur $MgSO_4$ et évapore sous vide le solvant. On obtient 38,8 g du produit attendu après cristallisation dans le mélange éther iso/pentane, F = 140°C.

B) 1-Benzyl-4-(méthylamino)-4-phénylpipéridine.

A une suspension de 12,5 g d'hydrure d'aluminium et de lithium dans 100 ml de THF, on ajoute lentement une solution de 38,8 g du composé obtenu à l'étape précédente dans 400 ml de THF et chauffe à reflux pendant 3 heures. Après refroidissement, on ajoute au mélange réactionnel une solution de 5 ml de NaOH concentrée dans 45 ml d'eau, filtre les sels minéraux et concentre sous vide le filtrat. On obtient 38 g du produit attendu.

C) 4-(Acétyl-N-méthylamino)-1-benzyl-4-phénylpipéridine.

On refroidit à 0-5°C une solution de 30 g du composé obtenu à l'étape précédente, 16,5 ml de triéthylamine dans 300 ml de DCM, ajoute goutte à goutte 8 ml de chlorure d'acétyle et laisse 30 minutes sous agitation à TA. On lave deux fois le mélange réactionnel à l'eau, par une solution de NaOH 2N, sèche la phase organique sur $MgSO_4$ et évapore sous vide le solvant. On obtient 31,6 g du produit attendu après cristallisation dans le mélange éther iso/pentane, F = 104°C.

D) *p*-Toluènesulfonate de 4-(acétyl-N-méthylamino)-4-phénylpipéridine.

On hydrogène pendant 3 heures à 25°C et à pression atmosphérique un mélange de 5 g du composé obtenu à l'étape précédente, 2,9 g d'acide *p*-toluènesulfonique monohydrate, 0,5 g de palladium sur charbon à 10 % et 80 ml d'EtOH. On filtre le catalyseur et concentre sous vide le filtrat. On obtient 5,7 g du produit attendu après cristallisation dans l'acétone, F = 165°C.

Préparation 2.4

Trifluoroacétate de 4-(éthoxycarbonylamino)-4-phénylpipéridine.

A) 1-*tert*-Butoxycarbonyl-4-isocyanato-4-phénylpipéridine.

On refroidit à 0-5°C une solution de 25 g du composé obtenu à l'étape A de la Préparation 2.1, 10,35 g de triéthylamine dans 100 ml d'acétone, ajoute goutte à goutte à une température inférieure à 5°C une solution de 8,7 g de chloroformiate de méthyle dans 30 ml d'acétone et laisse 30 minutes sous agitation à 5°C. Puis on ajoute à une température inférieure à 5°C et goutte à goutte une solution de 10,66 g d'azidure de sodium dans 30 ml d'eau et laisse 30 minutes sous agitation à 5°C. On verse le mélange réactionnel sur 500 ml d'eau glacée, extrait quatre fois au toluène, lave deux fois la phase organique par une solution tampon pH = 2, par une solution saturée de NaCl, sèche sur $MgSO_4$ et filtre. On chauffe à 90°C le filtrat pendant 1 heure et évapore sous vide le solvant. On obtient 18,9 g du produit attendu sous forme d'huile.

B) 1-*tert*-Butoxycarbonyl-4-(éthoxycarbonylamino)-4-phénylpipéridine.

On chauffe à reflux pendant 5 heures 30 minutes une solution de 6,28 g du composé obtenu à l'étape précédente dans 100 ml d'EtOH. On ajoute deux gouttes de triéthylamine puis laisse une nuit sous agitation à TA et concentre sous vide. On obtient 7,25 g du produit attendu.

C) Trifluoroacétate de 4-(éthoxycarbonylamino)-4-phénylpipéridine.

On laisse 30 minutes sous agitation à TA une solution de 7,25 g du composé obtenu à l'étape précédente dans 20 ml de TFA puis concentre sous vide. On reprend le résidu dans l'acétone et évapore sous vide le solvant. On obtient 6,02 g du produit attendu après cristallisation dans le mélange acétone/éther, F = 173°C.

Préparation 2.5

4-Benzylquinuclidine.

A) 1,4-Dibenzyl-4-cyanopipéridine.

On refroidit à -50°C une solution de 15 g de 4-cyanopipéridine dans 250 ml de THF, ajoute goutte à goutte 190 ml d'une solution 1,5M de diisopropylamidure de lithium dans le cyclohexane et laisse 30 minutes sous agitation à -50°C. Puis on ajoute 34 ml de bromure de benzyle et laisse 3 heures sous agitation après avoir laissé remonter la température à TA. On verse le mélange réactionnel sur un mélange glace/HCl concentré, ajoute de l'éther, essore le précipité formé et le lave à l'eau. On reprend le précipité dans l'eau, alcalinise à pH = 12 par addition d'une solution de NaOH concentrée, extrait à l'éther, sèche la phase organique sur $MgSO_4$ et évapore le solvant. On obtient 31,7 g du produit attendu après cristallisation dans le pentane, F = 92°C.

B) Chlorhydrate de 4-acétyl-1,4-dibenzylpipéridine.

A une solution de 20 g du composé obtenu à l'étape précédente dans 400 ml d'éther, on ajoute 55 ml d'une solution 1,6M de méthyllithium dans l'éther et laisse 3 heures sous agitation à TA. On verse le mélange réactionnel sur de l'eau glacée, après décantation sèche la phase organique sur $MgSO_4$ et évapore sous vide le solvant. On reprend le résidu dans 400 ml d'eau et 40 ml d'HCl concentré et chauffe à reflux pendant 2 heures. Après une nuit à TA, on essore les cristaux formés, lave à l'acétone puis à l'éther et sèche. On obtient 17,6 g du produit attendu, F = 246°C.

C) Bromhydrate de 1,4-dibenzyl-4-(2-bromoacétyl)pipéridine.

A une solution de 10 g du composé obtenu à l'étape précédente dans 40 ml d'acide acétique, on ajoute 1,6 ml de brome et laisse une nuit sous agitation à TA. On ajoute 50 ml d'éther au mélange réactionnel, essore les cristaux formés, lave par le mélange acétone/éther puis à l'éther. On obtient 12,5 g du produit attendu, F = 205°C.

D) Bromure de 1,4-dibenzyl-3-oxo-quinuclidinium.

A une suspension de 12,5 g du composé obtenu à l'étape précédente dans l'eau, on ajoute jusqu'à pH = 12 une solution de NaOH concentrée, extrait à l'éther, sèche la phase organique sur $MgSO_4$ et évapore sous vide le solvant. On reprend le résidu dans l'acétone et laisse 2 heures sous agitation à TA. On essore le précipité, lave à l'éther et sèche. On obtient 10,08 g du produit attendu, F = 234°C.

E) 4-Benzyl-3-oxo-quinuclidine.

On hydrogène à TA et à pression atmosphérique un mélange de 10 g du composé obtenu à l'étape précédente, 1 g de palladium sur charbon à 10 %, dans 200 ml de MeOH. On filtre le catalyseur et évapore sous vide le filtrat. On reprend le résidu à l'éther et essore le précipité formé. On dissout le précipité dans l'eau, alcalinise à pH = 12 par ajout d'une solution de NaOH concentrée, essore le précipité formé, lave à l'eau et sèche. On obtient 5 g du produit attendu, F = 111°C.

F) 4-Benzylquinuclidine.

On chauffe à 175°C pendant 2 heures un mélange de 5 g du composé obtenu à l'étape précédente, 2,5 g d'hydrate d'hydrazine, 4,3 g de KOH dans 25 ml d'éthylène glycol. On verse le mélange réactionnel sur de l'eau glacée, extrait deux fois à l'éther, lave la phase organique à l'eau, sèche sur $MgSO_4$ et évapore sous vide le solvant. On dissout le résidu dans l'acétone, acidifie à pH = 1 par ajout d'une solution saturée d'acide chlorhydrique dans l'éther, essore le précipité formé, et lave par un mélange acétone/éther (50/50 ;v/v) puis à l'éther. On dissout le précipité dans l'eau, alcalinise à pH = 12 par ajout d'une solution de NaOH concentrée, extrait à l'éther, sèche sur MgS04 et évapore sous vide le solvant. On obtient 1,8 g du produit attendu, F = 48°C.

Préparation 2.6

Benzènesulfonate de 4-phényl-4-(pyrrolidin-1-ylcarbonylamino)pipéridine.

A) 1-(Benzyloxycarbonyl)-4-carboxy-4-phénylpipéridine.

On refroidit à 5°C un mélange de 37,7 g de *p*-toluènesulfonate de 4-carboxy-4-phénylpipéridine, 53,3 g d'une solution aqueuse à 30 % de NaOH et 250 ml d'eau. On ajoute rapidement à 5°C une solution de 18 g de chloroformiate de benzyle dans 60 ml d'acétone et laisse une nuit sous agitation en laissant remonter la température à TA. On lave deux fois le mélange réactionnel à l'éther et acidifie, après décantation, la phase aqueuse à pH = 1 par ajout d'HCl concentré puis d'HCl 2N. On essore le précipité formé, le sèche, le reprend à l'éther et l'essore à nouveau. On obtient 30,6 g du produit attendu, F = 142-144°C.

B) 1-(Benzyloxycarbonyl)-4-phényl-4-(pyrrolidin-1-ylcarbonylamino)pipéridine.

On chauffe à reflux pendant 1 heure un mélange de 33,9 g du composé obtenu à l'étape précédente et 47,6 g de chlorure de thionyle dans 200 ml de 1,2-dichloroéthane. On concentre sous vide le mélange réactionnel, reprend le résidu à l'acétone et évapore sous vide le solvant. On dissout le résidu dans 200 ml d'acétone, refroidit à 5°C, ajoute goutte à goutte une solution de 13 g d'azidure de sodium dans 50 ml d'eau et laisse 1 heure sous agitation. On évapore sous vide à TA 100 ml d'acétone, ajoute à la solution restante une solution saturée de $NaHCO_3$, extrait au toluène, lave la phase organique à l'eau, par une solution saturée de NaCl, sèche sur $Na_2SO_4$ et évapore 50 % en volume de solvant. On chauffe à reflux pendant 30 minutes la solution toluénique restante puis concentre sous vide. On reprend le résidu dans 200 ml d'éther, ajoute goutte à goutte une solution de 7,1 g de pyrrolidine dans 20 ml d'éther et laisse 5 minutes sous agitation. On concentre sous vide, extrait le résidu au DCM, lave la phase organique par une solution d'HCl 2N, par une solution à 5 % de $NaHCO_3$, par une solution saturée de NaCl, sèche sur $Na_2SO_4$ et évapore sous vide le solvant. On dissout le résidu dans 40 ml d'AcOEt chaud, ajoute 200 ml d'éther et essore le produit cristallisé formé. On obtient 31,4 g du produit attendu.

C) Benzènesulfonate de 4-phényl-4-(pyrrolidin-1-ylcarbonylamino)pipéridine.

On hydrogène à 40°C et à pression atmosphérique un mélange de 29 g du composé obtenu à l'étape précédente, 11,27 g d'acide benzènesulfonique, 2 g de palladium sur charbon à 10 % et 250 ml d'EtOH. On filtre le catalyseur sur Célite® et concentre sous vide le filtrat. On cristallise le résidu dans un mélange EtOH/acétone. On obtient 29,4 g du produit attendu, F = 185°C.

Préparation 2.7

*p*-Toluènesulfonate de 4-[2-(diméthylamino)thiazol-4-yl]-4-phénylpipéridine.

A) 1,1-Diméthylthiourée.

On refroidit au bain de glace une solution de 10,67 g de thiocyanate de potassium dans 100 ml d'acétone, ajoute goutte à goutte une solution de 12,06 g de chlorure de pivaloyle dans 30 ml d'acétone et laisse 30 minutes sous agitation en laissant remonter la température à TA. On refroidit à -10°C le mélange réactionnel, ajoute goutte à goutte 17,9 ml d'une solution 5,6N de diméthylamine dans le MeOH et laisse sous agitation en laissant remonter la température à TA. On concentre sous vide, extrait le résidu à l'éther, lave deux fois la phase organique à l'eau, par une solution saturée de NaCl, sèche sur $MgSO_4$ et évapore sous vide le solvant. On reprend le produit obtenu dans 50 ml d'une solution d'HCl concentrée et chauffe à reflux pendant 1 heure. Après refroidissement à TA, on lave deux fois le mélange réactionnel à l'éther, alcalinise la phase aqueuse à pH = 9 par ajout d'une solution à 30 % de NaOH, extrait au DCM, lave la phase organique par une solution à 5 % de NaOH, par une solution saturée de NaCl, sèche sur $MgSO_4$ et évapore sous vide le solvant. On reprend le résidu dans l'éther et essore le précipité formé, on obtient 6,7 g du produit attendu.

B) Bromhydrate de 4-(2-bromoacétyl)-4-phénylpipéridine.

A une suspension de 14 g de bromhydrate de 4-acétyl-4-phénylpipéridine dans 200 ml de DCM on ajoute rapidement à TA 7,9 g de brome et laisse une nuit sous agitation à TA. On dilue le mélange réactionnel par ajout de 200 ml d'éther, essore le précipité formé et le lave à l'éther. On obtient 16,7 g du produit attendu après séchage sous vide.

C) *p*-Toluènesulfonate de 4-[2-(diméthylamino)thiazol-4-yl]-4-phénylpipéridine.

On chauffe à reflux pendant 1 heure 30 minutes un mélange de 7,26 g du composé obtenu à l'étape B), 2,08 g du composé obtenu à l'étape A) dans 150 ml d'EtOH. Après refroidissement à TA, on concentre sous vide, reprend

le résidu à l'eau, alcalinise à pH = 10 par ajout d'une solution à 10 % de NaOH, extrait au DCM, lave la phase organique par une solution à 10 % de NaOH, par une solution saturée de NaCl, sèche sur $MgSO_4$ et évapore sous vide le solvant. On dissout le produit obtenu dans l'acétone, ajoute goutte à goutte une solution de 3,31 g d'acide *p*- toluènesulfonique monohydrate dans 10 ml d'acétone et essore le produit cristallisé formé. On obtient 6,1 g du produit attendu, F = 164°C.

Préparation 2.8

*p*-Toluènesulfonate de 4-(morpholin-4-ylcarbonylamino)-4-phénylpipéridine, monohydrate.

A) 1-*tert*-Butoxycarbonyl-4-(morpholin-4-ylcarbonylamino)-4-phénylpipéridine.

A une solution de 6 g du composé obtenu à l'étape A de la Préparation 2.4 dans 100 ml d'acétone, on ajoute à TA et goutte à goutte une solution de 1,74 g de morpholine dans 10 ml d'acétone. On concentre sous vide le mélange réactionnel, extrait le résidu à l'AcOEt, lave la phase organique par une solution tampon pH = 2, par une solution saturée de NaCl, sèche sur $MgSO_4$ et évapore sous vide le solvant. On obtient 7,1 g du produit attendu.

B)*p*-Toluènesulfonate de 4-(morpholin-4-ylcarbonylamino)-4-phénylpipéridine, monohydrate.

A une solution de 7,1 g du composé obtenu à l'étape précédente dans 100 ml de MeOH, on ajoute 15 ml d'une solution d'HCl concentrée et laisse une nuit sous agitation à TA. On concentre sous vide le mélange réactionnel, reprend le résidu à l'eau, alcalinise à pH = 10 par ajout d'une solution de NaOH concentrée, extrait trois fois au DCM, lave les phases organiques jointes par une solution saturée de NaCl, sèche sur $MgSO_4$ et évapore sous vide le solvant. On dissout le produit obtenu dans l'acétone, ajoute goutte à goutte une solution de 3,46 g d'acide *p*-toluènesulfonique monohydrate dans 10 ml d'acétone, et concentre sous vide. On reprend le résidu dans l'éther et essore le précipité formé. On obtient 7 g du produit attendu, F = 93°C.

Préparation 2.9

Benzènesulfonate de 4-phényl-4-(pyrrolidin-1-ylaminocarbonyl)pipéridine.

A) Benzènesulfonate de 1-(benzyloxycarbonyl)-4-phényl-4-(pyrrolidin-1-ylaminocarbonyl)pipéridine.

On chauffe à reflux un mélange de 11,54 g du composé obtenu à l'étape A de la Préparation 2.6 dans 100 ml de 1,2-dichloroéthane, puis ajoute 16,18 g de chlorure de thionyle, chauffe à reflux pendant 1 heure et laisse une nuit sous agitation à TA. On concentre sous vide le mélange réactionnel, dissout le résidu dans 100 ml de DCM, refroidit à 5°C et ajoute successivement 10,3 g de triéthylamine puis 5 g de chlorhydrate de 1-aminopyrrolidine. Après 1 heure sous agitation, on concentre sous vide le mélange réactionnel, extrait le résidu à l'AcOEt, lave la phase organique à l'eau, par une solution saturée de NaCl, sèche sur $MgSO_4$ et évapore sous vide le solvant. On dissout le produit obtenu dans l'acétone, ajoute de l'éther jusqu'à précipitation et essore le précipité formé. On dissout le précipité dans l'EtOH, ajoute 2,61 g d'acide benzènesulfonique et essore le précipité formé. On obtient 9,34 g du produit attendu.

B) Benzènesulfonate de 4-phényl-4-(pyrrolidin-1-ylaminocarbonyl)pipéridine.

On hydrogène pendant 3 heures à 40°C et à pression atmosphérique un mélange de 9,34 g du composé obtenu à l'étape précédente, 1 g de palladium sur charbon à 5 % et 200 ml d'EtOH. On filtre le catalyseur et concentre sous vide le filtrat. On obtient 6,13 g du produit attendu après cristallisation dans le mélange EtOH/acétone.

Préparation 2.10

*p*-Toluènesulfonate de 4-benzyl-4-(pyrrolidin-1-ylcarbonylamino)pipéridine.

A) 4-Cyanopipéridine.

On ajoute par petites portions 25 g d'isonipécotamide (ou pipéridine-4-carboxamide) à 70 ml de $POCl_3$ et chauffe à reflux pendant 4 heures. On concentre sous vide le mélange réactionnel, reprend le résidu dans de la glace, alcalinise à pH = 13 par ajout d'une solution de NaOH concentrée, extrait au DCM puis 4 fois à l'éther, sèche les phases organiques jointes sur $MgSO_4$ et évapore sous vide les solvants. On distille sous pression réduite l'huile obtenue. On obtient 6,4 g du produit attendu, Eb = 108-110°C sous 2400 Pa.

B) 4-Cyano-1,4-dibenzylpipéridine.

On refroidit à -50°C une solution de 15 g du composé obtenu à l'étape précédente dans 250 ml de THF, ajoute goutte à goutte 190 ml d'une solution 1,5M de diisopropylamidure de lithium dans le cyclohexane et laisse 30 minutes sous agitation à -50°C. Puis on ajoute 34 ml de bromure de benzyle et laisse sous agitation en laissant remonter la température à TA. Après 3 heures à TA, on verse le mélange réactionnel sur un mélange de glace et d'HCl concentré, ajoute de l'éther, essore le précipité formé et le lave à l'eau. On reprend le précipité dans de l'eau, alcalinise à pH = 13 par ajout d'une solution de NaOH concentrée, extrait à l'éther, sèche la phase organique sur $MgSO_4$ et évapore sous vide le solvant. On obtient 31,7 g du produit attendu après cristallisation dans le pentane, F = 92°C.

C) 1,4-Dibenzyl-4-carboxypipéridine.

A une solution de 25 ml d'eau, 25 ml d'$H_2SO_4$ concentré et 25 ml d'AcOH, on ajoute 6 g du composé obtenu à l'étape précédente et chauffe à 140°C pendant 5 heures. Après refroidissement, on verse le mélange réactionnel sur de la glace, on amène à pH = 6,5 par ajout d'une solution de NaOH concentrée et laisse sous agitation jusqu'à cristallisation. On essore le produit cristallisé et le lave à l'eau. On reprend le produit dans du MeOH, l'essore et le lave à l'éther. On obtient 3 g du produit attendu, F = 262°C.

D) 1,4-Dibenzyl-4-isocyanatopipéridine.

On chauffe à 60°C pendant 1 heure un mélange de 2 g du composé obtenu à l'étape précédente et 1,6 g de pentachlorure de phosphore dans 40 ml de chloroforme. On concentre sous vide le mélange réactionnel, reprend le résidu dans 40 ml d'acétone, ajoute une solution de 2 g d'azidure de sodium dans 5 ml d'eau et laisse 30 minutes sous agitation à TA. On concentre sous vide à TA, reprend le résidu à l'éther, lave la phase organique par une solution saturée de $Na_2CO_3$, à l'eau, sèche sur $MgSO_4$ et évapore sous vide le solvant. On reprend le résidu dans 40 ml de toluène et chauffe à reflux pendant 1 heure. On concentre sous vide et obtient 2 g du produit attendu sous forme d'huile.

E) 1,4-Dibenzyl-4-(pyrrolidin-1-ylcarbonylamino)pipéridine.

A une solution de 5 g du composé obtenu à l'étape précédente dans 50 ml de DCM, on ajoute à TA 1,5 ml de pyrrolidine et laisse 2 heures sous agitation à TA. On concentre sous vide le mélange réactionnel, reprend le résidu au pentane, essore le précipité formé et le lave au pentane. On obtient 4,5 g du produit attendu après séchage, F = 126°C.

F) *p*-Toluènesulfonate de 4-benzyl-4-(pyrrolidin-1-ylcarbonylamino)pipéridine.

On hydrogène pendant 48 heures à TA et à pression atmosphérique un mélange de 4,2 g du composé obtenu à l'étape précédente, 2,1 g d'acide *p*-toluènesulfonique monohydrate, 0,4 g de palladium sur charbon à 10 % et 50 ml d'EtOH. On filtre le catalyseur sur Célite® et concentre sous vide le filtrat. On reprend le résidu dans l'éther et essore le précipité formé. On obtient 4,99 g du produit attendu, F > 180°C.

Préparation 2.11

*p*-Toluènesulfonate de 4-(méthoxycarbonylamino)-4-phénylpipéridine, hémihydrate.

On chauffe à reflux pendant 5 heures une solution de 6,05 g du composé obtenu à l'étape A de la Préparation 2.4 dans 100 ml de MeOH. On ajoute 1 goutte de triéthylamine et laisse une nuit sous agitation à TA. Puis on ajoute jusqu'à pH = 1 une solution d'HCl concentrée et concentre sous vide le mélange réactionnel. On reprend le résidu par une solution de NaOH à 10 %, extrait au DCM, lave la phase organique par une solution de NaOH à 10 %, par une solution saturée de NaCl, sèche sur $MgSO_4$ et évapore sous vide le solvant. On dissout le produit obtenu dans l'acétone, ajoute goutte à goutte une solution de 3,57 g d'acide *p*-toluènesulfonique monohydrate dans 10 ml d'acétone et concentre sous vide. On reprend le produit obtenu dans l'éther et évapore sous vide le solvant. On obtient 7,17 g du produit attendu, F = 159°C.

Préparation 2.12

*p*-Toluènesulfonate de 4-(2-amino-1,3,4-oxadiazol-5-yl)-4-phénylpipéridine, hémihydrate.

A) 1-(Benzyloxycarbonyl)-4-(chloroformyl)-4-phénylpipéridine.

On chauffe à reflux pendant 1 heure un mélange de 17,1 g du composé obtenu à l'étape A de la Préparation 2.6, 24 g de chlorure de thionyle dans 150 ml de 1,2-dichloroéthane. On concentre sous vide, reprend le résidu au

chloroforme et évapore sous vide le solvant. On reprend le résidu dans un mélange éther/pentane et évapore à nouveau sous vide les solvants. On obtient 20 g du produit attendu sous forme de gomme que l'on utilise tel quel.

B) 1-(Benzyloxycarbonyl)-4-carbazoyl-4-phénylpipéridine.

On refroidit à -50°C une solution de 16 g d'hydrazine monohydrate dans 40 ml d'EtOH, ajoute goutte à goutte une solution de 11,44 g du composé obtenu à l'étape précédente dans 20 ml de 1,2-diméthoxyéthane et laisse sous agitation en laissant remonter la température à TA. On concentre sous vide, reprend le résidu à l'eau, extrait au DCM, lave la phase organique à l'eau, par une solution saturée de NaCl, sèche sur $MgSO_4$ et évapore sous vide le solvant. On reprend le résidu par un mélange EtOH/benzène et évapore sous vide les solvants. On obtient 11,2 g du produit attendu sous forme de gomme que l'on utilise tel quel.

C) 4-(2-Amino-1,3,4-oxadiazol-5-yl)-1-(benzyloxycarbonyl)-4-phénylpipéridine.

A une solution de 11,2 g du composé obtenu à l'étape précédente dans 60 ml d'EtOH, on ajoute à TA une solution de 3,39 g de bromure de cyanogène dans 10 ml d'EtOH et chauffe à reflux pendant 1 heure. On concentre le mélange réactionnel jusqu'à 50 ml d'EtOH, puis ajoute goutte à goutte de l'eau jusqu'à obtenir un volume de 400 ml de mélange réactionnel. On essore le produit cristallisé formé, le lave à l'eau puis au DCM, à l'AcOEt, et à l'éther. On obtient 8 g du produit attendu.

D)p-Toluènesulfonate de 4-(2-amino-1,3,4-oxadiazol-5-yl)-4-phénylpipéridine hémihydrate.

On hydrogène à 50°C et à pression atmosphérique un mélange de 7,85 g du composé obtenu à l'étape précédente, 3,95 g d'acide *p*-toluènesulfonique monohydrate, 0,8 g de palladium sur charbon à 10 %, 350 ml d'EtOH 95 et 10 ml d'eau. Après 3 heures, on filtre le catalyseur sur Célite® et concentre sous vide le filtrat. On reprend le résidu à l'acétone, essore le produit cristallisé formé et le lave à l'acétone puis à l'éther. On obtient 7,65 g du produit attendu, F = 183-185°C.

Préparation 2.13

*p*-Toluènesulfonate de 4-[(acétyl-N-méthylamîno)méthyl]-4-phénylpipéridine.

A) 4-(aminométhyl)-1-benzyl-4-phénylpipéridine.

On refroidit à 0°C une suspension de 2,8 g d'hydrure d'aluminium et de lithium dans 50 ml de THF et ajoute, goutte à goutte, une solution de 20 g de 1-benzyl-4-cyano-4-phénylpipéridine dans 50 ml de THF. On laisse 1 heure sous agitation à TA puis chauffe à 40°C pendant 1 heure. On refroidit au bain de glace le mélange réactionnel, ajoute successivement 3 ml d'eau, 3 ml d'une solution de NaOH 4N et 12 ml d'eau. On filtre les sels minéraux et évapore sous vide le filtrat. On chromatographie le résidu sur silice H en éluant par le gradient du mélange DCM/MeOH de (100/3 ; v/v) à (100/10 ; v/v). On obtient 11 g du produit attendu.

B) 1-Benzyl-4-[(N-formylamino)méthyl]-4-phénylpipéridine.

A un mélange de 11 g du composé obtenu à l'étape précédente dans 76 ml d'acide formique on ajoute, à TA et goutte à goutte, 25 ml d'anhydride acétique puis on laisse 5 heures sous agitation. On concentre sous vide le mélange réactionnel, reprend le résidu à l'eau, alcalinise à pH = 14 par ajout de NaOH concentré, extrait à l'éther, lave à l'eau, sèche sur $Na_2SO_4$ et évapore sous vide le solvant. On obtient 12 g du produit attendu.

C) 1-Benzyl-4-[(N-méthylamino)méthyl]-4-phénylpipéridine.

On chauffe à 40°C une suspension de 3,9 g d'hydrure d'aluminium et de lithium dans 50 ml de THF, ajoute, goutte à goutte, une solution de 12 g du composé obtenu à l'étape précédente dans 50 ml de THF puis chauffe à reflux pendant 3 heures. Après refroidissement au bain de glace on ajoute successivement 4 ml d'eau, 4 ml d'une solution de NaOH 4N et 12 ml d'eau. On filtre les sels minéraux et concentre sous vide le filtrat. On extrait le résidu à l'éther, sèche sur $Na_2SO_4$ et évapore sous vide le solvant. On obtient 10 g du produit attendu.

D) 4-[(Acétyl-N-méthylamino)méthyl]-1-benzyl-4-phénylpipéridine.

A une solution de 3,3 g du composé obtenu à l'étape précédente, 1,4 g de triéthylamine dans 50 ml de DCM on ajoute 0,863 g de chlorure d'acétyle et laisse 2 heures sous agitation à TA. On concentre sous vide le mélange

réactionnel, reprend le résidu à l'eau, extrait à l'éther, lave à l'eau, sèche sur $Na_2SO_4$ et évapore le solvant. On chromatographie le résidu sur silice H en éluant par le mélange DCM/MeOH (100/3; v/v). On obtient 2,4 g du produit attendu.

E) *p*-Toluènesulfonate de 4-[(acétyl-N-méthylamino)méthyl]-4-phénylpipéridine.

On hydrogène, à TA et à pression atmosphérique, un mélange de 2,3 g du composé obtenu à l'étape précédente, 1,2 g d'acide *p*-toluènesulfonique monohydrate, 0,23 g du palladium sur charbon à 10 % et 100 ml de MeOH. On filtre le catalyseur et évapore sous vide le filtrat. On obtient 2,7 g du produit attendu après trituration dans l'éther et essorage.

Préparation 2.14

*p*-Toluènesulfonate de 4-[(éthoxycarbonyl-N-méthylamino)méthyl]-4-phénylpipéridine.

A) 1-Benzyl-4-[(éthoxycarbonyl-N-méthylamino)méthyl]-4-phénylpipéridine.

A une solution de 2,3 g du composé obtenu à l'étape C de la Préparation 2.13 et 1,03 g de triéthylamine dans 50 ml de DCM, on ajoute à TA et goutte à goutte une solution de 0,85 g de chloroformiate d'éthyle dans 10 ml de DCM et laisse 30 minutes sous agitation à TA. On concentre sous vide le mélange réactionnel, reprend le résidu à l'eau, extrait à l'éther, lave la phase organique à l'eau, par une solution saturée de NaCl, sèche sur $Na_2SO_4$ et évapore sous vide le solvant. On chromatographie le résidu sur silice H en éluant par le mélange DCM/MeOH (100/3 ; v/v). On obtient 2,3 g du produit attendu.

B)p-Toluènesulfonate de 4-[(éthoxycarbonyl-N-méthylamino)méthyl]-4-phénylpipéridine.

On hydrogène à 40°C et à pression atmosphérique, un mélange de 2,3 g du composé obtenu à l'étape précédente, 1,19 g d'acide *p*-toluènesulfonique monohydrate, 0,7 g de palladium sur charbon à 5 % et 50 ml de DCM. On filtre le catalyseur sur Célite® et concentre sous vide le filtrat. On obtient 2,7 g du produit attendu.

Préparation 2.15

*p*-Toluènesulfonate de 4-[N',N'-diméthyl-N-méthyluréido]méthyl]-4-phénylpipéridine.

A) 1-Benzyl-4-[(N',N'-diméthyl-N-méthyluréido)méthyl]-4-phénylpipéridine.

A une solution de 2,5 g du composé obtenu à l'étape C de la Préparation 2.13 et 1,11 g de triéthylamine dans 50 ml de DCM, on ajoute à TA et goutte à goutte une solution de 0,92 g de chlorure de N,N-diméthylcarbamoyle dans 20 ml de DCM puis chauffe à reflux pendant 3 heures. On concentre sous vide le mélange réactionnel, reprend le résidu à l'eau, extrait à l'éther, lave la phase organique à l'eau, par une solution saturée de NaCl, sèche sur $Na_2SO_4$ et évapore sous vide le solvant. On chromatographie le résidu sur silice H en éluant par le mélange DCM/MeOH (100/3 ; v/v). On obtient 2,92 g du produit attendu.

B)*p*-Toluènesulfonate de 4-[(N',N'-diméthyl-N-méthyluréido)méthyl]-4-phénylpipéridine.

On hydrogène à 40°C et à pression atmosphérique, un mélange de 2,92 g du composé obtenu à l'étape précédente, 1,52 g d'acide *p*-toluènesulfonique monohydrate, 0,3 g de palladium sur charbon à 10 % et 50 ml de MeOH. On filtre le catalyseur sur Célite® et concentre sous vide le filtrat. On obtient 2,6 g du produit attendu après trituration du résidu dans le mélange pentane/éther iso puis essorage.

Préparation 2.16

Dichlorhydrate de 4-carbamoyl-4-(pipérid-1-yl)pipéridine.

A) 1-Benzyl-4-cyano-4-(pipérid-1-yl)pipéridine.

A une solution de 18,9 g de 1-benzylpipérid-4-one, 12,16 g de chlorhydrate de pipéridine dans 25 ml de MeOH et 25 ml d'eau on ajoute, goutte à goutte, à TA une solution de 5,3 g de cyanure de sodium dans 20 ml d'eau et laisse 48 heures sous agitation à TA. On essore le précipité formé, le lave à l'eau et le sèche sous vide. On obtient 27 g du produit attendu.

B) 1-Benzyl-4-carbamoyl-4-(pipérid-1-yl)pipéridine.

On ajoute 10 g du composé obtenu à l'étape précédente à 50 ml d'acide sulfurique à 95 % et chauffe à 100°C pendant 45 minutes. Après refroidissement à TA, on verse le mélange réactionnel sur 100 g de glace, ajoute en refroidissant 250 ml de DCM, après décantation, sèche la phase organique sur $MgSO_4$ et évapore sous vide le solvant. On recristallise le produit solide obtenu dans 300 ml du mélange acétonitrile/toluène (65/35 ; v/v). On obtient 9,7 g du produit attendu, F =150-160°C.

C) Dichlorhydrate de 4-carbamoyl-4-(pipérid-1-yl)pipéridine.

A une solution de 9,7 g du composé obtenu à l'étape précédente dans 200 ml de MeOH on ajoute 10 g de formiate d'ammonium, 2,5 g de palladium sur charbon à 5 % et laisse 2 heures sous agitation à TA. On filtre sur Célite® et évapore sous vide le filtrat. On dissout le résidu dans une solution d'HCl 2N, alcalinise à pH = 13 par ajout d'une solution de NaOH à 40 %, extrait au chloroforme, sèche la phase organique sur $MgSO_4$ et évapore sous vide le solvant. On dissout le produit obtenu dans un mélange MeOH/DCM, acidifie à pH = 1 par ajout d'une solution saturée d'acide chlorhydrique dans l'éther et évapore sous vide. On obtient 5 g du produit attendu, F = 185°C.

Préparation 2.17

Benzènesulfonate de 4-phényl-4-uréidopipéridine.

A) 1-(Benzyloxycarbonyl)-4-isocyanato-4-phénylpipéridine.

On chauffe à reflux pendant 1 heure un mélange de 50,89 g du composé obtenu à l'étape A) de la Préparation 2.6 et 71,4 g de chlorure de thionyle dans 400 ml de 1,2-dichloroéthane. On concentre sous vide le mélange réactionnel, reprend le résidu à l'acétone et évapore sous vide le solvant. On dissout le résidu dans 200 ml d'acétone, refroidit à 5°C, ajoute goutte à goutte une solution de 19,5 g d'azidure de sodium dans 50 ml d'eau et laisse 2 heures sous agitation à TA. On évapore sous vide à TA l'acétone, ajoute à la solution restante une solution saturée de $NaHCO_3$, extrait au toluène, lave la phase organique à l'eau, par une solution saturée de NaCl, sèche sur $Na_2SO_4$ et évapore 50 % en volume de solvant. On chauffe à reflux pendant 1 heure la solution toluènique restante puis concentre sous vide. On obtient 54 g du produit attendu sous forme d'huile orange qui cristallise.

B) 1-(Benzyloxycarbonyl)-4-phényl-4-uréidopipéridine.

Dans une solution de 29 g du composé obtenu à l'étape précédente dans 300 ml d'éther et 300 ml de DCM, on fait barboter à TA et en excès de l'ammoniac gaz puis laisse une nuit sous agitation à TA. On concentre sous vide le mélange réactionnel, reprend le résidu dans de l'acétone chaud et laisse redescendre la température à TA. Dès que le produit cristallise on ajoute un mélange d'éther/AcOEt puis essore les cristaux formés. On obtient 26,4 g du produit attendu.

C) Benzènesulfonate de 4-phényl-4-uréidopipéridine..

On hydrogène à 40°C et à pression atmosphérique un mélange de 25 g du composé obtenu à l'étape précédente, 11,2 g d'acide benzènesulfonique, 3 g de palladium sur charbon à 5 % et 300 ml d'EtOH. On dilue le mélange réactionnel par ajout d'eau et de MeOH, filtre le catalyseur sur Célite® et concentre sous vide le filtrat. On obtient 26,45 g du produit attendu après cristallisation dans l'acétone, F = 235°C.

Préparation 2.18

Benzènesulfonate de 4-(N'-méthyluréido)-4-phénylpipéridine.

A) 1-(Benzyloxycarbonyl)-4-(N'-méthyluréido)-4-phénylpipéridine.

On refroidit à 5°C une solution de 25 g du composé obtenu à l'étape A de la Préparation 2.17 dans 300 ml d'éther et fait barboter en excès de la méthylamine gaz. On dilue le mélange réactionnel par ajout de 150 ml de DCM et laisse une nuit sous agitation à TA. On concentre sous vide, reprend le résidu dans de l'AcOEt chaud et laisse revenir à TA. On ajoute de l'éther jusqu'à précipitation et essore le précipité formé. On obtient 24 g du produit attendu.

B) Benzènesulfonate de 4-(N'-méthyluréido)-4-phénylpipéridine.

On hydrogène à 40°C et à pression atmosphérique un mélange de 23 g du composé obtenu à l'étape précédente, 9,9 g d'acide benzènesulfonique, 3 g de palladium sur charbon à 5 % et 300 ml d'EtOH 95. On filtre le catalyseur sur Célite® et concentre sous vide le filtrat. On reprend le résidu à l'acétone et essore le précipité formé. On obtient 22,36 g du produit attendu, F = 227°C.

Préparation 2.19

*p*-Toluènesulfonate de 4-(3,3-diméthylcarbazoyl)-4-phénylpipéridine.

A)p-Toluènesulfonate de 1-(benzyloxycarbonyl)-4-(3,3-diméthylcarbazoyl)-4-phénylpipéridine.

On refroidit à 5°C une solution de 7,15 g du composé obtenu à l'étape A de la Préparation 2.12 dans 60 ml de DCM, ajoute goutte à goutte une solution de 1,44 g de 1,1-diméthylhydrazine, 4,04 g de triéthylamine dans 20 ml de DCM et laisse une nuit sous agitation à TA. On concentre sous vide le mélange réactionnel, reprend le résidu à l'eau, extrait à l'AcOEt, lave la phase organique par une solution saturée de Nacl, sèche sur $NaiSO_4$ et évapore sous vide le solvant. On dissout le produit obtenu dans 100 ml d'acétone, ajoute rapidement une solution de 3,59 g d'acide *p*-toluènesulfonique monohydrate dans 10 ml d'acétone et concentre sous vide. On reprend le résidu dans un mélange éther/DCM et essore le précipité formé. On obtient 9,65 g du produit attendu.

B) *p*-Toluènesulfonate de 4-(3,3-diméthylcarbazoyl)-4-phénylpipéridine.

On hydrogène à 30°C et à pression atmosphérique un mélange de 9,5 g du composé obtenu à l'étape précédente, 0,9 g de palladium sur charbon à 10 % dans 100 ml d'EtOH 95. On filtre le catalyseur sur Célite ® et concentre sous vide le filtrat. On reprend le résidu dans 100 ml d'acétone, essore le produit cristallisé formé et le lave à l'éther. On obtient 7 g du produit attendu, F = 210-212°C.

Préparation 2.20

Chlorhydrate de 4-[(éthylaminocarbonyloxy)méthyl]-4-phénylpipéridine.

A) *p*-Toluènesulfonate de 4-méthoxycarbonyl-4-phénylpipéridine.

A une solution de 10 g de *p*-toluènesulfonate de 4-carboxy-4-phénylpipéridine dans 300 ml de MeOH, on ajoute 1 g d'acide *p*-toluènesulfonique monohydrate et chauffe à reflux pendant 3 jours. On concentre sous vide le mélange réactionnel, reprend le résidu dans l'acétone et ajoute de l'éther jusqu'à précipitation. Après essorage du précipité formé, on obtient 9,34 g du produit attendu.

B) 4-Hydroxyméthyl-4-phénylpipéridine.

On refroidit à -20°C une suspension de 1,16 g d'hydrure d'aluminium et de lithium dans 50 ml de THF, ajoute 4 g du composé obtenu à l'étape précédente et laisse sous agitation une nuit en laissant remonter la température à TA. On hydrolyse par ajout de 1,2 ml d'eau, puis 2,5 ml d'une solution à 10 % de NaOH et 2,5 ml d'eau. On dilue à l'éther, filtre les sels minéraux et évapore sous vide le filtrat. On obtient 1,8 g du produit attendu.

C) 1-*tert*-Butoxycarbonyl-4-(hydroxyméthyl)-4-phénylpipéridine.

A une solution de 22,8 g du composé obtenu à l'étape précédente dans 250 ml de 1,2-diméthoxyéthane on ajoute 26,05 g de di-*tert*-butyldicarbonate et chauffe à reflux pendant 2 heures. On concentre sous vide le mélange réactionnel, reprend le résidu au DCM, lave la phase organique par une solution tampon pH = 2, par une solution saturée de NaCl, sèche sur $MgSO_4$ et évapore sous vide le solvant. On obtient 17,86 g du produit attendu après cristallisation dans l'éther, F = 134°C.

D) 1-*tert*-Butoxycarbonyl-4-[(éthylaminocarbonyloxy)méthyl]-4-phénylpipéridine.

On laisse une nuit sous agitation à TA un mélange de 2,91 g du composé obtenu à l'étape précédente, 2,4 g d'isocyanate d'éthyle, 2 gouttes de triéthylamine dans 30 ml de toluène. Puis on chauffe à 100°C pendant 24 heures et concentre sous vide le mélange réactionnel. On reprend le résidu à l'éther, lave la phase organique par une solution tampon pH = 2, par une solution saturée de NaCl, sèche sur $MgSO_4$ et évapore sous vide le solvant. On obtient 3,85 g

du produit attendu sous forme d'huile.

E) Chlorhydrate de 4-[(éthylaminocarbonyloxy)méthyl]-4-phénylpipéridine.

A une solution de 3,85 g du composé obtenu à l'étape précédente dans 50 ml de MeOH, on ajoute 10 ml d'HCl concentré et chauffe à 60°C pendant 2 heures. On concentre sous vide, reprend le résidu à l'acétone et évapore sous vide le solvant. On obtient 2,6 g du produit attendu après cristallisation dans le mélange AcOEt/éther, F = 240-242°C.

Préparation 2.21

4-(Acétyloxyméthyl)-4-phénylpipéridine.

A une solution de 2,91 g du composé obtenu à l'étape C de la Préparation 2.20 et 1,31 g de triéthylamine dans 50 ml de DCM, on ajoute à TA 0,785 g de chlorure d'acétyle et laisse 30 minutes sous agitation à TA. On concentre sous vide, reprend le résidu à l'eau, extrait à l'AcOEt, lave deux fois la phase organique à l'eau, par une solution saturée de NaCl, sèche sur $MgSO_4$ et évapore sous vide le solvant. On reprend le résidu huileux dans 20 ml d'acide trifluoroacétique et laisse 10 minutes sous agitation à TA. On concentre sous vide, reprend le résidu à l'eau, alcalinise la phase aqueuse à pH = 11 par ajout d'une solution concentrée de NaOH, extrait au DCM, lave 5 fois la phase organique à l'eau, par une solution saturée de NaCl, sèche sur $MgSO_4$ et évapore sous vide le solvant. On obtient 1,6 g du produit attendu.

Préparation 2.22

Benzènesulfonate de 4-benzyl-4-[(éthoxycarbonyl-N-méthylamino)méthyl] pipéridine.

A) 1-Benzyl-4-carbamoylpipéridine.

A un mélange de 58,2 g d'isonipécotamide et 69 g de $K_2CO_3$ dans 275 ml de DMF, on ajoute à TA et goutte à goutte 85,5 g de bromure de benzyle et laisse 2 heures sous agitation à 50°C. On concentre sous vide le mélange réactionnel, reprend le résidu à l'eau, extrait au DCM, lave la phase organique à l'eau, sèche sur $Na_2SO_4$ et évapore sous vide le solvant. On obtient 62 g du produit attendu après cristallisation dans 300 ml d'eau et séchage sous vide.

B) 1-Benzyl-4-cyanopipéridine.

On chauffe à reflux pendant 2 heures un mélange de 62 g du composé obtenu à l'étape précédente et 200 ml de $POCl_3$. On concentre sous vide, reprend le résidu à l'eau, alcalinise à pH = 13 par ajout d'une solution concentrée de NaOH, extrait au DCM, lave la phase organique à l'eau, sèche sur $Na_2SO_4$ et évapore sous vide le solvant. On distille sous pression réduite l'huile obtenue. On obtient 52 g du produit attendu, Eb = 120°C sous 10 Pa.

C) 1,4-Dibenzyl-4-cyanopipéridine.

On refroidit à -50°C 200 ml d'une solution 1,5M de diisopropylamidure de lithium dans le cyclohexane diluée dans 100 ml de THF, ajoute goutte à goutte une solution de 52 g du composé obtenu à l'étape précédente dans 100 ml de THF et laisse 30 minutes sous agitation à -50°C. Puis on ajoute goutte à goutte et à température comprise entre -30°C et -25°C une solution de 51,3 g de bromure de benzyle dans 100 ml de THF et laisse sous agitation en laissant remonter la température à TA. On concentre sous vide, reprend le résidu à l'eau, extrait à l'éther, lave la phase organique à l'eau, sèche sur $Na_2SO_4$ et évapore sous vide le solvant. On obtient 64 g du produit attendu après cristallisation dans le pentane.

D) 4-(Aminométhyl)-1,4-dibenzylpipéridine.

A une suspension de 1,95 g d'hydrure d'aluminium et de lithium dans 50 ml de THF, on ajoute à TA et goutte à goutte une solution de 14,5 g du composé obtenu à l'étape précédente dans 50 ml de THF puis chauffe à reflux pendant 6 heures. Après refroidissement à TA on ajoute 2 ml d'eau, 2 ml d'une solution de NaOH 4N et 6 ml d'eau. On filtre les sels minéraux sur Célite®, décante le filtrat, sèche la phase organique sur $Na_2SO_4$ et évapore sous vide le solvant. On obtient 14,7 g du produit attendu.

E) 1,4-Dibenzyl-4-[(formylamino)méthyl]pipéridine.

A une solution de 14,7 g du composé obtenu à l'étape précédente dans 126 ml d'acide formique on ajoute à

TA et goutte à goutte 42 ml d'anhydride acétique et laisse 3 heures sous agitation à TA. On concentre sous vide, reprend le résidu à l'eau, alcalinise à pH = 13 par ajout d'une solution concentrée de NaOH, extrait à l'AcOEt, lave la phase organique à l'eau, sèche sur $Na_2SO_4$ et évapore sous vide le solvant. On obtient 14,5 g du produit attendu.

F) 1,4-Dibenzyl-4-[(méthylamino)méthyl]pipéridine.

A une suspension de 5 g d'hydrure d'aluminium et de lithium dans 100 ml de THF, on ajoute à 40°C et goutte à goutte une solution de 14,5 g du composé obtenu à l'étape précédente dans 100 ml de THF puis chauffe à reflux pendant 4 heures. Après refroidissement à TA on ajoute 5 ml d'eau, 5 ml d'une solution de NaOH 4N et 15 ml d'eau. On filtre les sels minéraux sur Célite® , décante le filtrat, sèche la phase organique sur $Na_2SO_4$ et évapore sous vide le solvant. on obtient 12,8 g du produit attendu.

G) 1,4-Dibenzyl-4-[(éthoxycarbonyl-N-méthylamino)méthyl]pipéridine.

A une solution de 3 g du composé obtenu à l'étape précédente et 1,18 g de triéthylamine dans 50 ml de 1,2-dichloroéthane, on ajoute à TA et goutte à goutte 1,26 g de chloroformiate d'éthyle et laisse 1 heure sous agitation à TA. On concentre sous vide, reprend le résidu à l'eau, extrait à l'éther, sèche la phase organique sur $Na_2SO_4$ et évapore sous vide le solvant. On chromatographie le résidu sur silice H en éluant par le mélange DCM/MeOH (100/3 ; v/v). On obtient 1,6 g du produit attendu.

H) Benzènesulfonate de 4-benzyl-4-[(éthoxycarbonyl-N-méthylamino)méthyl] pipéridine.

On hydrogène à 27°C et à pression atmosphérique un mélange de 1,6 g du composé obtenu à l'étape précédente, 0,66 g d'acide benzènesulfonique, 0,2 g de palladium sur charbon à 10 % et 30 ml de MeOH. On filtre le catalyseur sur Célite® et concentre sous vide le filtrat. On obtient 1,44 g du produit attendu.

Préparation 2.23

Benzènesulfonate de 4-benzyl-4-[(méthanesulfonyl-N-méthylamino)méthyl] pipéridine.

A) 1,4-Dibenzyl-4-[(méthanesulfonyl-N-méthylamino)méthyl]pipéridine.

A une solution de 3,2 g du composé obtenu à l'étape F de la Préparation 2.22 et 1,26 g de triéthylamine dans 50 ml de 1,2-dichloroéthane, on ajoute à TA et goutte à goutte 1,26 g de chlorure de méthanesulfonyle et laisse 1 heure sous agitation à TA. On concentre sous vide, reprend le résidu à l'eau, extrait à l'AcOEt, sèche la phase organique sur $Na_2SO_4$ et évapore sous vide le solvant. On obtient 3,8 g du produit attendu.

B) Benzènesulfonate de 4-benzyl-4-[(méthanesulfonyl-N-méthylamino)méthyl] pipéridine.

On hydrogène à TA et à pression atmosphérique un mélange de 3,8 g du composé obtenu à l'étape précédente, 1,58 g d'acide benzènesulfonique, 0,8 g de palladium sur charbon à 10 % et 30 ml de MeOH. On filtre le catalyseur sur Célite® et concentre sous vide le filtrat. On obtient 4,6 g du produit attendu, après cristallisation dans le mélange DCM/éther.

Préparation 2.24

Chlorhydrate de 4-benzyl-4-[(N',N'-diméthyl-N-méthyluréido)méthyl]pipéridine.

A) 1,4-Dibenzyl-4-[(N',N'-diméthyl-N-méthyluréido)méthyl]pipéridine.

A une solution de 3,2 g du composé obtenu à l'étape F de la Préparation 2.22 et 1,2 g de triéthylamine dans 40 ml de 1,2-dichloroéthane, on ajoute à TA et goutte à goutte 1,12 g de chlorure de N,N-diméthylcarbamoyle et chauffe à reflux pendant 4 heures. On concentre sous vide, reprend le résidu à l'eau, extrait à l'éther, sèche la phase organique sur $Na_2SO_4$ et évapore sous vide le solvant. On obtient 3,6 g du produit attendu.

B) Chlorhydrate de 4-benzyl-4-[(N',N'-diméthyl-N-méthyluréido)méthyl]pipéridine.

On laisse 1 heure 30 minutes sous agitation un mélange de 3,6 g du composé obtenu à l'étape précédente,

3,1 g de formiate d'ammonium et 0,8 g de palladium sur charbon à 5 % dans 50 ml de MeOH. On filtre le catalyseur et évapore sous vide le solvant. On dissout le résidu dans du DCM, ajoute une solution saturée d'acide chlorhydrique dans l'éther jusqu'à pH = 1 et évapore sous vide les solvants. On obtient 3,2 g du produit attendu.

Préparation 2.25

4-Carbamoyl-4-(morpholin-4-yl)pipéridine.

A) 1-Benzyl-4-cyano-4-(morpholin-4-yl)pipéridine.

A un mélange de 5 g de 1-benzylpipérid-4-one et 1,9 g de cyanure de potassium dans 50 ml d'un mélange EtOH/eau (50/50 ; v/v) on ajoute 2,5 ml de morpholine puis 5,1 g de $NaiS_2O_5$ et chauffe à 60°C pendant 2 heures. On rajoute 2,5 ml de morpholine et laisse une nuit sous agitation à TA. On ajoute de l'eau au mélange réactionnel et essore le produit cristallisé formé. On obtient 5,5 g du produit attendu.

B) 1-Benzyl-4-carbamoyl-4-(morpholin-4-yl)pipéridine.

On chauffe à 100°C pendant 2 heures un mélange de 14 g du composé obtenu à l'étape précédente et 50 ml d'acide sulfurique à 95 %. Après refroidissement à TA, on verse le mélange réactionnel sur 100 g de glace, amène à pH = 7 par ajout d'une solution concentrée de $NH_4OH$, extrait au DCM, lave la phase organique à l'eau, sèche sur $Na_2SO_4$ et évapore sous vide le solvant. On chromatographie le résidu sur silice H en éluant par le mélange DCM/MeOH (100/5 ; v/v) à (100/10 ; v/v). On obtient 3,4 g du produit attendu après cristallisation dans l'éther iso.

C) 4-Carbamoyl-4-(morpholin-4-yl)pipéridine.

A une solution de 3,4 g du composé obtenu à l'étape précédente dans 50 ml de MeOH on ajoute 3,1 g de formiate d'ammonium et 0,8 g de palladium sur charbon à 5 % et laisse 2 heures sous agitation à TA. On filtre le catalyseur sur Célite® et évapore sous vide le filtrat. On obtient 2,2 g du produit attendu après cristallisation dans le propan-2-ol.

EXEMPLE 1

Chlorhydrate de 3-benzyl-5-[2-(4-benzylpipérid-l-yl)éthyl]-5-(3,4-dichlorophényl)-tétrahydro-2*H*-1,3-oxazin-2-one, monohydrate.

A) 3-Benzyl-5-(3,4-dichlorophényl)-5-[2-(tétrahydropyran-2-yloxy)éthyl]tétrahydro-2*H*-1,3-oxazin-2-one.

On refroidit à +5°C une solution de 4 g du composé obtenu à la Préparation 1.1 dans 60 ml de THF, ajoute 1,37 g de *tert*-butylate de potassium puis, goutte à goutte, une solution de 1,92 g de bromure de benzyle dans 15 ml de THF. On laisse sous agitation en laissant remonter la température à TA et concentre sous vide le mélange réactionnel. On extrait le résidu à l'AcOEt, lave trois fois la phase organique à l'eau, deux fois par une solution saturée de NaCl, sèche sur $MgSO_4$ et évapore sous vide le solvant. On obtient 4,2 g du produit attendu que l'on utilise tel quel.

B) 3-Benzyl-5-(3,4-dichlorophényl)-5-(2-hydroxyéthyl)-tétrahydro-2*H*-1,3-oxazin-2-one, 0,25 hydrate.

A une solution de 4,2 g du composé obtenu à l'étape précédente dans 40 ml de MeOH, on ajoute jusqu'à pH = 1 une solution saturée d'HCl gaz dans l'éther et laisse 48 heures sous agitation à TA. On concentre sous vide le mélange réactionnel, reprend le résidu par une solution saturée d'HCl gaz dans le MeOH et évapore sous vide le solvant. On reprend le résidu à l'éther et essore le précipité formé. On obtient 3,44 g du produit attendu, F = 143°C.

C) 3-Benzyl-5-(3,4-dichlorophényl)-5-[2-(méthanesulfonyloxy)éthyl]tétrahydro-2*H*-1,3-oxazin-2-one.

On refroidit à +5°C une solution de 1,4 g du composé obtenu à l'étape précédente, 0,45 g de triéthylamine dans 50 ml de DCM, ajoute goutte à goutte une solution de 0,46 g de chlorure de méthanesulfonyle dans 5 ml de DCM. Après l'addition, on concentre sous vide le mélange réactionnel, reprend le résidu à l'eau, extrait à l'AcOEt, lave deux fois la phase organique à l'eau, par une solution saturée de NaCl, sèche sur $MgSO_4$ et évapore sous vide le solvant. On obtient 1,7 g du produit attendu.
Spectre de RMN du proton à 200 MHz dans DMSO-$d_6$

δ: 2,03 ppm : t : 2H

2,98 ppm : s : 3H
3,78 ppm : système AB : 2H
3,83 ppm : t : 2H
4,1 à 4,8 ppm : m : 4H
7,0 à 7,6 ppm : m : 8H

D) Chlorhydrate de 3-benzyl-5-[2-(4-benzylpipérid-1-yl)éthyl]-5-(3,4-dichloro phényl)-tétrahydro-2*H*-1,3-oxazin-2-one, monohydrate.

On chauffe à 60°C pendant 2 heures 30 minutes un mélange de 1,6 g de 4-benzylpipéridine, 1,7 g du composé obtenu à l'étape précédente, 0,6 g d'iodure de potassium dans 10 ml de DMF. Après refroidissement à TA, on verse le mélange réactionnel dans l'eau, extrait à l'AcOEt, lave deux fois la phase organique à l'eau, par une solution saturée de NaCl, sèche sur $MgSO_4$ et évapore sous vide le solvant. On chromatographie le résidu sur silice H en éluant au DCM. On reprend le produit obtenu par une solution saturée d'HCl gaz dans l'éther et essore le précipité formé. On obtient 0,9 g du produit attendu, F = 122°C.

EXEMPLE 2

Chlorhydrate de 3-benzyl-5-(3,4-dichlorophényl)-5-[2-(4-hydroxy-4-phényl pipérid-1-yl)éthyl]-tétrahydro-2*H*-1,3-oxazin-2-one.

On prépare ce composé selon le mode opératoire décrit à l'étape D de l'EXEMPLE 1 à partir de 1,32 g de 4-hydroxy-4-phénylpipéridine, 1,55 g du composé obtenu à l'étape C de l'EXEMPLE 1, 0,56 g d'iodure de potassium dans 10 ml de DMF. On chromatographie sur silice H en éluant au DCM puis par le mélange DCM/MeOH (96/4 ; v/v). On dissout le produit obtenu dans du DCM, acidifie à pH = 1 par ajout d'une solution saturée d'HCl gaz dans l'éther et évapore sous vide les solvants. On obtient 0,72 g du composé attendu après cristallisation dans l'éther, F = 172°C.

EXEMPLE 3

Chlorhydrate de 5-[2-(4-acétamido-4-phénylpipérid-1-yl)éthyl]-3-benzyl-5-(3,4-dichlorophényl)-tétrahydro-2*H*-1,3-oxazin-2-one, 1,5 hydrate.

On prépare ce composé selon le mode opératoire décrit à l'étape D de l'EXEMPLE 1 à partir de 2,77 g de 4-acétamido-4-phénylpipéridine, 1,7 g du composé obtenu à l'étape C de l'EXEMPLE 1, 0,61 g d'iodure de potassium dans 10 ml de DMF. On chromatographie sur silice H en éluant au DCM puis par le mélange DCM/MeOH (97/3 ; v/v). On reprend le résidu par une solution saturée d'HCl gaz dans l'éther et évapore sous vide le solvant. On obtient 0,6 g du produit attendu après cristallisation dans l'AcOEt, F = 168°C.

EXEMPLE 4

Chlorhydrate de 3-[3,5-bis(trifluorométhyl)benzyl]-5-(3,4-dichlorophényl)-5-[2-(4-phénylpipérid-1-yl)éthyl]-tétrahydro-2*H*-1,3-oxazin-2-one, hémihydrate.

A) 3-[3,5-Bis(trifluorométhyl)benzyl]-5-(3,4-dichlorophényl)-5-[2-(tétrahydropyran-2-yloxy)éthyl]-tétrahydro-2*H*-1,3-oxazin-2-one.

On refroidit à +5°C une solution de 3,7 g du composé obtenu à la Préparation 1.1 dans 60 ml de THF, ajoute 1,27 g de *tert*-butylate de potassium puis goutte à goutte une solution de 2,72 g de chlorure de 3,5-bis(trifluorométhyl) benzyle dans 15 ml de THF. On laisse sous agitation en laissant remonter la température à TA et concentre sous vide le mélange réactionnel. On reprend le résidu à l'eau, extrait à l'AcOEt, lave la phase organique à l'eau, par une solution saturée de NaCl, sèche sur $MgSO_4$ et évapore sous vide le solvant. On obtient 5,9 g du produit attendu que l'on utilise tel quel.

B) 3-[3,5-Bis(trifluorométhyl)benzyl]-5-(3,4-dichlorophényl)-5-(2-hydroxyéthyl)-tétrahydro-2*H*-1,3-oxazin-2-one.

A une solution de 5,9 g du composé obtenu à l'étape précédente dans 50 ml de MeOH, on ajoute jusqu'à pH = 1 une solution saturée d'HCl gaz dans l'éther et laisse 15 minutes sous agitation à TA. On concentre sous vide le mélange réactionnel, reprend deux fois le résidu par une solution saturée d'HCl gaz dans le MeOH et évapore sous

vide le solvant. On reprend le résidu à l'hexane et essore le précipité formé. On obtient 3,9 g du produit attendu.

Spectre de RMN du proton à 200 MHz dans DMSO-$d_6$

δ: 1,9 ppm : t : 2H

2,9 à 3,6 ppm : m : 4H
3,8 ppm : système AB : 2H
4,35 ppm : s : 2H
5,45 ppm : se : 1 H
7,1 à 7,8 ppm : m : 3H
8,0 à 8,5 ppm : m : 3H
8,9 ppm : se : 1H

C) 3-[3,5-Bis(trifluorométhyl)benzyl]-5-(3,4-dichlorophényl)-5-[2-(méthanesulfonyl oxy)éthyl]-tétrahydro-2*H*-1,3-oxazin-2-one.

On refroidit à +5°C une solution de 3 g du composé obtenu à l'étape précédente, 0,7 g de triéthylamine dans 50 ml de DCM, ajoute goutte à goutte une solution de 0,66 g de chlorure de méthanesulfonyle dans 6 ml de DCM et laisse sous agitation en laissant remonter la température à TA. On concentre sous vide le mélange réactionnel, reprend le résidu à l'eau, extrait à l'AcOEt, lave la phase organique à l'eau, par une solution d'HCl 2N, à l'eau, par une solution à 5 % de $NaHCO_3$, à l'eau, par une solution saturée de NaCl, sèche sur $MgSO_4$ et évapore sous vide le solvant. On obtient 3,4 g du produit attendu.

D) Chlorhydrate de 3-[3,5-bis(trifluorométhyl)benzyl]-5-(3,4-dichlorophényl)-5-[2-(4-phénylpipérid-1-yl)éthyl]-tétrahydro-2*H*-1,3-oxazin-2-one, hémihydrate.

On chauffe à 60°C pendant 3 heures un mélange de 0,32 g de 4-phénylpipéridine, 1 g du composé obtenu à l'étape précédente, 0,28 g d'iodure de potassium dans 5 ml de DMF. Après refroidissement on verse le mélange réactionnel dans l'eau, extrait à l'AcOEt, lave la phase organique à l'eau, par une solution saturée de NaCl, sèche sur $MgSO_4$ et évapore sous vide le solvant. On chromatographie le résidu sur silice H en éluant au DCM puis par le mélange DCM/MeOH (98/2 ; v/v). On reprend le produit obtenu par une solution saturée d'HCl gaz dans l'éther et essore le précipité formé. On obtient 0,22 g du produit attendu, F = 174°C.

EXEMPLE 5

Chlorhydrate de 5-[2-(4-benzylpipérid-1-yl)éthyl]-3-[3,5-bis(trifluorométhyl) benzyl]-5-(3,4-dichlorophényl)-tétrahydro-2*H*-1,3-oxazin-2-one, 1,5 hydrate.

On prépare ce composé selon le mode opératoire décrit à l'étape D de l'EXEMPLE 4 à partir de 0,3 g de 4-benzylpipéridine, 0,8 g du composé obtenu à l'étape C de l'EXEMPLE 4, 0,24 g d'iodure de potassium et 5 ml de DMF. On chromatographie sur silice H en éluant au DCM puis par le mélange DCM/MeOH (96/4 ; v/v). On reprend le produit obtenu par une solution saturée d'HCl gaz dans l'éther et essore le précipité formé. On obtient 0,13 g du produit attendu, F = 124°C.

EXEMPLE 6

Dichlorhydrate de 5-[2-(4-anilinopipérid-1-yl)éthyl]-3-[3,5-bis (trifluorométhyl) benzyl]-5-(3,4-dichlorophényl)-tétrahydro-2*H*-1,3-oxazin-2-one, monohydrate.

On chauffe à 50-60°C pendant 5 heures un mélange de 0,3 g de 4-anilinopipéridine, 0,8 g du composé obtenu à l'étape C de l'EXEMPLE 4, 0,69 g de carbonate de potassium dans 5 ml d'acétonitrile. On verse le mélange réactionnel dans l'eau, extrait à l'AcOEt, lave deux fois la phase organique à l'eau, par une solution saturée de NaCl, sèche sur $MgSO_4$ et évapore sous vide le solvant. On chromatographie le résidu sur silice H en éluant au DCM puis par le mélange DCM/MeOH (96/4 ; v/v). On reprend le produit obtenu par une solution saturée d'HCl gaz dans l'éther et essore le précipité formé. On obtient 0,12 g du produit attendu, F =158°C.

EXEMPLE 7

Chlorure de 5-(3,4-dichlorophényl)-5-[2-[4-phényl-1-azoniabicyclo [2.2.2] oct-1-yl]éthyl]-3-(3,5-bis(trifluorométhyl)benzyl)-tétrahydro-2H-1,3-oxazin-2-one, 2,5 hydrate.

On chauffe à 60°C pendant 5 heures un mélange de 0,53 g de 4-phényl-1-azabicyclo[2.2.2]octane, 1,4 g du

composé obtenu à l'étape C de l'EXEMPLE 4 dans 5 ml de DMF. Après refroidissement à TA, on verse le mélange réactionnel dans l'eau, extrait au DCM, lave deux fois la phase organique par une solution d'HCl 2N, trois fois par une solution saturée de NaCl, sèche sur $MgSO_4$ et évapore sous vide le solvant. On chromatographie le résidu sur silice en éluant au DCM puis par le mélange DCM/MeOH (92/8 ; v/v). On reprend le produit obtenu dans l'éther et essore le précipité formé. On obtient 0,21 g du produit attendu, F = 138°C.

EXEMPLE 8

Chlorhydrate de 3-benzyl-5-[3-(4-benzylpipérid-1-yl)propyl]-5-(3,4-dichloro phényl)-tétrahydro-2*H*-1,3-oxazin-2-one.

A) 3-Benzyl-5-(3,4-dichlorophényl)-5-[3-(tétrahydropyran-2-yloxy)propyl]-tétrahydro-2*H*-1,3-oxazin-2-one.

On refroidit à +5°C une solution de 12,5 g du composé obtenu à la Préparation 1.2 dans 150 ml de THF, ajoute 4,13 g de *tert*-butylate de potassium puis goutte à goutte une solution de 5,78 g de bromure de benzyle dans 30 ml de THF. On laisse 1 heure sous agitation à TA et concentre sous vide. On reprend le résidu à l'eau, extrait à l'AcOEt, lave deux fois la phase organique à l'eau, par une solution saturée de NaCl, sèche sur $MgSO_4$ et évapore sous vide le solvant. On obtient le produit attendu que l'on utilise tel quel.

B) 3-Benzyl-5-(3,4-dichlorophényl)-5-(3-hydroxypropyl)-tétrahydro-2*H*-1,3-oxazin-2-one.

On reprend le composé obtenu à l'étape précédente par une solution saturée d'HCl gaz dans le MeOH et laisse 2 heures sous agitation à TA. On concentre sous vide le mélange réactionnel, reprend deux fois le résidu par une solution saturée d'HCl gaz dans le MeOH et évapore sous vide le solvant. On chromatographie le résidu sur silice en éluant au DCM puis par le mélange DCM/MeOH (96/4 ; v/v). On obtient 10,6 g du produit attendu que l'on utilise tel quel.

C) 3-Benzyl-5-(3,4-dichlorophényl)-5-[3-(méthanesulfonyloxy)propyl]-tétrahydro-2*H*-1,3-oxazin-2-one.

On refroidit à +5°C une solution de 10 g du composé obtenu à l'étape précédente, 3,07 g de triéthylamine dans 350 ml de DCM, ajoute goutte à goutte une solution de 3,19 g de chlorure de méthanesulfonyle dans 35 ml de DCM. Après l'addition, on concentre sous vide le mélange réactionnel, reprend le résidu à l'eau, extrait à l'AcOEt, lave deux fois la phase organique à l'eau, par une solution saturée de NaCl, sèche sur $MgSO_4$ et évapore sous vide le solvant. On obtient 11 g du produit attendu que l'on utilise tel quel.

D) Chlorhydrate de 3-benzyl-5-[3-(4-benzylpipérid-1-yl)propyl]-5-(3,4-dichloro phényl)-tétrahydro-2*H*-1,3-oxazin-2-one.

On chauffe à 50°C pendant 2 heures un mélange de 1,86 g de 4-benzylpipéridine, 2 g du composé obtenu à l'étape précédente, 0,7 g d'iodure de potassium dans 10 ml de DMF. Après refroidissement à TA, on verse le mélange réactionnel dans l'eau, extrait à l'AcOEt, lave deux fois la phase organique à l'eau, par une solution saturée de NaCl, sèche sur $MgSO_4$ et évapore sous vide le solvant. On chromatographie le résidu sur silice H en éluant au DCM puis par le mélange DCM/MeOH (98/2 ; v/v). On reprend le produit obtenu par une solution saturée d'HCl gaz dans l'éther et évapore sous vide le solvant. On obtient 1,4 g du produit attendu après cristallisation dans l'AcOEt, F = 219°C.

EXEMPLE 9

Chlorhydrate de 3-benzyl-5-(3,4-dichlorophényl)-5-[3-(4-hydroxy-4-phényl pipérid-1-yl)propyl]-tétrahydro-2H-1,3-oxazin-2-one, hémihydrate.

On prépare ce composé selon le mode opératoire décrit à l'étape D de l'EXEMPLE 8 à partir de 1,6 g de 4-hydroxy-4-phénylpipéridine, 2 g du composé obtenu à l'étape C de l'EXEMPLE 8, 0,75 g d'iodure de potassium dans 10 ml de DMF. On chromatographie sur silice H en éluant au DCM puis par le mélange DCM/MeOH (96/4 ; v/v). On reprend le produit obtenu par une solution saturée d'HCl gaz dans l'éther et essore le précipité formé. On obtient 1,34 g du produit attendu, F = 125°C.

EXEMPLE 10

Chlorhydrate de 5-(3-(4-acétamido-4-phénylpipérid-1-yl)propyl)-3-benzyl-5-(3,4-dichlorophényl)-tétrahydro-2*H*-1,3-oxazin-2-one, 1,5 hydrate.

On dissout 2,02 g de chlorhydrate de 4-acétamido-4-phénylpipéridine dans 5 ml d'eau, alcalinise à pH = 13

par ajout de NaOH concentré, extrait au DCM, sèche la phase organique sur $MgSO_4$ et évapore sous vide le solvant. On reprend le résidu dans 10 ml de DMF, ajoute 1,5 g du composé obtenu à l'étape C de l'EXEMPLE 8, 0,53 g d'iodure de potassium et chauffe à 50°C pendant 2 heures 30 minutes. On verse le mélange réactionnel dans l'eau, extrait à l'AcOEt, lave trois fois la phase organique à l'eau, par une solution saturée de NaCl, sèche sur $MgSO_4$ et évapore sous vide le solvant. On chromatographie le résidu sur silice en éluant au DCM puis par le mélange DCM/MeOH (96/4 ; v/v). On reprend le produit obtenu par une solution saturée d'HCl gaz dans l'éther et évapore sous vide le solvant. On obtient 1,34 g du produit attendu après cristallisation dans l'AcOEt, F = 145°C.

EXEMPLE 11

Chlorhydrate de 3-benzyl-5-(3,4-dichlorophényl)-5-[3-[4-(N-phényl-acétamido) pipérid-1-yl]propyl]-tétrahydro-2*H*-1,3-oxazin-2-one.

On chauffe à 50°C pendant 1 heure 30 minutes un mélange de 1,76 g de 4-anilinopipéridine, 2 g du composé obtenu à l'étape C de l'EXEMPLE 8, 0,75 g d'iodure de potassium dans 10 ml de DMF. On verse le mélange réactionnel dans l'eau, extrait à l'AcOEt, lave trois fois la phase organique à l'eau, par une solution saturée de NaCl, sèche sur $MgSO_4$ et évapore sous vide le solvant. On chromatographie le résidu sur silice H en éluant au DCM puis par le mélange DCM/MeOH (96/4 ; v/v). On reprend le produit obtenu dans 10 ml d'anhydride acétique et laisse 48 heures sous agitation à TA. On concentre sous vide le mélange réactionnel, extrait le résidu à l'AcOEt, lave deux fois la phase organique par une solution à 20 % d'ammoniac dans l'eau, deux fois à l'eau, par une solution saturée de NaCl, sèche sur $MgSO_4$ et évapore sous vide le solvant. On reprend le produit obtenu par une solution saturée d'HCl gaz dans l'éther et évapore sous vide le solvant. On obtient 1,4 g du produit attendu après cristallisation dans l'AcOEt, F = 195°C.

EXEMPLE 12

Chlorure de 3-benzyl-5-(3,4-dichlorophényl)-5-[3-[4-phényl-1-azoniabicyclo [2.2.2]oct-1-yl]propyl]-tétrahydro-2*H*-1,3-oxazin-2-one, hémihydrate.

On chauffe à 50°C pendant 3 heures un mélange de 1,2 g de 4-phényl-1-azabicyclo[2.2.2]octane, 2 g du composé obtenu à l'étape C de l'EXEMPLE 8 dans 10 ml de DMF. Après refroidissement à TA, on verse le mélange réactionnel dans l'eau, extrait au DCM, lave la phase organique à l'eau, par une solution saturée de NaCl, deux fois par une solution d'HCl 2N, deux fois à l'eau, par une solution saturée de NaCl, sèche sur $MgSO_4$ et évapore sous vide le solvant. On reprend le résidu dans l'AcOEt et essore le précipité formé. On obtient 1,56 g du produit attendu, F = 235°C.

EXEMPLE 13

Chlorure de 3-benzyl-5-(3,4-dichlorophényl)-5-[3-(4-benzylpyridinio-1) propyl]-tétrahydro-2*H*-1,3-oxazin-2-one, dihydrate.

On chauffe à 90°C pendant 6 heures un mélange de 1,07 g de 4-benzylpyridine, 2 g du composé obtenu à l'étape C de l'EXEMPLE 8 dans 10 ml de DMF. Après refroidissement à TA, on verse le mélange réactionnel dans l'eau, extrait au DCM, lave la phase organique à l'eau, trois fois par une solution saturée de NaCl, sèche sur $MgSO_4$ et évapore sous vide le solvant. On chromatographie le résidu sur silice en éluant au DCM puis par le mélange DCM/MeOH (90/10 ; v/v).On dissout le produit obtenu dans un minimum d'acétone, ajoute de l'éther jusqu'à précipitation et essore le précipité formé. On obtient 0,8 g du produit attendu, F = 84°C.

EXEMPLE 14

Chlorhydrate de 4-benzyl-6-(3,4-dichlorophényl)-6-[2-(4-hydroxy-4-phényl pipérid-1-yl)éthyl]morpholin-3-one, hémihydrate.

A) 4-Benzyl-6-(3,4-dichlorophényl)-6-(2-hydroxyéthyl)morpholin-3-one.

A une solution de 1,7 g du composé obtenu à la Préparation 1.3 dans 30 ml de THF on ajoute à TA 0,51 g de *tert*-butylate de potassium puis lentement 0,77 g de bromure de benzyle et chauffe 2 heures à 50°C. On concentre sous vide le mélange réactionnel, reprend le résidu à l'eau, extrait à l'éther, lave la phase organique à l'eau, sèche sur $Na_2SO_4$ et évapore sous vide le solvant. On chromatographie le résidu sur silice H en éluant par le mélange DCM/MeOH (100/0,5 ; v/v). On dissout le produit obtenu (1 g) dans 10 ml de MeOH, acidifie à pH = 1 par ajout d'une solution saturée d'HCl gaz dans l'éther et évapore sous vide les solvants. On extrait le résidu à l'éther, lave la phase organique à l'eau, sèche sur $Na_2SO_4$ et évapore sous vide le solvant. On obtient 0,65 g du produit attendu.

Spectre de RMN du proton à 200 MHz dans DMSO-$d_6$

δ : 1,9 ppm : mt : 2H

2,7 à 3,3 ppm : mt : 2H
3,65 ppm : système AB : 2H
4,0 ppm : système AB : 2H
4,3ppm:t:1H
4,45 ppm : système AB : 2H
7,0 à 7,6 : m : 11H

On peut également obtenir ce composé en utilisant le procédé décrit ci-après. A') 4-Benzyl-6-(3,4-dichlorophényl)-6-(2-hydroxyéthyl)morpholin-3-one.

On laisse deux heures sous agitation à TA un mélange de 6 g du composé obtenu à la Préparation 1.4, 1,87 g de *tert*-butylate de potassium dans 100 ml de THF, puis ajoute lentement 2,85 g de bromure de benzyle et chauffe à reflux pendant 3 heures. On concentre sous vide le mélange réactionnel, reprend le résidu par une solution tampon pH = 2, extrait à l'éther, lave la phase organique à l'eau, sèche sur $Na_2SO_4$ et évapore sous vide le solvant. On dissout le produit obtenu dans du MeOH, ajoute 1,3 g d'hydroxyde de lithium monohydrate et laisse 30 minutes sous agitation à TA. On concentre sous vide le mélange réactionnel, extrait le résidu à l'éther, lave la phase organique à l'eau, sèche sur $Na_2SO_4$ et évapore sous vide le solvant. On chromatographie le résidu sur silice H en éluant par le mélange DCM/MeOH (100/1 ; v/v). On obtient 4,6 g du produit attendu.

B) 4-Benzyl-6-(3,4-dichlorophényl)-6-[2-(méthanesulfonyloxy)éthyl]morpholin-3-one.

On refroidit à 0°C une solution de 0,65 g du composé obtenu à l'étape précédente, 0,34 g de triéthylamine dans 30 ml de DCM et ajoute 0,25 g de chlorure de méthanesulfonyle. Après l'addition, on concentre sous vide le mélange réactionnel, extrait le résidu à l'AcOEt, lave la phase organique à l'eau, sèche sur $Na_2SO_4$ et évapore sous vide le solvant. On obtient 0,71 g du produit attendu que l'on utilise tel quel.

C) Chlorhydrate de 4-benzyl-6-(3,4-dichlorophényl)-6-(2-(4-hydroxy-4-phényl pipérid-1-yl)éthyl]morpholin-3-one, hémihydrate.

On chauffe à reflux pendant 4 heures un mélange de 0,7 g du composé obtenu à l'étape précédente, 0,378 g de 4-hydroxy-4-phénylpipéridine, 0,8 g de $K_2CO_3$ dans 5 ml d'acétonitrile. Après refroidissement à TA, on verse le mélange réactionnel dans l'eau, extrait à l'AcOEt, lave la phase organique à l'eau, sèche sur $Na_2SO_4$ et évapore sous vide le solvant. On chromatographie le résidu sur silice H en éluant par le mélange DCM/MeOH (100/3 ; v/v). On dissout le produit obtenu dans un minimum de DCM, acidifie à pH = 1 par ajout d'une solution saturée d'HCl gaz dans l'éther et essore le précipité formé. On obtient 0,43 g du produit attendu, F = 160-162°C.

EXEMPLE 15

Chlorhydrate de 6-(3,4-dichlorophényl)-6-[2-(4-hydroxy-4-phénylpipérid-1-yl) éthyl]-4-(4-phénylbenzyl)morpholin-3-one, monohydrate.

A) 6-[2-(Benzoyloxy)éthyl]-6-(3,4-dichlorophényl)-4-(4-phénylbenzyl) morpholin-3-one.

On laisse 30 minutes sous agitation à TA un mélange de 1,3 g du composé obtenu à la Préparation 1.4, 0,36 g de *tert*-butylate de potassium dans 30 ml de THF, ajoute 0,79 g de 4-(bromométhyl)biphényle et chauffe à reflux pendant 2 heures. On concentre sous vide le mélange réactionnel, extrait le résidu à l'éther, lave la phase organique à l'eau, sèche sur $Na_2SO_4$ et évapore sous vide le solvant. On chromatographie le résidu sur silice H en éluant au DCM puis par le mélange DCM/MeOH (100/0,5 ; v/v). On obtient 1,1 g du produit attendu.

Spectre de RMN du proton à 200 MHz dans DMSO-$d_6$
δ : 2,3 ppm : mt : 2H

3,55 ppm : système AB : 2H
3,9 à 4,4 ppm : m : 4H
4,5 ppm : s : 2H
7,0 à 7,8 ppm : m : 17H

B) 6-(3,4-Dichlorophényl)-6-(2-hydroxyéthyl)-4-(4-phénylbenzyl)morpholin-3-one.

On laisse 30 minutes sous agitation à TA un mélange de 1,1 g du composé obtenu à l'étape précédente, 2 ml d'une solution concentrée de NaOH dans 20 ml de MeOH. On concentre sous vide le mélange réactionnel, extrait le résidu à l'éther, lave la phase organique à l'eau, sèche sur $Na_2SO_4$ et évapore sous vide le solvant. On obtient 0,84 g du produit attendu.

Spectre de RMN du proton à 200 MHz dans DMSO-$d_6$

δ : 1,85 ppm : mt : 2H

2,7 à 3,3 ppm : mt : 2H
3,5 ppm : système AB : 2H
3,95 ppm : système AB : 2H
4,35 ppm : t : 1H
4,4 ppm : système AB : 2H
7,0 à 7,6 ppm : m : 12H

C) 6-(3,4-Dichlorophényl)-6-[2-(méthanesulfonyloxy)éthyl]-4-(4-phénylbenzyl) morpholin-3-one.

A une solution de 0,84 g du composé obtenu à l'étape précédente, 0,223 g de triéthylamine dans 30 ml de DCM, on ajoute à TA 0,252 g de chlorure de méthanesulfonyle et laisse 30 minutes sous agitation à TA. On concentre sous vide, extrait le résidu à l'éther, lave la phase organique à l'eau, sèche sur $Na_2SO_4$ et évapore sous vide le solvant. On obtient 0,89 g du produit attendu que l'on utilise tel quel.

D) Chlorhydrate de 6-(3,4-dichlorophényl)-6-[2-(4-hydroxy-4-phénylpipérid-1-yl) éthyl]-4-(4-phénylbenzyl)morpholin-3-one, monohydrate.

On chauffe à 80-100°C pendant 1 heure un mélange de 0,89 g du composé obtenu à l'étape précédente, 0,875 g de *p*-toluènesulfonate de 4-hydroxy-4-phénylpipéridine, 0,483 g de $K_2CO_3$ dans 2 ml de DMF. Après refroidissement à TA, on verse le mélange réactionnel dans l'eau, extrait à l'éther, lave la phase organique à l'eau, sèche sur $Na_2SO_4$ et évapore sous vide le solvant. On chromatographie le résidu sur silice H en éluant par le gradient du mélange DCM/MeOH de (100/2 ; v/v) à (100/5 ; v/v). On dissout le produit obtenu dans un minimum de DCM, acidifie à pH = 1 par ajout d'une solution saturée d'HCl gaz dans l'éther et essore le précipité formé. On obtient 0,45 g du produit attendu, F = 143-150°C.

EXEMPLE 16

Chlorure de 6-(3,4-dichlorophényl)-6-[2-[4-phényl-1-azoniabicyclo[2.2.2] oct-1-yl]éthyl]-4-[3,5-bis(trifluorométhyl)benzyl]morpholin-3-one, monohydrate.

A) 6-[2-(Benzoyloxy)éthyl]-6-(3,4-dichlorophényl)-4-[3,5-bis(trifluorométhyl) benzyl]morpholin-3-one.

On laisse 30 minutes sous agitation à TA un mélange de 2,1 g du composé obtenu à la Préparation 1.4, 0,616 g de *tert*-butylate de potassium dans 50 ml de THF, ajoute 1,44 g de chlorure de 3,5-bis(trifluorométhyl)benzyle et chauffe à reflux pendant 1 heure. On concentre sous vide le mélange réactionnel, reprend le résidu par une solution tampon pH = 4, extrait à l'éther, lave la phase organique à l'eau, sèche sur $Na_2SO_4$ et évapore sous vide le solvant. On chromatographie le résidu sur silice H en éluant par le mélange DCM/MeOH (100/0,5 ; v/v). On obtient 1,8 g du produit attendu.

Spectre de RMN du proton à 200 MHz dans DMSO-$d_6$

δ : 2,4 ppm : mt : 2H

4,05 ppm : système AB : 2H
4,15 à 4,6 ppm : m : 4H
4,75 ppm : système AB : 2H
7,3 à 8,2 ppm : m : 11H

B) 6-(3,4-Dichlorophényl)-6-(2-hydroxyéthyl)-4-[3,5-bis(trifluorométhyl) benzyl]morpholin-3-one.

On laisse 30 minutes sous agitation à 0°C un mélange de 1,8 g du composé obtenu à l'étape précédente, 4

ml d'une solution concentrée de NaOH dans 30 ml de MeOH puis poursuit l'agitation pendant 1 heure à TA. On concentre sous vide le mélange réactionnel, extrait le résidu à l'éther, lave la phase organique à l'eau, sèche sur $Na_2SO_4$ et évapore sous vide le solvant. On obtient 1,1 g du produit attendu.

Spectre de RMN du proton à 200 MHz dans DMSO-$d_6$
δ : 2,0 ppm : t : 2H

2,9 à 3,45 ppm : mt : 2H
3,9 ppm : système AB : 2H
4,2 ppm : système AB : 2H
4,45 ppm : t : 1H
4,7 ppm : système AB : 2H
7,1 à 8,2 ppm: m : 6H

C) 6-(3,4-Dichlorophényl)-6-[2-(méthanesulfonyloxy)éthyl]-4-[3,5-bis(trifluoro méthyl)benzyl]morpholin-3-one.

A une solution de 1,1 g du composé obtenu à l'étape précédente, 0,22 g de triéthylamine dans 30 ml de DCM, on ajoute à TA 0,25 g de chlorure de méthanesulfonyle et laisse 1 heure sous agitation. On concentre sous vide le mélange réactionnel, extrait le résidu à l'AcOEt, lave la phase organique à l'eau, sèche sur $Na_2SO_4$ et évapore sous vide le solvant. On obtient 1,2 g du produit attendu que l'on utilise tel quel.

D) Chlorure de 6-(3,4-dichlorophényl)-6-[2-[4-phényl-1-azoniabicyclo[2.2.2] oct-1-yl]éthyl]-4-[3,5-bis(trifluorométhyl) benzyl]morpholin-3-one, monohydrate.

On chauffe à 100°C pendant 1 heure un mélange de 1,2 g du composé obtenu à l'étape précédente, 0,45 g de 4-phényl-1-azabicyclo[2.2.2]octane dans 4 ml de DMF. Après refroidissement à TA, on verse le mélange réactionnel dans l'eau, extrait au DCM, lave la phase organique par une solution d'HCl 2N, par une solution saturée de NaCl, à l'eau, sèche sur $Na_2SO_4$ et évapore sous vide le solvant. On obtient 0,9 g du produit attendu après cristallisation dans le mélange DCM/éther iso/pentane, F = 160°C.

EXEMPLE 17

Chlorhydrate de 2-[2-(4-acétamido-4-phénylpipérid-1-yl)éthyl]-4-benzoyl-2-(3,4-dichlorophényl)morpholine, monohydrate.

A) 4-Benzoyl-2-(3,4-dichlorophényl)-2-(2-hydroxyéthyl)morpholine.

On refroidit à -60°C une solution de 3,6 g du composé obtenu à la Préparation 1.5, 2,9 g de triéthylamine dans 50 ml de DCM, ajoute goutte à goutte une solution de 1,83 g de chlorure de benzoyle dans 20 ml de DCM et laisse 30 minutes sous agitation. On concentre sous vide le mélange réactionnel, extrait le résidu à l'éther, lave la phase organique à l'eau, par une solution à 10 % de NaOH, sèche sur $Na_2SO_4$ et évapore sous vide le solvant. On chromatographie le résidu sur silice H en éluant par le gradient du mélange DCM/MeOH de (100/1 ; v/v) à (100/5 ; v/v). On obtient 1,2 g du produit attendu.

Spectre de RMN du proton à 200 MHz dans DMSO-$d_6$
δ : 1,95 ppm : mt : 2H

2,8 à 4,8 ppm : m : 9H
6,8 à 7,9 ppm : m : 8H

B) 4-Benzoyl-2-(3,4-dichlorophényl)-2-[2-(méthanesulfonyloxy)éthyl]morpholine.

On chauffe à 60°C une solution de 1,2 g du composé obtenu à l'étape précédente, 0,637 g de triéthylamine dans 50 ml de DCM, ajoute goutte à goutte une solution de 0,414 g de chlorure de méthanesulfonyle dans 10 ml de DCM et laisse 1 heure sous agitation à 60°C. On concentre sous vide le mélange réactionnel, extrait le résidu par un mélange AcOEt/éther (50/50 ; v/v), lave la phase organique à l'eau, par une solution saturée de $NaHCO_3$, sèche sur $Na_2SO_4$ et évapore sous vide le solvant. On obtient 1 g du produit attendu que l'on utilise tel quel.

C) Chlorhydrate de 2-[2-(4-acétamido-4-phénylpipérid-1-yl)éthyl]-4-benzoyl-2-(3,4-dichlorophényl)morpholine, monohydrate.

On chauffe à reflux pendant 4 heures un mélange de 1 g du composé obtenu à l'étape précédente, 1 g de *p*-toluènesulfonate de 4-acétamido-4-phénylpipéridine, 1 g de $K_2CO_3$ dans 10 ml d'acétonitrile et 5 ml de DMF. On concentre sous vide le mélange réactionnel, extrait le résidu à l'AcOEt, lave la phase organique à l'eau, sèche sur $Na_2SO_4$ et évapore sous vide le solvant. On chromatographie le résidu sur silice H en éluant par le mélange DCM/MeOH (100/3 ; v/v). On dissout le produit obtenu dans un minimum de DCM, acidifie à pH = 1 par ajout d'une solution saturée de HCl gaz dans l'éther et essore le précipité formé. On obtient 0,4 g du produit attendu, F = 184-187°C.

EXEMPLE 18

Chlorure de 2-(3,4-dichlorophényl)-4-[(3-isopropoxyphényl)acétyl]-2-[2-[4-phényl-1-azoniabicyclo[2.2.2]oct-1-yl]éthyl]morpholine, monohydrate.

A) 2-(3,4-Dichlorophényl)-2-(2-hydroxyéthyl)-4-[(3-isopropoxyphényl)acétyl] morpholine.

On refroidit à 0°C une solution de 1,2 g du composé obtenu à la Préparation 1.5, 0,83 g d'acide 3-isopropoxyphénylacétique, 0,86 g de triéthylamine dans 15 ml de DCM, ajoute 2 g de BOP et laisse 30 minutes sous agitation. On concentre sous vide le mélange réactionnel, extrait le résidu au DCM, lave la phase organique à l'eau, par une solution tampon pH = 2, à l'eau, sèche sur $Na_2SO_4$ et évapore sous vide le solvant. On chromatographie le résidu sur silice H en éluant par le gradient du mélange DCM/MeOH de (100/0,5 ; v/v) à (100/2 ; v/v). On obtient 0,25 g du produit attendu.

Spectre de RMN du proton à 200 MHz dans DMSO-$d_6$

$\delta$ : 1,2 ppm : d : 6H

1,8 ppm : t : 2H
2,8 à 4,8 ppm: m: 11H
6,4 à 7,8 ppm : m : 7H

B) 2-(3,4-Dichlorophényl)-4-[(3-isopropoxyphényl)acétyl]-2-[2-(méthanesulfonyl oxy)éthyl]morpholine.

A une solution de 0,25 g du composé obtenu à l'étape précédente, 0,061 g de triéthylamine dans 10 ml de DCM, on ajoute à TA 0,069 g de chlorure de méthanesulfonyle et laisse 30 minutes sous agitation. On concentre sous vide le mélange réactionnel, extrait le résidu à l'AcOEt, lave la phase organique à l'eau, par une solution saturée de NaCl, sèche sur $Na_2SO_4$ et évapore sous vide le solvant. On obtient 0,285 g du produit attendu que l'on utilise tel quel.

C) Chlorure de 2-(3,4-dichlorophényl)-4-[(3-isopropoxyphényl)acétyl]-2-[2-[4-phényl-1-azoniabicyclo[2.2.2]oct-1-yl]éthyl]morpholine, monohydrate.

On chauffe à 100°C pendant 1 heure un mélange de 0,28 g du composé obtenu à l'étape précédente, 0,15 g de 4-phényl-1-azabicyclo[2.2.2]octane dans 2 ml de DMF. On concentre sous vide le mélange réactionnel, extrait le résidu au DCM, lave la phase organique à l'eau, par une solution à 10 % d'HCl, par une solution saturée de NaCl, sèche sur $Na_2SO_4$ et évapore sous vide le solvant. On obtient 0,17 g du produit attendu après cristallisation dans le mélange acétone/éther, F = 125-130°C.

EXEMPLE 19

Chlorhydrate de 2-[3-(4-acétamido-4-phénylpipérid-1-yl)propyl]-4-benzoyl-2-(3,4-dichlorophényl)morpholine, monohydrate.

A) 4-Benzoyl-2-(3,4-dichlorophényl)-2-(3-hydroxypropyl)morpholine.

On refroidit à -10°C une solution de 2 g du composé obtenu à la Préparation 1.6, 0,83 g de triéthylamine dans 30 ml de DCM, ajoute goutte à goutte une solution de 0,97 g de chlorure de benzoyle dans 10 ml de DCM et laisse 30 minutes sous agitation. On concentre sous vide le mélange réactionnel, extrait le résidu à l'AcOEt, lave la phase organique à l'eau, par une solution à 10 % de NaOH, sèche sur $Na_2SO_4$ et évapore sous vide le solvant. On chromatographie le résidu sur silice H en éluant par le mélange DCM/MeOH (100/1 ; v/v). On obtient 1,7 g du produit attendu.

Spectre de RMN du proton à 200 MHz dans DMSO-$d_6$

δ : 1,9 à 2,2 ppm : m : 4H

3,1 à 4,6 ppm : m : 9H
7,0 à 7,9 ppm : m : 8H

B) 4-Benzoyl-2-(3,4-dichlorophényl)-2-(3-(méthanesulfonyloxy)propyl)morpholine.

On refroidit à 0°C une solution de 1,7 g du composé obtenu à l'étape précédente, 0,52 g de triéthylamine dans 30 ml de DCM, ajoute 0,6 g de chlorure de méthane sulfonyle et laisse 30 minutes sous agitation. On concentre sous vide, extrait le résidu à l'AcOEt, lave la phase organique à l'eau, sèche sur $Na_2SO_4$ et évapore sous vide le solvant. On obtient 1,7 g du produit attendu que l'on utilise tel quel.

C) Chlorhydrate de 2-[3-(4-acétamido-4-phénylpipérid-1-yl)propyl]-4-benzoyl-2-(3,4-dichlorophényl)morpholine, monohydrate.

On chauffe à reflux pendant 3 heures un mélange de 1,7 g du composé obtenu à l'étape précédente, 2,18 g de *p*-toluènesulfonate de 4-acétamido-4-phénylpipéridine, 2,5 g de $K_2CO_3$ dans 10 ml de DMF et 10 ml d'acétonitrile. On concentre sous vide le mélange réactionnel, reprend le résidu à l'eau, extrait à l'éther, lave la phase organique à l'eau, sèche sur $Na_2SO_4$ et évapore sous vide le solvant. On chromatographie le résidu sur silice H en éluant par le mélange DCM/MeOH (100/3 ; v/v). On dissout le produit obtenu dans un minimum de DCM, ajoute jusqu'à pH = 1 une solution saturée d'HCl gaz dans l'éther et essore le précipité formé. On obtient 1 g du produit attendu, F = 170-173°C.

EXEMPLE 20

Chlorhydrate de 3-benzyl-5-(3,4-dichlorophényl)-5-[2-(4-hydroxy-4-phényl pipérid-1-yl)éthyl]oxazolidin-2-one.

A) 5-[2-(Benzoyloxy)éthyl]-3-benzyl-5-(3,4-dichlorophényl)oxazolidin-2-one.

A une solution de 1,5 g du composé obtenu à la Préparation 1.7 dans 30 ml de THF on ajoute à TA 0,44 g de *tert*-butylate de potassium et laisse 1 heure sous agitation à TA. On ajoute ensuite goutte à goutte 0,667 g de bromure de benzyle et chauffe à reflux pendant 3 heures. On concentre sous vide le mélange réactionnel, extrait le résidu à l'éther, lave la phase organique par une solution tampon pH = 2, à l'eau, sèche sur $Na_2SO_4$ et évapore sous vide le solvant. On chromatographie le résidu sur silice H en éluant au DCM puis par le mélange DCM/MeOH (99,5/0,5 ; v/v). On obtient 0,9 g du produit attendu.

Spectre de RMN du proton à 200 MHz dans DMSO-$d_6$
δ : 2,6 ppm : mt : 2H

3,5 à 4,0 ppm : système AB : 2H
4,2 à 4,7 ppm : m : 4H
7,2 à 8,0 ppm : m : 13H

B) 3-Benzyl-5-(3,4-dichlorophényl)-5-(2-hydroxyéthyl)oxazolidin-2-one.

On laisse 30 minutes sous agitation à TA un mélange de 0,9 g du composé obtenu à l'étape précédente, 0,5 ml d'une solution de NaOH concentrée dans 30 ml de MeOH et 15 ml de DCM. On concentre sous vide le mélange réactionnel, extrait le résidu à l'éther, lave la phase organique à l'eau, sèche sur $Na_2SO_4$ et évapore sous vide le solvant. On obtient 0,7 g du produit attendu que l'on utilise tel quel.

C) 3-Benzyl-5-(3,4-dichlorophényl)-5-[2-(méthanesulfonyloxy)éthyl]oxazolidin-2-one.

A une solution de 0,7 g du composé obtenu à l'étape précédente, 0,21 g de triéthylamine dans 20 ml de DCM, on ajoute à TA 0,24 g de chlorure de méthane sulfonyle et laisse 1 heure sous agitation à TA. On concentre sous vide le mélange réactionnel, extrait le résidu à l'AcOEt, lave la phase organique à l'eau, sèche sur $Na_2SO_4$ et évapore sous vide le solvant. On obtient 0,76 g du produit attendu que l'on utilise tel quel.

D) Chlorhydrate de 3-benzyl-5-(3,4-dichlorophényl)-5-[2-(4-hydroxy-4-phényl pipérid-1-yl)éthyl]oxazolidin-2-one.

On chauffe à 50-60°C pendant 3 heures un mélange de 0,76 g de 4-hydroxy-4-phénylpipéridine, 0,282 g

d'iodure de potassium dans 2 ml de DMF. Après refroidissement à TA, on verse le mélange réactionnel dans l'eau, extrait à l'AcOEt, lave la phase organique à l'eau, sèche sur $Na_2SO_4$ et évapore sous vide le solvant. On chromatographie le résidu sur silice H en éluant par le gradient du mélange DCM/MeOH de (100/2 ; v/v) à (100/3 ; v/v). On dissout le produit obtenu dans un minimum de DCM, acidifie à pH = 1 par ajout d'une solution saturée d'HCl gaz dans l'éther et essore le précipité formé. On obtient 0,36 g du produit attendu, F = 223-225°C.

EXEMPLE 21

Chlorure de 5-(3,4-dichlorophényl)-5-[2-[4-phényl-1-azoniabicyclo[2.2.2] oct-1-yl]éthyl]-3-[3,5-bis(trifluorométhyl)benzyl]oxazolidin-2-one, dihydrate.

A) 5-(3,4-dichlorophényl)-5-(2-hydroxyéthyl)-3-[3,5-bis(trifluorométhyl)benzyl] oxazolidin-2-one.

On laisse 30 minutes sous agitation à TA un mélange de 1,4 g du composé obtenu à la Préparation 1.7, 0,45 g de *tert*-butylate de potassium dans 30 ml de THF, ajoute 1,05 g de chlorure de 3,5-bis(trifluorométhyl)benzyle et chauffe à reflux pendant 5 heures. On concentre sous vide, extrait le résidu à l'éther, lave la phase organique à l'eau, sèche sur $MgSO_4$ et évapore sous vide le solvant. On reprend le produit obtenu dans 30 ml de MeOH, ajoute 0,18 g d'hydroxyde de lithium monohydrate et 1 ml d'eau et laisse 2 heures sous agitation à TA. On concentre sous vide, extrait le résidu à l'éther, lave la phase organique à l'eau, sèche sur $MgSO_4$ et évapore sous vide le solvant. On chromatographie le résidu sur silice H en éluant par le mélange DCM/MeOH (100/0,5 ; v/v). On obtient 0,96 g du produit attendu.

Spectre de RMN du proton à 200 MHz dans DMSO-$d_6$
$\delta$: 2,2 ppm : mt : 2H

3,0 à 3,55 ppm : mt : 2H
3,57 à 3,9 ppm : système AB : 2H
4,35 à 4,7 ppm : m : 3H
7,2 à 8,1 ppm : m : 6H

B) 5-(3,4-dichlorophényl)-5-[2-(méthanesulfonyloxy)éthyl]-3-[3,5-bis(trifluoro méthyl)benzyl]oxazolidin-2-one.

A une solution de 0,95 g du composé obtenu à l'étape précédente, 0,21 g de triéthylamine dans 20 ml de DCM, on ajoute à TA 0,24 g de chlorure de méthane sulfonyle et laisse 1 heure sous agitation à TA. On concentre sous vide le mélange réactionnel, extrait le résidu à l'AcOEt, lave la phase organique à l'eau, sèche sur $Na_2SO_4$ et évapore sous vide le solvant. On obtient 1,1 g du produit attendu que l'on utilise tel quel.

C) Chlorure de 5-(3,4-dichlorophényl)-5-[2-[4-phényl-1-azoniabicyclo[2.2.2] oct-1-yl]éthyl]-3-[3,5-bis(trifluorométhyl) benzyl]oxazolidin-2-one, dihydrate.

On chauffe à 60°C pendant 5 heures un mélange de 0,46 g de 4-phényl-1-azabicyclo[2.2.2]octane, 1,1 g du composé obtenu à l'étape précédente dans 2 ml de DMF. Après refroidissement à TA, on verse le mélange réactionnel dans l'eau, extrait au DCM, lave la phase organique à l'eau, par une solution saturée de NaCl, par une solution d'HCl 2N, à l'eau, sèche sur $MgSO_4$ et évapore sous vide le solvant. On obtient 0,6 g du produit attendu après cristallisation dans le mélange DCM/pentane, F =127-130°C (déc).

EXEMPLE 22

Chlorhydrate de 5-[2-(4-acétamido-4-phénylpipérid-1-yl)éthyl]-1-benzyl-5-(3,4-dichlorophényl)-4-méthylpipérazin-2,3-dione.

A) 1-Benzyl-5-(3,4-dichlorophényl)-4-méthyl-5-[2-(tétrahydropyran-2-yloxy)éthyl] pipérazin-2,3-dione.

A une solution de 0,6 g du composé obtenu à la Préparation 1.8 dans 10 ml de THF on ajoute 0,2 g de *tert*-butylate de potassium et laisse 1 heure sous agitation à TA. Puis on ajoute 0,21 ml de bromure de benzyle et chauffe à 40°C pendant 2 heures. On concentre sous vide le mélange réactionnel, extrait le résidu au DCM, lave la phase organique à l'eau, sèche sur $MgSO_4$ et évapore sous vide le solvant. On obtient 0,58 g du produit attendu après cristallisation dans l'éther, F = 178°C.

B) 1-Benzyl-5-(3,4-dichlorophényl)-5-(2-hydroxyéthyl)-4-méthylpipérazin-2,3-dione.

On laisse 1 heure sous agitation un mélange de 0,56 g du composé obtenu à l'étape précédente, 10 ml de MeOH et 1 ml d'une solution saturée d'HCl gaz dans l'éther. On concentre sous vide le mélange réactionnel, reprend le résidu dans l'éther iso et essore le précipité formé. On obtient 0,45 g du produit attendu, F = 198°C.

C) 1-Benzyl-5-(3,4-dichlorophényl)-5-[2-(méthanesulfonyloxy)éthyl]-4-méthylpipérazin-2,3-dione.

On refroidit à 0°C une solution de 0,44 g du composé obtenu à l'étape précédente, 0,24 ml de triéthylamine dans 10 ml de DCM, ajoute lentement 0,12 ml de chlorure de méthanesulfonyle et laisse 15 minutes sous agitation. On lave deux fois le mélange réactionnel à l'eau, sèche la phase organique sur $MgSO_4$ et évapore sous vide le solvant. On obtient 0,5 g du produit attendu que l'on utilise tel quel.

D) Chlorhydrate de 5-[2-(4-acétamido-4-phénylpipérid-1-yl)éthyl]-1-benzyl-5-(3,4-dichlorophényl)-4-méthylpipérazin-2,3-dione.

On chauffe à 80°C pendant 8 heures 30 minutes un mélange de 0,5 g du composé obtenu à l'étape précédente, 0,85 g de *p*-toluènesulfonate de 4-acétamido-4-phénylpipéridine, 0,6 g de $K_2CO_3$ dans 5 ml de DMF. Après refroidissement à TA, on verse le mélange réactionnel dans l'eau, essore le précipité formé et le lave à l'eau. On dissout le précipité dans du DCM, sèche la phase organique sur $MgSO_4$ et évapore sous vide le solvant. On chromatographie le résidu sur silice H en éluant au DCM puis par le mélange DCM/MeOH (93/7 ; v/v). On dissout le produit obtenu dans du DCM, acidifie à pH = 1 par ajout d'une solution saturée d'HCl gaz dans l'éther et concentre sous vide. On obtient 0,105 g du produit attendu après cristallisation dans l'éther, F = 220°C (déc).

EXEMPLE 23

Dichlorhydrate de 3-[2-(4-acétamido-4-phénylpipérid-1-yl)éthyl]-1-benzoyl-3-(3,4-dichlorophényl)-4-méthylpipérazine, hémihydrate.

A) 1-Benzoyl-3-(3,4-dichlorophényl)-4-méthyl-3-[2-(tétrahydropyran-2-yloxy)éthyl] pipérazine.

A une solution de 1 g du composé obtenu à la Préparation 1.9 dans 15 ml de DCM, on ajoute 0,45 ml de triéthylamine puis on refroidit le mélange réactionnel à 0°C et ajoute goutte à goutte 0,32 ml de chlorure de benzoyle. On lave ensuite le mélange réactionnel à l'eau, par une solution de NaOH 1N, sèche la phase organique sur $MgSO_4$ et évapore sous vide le solvant. On chromatographie le résidu sur silice en éluant à l'heptane puis par le mélange heptanc/AcOEt (50/50 ; v/v). On obtient 0,95 g du produit attendu que l'on utilise tel quel.

B) Chlorhydrate de 1-benzoyl-3-(3,4-dichlorophényl)-3-(2-hydroxyéthyl)-4-méthylpipérazine.

A une solution de 0,95 g du composé obtenu à l'étape précédente dans 15 ml de MeOH on ajoute jusqu'à pH = 1 une solution saturée d'HCl gaz dans l'éther et laisse 2 heures à TA. On concentre sous vide, reprend le résidu à l'éther, essore le précipité formé et le sèche. On obtient 0,8 g du composé attendu.

Spectre de RMN du proton à 200 MHz dans DMSO-$d_6$

δ: 1,1 à 1,9 ppm : m : 2H

2,1 à 5,1 ppm : m : 12H

7,1 à 8,5 ppm : m : 8H

C) Dichlorhydrate de 3-[2-(4-acétamido-4-phénylpipérid-1-yl)éthyl]-1-benzoyl-3-(3,4-dichlorophényl)-4-méthylpipérazine, hémihydrate.

A une solution de 0,8 g du composé obtenu à l'étape précédente dans 15 ml de DCM, on ajoute 0,78 ml de triéthylamine puis refroidit à -20°C et ajoute goutte à goutte 0,25 ml de chlorure de méthanesulfonyle. On laisse 10 minutes sous agitation, puis lave le mélange réactionnel à l'eau, par une solution à 10 % de $Na_2CO_3$, sèche la phase organique sur $MgSO_4$ et filtre. On ajoute au filtrat 1,5 g de *p*-toluènesulfonate de 4-acétamido-4-phénylpipéridine et concentre sous vide. On reprend le résidu dans 10 ml de DMF, ajoute 1,5 g de $K_2CO_3$ et chauffe à 80°C pendant 2 heures 30 minutes. Après refroidissement, on verse le mélange réactionnel dans de l'eau glacée, extrait à l'AcOEt, lave la phase organique par une solution de NaOH 1N, à l'eau, sèche sur $MgSO_4$ et évapore sous vide le solvant. On

chromatographie le résidu sur silice H en éluant au DCM puis par le mélange DCM/MeOH (93/7 ; v/v). On reprend le produit obtenu par une solution saturée d'HCl gaz dans l'éther et essore le précipité formé. On obtient 0,86 g du produit attendu, F = 210°C (déc).

EXEMPLE 24

Chlorure, chlorhydrate de 3-(3,4-dichlorophényl)-1-[(3-isopropoxyphényl) acétyl]-4-méthyl-3-[2-[4-phényl-1-azoniabicyclo[2.2.2]oct-1-yl]éthyl]pipérazine.

A) 3-(3,4-dichlorophényl)-1-[(3-isopropoxyphényl)acétyl]-4-méthyl-3-[2-(tétrahydropyran-2-yloxy)éthyl]pipérazine.

A une solution de 1 g du composé obtenu à la Préparation 1.9 dans 15 ml de DCM, on ajoute 0,57 ml de triéthylamine puis 0,52 g d'acide 3-isopropoxyphénylacétique et 1,3 g de BOP. On laisse 15 minutes sous agitation à TA et concentre sous vide. On extrait le résidu à l'AcOEt, lave la phase organique à l'eau, par une solution de NaOH 1N, par une solution saturée de NaCl, sèche sur $MgSO_4$ et évapore sous vide le solvant. On chromatographie le résidu sur silice en éluant à l'heptane puis par le mélange heptane/AcOEt (50/50 ; v/v). On obtient 1,1 g du produit attendu que l'on utilise tel quel.

B) Chlorhydrate de 3-(3,4-dichlorophényl)-3-(2-hydroxyéthyl)-1-[(3-isopropoxy phényl) acétyl]-4-méthylpipérazine.

A une solution de 1,1 g du composé obtenu à l'étape précédente dans 15 ml de MeOH on ajoute jusqu'à pH = 1 une solution saturée d'HCl gaz dans l'éther et laisse 2 heures 30 minutes à TA. On concentre sous vide le mélange réactionnel, reprend le résidu à l'éther, essore le précipité formé et le sèche. On obtient 1,05 g du produit attendu.

C) Chlorure, chlorhydrate de 3-(3,4-dichlorophényl)-1-[(3-isopropoxyphényl) acétyl]-4-méthyl-3-[2-[4-phényl-1-azoniabicyclo[2.2.2]oct-1-yl]éthyl]pipérazine.

A une solution de 1,05 g du composé obtenu à l'étape précédente dans 15 ml de DCM, on ajouté 0,8 ml de triéthylamine puis refroidit à -20°C et ajoute goutte à goutte 0,22 ml de chlorure de méthanesulfonyle. Après 5 minutes, on lave le mélange réactionnel à l'eau, par une solution à 10 % de $Na_2CO_3$, on sèche la phase organique sur $MgSO_4$ et filtre. Puis on ajoute au filtrat 1 g de 4-phénylquinuclidine et concentre sous vide. On reprend le résidu dans 1 ml de DMF et chauffe à 80°C pendant 1 heure. Après refroidissement à TA, on dilue le mélange réactionnel au DCM, lave deux fois par une solution d'HCl 2N, par une solution saturée de NaCl, sèche la phase organique sur $MgSO_4$ et évapore sous vide le solvant. On chromatographie le résidu sur silice H en éluant au DCM puis par le mélange DCM/MeOH (90/10 ; v/v). On reprend le produit obtenu dans l'éther iso et essore le précipité formé. On obtient 1,05 g du produit attendu, F = 168°C (déc).

EXEMPLE 25

Chlorhydrate de 6-[2-(4-acétamido-4-phénylpipérid-1-yl)éthyl]-4-benzyl-6-(3,4-difluorophényl)morpholin-3-one, hémihydrate.

A) 6-[2-(Benzoyloxy)éthyl]-4-benzyl-6-(3,4-difluorophényl)morpholin-3-one.

On laisse 30 minutes sous agitation à TA un mélange de 1,1 g du composé obtenu à la Préparation 1.10, 0,38 g de *tert*-butylate de potassium dans 50 ml de THF, ajoute 0,51 g de bromure de benzyle et chauffe à reflux pendant 2 heures. On concentre sous vide le mélange réactionnel, extrait le résidu à l'éther, lave la phase organique à l'eau, par une solution tampon pH = 2, sèche sur $Na_2SO_4$ et évapore sous vide le solvant. On chromatographie le résidu sur silice H en éluant au DCM puis par le mélange DCM/MeOH (100/2 ; v/v). On obtient 0,6 g du produit attendu.

Spectre de RMN du proton à 200 MHz dans DMSO-$d_6$
δ : 2,3 ppm : mt : 2H

3,75 ppm : système AB : 2H
3,9 à 4,4 ppm : m : 4H
4,5 ppm : s : 2H
7,0 à 8,0 ppm : m : 13H

B) 4-Benzyl-6-(3,4-difluorophényl)-6-(2-hydroxyéthyl)morpholin-3-one.

On laisse 30 minutes sous agitation à TA un mélange de 0,6 g du composé obtenu à l'étape précédente, 0,5 ml d'une solution concentrée de NaOH dans 10 ml de MeOH. On concentre sous vide le mélange réactionnel, extrait le résidu à l'AcOEt, lave la phase organique à l'eau, sèche sur $Na_2SO_4$ et évapore sous vide le solvant. On obtient 0,42 g du produit attendu que l'on utilise tel quel.

C) 4-Benzyl-6-(3,4-difluorophényl)-6-[2-(méthanesulfonyloxy)éthyl]morpholin-3-one.

A une solution de 0,42 g du composé obtenu à l'étape précédente, 0,146 g de triéthylamine dans 20 ml de DCM, on ajoute à TA 0,137 g de chlorure de méthanesulfonyle et laisse 30 minutes sous agitation à TA. On concentre sous vide, extrait le résidu à l'éther, lave la phase organique à l'eau, sèche sur $Na_2SO_4$ et évapore sous vide le solvant. On obtient 0,49 g du produit attendu que l'on utilise tel quel.

D) Chlorhydrate de 6-[2-(4-acétamido-4-phénylpipérid-1-yl)éthyl]-4-benzyl-6-(3,4-difluorophényl)morpholin-3-one, hémihydrate.

On chauffe à 80°C pendant 2 heures un mélange de 0,49 g du composé obtenu à l'étape précédente, 0,94 g de *p*-toluènesulfonate de 4-acétamido-4-phénylpipéridine, 0,48 g de $K_2CO_3$ dans 1 ml de DMF. Après refroidissement à TA, on verse le mélange réactionnel dans l'eau, extrait à l'AcOEt, lave la phase organique à l'eau, sèche sur $Na_2SO_4$ et évapore sous vide le solvant. On chromatographie le résidu sur silice H en éluant par le mélange DCM/MeOH (100/3 ; v/v). On dissout le produit obtenu dans un mininum de DCM, acidifie à pH = 1 par ajout d'une solution saturée d'HCl gaz dans l'éther et essore le précipité formé. On obtient 0,5 g du produit attendu, F = 273-275°C.

EXEMPLE 26

Chlorure de 6-(3,4-difluorophényl)-6-[2-[4-phényl-l-azoniabicyclo[2.2.2] oct-1-yl]éthyl]-4-[3,5-bis(trifluorométhyl)benzyl]morpholin-3-one, dihydrate.

A) 6-[2-(Benzoyloxy)éthyl]-6-(3,4-difluorophényl)-4-[3,5-bis(trifluorométhyl) benzyl]morpholin-3-one.

On laisse 1 heure sous agitation à TA un mélange de 1 g du composé obtenu à la Préparation 1.10, 0,32 g de *tert*-butylate de potassium dans 20 ml de THF, puis on ajoute 0,735 g de chlorure de 3,5-bis(trifluorométhyl)benzyle et chauffe à reflux pendant 5 heures. On concentre sous vide le mélange réactionnel, extrait le résidu à l'éther, lave la phase organique à l'eau, sèche sur $MgSO_4$ et évapore sous vide le solvant. On obtient le produit attendu que l'on utilise tel quel.

B) 6-(3,4-Difluorophényl)-6-(2-hydroxyéthyl)-4-[3,5-bis(trifluorométhyl)benzyl] morpholin-3-one.

On dissout le produit obtenu à l'étape précédente dans 15 ml de MeOH, ajoute 0,235 g d'hydroxyde de lithium monohydrate et laisse 30 minutes sous agitation à TA. On concentre sous vide le mélange réactionnel, extrait le résidu à l'éther, lave la phase organique à l'eau, sèche sur $MgSO_4$ et évapore sous vide le solvant. On chromatographie le résidu sur silice H en éluant par le mélange DCM/MeOH (100/1; v/v). On obtient 0,84 g du produit attendu.

Spectre de RMN du proton à 200 MHz dans DMSO-$d_6$

$\delta$ : 1,95 ppm : t : 2H

2,95 à 3,5 ppm : mt : 2H
3,95 ppm : système AB : 2H
4,25 ppm : système AB : 2H
4,5 ppm : t : 1H
4,7 ppm : système AB : 2H
7,0 à 8,2 ppm : m : 6H

C) 6-(3,4-Difluorophényl)-6-[2-(méthanesulfonyloxy)éthyl]-4-[3,5-bis(trifluorométhyl)benzyl]morpholin-3-one.

A une solution de 0,8 g du composé obtenu à l'étape précédente, 0,2 g de triéthylamine dans 15 ml de DCM, on ajoute à TA 0,22 g de chlorure de méthanesulfonyle et laisse 30 minutes sous agitation à TA. On concentre sous vide le mélange réactionnel, extrait le résidu à l'AcOEt, lave la phase organique à l'eau, sèche sur $MgSO_4$ et évapore

sous vide le solvant. On obtient 0,9 g du produit attendu que l'on utilise tel quel.

D) Chlorure de 6-(3,4-difluorophényl)-6-[2-[4-phényl-1-azoniabicyclo[2.2.2] oct-1-yl]éthyl]-4-[3,5-bis(trifluorométhyl)benzyl]morpholin-3-one, dihydrate.

On chauffe à 80°C pendant 4 heures un mélange de 0,9 g du composé obtenu à l'étape précédente, 0,38 g de 4-phényl-1-azabicyclo[2.2.2]octane dans 2 ml de DMF. Après refroidissement à TA, on verse le mélange réactionnel dans l'eau, extrait à l'AcOEt, lave la phase organique par une solution d'HCl 2N, à l'eau, par une solution saturée de NaCl, à l'eau, sèche sur $Na_2SO_4$ et évapore sous vide le solvant. On obtient 0,41 g du produit attendu.
Spectre de RMN du proton à 200 MHz dans DMSO-$d_6$
δ : 2,0 à 2,6 ppm : m : 8H

2,7 à 3,7 ppm : m : 8H
3,9 ppm : système AB : 2H
4,1 à 5,1 ppm : m: 4H
7,0 à 8,2 ppm: m: 11H

EXEMPLE 27

Chlorhydrate de 2-[2-(4-acétamido-4-phénylpipérid-1-yl)éthyl]-4-benzoyl-2-(3,4-difluorophényl)morpholine, monohydrate.

A) 4-Benzoyl-2-(3,4-difluorophényl)-2-(2-hydroxyéthyl)morpholine.

A une solution de 0,77 g du composé obtenu à la Préparation 1.11, 0,7 g de triéthylamine dans 30 ml de DCM, on ajoute goutte à goutte à TA une solution de 0,9 g de chlorure de benzoyle dans 20 ml de DCM et laisse 1 heure sous agitation à TA. On concentre sous vide le mélange réactionnel, extrait le résidu à l'éther, lave la phase organique à l'eau et évapore sous vide le solvant. On dissout le résidu dans du MeOH, ajoute 0,53 g d'hydroxyde de lithium monohydrate et laisse 30 minutes sous agitation à TA. On concentre sous vide le mélange réactionnel, extrait le résidu au DCM, lave la phase organique à l'eau, sèche sur $MgSO_4$ et évapore sous vide le solvant. On chromatographie le résidu sur silice H en éluant par le mélange DCM/MeOH (100/0,5 ; v/v). On obtient 0,5 g du produit attendu.
Spectre de RMN du proton à 200 MHz dans DMSO-$d_6$
δ : 1,95 ppm : mt : 2H

2,8 à 4,9 ppm : m : 9H
6,8 à 7,8 ppm : m : 8H

B) 4-Benzoyl-2-(3,4-difluorophényl)-2-[2-(méthanesulfonyloxy) éthyl] morpholine.

A une solution de 0,5 g du composé obtenu à l'étape précédente, 0,17 g de triéthylamine dans 20 ml de DCM, on ajoute goutte à goutte à TA une solution de 0,19 g de chlorure de méthanesulfonyle dans 5 ml de DCM et laisse 1 heure sous agitation à TA. On concentre sous vide le mélange réactionnel, extrait le résidu à l'AcOEt, lave la phase organique à l'eau, sèche sur $Na_2SO_4$ et évapore sous vide le solvant. On obtient 0,56 g du produit attendu que l'on utilise tel quel.

C) Chlorhydrate de 2-[2-(4-acétamido-4-phénylpipérid-1-yl)éthyl]-4-benzoyl-2-(3,4-difluorophényl)morpholine, monohydrate.

On chauffe à 80°C pendant 3 heures un mélange de 0,56 g du composé obtenu à l'étape précédente, 1 g de *p*-toluènesulfonate de 4-acétamido-4-phénylpipéridine, 0,7 g de $K_2CO_3$ dans 2 ml de DMF. On verse le mélange réactionnel dans l'eau, extrait à l'AcOEt, lave la phase organique à l'eau, sèche sur $Na_2SO_4$ et évapore sous vide le solvant. On chromatographie le résidu sur silice H en éluant par le mélange DCM/MeOH (100/3 ; v/v). On dissout le produit obtenu dans un minimum de DCM, acidifie à pH = 1 par ajout d'une solution saturée de HCl gaz dans l'éther et concentre sous vide. On obtient 0,4 g du produit attendu après cristallisation dans le mélange EtOH/éther, F = 179-182°C.

EXEMPLE 28

Chlorure de 5-(3,4-difluorophényl)-5-[2-[4-phényl-1-azoniabicyclo[2.2.2] oct-1-yl]éthyl]-3-[3,5-bis(trifluorométhyl)benzyl]-

thyl)benzyl]oxazolidin-2-one, 1,5 hydrate.

A) 5-(3,4-Difluorophényl)-5-(2-hydroxyéthyl)-3-[3,5-bis(trifluorométhyl)benzyl] oxazolidin-2-one.

On laisse 1 heure sous agitation à TA un mélange de 1,5 g du composé obtenu à la Préparation 1.12, 0,48 g de *tert*-butylate de potassium dans 15 ml de THF, ajoute 1,1 g de chlorure de 3,5-bis(trifluorométhyl)benzyle et chauffe à reflux pendant 5 heures. On concentre sous vide, reprend le résidu par une solution tampon pH = 2, extrait à l'éther, lave la phase organique à l'eau, sèche sur $MgSO_4$ et évapore sous vide le solvant. On reprend le produit obtenu dans 20 ml de MeOH, ajoute 0,2 g d'hydroxyde de lithium monohydrate et laisse 30 minutes sous agitation à TA. On concentre sous vide, extrait le résidu au DCM, lave la phase organique à l'eau, sèche sur $MgSO_4$ et évapore sous vide le solvant. On chromatographie le résidu sur silice H en éluant par le mélange DCM/MeOH (100/1 ; v/v). On obtient 1,3 g du produit attendu.

Spectre de RMN du proton à 200 MHz dans DMSO-$d_6$

δ : 2,2 ppm : mt : 2H

3,0 à 3,55 ppm : mt : 2H
3,75 ppm : système AB : 2H
4,4 à 4,6 ppm : m : 3H
7,1 à 8,2 ppm : m : 6H

B) 5-(3,4-Difluorophényl)-5-[2-(méthanesulfonyloxyfithyl]-3-(3,5-bis(trifluorométhyl) benzyl]oxazolidin-2-one.

A une solution de 1,3 g du composé obtenu à l'étape précédente, 0,3 g de triéthylamine dans 20 ml de DCM, on ajoute à TA 0,34 g de chlorure de méthanesulfonyle et laisse 30 minutes sous agitation à TA. On concentre sous vide le mélange réactionnel, extrait le résidu à l'AcOEt, lave la phase organique à l'eau, sèche sur $Na_2SO_4$ et évapore sous vide le solvant. On obtient 1,1 g du produit attendu que l'on utilise tel quel.

C) Chlorure de 5-(3,4-difluorophényl)-5-[2-[4-phényl-l-azoniabicyclo[2.2.2]oct -1-yl]éthyl]-3-[3,5-bis(trifluorométhyl)benzyl]oxazolidin-2-one, 1,5 hydrate.

On chauffe à 60° c pendant 5 heures un mélange de 0,46 g de 4-phényl-1-azabicyclo[2.2.2]octane, 1,1 g du composé obtenu à l'étape précédente dans 2 ml de DMF. Après refroidissement à TA, on verse le mélange réactionnel dans l'eau, extrait au DCM, lave la phase organique à l'eau, par une solution saturée de NaCl, par une solution d'HCl 2N, à l'eau, sèche sur $MgSO_4$ et évapore sous vide le solvant. On obtient 0,6 g du produit attendu.

Spectre de RMN du proton à 200 MHz dans DMSO-$d_6$

δ : 2,1 ppm : mt : 6H

2,7 ppm : t : 2H
2,85 à 4,0 ppm : m : 10H
4,6 ppm : système AB : 2H
7,1 à 8,2 ppm:m:11H

EXEMPLE 29

Chlorhydrate de 4-[2-(4-acétamido-4-phénylpipérid-1-yl)éthyl]-1-benzyl-4-(3,4-dichlorophényl)-3-méthylimidazolidin-2-one.

A) 1-Benzyl-4-(3,4-dichlorophényl)-3-méthyl-4-[2-(tétrahydropyran-2-yloxy)éthyl] imidazolidin-2-one.

A une solution de 1,45 g du composé obtenu à la Préparation 1.13 dans 20 ml de THF, on ajoute 0,5 g de *tert*-butylate de potassium et laisse 1 heure sous agitation à TA. Puis on ajoute 0,48 ml de bromure de benzyle et laisse 30 minutes sous agitation à TA. On concentre sous vide le mélange réactionnel, extrait le résidu à l'éther, lave la phase organique à l'eau, sèche sur $MgSO_4$ et concentre sous vide le solvant. On chromatographie le résidu sur silice en éluant à l'heptane puis par le mélange heptane/AcOEt (40/60 ; v/v). On obtient 1.5 g du produit attendu que l'on utilise tel quel.

B) 1-Benzyl-4-(3,4-dichlorophényl)-4-(2-hydroxyéthyl)-3-méthylimidazolidin-2-one.

A une solution de 1,5 g du composé obtenu à l'étape précédente dans 20 ml de MeOH, on ajoute jusqu'à pH

= 1 une solution saturée d'HCl gaz dans l'éther et laisse 30 minutes sous agitation à TA. On concentre sous vide le mélange réactionnel, reprend le résidu au MeOH et évapore sous vide le solvant. On obtient 1,05 g du produit attendu après cristallisation dans l'éther iso, F = 118°C.

C) 1-Benzyl-4-(3,4'-dichlorophényl)-4-[2-(méthanesulfonyloxy)éthyl]-3-méthyl imidazolidin-2-one.

On refroidit à 0°C une solution de 0,5 g du composé obtenu à l'étape précédente, 0,34 ml de triéthylamine dans 10 ml de DCM, ajoute 0,17 ml de chlorure de méthanesulfonyle et laisse 15 minutes sous agitation. On lave le mélange réactionnel à l'eau, sèche la phase organique sur $MgSO_4$ et évapore sous vide le solvant. On obtient le produit attendu que l'on utilise tel quel.

D) Chlorhydrate de 4-[2-(4-acétamido-4-phénylpipérid-1-yl)éthyl]-1-benzyl-4-(3,4-dichlorophényl)-3-méthylimidazolidin-2-one.

On chauffe à 80°C pendant 3 heures un mélange du composé obtenu à l'étape précédente, 0,8 g de *p*-toluènesulfonate de 4-acétamido-4-phénylpipéridine, 0,8 g de $K_2CO_3$ dans 5 ml de DMF. Après refroidissement à TA, on verse le mélange réactionnel dans l'eau, extrait à l'AcOEt, lave la phase organique par une solution 1N de NaOH, par une solution saturée de NaCl, sèche sur $MgSO_4$ et évapore sous vide le solvant. On chromatographie le résidu sur silice H en éluant au DCM puis par le mélange DCM/MeOH (95/5 ; v/v). On dissout le produit obtenu dans un minimum de DCM, acidifie à pH = 1 par ajout d'une solution saturée d'HCl gaz dans l'éther et concentre sous vide. On obtient 0,3 g du produit attendu après cristallisation dans l'éther iso, F = 206°C.

EXEMPLE 30

Chlorure de 4-(3,4-dichlorophényl)-4-[2-[4-phényl-1-azoniabicyclo[2.2.2] oct-1-yl[éthyl]-3-méthyl-1-[3,5-bis(trifluorométhyl)benzyl)imidazolidin-2-one.

A) 4-(3,4-Dichlorophényl)-3-méthyl-4-[2-(tétrahydropyran-2-yloxy)éthyl]-1-[3,5-bis(trifluorométhyl)b=yl]imidazolidin-2-one.

On laisse 1 heure sous agitation à TA un mélange de 1,45 g du composé obtenu à la Préparation 1.13, 0,5 g de *tert*-butylate de potassium dans 20 ml de THF puis on ajoute 1,04 g de chlorure de 3,5-bis(trifluorométhyl)benzyle et laisse 2 heures sous agitation à TA. On concentre sous vide le mélange réactionnel, extrait le résidu à l'éther, lave la phase organique à l'eau, par une solution saturée de NaCl, sèche sur $MgSO_4$ et évapore sous vide le solvant. On chromatographie le résidu sur silice en éluant à l'heptane puis par le mélange heptane/AcOEt (50/50 ; v/v). On obtient 2,1 g du produit attendu que l'on utilise tel quel.

B) 4-(3,4-Dichlorophényl)-4-(2-hydroxyéthyl)-3-méthyl-1-[3,5-bis(trifluorométhyl) benzyl]imidazolidin-2-one.

A une solution de 2,1 g du composé obtenu à l'étape précédente dans 20 ml de MeOH, on ajoute 1 ml d'une solution saturée d'HCl gaz dans l'éther et laisse 30 minutes sous agitation à TA. On concentre sous vide, reprend le résidu au MeOH et évapore sous vide le solvant. On obtient 1,7 g du produit attendu après cristallisation dans le mélange éther iso/pentane, F = 96°C.

C) 4-(3,4-Dichlorophényl)-4-[2-(méthanesulfonyloxyfithyl]-3-méthyl-1-[3,5-bis(trifluorométhyl)benzyl]imidazolidin-2-one.

On refroidit à 0°C une solution de 0,56 g du composé obtenu à l'étape précédente, 0,3 ml de triéthylamine dans 10 ml de DCM, ajoute 0,15 ml de chlorure de méthanesulfonyle et laisse 15 minutes sous agitation. On lave le mélange réactionnel à l'eau, sèche la phase organique sur $MgSO_4$ et évapore sous vide le solvant. On obtient le produit attendu que l'on utilise tel quel.

D) Chlorure de 4-(3,4-dichlorophényl)-4-[2-[4-phényl-1-azoniabicyclo[2.2.2]oct -1-yl]éthyl]-3-méthyl-1-[3,5-bis(trifluorométhyl)benzyl]imidazolidin-2-one.

On chauffe à 80°C pendant 3 heures un mélange du composé obtenu à l'étape précédente, 0,35 g de 4-phényl-1-azabicyclo[2.2.2]octane dans 1 ml de DMF. Après refroidissement à TA, on verse le mélange réactionnel dans un mélange de 50 ml d'eau, 50 ml de DCM et 3 ml d'HCl concentré et laisse 5 minutes sous agitation. On essore le précipité

formé, le lave à l'eau, au DCM puis à l'éther et le sèche. On obtient 0,3 g du produit attendu, F = 290°C.

EXEMPLE 31

Chlorhydrate de 6-[3-(4-acétamido-4-phénylpipérid-1-yl)propyl]-4-benzyl-6-(3,4-dichlorophényl)morpholin-3-one.

A) 4-Benzyl-6-[3-(benzoyloxy)propyl]-6-(3,4-dichlorophényl)morpholin-3-one.

A une solution de 1,34 g du composé obtenu à la Préparation 1.14 dans 20 ml de THF, on ajoute 0,369 g de *tert*-butylate de potassium et laisse 1 heure sous agitation à TA. Puis on ajoute 0,564 g de bromure de benzyle et chauffe à reflux le mélange réactionnel pendant 2 heures. On concentre sous vide, reprend le résidu par une solution tampon pH = 2, extrait à l'éther, lave la phase organique à l'eau, sèche sur $MgSO_4$ et évapore sous vide le solvant. On obtient le produit attendu que l'on utilise tel quel.

B) 4-Benzyl-6-(3,4-dichlorophényl)-6-(3-hydroxypropyl)morpholin-3-one.

A une solution du composé obtenu à l'étape précédente dans 20 ml de MeOH on ajoute 0,277 g d'hydroxyde de lithium monohydrate et laisse 1 heure sous agitation à TA. On concentre sous vide, extrait le résidu à l'éther, lave la phase organique à l'eau, sèche sur $MgSO_4$ et évapore sous vide le solvant. On obtient 0,9 g du produit attendu que l'on utilise tel quel.

Spectre de RMN du proton à 200 MHz dans DMSO-$d_6$
$\delta$: 0,9 à 2,1 ppm : m : 4H

3,3 ppm : qd : 2H
3,7 ppm : système AB : 2H
4,15 ppm : système AB : 2H
4,4ppm:t:1H
4,5 ppm : système AB : 2H
7,1 à 7,8 ppm : m : 8H

C) 4-Benzyl-6-(3,4-dichlorophényl)-6-[3-(méthanesulfonyloxy)propyl]morpholin-3-one.

A une solution de 0,9 g du composé obtenu à l'étape précédente, 0,288 g de triéthylamine dans 50 ml de DCM, on ajoute à TA 0,316 g de chlorure de méthanesulfonyle et laisse 30 minutes sous agitation à TA. On concentre sous vide, extrait le résidu à l'AcOEt, lave la phase organique à l'eau, sèche sur $MgSO_4$ et évapore sous vide le solvant. On obtient 1,08 g du produit attendu que l'on utilise tel quel.

D) Chlorhydrate de 6-[3-(4-acétamido-4-phénylpipérid-1-yl)propyl]-4-benzyl-6-(3,4-dichlorophényl)morpholin-3-one.

On chauffe à 80°C pendant 3 heures un mélange de 1,08 g du composé obtenu à l'étape précédente, 1,4 g de *p*-toluènesulfonate de 4-acétamido-4-phénylpipéridine, 1,3 g de $K_2CO_3$ dans 5 ml de DMF. Après refroidissement, on verse le mélange réactionnel dans l'eau, extrait à l'AcOEt, lave la phase organique à l'eau, sèche sur $MgSO_4$ et évapore sous vide le solvant. On chromatographie le résidu sur silice H en éluant par le mélange DCM/MeOH (100/3 ; v/v). On dissout le produit obtenu dans du DCM, ajoute jusqu'à pH = 1 une solution saturée d'HCl gaz dans l'éther et évapore sous vide. On obtient 0,6 g du produit attendu après cristallisation dans le mélange EtOH/éther, F = 151-153°C.

EXEMPLE 32

Chlorhydrate de 4-(3,4-dichlorophényl)-3-méthyl-4-[2-(4-phénylpipérid-1-yl)éthyl]-1-[3,5-bis(trifluorométhyl)benzyl]imidazolidin-2-one.

On chauffe à 80°C pendant 5 heures un mélange de 0,55 g du composé obtenu à l'étape C de l'EXEMPLE 30, 0,47 g de 4-phénylpipéridine, 0,5 g de $K_2CO_3$ dans 4 ml de DMF. Après refroidissement, on verse le mélange réactionnel dans l'eau glacée, extrait à l'AcOEt, lave la phase organique par une solution de NaOH 1N, à l'eau, sèche sur $MgSO_4$ et évapore sous vide le solvant. On chromatographie le résidu sur silice H en éluant au DCM puis par le mélange DCM/MeOH (98/2 ; v/v). On dissout le produit obtenu dans l'éther, ajoute jusqu'à pH = 1 une solution saturée d'HCl gaz dans l'éther et essore le précipité formé. On obtient 0,25 g du produit attendu, F = 208°C.

EXEMPLE 33

Chlorhydrate de 4-benzyl-6-(3,4-dichlorophényl)-6-[2-[4-phényl-4-(pyrrolidin-1-ylcarbonyl)pipérid-1-yl]éthyl] morpholin-3-one, monohydrate.

On chauffe à 80°C pendant 2 heures un mélange de 0,98 g du composé obtenu à l'étape B de l'EXEMPLE 14, 0,645 g de 4-phényl-4-(pyrrolidin-1-ylcarbonyl)pipéridine, 0,69 g de $K_2CO_3$ dans 3 ml de DMF. Après refroidissement à TA, on verse le mélange réactionnel dans l'eau, extrait par le mélange AcOEt/éther, lave la phase organique à l'eau, sèche sur $MgSO_4$ et évapore sous vide le solvant. On chromatographie le résidu sur silice H en éluant par le mélange DCM/MeOH (100/2 ; v/v). On dissout le produit obtenu dans un minimum de DCM, acidifie à pH = 1 par ajout d'une solution saturée d'HCl gaz dans l'éther et essore le précipité formé. On obtient 0,42 g du produit attendu, F = 155-178°C.

EXEMPLE 34

Chlorhydrate de 6-[2-[4-(acétyl-N-méthylamino)-4-phénylpipérid-1-yl]éthyl] -4-benzyl-6-(3,4-dichlorophényl) morpholin-3-one, monohydrate.

On chauffe à 80-100°C pendant 2 heures un mélange de 1,1 g du composé obtenu à l'étape B de l'EXEMPLE 14, 1,85 g de *p*-toluènesulfonate de 4-(acétyl-N-méthylamino)-4-phénylpipéridine, 1,3 g de $K_2CO_3$ dans 3 ml de DMF. Après refroidissement à TA, on verse le mélange réactionnel dans l'eau, extrait à l'éther, lave la phase organique à l'eau, sèche sur $MgSO_4$ et évapore sous vide le solvant. On chromatographie le résidu sur silice H en éluant par le mélange DCM/MeOH de (100/1 ; v/v) à (100/4 ; v/v). On dissout le produit obtenu dans du DCM, ajoute jusqu'à pH = 1 une solution saturée d'acide chlorhydrique dans l'éther et essore le précipité formé. On obtient 0,62 g du produit attendu, F = 148-150°C.

EXEMPLE 35

Chlorhydrate de 4-benzyl-6-[2-[4-(éthoxycarbonylamino)-4-phénylpipérid-1-yl] éthyl]-6-(3,4-dichlorophényl) morpholin-3-one, monohydrate.

On chauffe à 80-100°C pendant 2 heures un mélange de 1,1 g du composé obtenu à l'étape B de l'EXEMPLE 14, 1g de trifluoroacétate de 4-(éthoxycarbonylamino)-4-phénylpipéridine, 0,7 g de $K_2CO_3$ dans 3 ml de DMF. Après refroidissement à TA, on verse le mélange réactionnel dans l'eau, laisse 30 minutes sous agitation, essore le précipité formé, le lave à l'eau, et le sèche sous vide à 60°C. On chromatographie le précipité sur silice H en éluant par le mélange DCM/MeOH de (100/1 ; v/v) à (100/4,5 ; v/v). On dissout le produit obtenu dans du DCM, ajoute jusqu'à pH = 1 une solution saturée d'acide chlorhydrique dans l'éther et essore le précipité formé. On obtient 0,52 g du produit attendu, F =148-150°C.

EXEMPLE 36

Chlorhydrate de 4-benzoyl-2-(3,4-difluorophényl)-2-[2-[4-(N',N'-diméthyl uréido)-4-phénylpipérid-1-yl]éthyl] morpholine, monohydrate, isomère (-).

A) 4-Benzoyl-2-[2-(benzoyloxy)éthyl]-2-(3,4-difluorophényl)morpholine, isomère (-).

On refroidit à 0°C une solution de 8,9 g du composé obtenu à la Préparation 1.17 (isomère (-)), 3 g de triéthylamine dans 100 ml de DCM, ajoute goutte à goutte une solution de 3,59 g de chlorure de benzoyle dans 20 ml de DCM et laisse 30 minutes sous agitation. On concentre sous vide le mélange réactionnel, extrait le résidu à l'éther, lave la phase organique à l'eau, sèche sur $MgSO_4$ et évapore sous vide le solvant. On chromatographie le résidu sur silice H en éluant par le mélange DCM/MeOH (100/0,5 ; v/v). On obtient 9,3 g du produit attendu.

$$\alpha_D^{20} = -4{,}7^\circ \ (c = 1 \ ; \ MeOH)$$

B) 4-Benzoyl-2-(3,4-difluorophényl)-2-(2-hydroxyéthyl)morpholine, isomère (-).

A une solution de 11,5 g du composé obtenu à l'étape précédente dans 60 ml de MeOH, on ajoute à TA et goutte à goutte 6,6 g d'une solution de NaOH à 30 % dans l'eau et laisse 1 heure sous agitation. On concentre sous vide le mélange réactionnel, reprend le résidu à l'eau, extrait à l'éther, lave la phase organique à l'eau, sèche sur $MgSO_4$

et évapore sous vide le solvant. On chromatographie le résidu sur silice H en éluant par le mélange DCM/MeOH de (100/1 ; v/v) à (100/5 ; v/v). On obtient 8,4 g du produit attendu après cristallisation dans l'éther iso, F = 80°C.

$$\alpha_D^{20} = -56{,}1° \text{ (c = 1 ; MeOH)}$$

C) 4-Benzoyl-2-(3,4-difluorophényl)-2-[2-(méthanesulfonyloxy)éthyl]morpholine, isomère (-).

A une solution de 1 g du composé obtenu à l'étape précédente, 0,3 g de triéthylamine dans 25 ml de DCM, on ajoute à TA et goutte à goutte une solution de 0,36 g de chlorure de méthanesulfonyle dans 3 ml de DCM et laisse 30 minutes sous agitation à TA. On concentre sous vide le mélange réactionnel, extrait le résidu à l'éther, lave la phase organique à l'eau, par une solution saturée de $NaHCO_3$, à l'eau, sèche sur $MgSO_4$ et évapore sous vide le solvant. On obtient 1,05 g du produit attendu.

$$\alpha_D^{20} = -36{,}1° \text{ (c = 1 ; MeOH)}$$

D) Chlorhydrate de 4-benzoyl-2-(3,4-difluorophényl)-2-[2-[4-(N',N'-diméthyl uréido)-4-phénylpipérid-1-yl]éthyl]morpholine, monohydrate, isomère (-).

On dissout 2 g du composé obtenu à la Préparation 2.2 dans de l'eau, alcalinise par ajout d'une solution de NaOH concentrée, extrait au DCM, sèche la phase organique sur $MgSO_4$, évapore sous vide le solvant et obtient 1,08 g de base libre. On reprend le produit obtenu dans 3 ml de DMF, ajoute 0,95 g du composé obtenu à l'étape précédente et chauffe à 80°C pendant 3 heures. Après refroidissement, on verse le mélange réactionnel dans l'eau, essore le précipité formé et le lave à l'eau. On dissout le précipité dans du DCM, lave la phase organique par une solution de NaOH à 10 %, sèche sur $MgSO_4$ et évapore sous vide le solvant. On chromatographie le résidu sur silice H en éluant par le mélange DCM/MeOH de (100/1 ; v/v) à (100/7,5 ; v/v). On dissout le produit obtenu dans du DCM, ajoute jusqu'à pH = 1 une solution saturée d'acide chlorhydrique dans l'éthere et évapore sous vide les solvants. On obtient 0,3 g du produit attendu après cristallisation dans le mélange DCM/pentane, F = 168-172°C.

$$\alpha_D^{20} = -22{,}2° \text{ (c = 1 ; MeOH)}$$

EXEMPLE 37

Chlorhydrate de 4-benzoyl-2-(3,4-difluorophényl)-2-[2-[4-(N',N'-diméthyl uréido)-4-phénylpipérid-1-yl]éthyl] morpholine, hémihydrate, isomère (+).

A) 4-Benzoyl-2-[2-benzoyloxy)éthyl]-2-(3,4-difluorophényl)morpholine, isomère (+).

On refroidit à 0°C une solution de 20 g du composé obtenu à la Préparation 1.18 (isomère (+)), 8 ml de triéthylamine dans 200 ml de DCM, ajoute goutte à goutte 6 ml de chlorure de benzoyle et laisse 15 minutes sous agitation. On concentre sous vide le mélange réactionnel, extrait le résidu à l'éther, lave la phase organique à l'eau, par une solution d'HCl 1N, par une solution de $Na_2CO_3$ à 10 %, sèche sur $MgSO_4$ et évapore sous vide le solvant. On obtient 22 g du produit attendu sous forme d'huile.

$$\alpha_D^{20} = +4{,}7° \text{ (c = 1 ; MeOH)}$$

B) 4-Benzoyl-2-(3,4-difluorophényl)-2-(2-hydroxyéthyl)morpholine, isomère (+).

On laisse 1 heure sous agitation à TA un mélange de 22 g du composé obtenu à l'étape précédente, 9 ml d'une solution de NaOH à 30 % dans l'eau et 200 ml d'EtOH 95. On concentre sous vide le mélange réactionnel, extrait le résidu à l'éther, lave la phase organique trois fois à l'eau, sèche sur $MgSO_4$ et évapore sous vide le solvant. On obtient 13,7 g du produit attendu après cristallisation dans le mélange éther/éther iso, F = 80°C.

$$\alpha_D^{20} = +59{,}5° \text{ (c = 1 ; MeOH)}$$

C) 4-Benzoyl-2-(3,4-difluorophényl)-2-[2-(méthanesulfonyloxy)éthyl]morpholine, isomère (+).

On refroidit à 0°C une solution de 1 g du composé obtenu à l'étape précédente, 0,45 ml de triéthylamine dans 10 ml de DCM, ajoute 0,25 ml de chlorure de méthanesulfonyle et laisse 15 minutes sous agitation. On lave le mélange réactionnel à l'eau, par une solution de $Na_2CO_3$ à 10 %, sèche la phase organique sur $MgSO_4$ et évapore sous vide le solvant. On obtient 1,2 g du produit attendu sous forme d'huile.

D) Chlorhydrate de 4-benzoyl-2-(3,4-difluorophényl)-2-[2-[4-(N',N'-diméthyl uréido)-4-phénylpipérid-1-yl]éthyl]morpholine, hémihydrate, isomère (+).

On chauffe à 80°C pendant 3 heures un mélange de 1,2 g du composé obtenu à l'étape précédente, 3 g du composé obtenu à la Préparation 2.2, 2 g de $K_2CO_3$ dans 10 ml de DMF. On verse le mélange réactionnel dans de l'eau glacée, extrait à l'AcOEt, lave la phase organique par une solution de NaOH 1N, par une solution saturée de NaCl, sèche sur $MgSO_4$ et évapore sous vide le solvant. On chromatographie le résidu sur silice H en éluant au DCM puis par le mélange DCM/MeOH (96/4 ; v/v). On dissout le produit obtenu dans du DCM, ajoute jusqu'à pH = 1 une solution saturée d'HCl gaz dans l'éther et évapore sous vide. On obtient 0,66 g du produit attendu après cristallisation dans l'éther iso.

$$\alpha_D^{20} = +22{,}2° \text{ (c = 1 ; MeOH)}$$

EXEMPLE 38

Chlorhydrate de 5-(3,4-dichlorophényl)-5-[2-(4-phénylpipérid-1-yl)éthyl]-3-[3,5-bis(trifluorométhyl)benzyl]oxazolidin-2-one.

On chauffe à 80°C pendant 3 heures un mélange de 1,5 g du composé obtenu à l'étape B de l'EXEMPLE 21, 1,03 g de 4-phénylpipéridine dans 3 ml de DMF. Après refroidissement, on verse le mélange réactionnel dans l'eau, extrait à l'AcOEt, lave la phase organique à l'eau, sèche sur $MgSO_4$ et évapore sous vide le solvant. On chromatographie le résidu sur silice H en éluant par le mélange DCM/MeOH (100/1 ; v/v). On dissout le produit obtenu dans du DCM, ajoute jusqu'à pH = 1 une solution saturée d'HCl gaz dans l'éther et évapore sous vide. On obtient 0,7 g du produit attendu après cristallisation dans le mélange DCM/pentane, F = 112-114°C.

EXEMPLE 39

Chlorhydrate de 5-[2-(4-acétamido-4-phénylpipérid-1-yl)éthyl]-5-(3,4-dichlorophényl)-3-[3,5-bis(trifluorométhyl)benzyl]oxazolidin-2-one, monohydrate.

On chauffe à 80°C pendant 3 heures un mélange de 1,5 g du composé obtenu à l'étape B de l'EXEMPLE 21, 1,5 g de *p*-toluènesulfonate de 4-acétamido-4-phénylpipéridine, 1,6 g de $K_2CO_3$ dans 4 ml de DMF. Après refroidissement, on verse le mélange réactionnel dans l'eau, extrait par le mélange AcOEt/éther (50/50 ; v/v), lave la phase organique à l'eau, sèche sur $MgSO_4$ et évapore sous vide le solvant. On chromatographie le résidu sur silice H en éluant par le mélange DCM/MeOH (100/3 ; v/v). On dissout le produit obtenu dans du DCM, ajoute jusqu'à pH = 1 une solution saturée d'HCl gaz dans l'éther et évapore sous vide. On obtient 0,66 g du produit attendu après cristallisation dans le mélange DCM/pentane, F = 165-170°C.

EXEMPLE 40

Chlorure de 5-[2-[4-benzyl-1-azoniabicyclo[2.2.2]oct-1-yl]éthyl]-5-(3,4-dichlorophényl)-3-[3,5-bis(trifluorométhyl)benzyl]oxazolidin-2-one, monohydrate.

On chauffe à 80°C pendant 2 heures un mélange de 1,4 g du composé obtenu à l'étape B de l'EXEMPLE 21, 0,6 g de 4-benzylquinuclidine dans 3 ml de DMF. Après refroidissement à TA, on verse le mélange réactionnel dans l'eau, extrait à l'AcOEt, lave la phase organique à l'eau, par 300 ml d'une solution d'HCl à 10 %, par 300 ml d'une solution saturée de NaCl, sèche sur $MgSO_4$ et évapore sous vide le solvant. On obtient 1,35 g du produit attendu après cristallisation dans l'AcOEt, F = 172-175°C.

EXEMPLE 41

Chlorhydrate de 4-benzyl-6-(3,4-dichlorophényl)-6-[2-[4-phényl-4-(pyrrolidin-1-ylcarbonylamino)pipérid-1-yl] éthyl]morpholin-3-one, monohydrate.

On chauffe à 80-100°C pendant 3 heures un mélange de 1,1 g du composé obtenu à l'étape B de l'EXEMPLE 14, 1,2 g de benzènesulfonate de 4-phényl-4-(pyrrolidin-1-ylcarbonylamino)pipéridine, 0,95 g de $K_2CO_3$ dans 3 ml de DMF. Après refroidissement à TA, on verse le mélange réactionnel dans l'eau, essore le précipité formé, le lave à l'eau et le sèche sous vide. On chromatographie le précipité sur silice H en éluant par le mélange DCM/MeOH de (100/2 ; v/v) à (100/3 ; v/v). On dissout le produit obtenu dans un minimum de DCM, acidifie à pH = 1 par ajout d'une solution saturée d'HCl gaz dans l'éther et essore le précipité formé. On obtient 0,54 g du produit attendu, F = 158-160°C.

EXEMPLE 42

Dichlorhydrate de 4-benzyl-6-(3,4-dichlorophényl)-6-[2-[4-(2-diméthylamino thiazol-4-yl)-4-phénylpipérid-1-yl] éthyl]morpholin-3-one.

On prépare ce composé selon le mode opératoire décrit à l'EXEMPLE 41 à partir de 1,1 g du composé obtenu à l'étape B de l'EXEMPLE 14, 1,3 g de *p*-toluènesulfonate de 4-[2-(diméthylamino)thiazol-4-yl]-4-phénylpipéridine, 0,97 g de $K_2CO_3$ et 4 ml de DMF. On obtient 0,76 g du produit attendu, F = 155-160°C.

EXEMPLE 43

Chlorhydrate de 4-benzoyl-2-(3,4-dichlorophényl)-2-[2-[4-(morpholin-4-ylcarbonylamino)-4-phénylpipérid-1-yl]éthyl]morpholine, monohydrate.

A) 4-Benzoyl-2-[2-(benzoyloxy)éthyl]-2-(3,4-dichlorophényl)morpholine.

A une solution de 5 g du composé obtenu à la Préparation 1.19, 1,4 g de triéthylamine dans 100 ml de DCM, on ajoute à TA et goutte à goutte 2,03 g de chlorure de benzoyle et laisse 30 minutes sous agitation à TA. On concentre sous vide le mélange réactionnel, extrait le résidu à l'éther, lave la phase organique à l'eau, sèche sur $MgSO_4$ et évapore sous vide le solvant. On chromatographie le résidu sur silice H en éluant par le mélange DCM/MeOH (100/1 ; v/v). On obtient 4 g du produit attendu.

B) 4-Benzoyl-2-(3,4-dichlorophényl)-2-(2-hydroxyéthyl)morpholine.

On laisse 30 minutes sous agitation à TA un mélange de 4 g du composé obtenu à l'étape précédente, 0,7 g d'hydroxyde de lithium monohydrate dans 50 ml de MeOH. On concentre sous vide le mélange réactionnel, extrait le résidu à l'éther, lave la phase organique à l'eau, sèche sur $MgSO_4$ et évapore sous vide le solvant. On obtient 3,1 g du produit attendu.

On prépare également ce composé en suivant le mode opératoire décrit à l'étape A de l'EXEMPLE 17.

C) 4-Benzoyl-2-(3,4-dichlorophényl)-2-[2-(méthanesulfonyloxy)éthyl] morpholine.

A une solution de 3,1 g du composé obtenu à l'étape précédente, 0,95 g de triéthylamine dans 50 ml de DCM, on ajoute à TA et goutte à goutte 1,07 g de chlorure de méthanesulfonyle et laisse 1 heure sous agitation à TA. On concentre sous vide le mélange réactionnel, extrait le résidu par un mélange AcOEt/éther (50/50 ; v/v), lave la phase organique à l'eau, sèche sur $MgSO_4$ et évapore sous vide le solvant. On obtient 3,7 g du produit attendu que l'on utilise tel quel.

On prépare également ce composé en suivant le mode opératoire décrit à l'étape B de l'EXEMPLE 17.

D) Chlorhydrate de 4-benzoyl-2-(3,4-dichlorophényl)-2-[2-[4-(morpholin-4-ylcarbonylamino)-4-phénylpipérid-1-yl]éthyl] morpholine, monohydrate.

On chauffe à 100°C pendant 3 heures un mélange de 1,3 g du composé obtenu à l'étape précédente, 1,5 g de *p*-toluènesulfonate de 4-(morpholin-4-ylcarbonylamino)-4-phénylpipéridine, 1,2 g de $K_2CO_3$ dans 3 ml de DMF. Après refroidissement à TA, on verse le mélange réactionnel dans l'eau, essore le précipité formé et le sèche sous vide. On chromatographie le précipité sur silice H en éluant par le mélange DCM/MeOH de (100/2 ; v/v) à (100/3 ; v/v). On dissout le produit obtenu dans un minimum de DCM, acidifie à pH = 1 par ajout d'une solution saturée d'HCl gaz dans l'éther et concentre sous vide. On obtient 0,13 g du produit attendu après cristallisation dans l'éther iso.

Spectre de RMN du proton à 200 MHz dans DMSO-$d_6$.

δ: 1,8 à 4,0 ppm : m : 26H

6,Sppm:s:1H
6,9 à 7,8 ppm : m : 13H
10,65 ppm : s : 1H

EXEMPLE 44

Dichlorhydrate de 4-benzoyl-2-(3,4-dichlorophényl)-2-[2-[4-phényl-4-(pyrrolidin-1-ylaminocarbonyl)pipérid-1-yl]éthyl]morpholine, dihydrate.

On chauffe à 100°C pendant 4 heures un mélange de 1,3 g du composé obtenu à l'étape C de l'EXEMPLE 43, 1,46 g de benzènesulfonate de 4-phényl-4-(pyrrolidin-1-ylaminocarbonyl)pipéridine, 1,17 g de $K_2CO_3$ dans 5 ml d'acétonitrile et 5 ml de DMF. Après refroidissement à TA, on verse le mélange réactionnel dans l'eau, extrait à l'AcOEt, lave deux fois la phase organique à l'eau, par une solution saturée de NaCl, sèche sur $MgSO_4$ et évapore sous vide le solvant. On chromatographie le résidu sur silice H en éluant au DCM puis par le mélange DCM/MeOH (97/3 ; v/v). On dissout le produit obtenu dans l'acétone, ajoute jusqu'à pH = 1 une solution saturée d'acide chlorhydrique dans l'éther et essore le précipité formé. On obtient 1,01 g du produit attendu, F = 170-174°C.

EXEMPLE 45

Chlorhydrate de 4-benzoyl-2-(3,4-difluorophényl)-2-[2-[4-(méthoxycarbonyl amino)-4-phénylpipérid-1-yl]éthyl] morpholine, monohydrate, isomère (+).

On chauffe à 80-100°C pendant 2 heures un mélange de 1,2 g du composé obtenu à l'étape C de l'EXEMPLE 37, 1,3 g de *p*-toluènesulfonate de 4-(méthoxycarbonyl amino)-4-phénylpipéridine, 1,2 g de $K_2CO_3$ dans 3 ml de DMF. Après refroidissement à TA, on verse le mélange réactionnel dans l'eau, essore le précipité formé et le sèche sous vide. On chromatographie le précipité sur silice H en éluant par le mélange DCM/MeOH de (100/1,5 ; v/v) à (100/3 ; v/v). On dissout le produit obtenu dans du DCM, ajoute jusqu'à pH = 1 une solution saturée d'acide chlorhydrique dans l'éther et essore le précipité formé. On obtient 0,8 g du produit attendu, F =170°C.

$$\alpha_D^{20} = +26{,}7^\circ \ (c = 1 \ ; \ MeOH)$$

EXEMPLE 46

Chlorhydrate de 4-benzoyl-2-(3,4-difluorophényl)-2-[2-(4-phényl-4-(pyrrolidin-1-ylcarbonylamino)pipérid-1-yl]éthyl]morpholine, monohydrate, isomère (+).

On prépare ce composé selon le mode opératoire décrit à l'EXEMPLE 45 à partir de 1,2 g du composé obtenu à l'étape C de l'EXEMPLE 37, 1,4 g de benzènesulfonate de 4-phényl-4-(pyrrolidin-1-ylcarbonylamino)pipéridine, 1,2 g de $K_2CO_3$ et 2 ml de DMF. On obtient 0,3 g du produit attendu, F = 163-168°C.

$$\alpha_D^{20} = +22{,}8^\circ \ (c = 1 \ ; \ MeOH)$$

EXEMPLE 47

Chlorhydrate de 4-benzoyl-2-[2-[4-benzyl-4-(pyrrolidin-1-ylcarbonylamino) pipérid-1-yl]éthyl]-2-(3,4-difluorophényl)morpholine, 1,5 hydrate, isomère (+).

On prépare ce composé selon le mode opératoire décrit à l'EXEMPLE 45 à partir de 1,2 g du composé obtenu à l'étape C de l'EXEMPLE 37, 1,5 g de *p*-toluènesulfonate de 4-benzyl-4-(pyrrolidin-1-ylcarbonylamino)pipéridine, 1,2 g de $K_2CO_3$ et 3 ml de DMF. On obtient 0,83 g du produit attendu, F = 140°C.

$$\alpha_D^{20} = +28,4° \ (c = 1 ; MeOH)$$

EXEMPLE 48

Dichlorhydrate de 4-benzoyl-2-(3,4-difluorophényl)-2-(2-(4-(2-(diméthylamino) thiazol-4-yl]-4-phénylpipérid-1-yl]éthyl]morpholine, hémihydrate, isomère (+).

On prépare ce composé selon le mode opératoire décrit à l'EXEMPLE 45 à partir de 1,2 g du composé obtenu à l'étape C de l'EXEMPLE 37, 1,55 g de *p*-toluènesulfonate de 4-[2-(diméthylamino)thiazol-4-yl]-4-phénylpipéridine, 1,2 g de $K_2CO_3$ et 3 ml de DMF. On obtient 0,7 g du produit attendu, F = 147°C.

$$\alpha_D^{20} = +20,3° \ (c = 1 ; MeOH)$$

EXEMPLE 49

Dichlorhydrate de 2-[2-[4-(2-amino-1,3,4-oxadiazol-5-yl)-4-phénylpipérid-1-yl]éthyl]-4-benzoyl-2-(3,4-difluorophényl)morpholine, isomère (+).

On prépare ce composé selon le mode opératoire décrit à l'EXEMPLE 45 à partir de 1,2 g du composé obtenu à l'étape C de l'EXEMPLE 37, 1,4 g de *p*-toluènesulfonate de 4-(2-amino-1,3,4-oxadiazol-5-yl)-4-phénylpipéridine, 1,2 g de $K_2CO_3$ et 3 ml de DMF. On obtient 0,6 g du produit attendu, F = 153°C.

$$\alpha_D^{20} = +27,2° \ (c = 1 ; MeOH)$$

EXEMPLE 50

Chlorure de 5-(3,4-difluorophényl)-5-[2-[4-phényl-1-azoniabicyclo[2.2.2]oct-1-yl] éthyl]-3-[3,5-bis(trifluorométhyl)benzyl]oxazolidin-2-one, monohydrate, isomère (-).

A) 5-[2-(Benzoyloxy)éthyl]-5-(3,4-difluorophényl)-3-[3,5-bis(trifluorométhyl)benzyl] oxazolidin-2-one, isomère (-).

A une solution de 3,5 g du composé obtenu à la Préparation 1.20 (isomère (-)) dans un mélange de 50 ml de THF et 10 ml de DMF, on ajoute à TA 1,2 g de *tert*-butylate de potassium et laisse 30 minutes sous agitation à TA. Puis on ajoute 2,7 g de chlorure de 3,5-bis(trifluorométhyl)benzyle et chauffe à 60°C pendant 6 heures. On verse le mélange réactionnel sur 200 ml de tampon pH = 2, extrait à l'éther, lave la phase organique à l'eau, sèche sur $MgSO_4$ et évapore sous vide le solvant. On chromatographie le résidu sur silice H en éluant au DCM. On obtient 3,6 g du produit attendu.

B) 5-(3,4-Difluorophényl)-5-(2-hydroxyéthyl)-3-[3,5-bis(trifluorométhyl)benzyl) oxazolidin-2-one, isomère (-).

On laisse 2 heures sous agitation à TA un mélange de 3,6 g du composé obtenu à l'étape précédente, 0,32 g d'hydroxyde de lithium monohydrate dans 50 ml de MeOH et 5 ml d'eau. On concentre sous vide le mélange réactionnel, reprend le résidu à l'eau, extrait à l'éther, lave deux fois la phase organique à l'eau, sèche sur $MgSO_4$ et évapore sous vide le solvant. On chromatographie le résidu sur silice H en éluant au DCM puis par le mélange DCM/MeOH (98/2 ;

v/v). On obtient 2,6 g du produit attendu.

C) 5-(3,4-Difluorophényl)-5-[2-(méthanesulfonyloxy)éthyl]-3-[3,5-bis(trifluorométhyl) benzyl]oxazolidin-2-one, isomère (-).

On refroidit à 0°C une solution de 2,6 g du composé obtenu à l'étape précédente dans 50 ml de DCM, ajoute 1,14 ml de triéthylamine puis 0,62 ml de chlorure de méthanesulfonyle et laisse 10 minutes sous agitation. On concentre sous vide le mélange réactionnel, extrait le résidu à l'éther, lave deux fois la phase organique à l'eau, sèche sur $MgSO_4$ et évapore sous vide le solvant. On obtient 3 g du produit attendu que l'on utilise tel quel.

D) Chlorure de 5-(3,4-difluorophényl)-5-[2-(4-phényl-1-azoniabicyclo[2.2.2] oct-1-yl]éthyl]-3-(3,5-bis(trifluorométhyl) benzyl)oxazolidin-2-one, monohydrate, isomère (-).

On chauffe à 90°C pendant 2 heures un mélange de 2 g du composé obtenu à l'étape précédente, 1 g de 4-phényl-1-azabicyclo[2.2.2]octane dans 1 ml de DMF. Après refroidissement à TA, on verse le mélange réactionnel dans une solution d'HCl 1N, extrait au DCM, lave la phase organique par une solution d'HCl 1N, par une solution saturée de NaCl, sèche sur $MgSO_4$ et évapore sous vide le solvant. On chromatographie le résidu sur silice H en éluant au DCM puis par le mélange DCM/MeOH (90/10 ; v/v). On obtient 1,45 g du produit attendu après cristallisation dans l'éther iso, F = 130°C (dec).

$$\alpha_D^{20} = -36{,}2^\circ \ (c = 1 \ ; \ MeOH)$$

EXEMPLE 51

Fumarate de 4-benzoyl-2-(3,4-difluorophényl)-2-[2-[4-(N',N'-diméthyluréido)-4-phénylpipérid-1-yl]éthyl]morpholine.

A) 4-Benzoyl-2-(3,4-difluorophényl)-2-(formylméthyl)morpholine.

On refroidit à -78°C, sous atmosphère d'azote, une solution de 0,32 ml de chlorure d'oxalyle dans 7,3 ml de DCM, ajoute goutte à goutte une solution de 0,51 ml de DMSO dans 3,5 ml de DCM, puis une solution de 1 g du composé obtenu à l'étape A de l'EXEMPLE 27 dans 7,5 ml de DCM et 0,7 ml de DMSO. On laisse 30 minutes sous agitation à -60°C le mélange réactionnel, puis ajoute 2 ml de triéthylamine et laisse sous agitation en laissant remonter la température à TA. On lave le mélange réactionnel par une solution d'HCl 1N, par une solution saturée de $Na_2CO_3$, à l'eau, sèche la phase organique sur $MgSO_4$ et évapore sous vide le solvant. On obtient 1,03 g du produit attendu sous forme d'huile.

Spectre de RMN du proton à 200 MHz dans DMSO-$d_6$

δ : 2,7 à 4,8 ppm : m : 8H

7,0 à 8,0 ppm : m : 8H
9,6 ppm : s : 1H

B) Fumarate de 4-benzoyl-2-(3,4-difluorophényl)-2-[2-[4-(N',N'-diméthyluréido)-4-phénylpipérid-1-yl]éthyl]morpholine.

A une solution de 0,72 g de 4-(N',N'-diméthyluréido)-4-phénylpipéridine (base libre), 0,16 ml d'acide acétique dans 10 ml de MeOH, on ajoute à TA une solution de 1 g du composé obtenu à l'étape précédente dans 10 ml de MeOH et laisse 5 minutes sous agitation à TA. Puis on ajoute en une fois une solution de 0,19 g de cyanoborohydrure de sodium dans 10 ml de MeOH et laisse 4 heures sous agitation à TA. On neutralise le mélange réactionnel par ajout d'une solution à 10 % de $Na_2CO_3$, extrait au DCM, sèche la phase organique sur $MgSO_4$ et évapore sous vide le solvant. On chromatographie le résidu sur silice H en éluant au DCM puis par le mélange DCM/MeOH (96/4 ; v/v). On dissout le produit obtenu (1,1 g) dans 20 ml d'acétone, ajoute 0,24 g d'acide fumarique et laisse 1 heure sous agitation à TA. On essore le produit cristallisé formé, le lave à l'acétone puis à l'éther. On obtient 1,35 g du produit attendu, F = 204°C.

En procédant selon le mode opératoire décrit à l'EXEMPLE 34 à partir du composé obtenu à l'étape B de l'EXEMPLE 14 et des pipéridines décrites dans les Préparations, on prépare les composés selon l'invention rassemblés dans le TABLEAU I ci-après.

TABLEAU 1

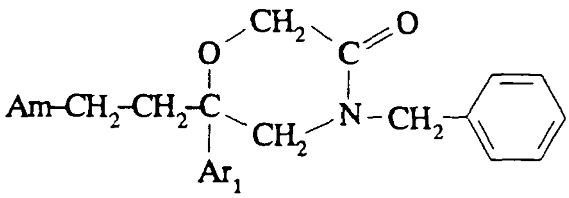

(I)

| Exemples | Am | $Ar_1$ | Sel, solvate ; F°C ou RMN ; solvant de recristallisation |
|---|---|---|---|
| 52 | 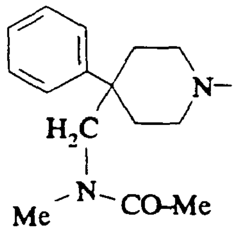 | 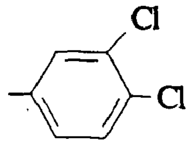 | HCl, 2,5 $H_2O$ 138-140 pentane/éther |
| 53 | 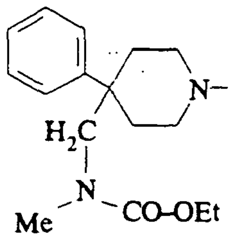 | 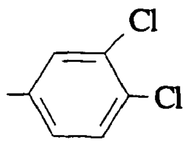 | HCl, 2 $H_2O$ 128 (dèc) pentane/éther |
| 54 | 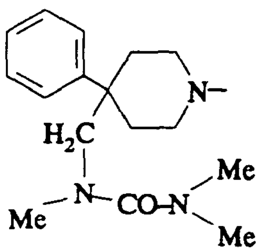 | 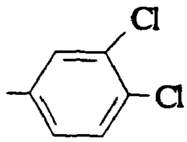 | HCl, 2,5 $H_2O$ 138-140 pentane/éther |
| 55 | 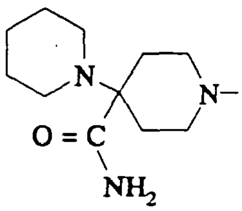 | 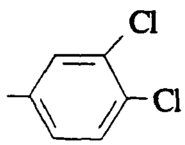 | 2 HCl, 2 $H_2O$ 198 (dèc) pentane/éther iso |
| 56 | 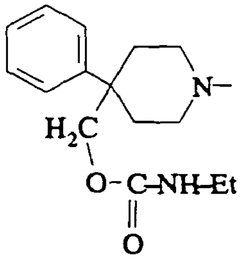 | 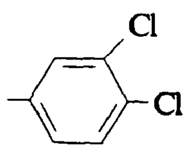 | HCl, 1 $H_2O$ 130-134 pentane |

(suite)

| Exemples | Am | $Ar_1$ | Sel, solvate ; F°C ou RMN ; solvant de recristallisation |
|---|---|---|---|
| 57 | 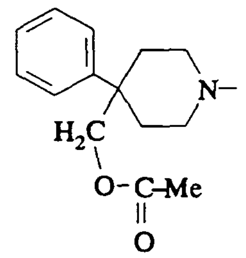 | 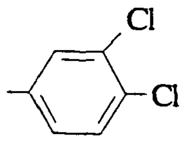 | HCl, 1 $H_2O$ RMN pentane/éther |
| 58 | 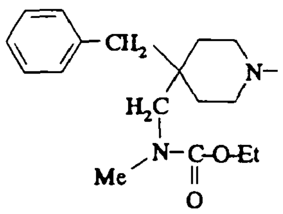 | 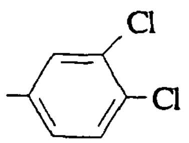 | HCl, 0,5 $H_2O$ RMN DCM/éther |
| 59 | 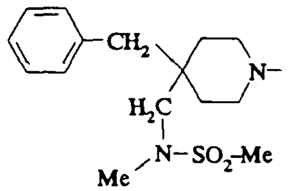 | 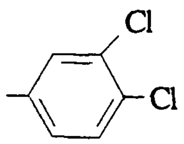 | HCl, 1 $H_2O$ RMN DCM/éther |
| 60 | 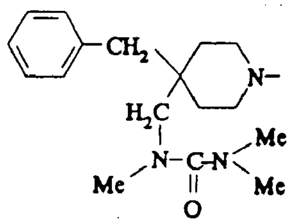 | 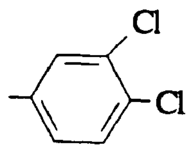 | HCl, 1 $H_2O$ 148 DCM/pentane |
| 61 | 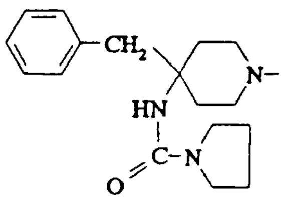 | 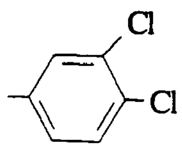 | HCl, 1,5 $H_2O$ 158 pentane/éther |
| 62 | 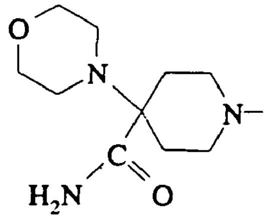 | 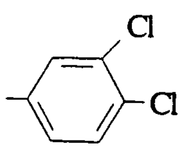 | 2 HCl, 1,5 $H_2O$ 140-145 éther |

Spectre de RMN du proton à 200 MHz dans DMSO-$d_6$ de l'EXEMPLE 57 :

δ : 1,6 à 3,7 ppm : m : 17 H ; 3,75 à 4,8 ppm : m : 6 H ; 7,0 à 7,6 ppm : m : 13 H ; 10,2 à 11 ppm: 2s : 1 H

Spectre de RMN du proton à 200 MHz dans DMSO-$d_6$ de l'EXEMPLE 58 :

δ : 0,8 à 4,9 ppm : m : 30 H ; 7,0 à 7,8 ppm : m : 13 H ; 10,0 ppm : s : 1 H ;

Spectre de RMN du proton à 200 MHz dans DMSO-$d_6$ de l'EXEMPLE 59 :

δ : 1,1 à 3,8 ppm : m : 24 H ; 3,9 à 4,75 ppm : m : 4 H ; 6,9 à 7,7 ppm : m : 13 H ; 9,7 ppm : s : 1 H

EXEMPLE 63

Chlorure de 6-[2-[4-benzyl-1-azoniabicydo[2.2.2]oct-1-yl]éthyl]-6-(3,4-difluorophényl)-4-[3,5-bis(trifluorométhyl)benzyl]morpholin-3-one, dihydrate.

On chauffe à 90°C pendant 9 heures un mélange de 0,86 g du composé obtenu à l'étape C de l'EXEMPLE 26, et 0,47 g de 4-benzyl-1-azabicyclo[2.2.2]octane (ou 4-benzylquinuclidine) dans 1 ml de DMF. Après refroidissement à TA, on verse le mélange réactionnel dans l'eau, extrait au DCM, lave deux fois la phase organique par une solution d'HCl 2N, deux fois par une solution saturée de NaCl, sèche sur $Na_2SO_4$ et évapore sous vide le solvant. On chromatographie le résidu sur silice H en éluant au DCM puis par le mélange DCM/MeOH (90/10 ; v/v). On obtient 0,4 g du produit attendu après cristallisation dans l'éther, F = 135°C (dèc).

EXEMPLE 64

Dichlorhydrate de 6-(3,4-difluorophényl)-4-[3,5-bis(trifluorométhyl)benzyl]-6-[2-[4-(pipérid-1-yl)pipérid-1-yl] éthyl]morpholin-3-one, hémihydrate.

On chauffe à 70°C pendant 4 heures et 30 minutes un mélange de 0,86 g du composé obtenu à l'étape C de l'EXEMPLE 26 et 0,7 g de 4-(pipérid-1-yl)pipéridine dans 3 ml de DMF. Après refroidissement à TA, on verse le mélange réactionnel dans l'eau, extrait à l'AcOEt, lave la phase organique par une solution de NaOH 1N, par une solution saturée de NaCl, sèche sur $Na_2SO_4$ et évapore sous vide le solvant. On chromatographie le résidu sur silice H en éluant au DCM puis par le mélange DCM/MeOH (90/10 ; v/v). On reprend le produit obtenu dans une solution saturée d'acide chlorhydrique dans l'éther et essore le précipité formé. On obtient 0,49 g du produit attendu, F = 260°C (dèc.).

En procédant selon les modes opératoires décrits dans les EXEMPLES précédents, on prépare les composés selon l'invention rassemblés dans le TABLEAU II ci-après.

TABLEAU II

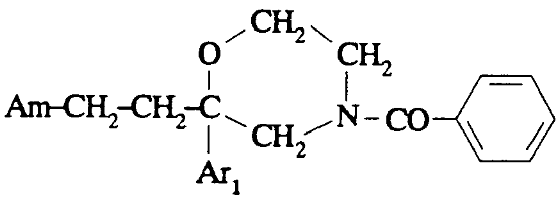

(I)

| Exemples | Am | $Ar_1$ | Sel, solvate ; F°C ou RMN ; solvant de recristallisation |
|---|---|---|---|
| 65 (a) | 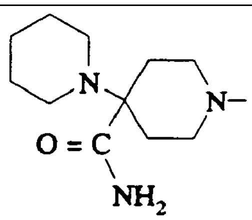 | 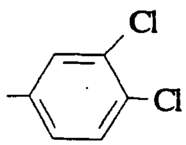 | 2HCl, 1 $H_2O$ RMN |

(suite)

| Exemples | Am | $Ar_1$ | Sel, solvate ; F°C ou RMN ; solvant de recristallisation |
|---|---|---|---|
| 66 (b) | 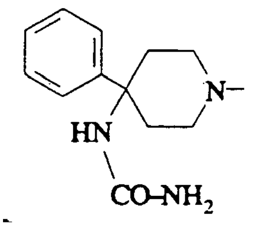 | 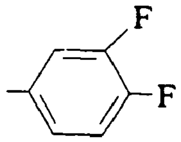 | HCl, 1,5 $H_2O$ 192-195 DCM/éther |
| 67 (c) | 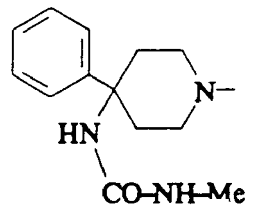 | 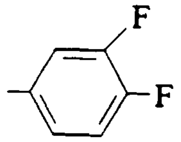 | HCl, 1 $H_2O$ RMN éther |

(a) On prépare ce composé selon le mode opératoire décrit à l'EXEMPLE 44 à partir du composé obtenu à l'étape C de l'EXEMPLE 43 et du composé obtenu à la Préparation 2.16 sous forme de base libre.
(b) On prépare ce composé selon le mode opératoire décrit à l'étape B de l'EXEMPLE 51 à partir du composé obtenu à l'étape A de l'EXEMPLE 51 et du composé obtenu à la Préparation 2.17 (base libre). Après chromatographie on dissout le produit obtenu dans du DCM, ajoute jusqu'à pH = 1 une solution saturée d'acide chlorhydrique dans l'éther et essore le chlorhydrate attendu.
(c) On prépare ce composé selon le mode opératoire décrit à l'étape B de l'EXEMPLE 51 à partir du composé obtenu à l'étape A de l'EXEMPLE 51 et du composé obtenu à la Préparation 2.18 (base libre). Après chromatographie on reprend le produit obtenu dans une solution saturée d'acide chlorhydrique dans l'éther et essore le chlorhydrate attendu.

Spectre de RMN du proton à 200 MHz dans DMSO-$d_6$ de l'EXEMPLE 65
δ : 0,8 à 4,2 ppm : m : 28H ; 7,0 à 7,8 ppm : m : 8H ; 7,9 à 8,6 ppm : 2s : 2H ; 10,4 à 11,6 ppm : m : 2H.
Spectre de RMN du proton à 200 MHz dans DMSO-$d_6$ de l'EXEMPLE 67 :

δ: 2,0 à 4,2 ppm : m : 21 H ; 5,9 ppm : mt : 1 H ; 6,75 ppm : s : 1 H ;
7,0 à 7,8 ppm : m : 8 H ; 10,95 ppm : s : 1 H.

EXEMPLE 68

Chlorhydrate de 4-benzoyl-2-(3,4-difluorophényl)-2-[2-(4-phényl-4-uréidopipérid-1-yl)éthyl]morpholine, dihydrate, isomère (+).

A) 4-Benzoyl-2-(3,4-difluorophényl)-2-(formylméthyl)morpholine, isomère (+).

On refroidit à -78°C, sous atmosphère d'azote, une solution de 2,6 ml de chlorure d'oxalyle dans 59 ml de DCM, ajoute goutte à goutte une solution de 4,6 ml de DMSO dans 29,5 ml de DCM, puis une solution de 8,2 g du composé obtenu à l'étape B de l'EXEMPLE 37 dans 59 ml de DCM et 5,7 ml de DMSO. On laisse 30 minutes sous agitation à -60°C le mélange réactionnel, puis ajoute 16 ml de triéthylamine et laisse sous agitation en laissant remonter la température à TA. On lave le mélange réactionnel par une solution d'HCl 1N, à l'eau, par une solution saturée de $NaHCO_3$, à l'eau, sèche la phase organique sur $MgSO_4$ et évapore sous vide le solvant. On obtient 7,5 g du produit attendu sous forme d'huile.

B) Chlorhydrate de 4-benzoyl-2-(3,4-difluorophényl)-2-[2-(4-phényl-4-uréidopipérid-1-yl)éthyl]morpholine, dihydrate, isomère (+).

A une solution de 1,6 g de 4-phényl-4-uréidopipéridine (base libre) et 0,4 ml d'acide acétique dans 26 ml de MeOH, on ajoute à TA une solution de 2,5 g du composé obtenu à l'étape précédente dans 26 ml de MeOH et laisse 5 minutes sous agitation à TA. Puis on ajoute en une fois une solution de 0,5 g de cyanoborohydrure de sodium dans 26 ml de MeOH et laisse 4 heures sous agitation à TA. On verse le mélange réactionnel dans 200 ml d'une solution à 10 % de $Na_2CO_3$, extrait à l'AcOEt, lave la phase organique à l'eau, sèche sur $MgSO_4$ et évapore sous vide le solvant. On chromatographie le résidu sur silice H en éluant par le gradient du mélange DCM/MeOH de (100/5 ; v/v) à (100/7,5 ; v/v). On dissout le produit obtenu dans du DCM, ajoute jusqu'à pH = 1 une solution saturée d'acide chlorhydrique dans l'éther et essore le précipité formé. On obtient 1,6 g du produit attendu, F = 187-190°C.

$$\alpha_D^{20} = +22,5° \text{ (c = 1 ; MeOH)}$$

EXEMPLE 69

Chlorhydrate de 4-benzoyl-2-(3,4-difluorophényl)-2-[2-[4-(N'-méthyluréido)-4-phénylpipérid-1-yl]éthyl]morpholine, monohydrate, isomère (+).

On prépare ce composé selon le mode opératoire décrit à l'étape B de l'EXEMPLE 68 à partir de 1,85 g de 4-(N'-méthyluréido)-4-phénylpipéridine (base libre), 0,44 ml d'acide acétique et 26 ml de MeOH, puis 2,75 g du composé obtenu à l'étape A de l'EXEMPLE 68 dans 26 ml de MeOH et 0,55 g de cyanoborohydrure de sodium dans 26 ml de MeOH. On obtient 2 g du produit attendu, F = 170-173°C.

$$\alpha_D^{20} = +23,4° \text{ (c = 1 ; MeOH)}$$

EXEMPLE 70

Dichlorhydrate de 4-benzoyl-2-(3,4-difluorophényl)-2-[2-[4-(3,3-diméthyl carbazoyl)-4-phénylpipérid-1-yl]éthyl] morpholine, 1,5 hydrate, isomère (+).

On prépare ce composé selon le mode opératoire décrit à l'étape B de l'EXEMPLE 68 à partir de 1,55 g de 4-(3,3-diméthylcarbazoyl)-4-phénylpipéridine (base libre), 0,38 ml d'acide acétique dans 26 ml de MeOH, puis 2,4 g du composé obtenu à l'étape A de l'EXEMPLE 68 dans 26 ml de MeOH et 0,47 g de cyanoborohydrure de sodium dans 26 ml de MeOH. On obtient 1,82 g du produit attendu.

$$\alpha_D^{20} = +22,5° \text{ (c = 1 ; MeOH)}$$

EXEMPLE 71

Chlorure de 2-(3,4-difluorophényl)-4-[2-(3-isopropoxyphényl)acétyl]-2-[2-[4-phényl-1-azoniabicyclo[2.2.2]oct-1-yl]éthyl]morpholine, monohydrate.

A) 2-[2-(Benzoyloxy)éthyl]-2-(3,4-difluorophényl)-4-[2-(3-isopropoxyphényl)acétyl] morpholine.

A une solution de 2,1 g du composé obtenu à la Préparation 1.21, 1,2 g d'acide 2-(3-isopropoxyphényl)acétique et 2,2 ml de triéthylamine dans 50 ml de DCM, on ajoute à TA 3,2 g de BOP et laisse 15 minutes sous agitation à TA. On concentre sous vide le mélange réactionnel, extrait le résidu à l'éther, lave la phase organique par une solution d'HCl 1N, à l'eau, par une solution à 10 % de $Na_2CO_3$, sèche sur $MgSO_4$ et évapore le solvant sous vide. On obtient 3,2 g du produit attendu sous forme d'huile.

B) 2-(3,4-Difluorophényl)-2-(2-hydroxyéthyl)-4-[2-(3-isopropoxyphényl)acétyl] morpholine.

On laisse 2 heures sous agitation à TA un mélange de 3,2 g du composé obtenu à l'étape précédente, 2 ml d'une solution concentrée de NaOH et 50 ml de MeOH. On concentre sous vide, extrait le résidu à l'éther, lave deux fois la phase organique à l'eau, sèche sur $MgSO_4$ et évapore sous vide le solvant. On obtient 2,3 g du produit attendu sous forme d'huile.

C) 2-(3,4-Difluorophényl)-4-[2-(3-isopropoxyphényl)acétyl]-2-[2-(méthane sulfonyloxy)éthyl]morpholine.

A une solution de 2,3 g du composé obtenu à l'étape précédente dans 50 ml de DCM, on ajoute 0,93 ml de triéthylamine puis 0,51 ml de chlorure de méthanesulfonyle et laisse 15 minutes sous agitation à TA. On concentre sous vide, extrait le résidu à l'éther, lave la phase organique à l'eau, par une solution à 10 % de $Na_2CO_3$, sèche sur $MgSO_4$ et évapore sous vide le solvant. On obtient 2,7 g du produit attendu sous forme d'huile.

D) Chlorure de 2-(3,4-difluorophényl)-4-[2-(3-isopropoxyphényl)acétyl]-2-[2-[4-phényl-1-azoniabicyclo[2.2.2]oct-1-yl] éthyl]morpholine, monohydrate.

On chauffe à 90°C pendant 5 heures un mélange de 1,3 g du composé obtenu à l'étape précédente, 0,72 g de 4-phényl-1-azabicyclo[2.2.2]octane dans 1,5 ml de DMF. Après refroidissement à TA, on verse le mélange réactionnel dans une solution d'HCl 1N, extrait au DCM, lave la phase organique par une solution d'HCl 1N, par une solution saturée de NaCl, sèche sur $MgSO_4$ et évapore sous vide le solvant. On chromatographie le résidu sur silice H en éluant au DCM puis par le mélange DCM/MeOH (90/10 ; v/v). On obtient 0,38 g du produit attendu après cristallisation dans l'éther iso, F = 136°C (déc.).

EXEMPLE 72

Dichlorhydrate de 2-(3,4-difluorophényl)-4-[2-(3-isopropoxyphényl)acétyl]-2-[2-[4-(pipérid-1-yl)pipérid-1-yl] éthyl]morpholine, hémihydrate.

On chauffe à 70°C pendant 4 heures un mélange de 1,3 g du composé obtenu à l'étape C de l'EXEMPLE 71 et 1,1 g de 4-(pipérid-1-yl)pipéridine dans 4 ml de DMF. Après refroidissement à TA, on verse le mélange réactionnel dans l'eau glacée, extrait à l'AcOEt, lave la phase organique par une solution de NaOH 1N, par une solution saturée de NaCl, sèche sur $Na_2SO_4$ et évapore sous vide le solvant. On chromatographie le résidu sur silice H en éluant au DCM puis par le mélange DCM/MeOH (90/10 ; v/v). On reprend le produit obtenu dans une solution saturée d'acide chlorhydrique dans l'éther et essore le précipité formé. On obtient 0,49 g du produit attendu, F = 270°C (dèc.).

EXEMPLE 73

Chlorure de 2-(3,4-difluorophényl)-2-(2-[4-phényl-1-azoniabicyclo [2.2.2]oct-1-yl]éthyl]-4-[3,5-bis(trifluorométhyl)benzoyl]morpholine, 1,5 hydrate.

A) 2-[2-(Benzoyloxy)éthyl]-2-(3,4-difluorophényl)-4-[3,5-bis(trifluorométhyl) benzoyl] morpholine.

On refroidit à 0°C une solution de 2,1 g du composé obtenu à la Préparation 1.21 et 1 ml de triéthylamine dans 50 ml de DCM, ajoute goutte à goutte 1,24 ml de chlorure de 3,5-bis(trifluorométhyl)benzoyle et laisse 5 minutes sous agitation. On lave deux fois le mélange réactionnel à l'eau, sèche la phase organique sur $MgSO_4$ et évapore sous vide le solvant. On obtient 3,5 g du produit attendu sous forme d'huile.

B) 2-(3,4-Difluorophényl)-2-(2-hydroxyéthyl)-4-[3,5-bis(trifluorométhyl)benzoyl] morpholine.

On laisse 2 heures sous agitation à TA un mélange de 3,5 g du composé obtenu à l'étape précédente, 2 ml d'une solution concentrée de NaOH et 50 ml de MeOH. On concentre sous vide, extrait le résidu à l'éther, lave deux fois la phase organique à l'eau, sèche sur $MgSO_4$ et évapore sous vide le solvant. On obtient 0,84 g du produit attendu après cristallisation dans le mélange éther isô/pentane, F = 101°C.

C) 2-(3,4-Difluorophényl)-2-[2-(méthanesulfonyloxy)éthyl]-4-[3,5-bis(trifluorométhyl) benzoyl]morpholine.

A une solution de 0,84 g du composé obtenu à l'étape précédente dans 10 ml de DCM, on ajoute 0,5 ml de triéthylamine, refroidit à 0°C puis ajoute 0,26 ml de chlorure de méthanesulfonyle et laisse 5 minutes sous agitation à

TA. On lave deux fois le mélange réactionnel à l'eau, sèche la phase organique sur $MgSO_4$ et évapore sous vide le solvant. On obtient 0,98 g du produit attendu.

D) Chlorure de 2-(3,4-difluorophényl)-2-[2-[4-phényl-1-azoniabicyclo[2.2.2] oct-1-yl]éthyl]-4-[3,5-bis(trifluorométhyl)benzoyl]morpholine, 1,5 hydrate.

On chauffe à 100°C pendant 3 heures un mélange de 0,98 g du composé obtenu à l'étape précédente, 0,65 g de 4-phényl-1-azabicyclo[2.2.2]octane dans 1 ml de DMF. Après refroidissement à TA, on verse le mélange réactionnel dans une solution d'HCl 1N, extrait au DCM, lave la phase organique par une solution d'HCl 1N, par une solution saturée de NaCl, sèche sur $MgSO_4$ et évapore sous vide le solvant. On chromatographie le résidu sur silice H en éluant au DCM puis par le mélange DCM/MeOH (90/10 ; v/v). On obtient 0,42 g du produit attendu après cristallisation dans l'éther iso, F = 170°C (dèc.).

EXEMPLE 74

Chlorhydrate de 5-[2-(4-acétamido-4-phénylpipérid-1-yl)éthyl]-3-benzoyl-5-(3,4-dichlorophényl)oxazolidine, monohydrate.

A) 3-Benzoyl-5-(3,4-dichlorophényl)-5-(2-hydroxyéthyl)oxazolidine.

A une solution de 3,2 g du composé obtenu à la Préparation 1.22 et 1 g de triéthylamine dans 50 ml de DCM on ajoute à TA et goutte à goutte une solution de 1,23 g de chlorure de benzoyle dans 10 ml de DCM et laisse 1 heure sous agitation à TA. On rajoute 1,76 g de triéthylamine puis 2,46 g de chlorure de benzoyle et laisse 1 heure sous agitation à TA. On concentre sous vide, reprend le résidu à l'eau, extrait à l'AcOEt, lave la phase organique par une solution tampon pH = 2, par une solution saturée de NaCl, sèche sur $Na_2SO_4$ et évapore sous vide le solvant. On reprend le résidu dans 30 ml de MeOH, ajoute 2 ml d'une solution à 30 % de NaOH et laisse 30 minutes sous agitation à TA. On concentre sous vide, extrait le résidu à l'éther, lave la phase organique à l'eau, sèche sur $Na_2SO_4$ et évapore sous vide le solvant. On chromatographie le résidu sur silice H en éluant par le mélange DCM/MeOH (100/1,5 ; v/v). On obtient 1,6 g du produit attendu.

B) 3-Benzoyl-5-(3,4-dichlorophényl)-5-[2-(méthanesulfonyloxy)éthyl]oxazolidine.

A une solution de 1,6 g du composé obtenu à l'étape précédente et 0,485 g de triéthylamine dans 50 ml de DCM, on ajoute à TA et goutte à goutte une solution de 0,55 g de chlorure de méthanesulfonyle dans 5 ml de DCM et laisse 30 minutes sous agitation à TA. On concentre sous vide, extrait le résidu à l'AcOEt, lave la phase organique à l'eau, sèche sur $Na_2SO_4$ et évapore sous vide le solvant. On obtient 1,9 g du produit attendu.

C) Chlorhydrate de 5-[2-(4-acétamido-4-phénylpipérid-1-yl)éthyl]-3-benzoyl-5-(3,4-dichlorophényl)oxazolidine, monohydrate.

On chauffe à 80-100°C pendant 2 heures un mélange de 1,8 g du composé obtenu à l'étape précédente, 1,7 g de *p*-toluènesulfonate de 4-acétamido-4-phénylpipéridine, 1,7 g de $K_2CO_3$ dans 4 ml de DMF. Après refroidissement à TA, on verse le mélange réactionnel dans l'eau, essore le précipité formé et le sèche sous vide. On chromatographie le précipité sur silice H en éluant par le mélange DCM/MeOH (100/3 ; v/v). On dissout le produit dans du DCM, ajoute jusqu'à pH = 1 une solution saturée d'acide chlorhydrique dans l'éther et essore le précipité formé. On obtient 1 g du produit attendu, F = 165-170°C.

EXEMPLE 75

Chlorure de 5-(3,4-difluorophényl)-5-[2-[4-phényl-1-azoniabicyclo[2.2.2] oct-1-yl]éthyl]-3-[3,5-bis(trifluorométhyl)benzyl]oxazolidin-2-one, monohydrate, isomère (+).

A) 5-[2-(Benzoyloxy)éthyl]-5-(3,4-difluorophényl)-3-[3,5-bis(trifluorométhyl)benzyl] oxazolidin-2-one, isomère (+).
On prépare ce composé selon le mode opératoire décrit à l'étape A de l'EXEMPLE 50 à partir du composé obtenu à la Préparation 1.23 (isomère (+)).
B) 5-(3,4-Difluorophényl)-5-(2-hydroxyéthyl)-3-[3,5-bis(trifluorométhyl)benzyl]oxazolidin-2-one, isomère (+).
On prépare ce composé selon le mode opératoire décrit à l'étape B de l'EXEMPLE 50 à partir du composé obtenu à l'étape précédente.

C) 5-(3,4-Difluorophényl)-5-[2-(méthanesulfonyloxy)éthyl]-3-[3,5-bis(trifluorométhyl) benzyl]oxazolidin-2-one, isomère (+).

On prépare ce composé selon le mode opératoire décrit à l'étape C de l'EXEMPLE 50 à partir du composé obtenu à l'étape précédente.

D) Chlorure de 5-(3,4-difluorophényl)-5-[2-[4-phényl-1-azoniabicyclo[2.2.2] oct-1-yl]éthyl]-3-[3,5-bis(trifluorométhyl) benzyl]oxazolidin-2-one, monohydrate, isomère (+).

On prépare ce composé selon le mode opératoire décrit à l'étape D de l'EXEMPLE 50 à partir du composé obtenu à l'étape précédente et de 4-phényl-1-azabicyclo[2.2.2]octane.

$$\alpha_D^{20} = +36{,}2° \ (c = 1 \ ; \ MeOH).$$

EXEMPLE 76

Dichlorhydrate de 4-benzoyl-2-(3[4-carbamoyl-4-(pipérid-1-yl)pipérid-1-yl]propyl]-2-(3,4-dichlorophényl)morpholine.

On prépare ce composé selon le mode opératoire décrit à l'étape C de l'EXEMPLE 19 à partir du composé obtenu à l'étape B de l'EXEMPLE 19 et du composé obtenu à la Préparation 2.16.

EXEMPLE 77

Chlorure de 2-(3,4-dichlorophényl)-4-[2-(3-chlorophényl)acétyl]-2-[2-[4-phényl-1-azoniabicyclo[2.2.2]oct-1-yl] éthyl]morpholine.

A) 2-[2-(Benzoyloxy)éthyl]-2-(3,4-dichlorophényl)-4-[2-(3-chlorophényl) acétyl] morpholine.

On prépare ce composé selon le mode opératoire décrit à l'étape A de l'EXEMPLE 71 à partir du composé obtenu à la Préparation 1.19 et l'acide 2-(3-chlorophényl)acétique.

B) 2-(3,4-Dichlorophényl)-4-[2-(3-chlorophényl)acétyl]-2-(2-hydroxyéthyl) morpholine;

On prépare ce composé selon le mode opératoire décrit à l'étape B de l'EXEMPLE 71 à partir du composé obtenu à l'étape précédente.

C) 2-(3,4-Dichlorophényl)-4-[2-(3-chlorophényl)acétyl]-2-[2-(méthanesulfonyloxy) éthyl] morpholine;

On prépare ce composé selon le mode opératoire décrit à l'étape C de l'EXEMPLE 71 à partir du composé obtenu à l'étape précédente et du chlorure de méthanesulfonyle.

D) Chlorure de 2-(3,4-dichlorophényl)-4-[2-(3-chlorophényl)acétyl]-2-[2-[4-phényl-1-azoniabicyclo[2.2.2]oct-1-yl] éthyl]morpholine.

On prépare ce composé selon le mode opératoire décrit à l'étape D de l'EXEMPLE 71 à partir du composé obtenu à l'étape précédente et de 4-phényl-1-azabicyclo[2.2.2]octane.

Spectre de RMN du proton à 200 MHz dans DMSO-$d_6$

$\delta$ : 2,0 à 2,5 ppm : m : 8H ; 2,55 à 4,6 ppm : m : 16H ; 7,0 à 8,0 ppm : m : 12H.

EXEMPLE 78

Chlorure de 2-(3,4-dichlorophényl)-4-[2-(3-chlorophényl)acétyl]-2-[4-benzyl-1-azoniabicyclo[2.2.2]oct-1-yl] éthyl]morpholine.

On prépare ce composé selon le mode opératoire décrit à l'étape D de l'EXEMPLE 71 à partir du composé obtenu à l'étape C de l'EXEMPLE 77 et de 4-benzyl-1-azabicyclo[2.2.2]octane.

## Revendications

**1.** Composé de formule :

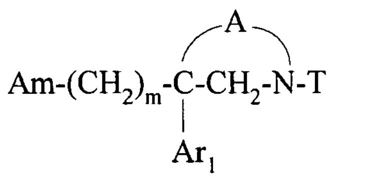

(I)

dans laquelle :

- A représente un radical bivalent choisi parmi :

$A_1$) -O-CO-
$A_2$) $-CH_2$-O-CO-
$A_3$) -O-$CH_2$-CO-
$A_4$) -O-$CH_2$-$CH_2$-
$A_5$) -N($R_1$)-CO-
$A_6$) -N($R_1$)-CO-CO-
$A_7$) -N($R_1$)-$CH_2$-$CH_2$-
$A_8$) -O-$CH_2$-

dans lesquels $R_1$ représente un hydrogène ou un ($C_1$-$C_4$)alkyle ;

- m est 2 ou 3 ;
- $Ar_1$ représente un phényle non substitué ou substitué une ou plusieurs fois par un substituant choisi parmi : un atome d'halogène, un hydroxy, un ($C_1$-$C_4$)alcoxy, un ($C_1$-$C_4$)alkyle, un trifluorométhyle, un méthylènedioxy, lesdits substituants étant identiques ou différents ; un thiényle non substitué ou substitué par un atome d'halogène ; un benzothiényle non substitué ou substitué par un atome d'halogène ; un naphtyle non substitué ou substitué par un atome d'halogène ; un indolyle non substitué ou N-substitué par un ($C_1$-$C_4$)alkyle ou un benzyle ; un imidazolyle non substitué ou substitué par un atome d'halogène ; un pyridyle non substitué ou substitué par un atome d'halogène ; un biphényle ;
- T représente un groupe choisi parmi : $CH_2$-Z, -CH($C_6H_5$)$_2$, -C($C_6H_5$)$_3$ ; T peut de plus représenter le groupe -CO-B-Z lorsque A représente un radical bivalent choisi parmi : -O-$CH_2$-$CH_2$- ou -N($R_1$)-$CH_2$-$CH_2$ - ou -O-$CH_2$- ;
- B représente une liaison directe ou un méthylène ;
- Z représente un phényle non substitué ou substitué une ou plusieurs fois par un substituant choisi parmi : un atome d'halogène ; un trifluorométhyle ; un cyano ; un hydroxy ; un nitro ; un phényle non substitué ou substitué une ou plusieurs fois par un halogène, un trifluorométhyle, un ($C_1$-$C_4$)alkyle, un hydroxy, un ($C_1$-$C_4$)alkoxy, lesdits substituants étant identiques ou différents ; un amino non substitué ou substitué une ou plusieurs fois par un ($C_1$-$C_4$)alkyle ; un benzylamino ; un carboxy ; un ($C_1$-$C_{10}$)alkyle ; un ($C_3$-$C_8$)cycloalkyle non substitué ou substitué une ou plusieurs fois par un méthyle ; un ($C_1$-$C_{10}$)alcoxy ; un ($C_3$-$C_8$)cycloalkyloxy non substitué ou substitué une ou plusieurs fois par un méthyle ; un mercapto ; un ($C_1$-$C_{10}$)alkylthio ; un formyloxy ; un ($C_1$-$C_6$) alkylcarbonyloxy ; un formylamino un ($C_1$-$C_6$)alkylcarbonylamino ; un benzoylamino ; un ($C_1$-$C_4$) alcoxycarbonyle ; un ($C_3$-$C_7$)cycloalkylcarbonyle ; un ($C_3$-$C_7$)cycloalkylcarbonyle ; un carbamoyle non substitué ou substitué une ou deux fois par un ($C_1$-$C_4$)alkyle ; un uréido non substitué ou substitué une ou deux fois en position 3 par un ($C_1$-$C_4$)alkyle ou un ($C_3$-$C_7$)cycloalkyle ; un (pyrrolidin-1-yl)carbonylamino, lesdits substituants étant identiques ou différents ; - un 1- ou 2-naphthyle ; 1-, 2, 3-, 4-, 5-, 6-, 7-indényle, dont une ou plusieurs liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, oxo, alkylcarbonylamino, alcoxycarbonyle, thioalkyle, halogène, alcoxy et trifluorométhyle, dans lesquels les alkyles sont en $C_1$-$C_4$ ;
- un groupe pyridyle, thiadiazolyle, indolyle, indazolyle, imidazolyle, benzimidazolyle, benzotriazolyle, benzofurannyle, benzothiényle, benzothiazolyle, benzisothiazolyle, quinolyle, isoquinolyle, benzoxazolyle, benzisoxazolyle, benzoxazinyle, benzodioxinyle, isoxazolyle, benzopyrannyle, thiazolyle, thiényle, furyle, pyrrannyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromannyle, chromannyle, dont une ou plusieurs doubles liaisons peuvent être hydrogénées, lesdits groupes pouvant être non substitués ou contenir éventuellement un ou plusieurs substituants tels que : le groupe alkyle, phényle, cyano, hydroxyalkyle, hydroxy, alkylcarbonylamino, alcoxycarbonyle, thioalkyle dans lesquels les alkyles sont en $C_1$-$C_4$;

- Am représente :

i- soit un groupe $Am_1$ de formule :

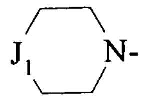

dans laquelle $J_1$ représente :
$i_1$ - soit un groupe

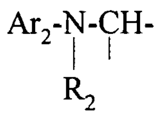

dans lequel :

- $Ar_2$ représente un pyridyle ; un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, un nitro, un méthylènedioxy, lesdits substituants étant identiques ou différents ; un thiényle ; un pyrimidyle ; un imidazolyle non substitué ou substitué par un $(C_1-C_4)$alkyle ;
- $R_2$ représente un hydrogène ; un $(C_1-C_7)$alkyle ; un benzyle ; un formyle ; un $(C_1-C_7)$alkylcarbonyle ;

$i_2$- soit un groupe ,

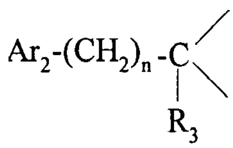

dans lequel :

- $Ar_2$ est tel que défini ci-dessus ;
- n est 0 ou 1 ;
- $R_3$ représente un groupe choisi parmi :

(1) hydrogène ;
(2) $(C_1-C_7)$alkyle ;
(3) formyle ;
(4) $(C_1-C_7)$alkylcarbonyle ;
(5) cyano ;
(6) $-(CH_2)_q-OH$ ;
(7) $-(CH_2)_q-O-(C_1-C_7)$alkyle ;
(8) $-(CH_2)_q-OCHO$ ;
(9) $-(CH_2)_q-OCOR_{17}$ ;
(10) $-(CH_2)_q-OCONH-(C_1-C_7)$alkyle ;
(11)$-NR_4R_5$ ;
(12) $-(CH_2)_q-NR_6C(=W_1)R_7$ ;
(13) $-(CH_2)_q-NR_6COOR_8$ ;
(14) $-(CH_2)_q-NR_6SO_2R_9$ ;
(15)$-(CH_2)_q-NR_6C(=W_1)NR_{10}R_{11}$ ;
(16) $-CH_2-NR_{12}R_{13}$ ;
(17) $-CH_2-CH_2-NR_{12}R_{13}$ ;

(18) -COOH ;
(19) $(C_1-C_7)$alcoxycarbonyle ;
(20) $-C(=W_1)NR_{10}R_{11}$ ;
(21) -CH2-COOH ;
(22) $(C_1-C_7)$alcoxycarbonylméthyle ;
(23) $-CH_2-C(=W_1)NR_{10}R_{11}$ ;
(24) $-O-CH_2CH_2-OR_{18}$ ;
(25) $-NR_6COCOR_{19}$ ;
(26) $-CO-NR_{20}-NR_{21}R_{22}$ ;
(27)

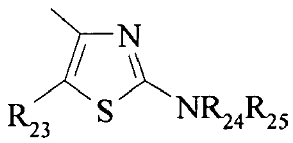

;

(28)

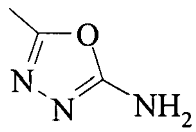

;

- ou $R_3$ constitue une double liaison entre l'atome de carbone auquel il est lié et l'atome de carbone voisin du cycle pipéridine ;
- q est 0, 1 ou 2 ;
- $W_1$ représente un atome d'oxygène ou un atome de soufre ;
- $R_4$ et $R_5$ représentent chacun indépendamment un hydrogène ou un $(C_1-C_7)$alkyle ; $R_5$ peut de plus représenter un $(C_3-C_7)$cycloalkylméthyle, un benzyle ou un phényle ; ou $R_4$ et $R_5$ ensemble avec l'atome d'azote auquel ils sont liés constituent un hétérocycle choisi parmi l'azétidine, la pyrrolidine, la pipéridine, la morpholine, la thiomorpholine, la perhydroazépine ou la pipérazine non substituée ou substituée en position 4 par un $(C_1-C_4)$alkyle ;
- $R_6$ représente un hydrogène ou un $(C_1-C_4)$alkyle ;
- $R_7$ représente un hydrogène ; un $(C_1-C_7)$alkyle ; un vinyle ; un phényle ; un benzyle ; un pyridyle ; un $(C_3-C_7)$cycloalkyle non substitué ou substitué par un ou plusieurs méthyles ; un furyle ; un thiényle ; un pyrrolyle ; un imidazolyle ;
- ou $R_6$ et $R_7$ ensemble représentent un groupe $-(CH_2)_p-$ ;
- p est 3 ou 4 ;
- $R_8$ représente un $(C_1-C_7)$alkyle ou un phényle ;
- $R_9$ représente un $(C_1-C_7)$alkyle ; un amino libre ou substitué par un ou deux $(C_1-C_7)$alkyles ; un phényle non substitué ou substitué une ou plusieurs fois par un substituant choisi parmi : un atome d'halogène, un $(C_1-C_7)$alkyle, un trifluorométhyle, un hydroxy, un $(C_1-C_7)$alcoxy, un carboxy, un $(C_1-C_7)$ alcoxycarbonyle, un $(C_1-C_7)$alkylcarbonyloxy, un cyano, un nitro, un amino libre ou substitué par un ou deux $(C_1-C_7)$alkyles, lesdits substituants étant identiques ou différents ;
- $R_{10}$ et $R_{11}$ représentent chacun indépendamment un hydrogène ou un $(C_1-C_7)$alkyle ; $R_{11}$ peut de plus représenter un $(C_3-C_7)$cycloalkyle, un $(C_3-C_7)$cycloalkylméthyle, un hydroxy, un $(C_1-C_4)$alcoxy, un benzyle ou un phényle ; ou $R_{10}$ et $R_{11}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;
- $R_{12}$ et $R_{13}$ représentent chacun indépendamment un hydrogène ou un $(C_1-C_7)$alkyle ; $R_{13}$ peut de plus représenter un $(C_3-C_7)$cycloalkylméthyle ou un benzyle ;
- $R_{17}$ représente un $(C_1-C_7)$alkyle ; un $(C_3-C_7)$cycloalkyle non substitué ou substitué par un ou plusieurs méthyles ; un phényle ; un pyridyle ;

- $R_{18}$ représente un hydrogène ; un $(C_1-C_7)$alkyle ; un formyle ; un $(C_1-C_7)$alkylcarbonyle ;
- $R_{19}$ représente un $(C_1-C_4)$alcoxy ;
- $R_{20}$ représente un hydrogène ou un $(C_1-C_7)$alkyle ;
- $R_{21}$ et $R_{22}$ représentent chacun indépendamment un hydrogène ou un $(C_1-C_7)$alkyle ;
- ou bien $R_{21}$ et $R_{22}$ ensemble avec l'atome d'azote auquel ils sont liés constituent un hétérocycle choisi parmi la pyrrolidine, la pipéridine ou la morpholine ;
- $R_{23}$ représente un hydrogène ou un $(C_1-C_7)$alkyle ;
- $R_{24}$ et $R_{25}$ représentent chacun indépendamment un hydrogène ou un $(C_1-C_7)$alkyle ; $R_{25}$ peut de plus représenter un formyle ou un $(C_1-C_7)$alkylcarbonyle ;

$i_3$ - soit un groupe

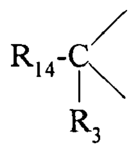

dans lequel :

- $R_3$ est tel que défini ci-dessus ;
- $R_{14}$ représente un $(C_1-C_7)$alkyle ; un $(C_3-C_7)$cycloalkyle ; $R_{14}$ peut de plus représenter soit un groupe $-CONR_{15}R_{16}$ lorsque $R_3$ représente l'hydrogène, soit un groupe $-NR_{15}R_{16}$ lorsque $R_3$ représente l'hydrogène, un cyano, un carboxy, un $(C_1-C_7)$alcoxycarbonyle ou un groupe $-C(=W_1)NR_{10}R_{11}$ ;
- $R_{15}$ et $R_{16}$ représentent chacun indépendamment un $(C_1-C_7)$alkyle ; ou $R_{15}$ et $R_{16}$ ensemble avec l'atome d'azote auquel ils sont liés constituent un hétérocycle choisi parmi l'azétidine, la pyrrolidine, la pipéridine, la morpholine, la thiomorpholine ou la perhydroazépine ;

ii- soit un groupe $Am_2$ de formule :

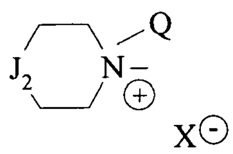

dans laquelle $J_2$ représente :

$ii_1$- soit un groupe

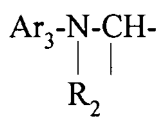

dans lequel :

- $Ar_3$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- $R_2$ est tel que défini ci-dessus pour $J_1$ ;

$ii_2$- soit un groupe

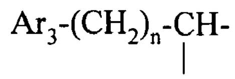

dans lequel :

- $Ar_3$ est tel que défini ci-dessus ;
- n est 0 ou 1 ;
- Q représente un $(C_1-C_6)$alkyle ou un benzyle ; ledit substituant étant soit en position axiale soit en position équatoriale ;
- $X^\ominus$ est un anion ;

iii- soit un groupe $Am_3$ de formule :

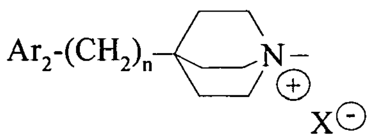

dans laquelle :

- $Ar_2$ est tel que défini ci-dessus ;
- n est 0 ou 1 ;
- $X^\ominus$ représente un anion ;

iv- soit un groupe $Am_4$ de formule :

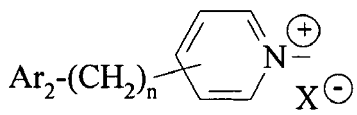

dans laquelle :

- $Ar_2$ est tel que défini ci-dessus ;
- n est 0 ou 1 ;
- $X^\ominus$ représente un anion ;

à la condition que :

1) lorsque A est $-O-CH_2-CH_2-$, $-N(R_1)-CH_2-CH_2-$ ou $-O-CH_2-$, m est 2, $Ar_1$ est $Ar_{1a}$, $Ar_{1a}$ étant un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, T est -CO-Za, Za étant un phényle non substitué ou substitué une ou plusieurs fois par un substituant choisi parmi : un atome d'halogène, un trifluorométhyle, un $(C_1-C_{10})$ alkyle, un $(C_1-C_{10})$alcoxy, un hydroxy, lesdits substituants étant identiques ou différents; ou bien
2) lorsque A est $-O-CH_2-CO-$, $-CH_2-O-CO-$ ou $-O-CO-$, m est 2, $Ar_1$ est $Ar_{1a}$, $Ar_{1a}$ étant tel que défini ci-dessus, T est $-CH_2-Za$, Za étant tel que défini ci-dessus, alors Am n'est pas un groupe $Am_1$ de formule :

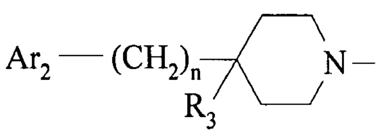 ;

dans lequel $Ar_2$, $R_3$ et n sont tels que définis ci-dessus ;
3) lorsque A est $-O-CH_2-CH_2-$, $-N(R_1)-CH_2-CH_2-$ ou $-O-CH_2-$, m est 2, $Ar_1$ est $Ar_{1a}$, $Ar_{1a}$ étant tel que défini ci-dessus ; T est $-O-CH_2-$ Za, Za étant tel que défini ci- dessus, alors Am n'est pas :

* soit un groupe $Am_{2a}$ de formule :

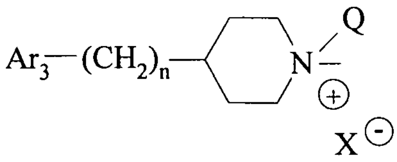

* soit un groupe $Am_3$ de formule :

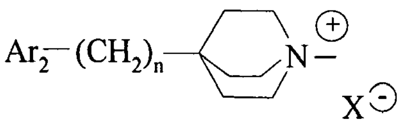

* soit un groupe $Am_{1a}$ de formule :

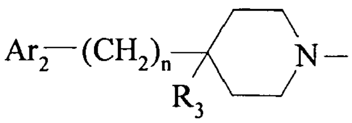

dans lesquels :

- n est 0 ou 1 ;
- Q est tel que défini ci-dessus et est en position axiale ;
- $X^{\ominus}$ représente un anion pharmaceutiquement acceptable ;
- $Ar_2$, $Ar_3$ et $R_3$ sont tels que définis ci-dessus;

et ses sels avec des acides minéraux ou organiques.

**2.** Composé optiquement pur selon la revendication 1 de formule :

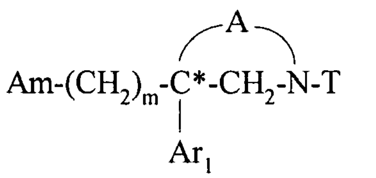

(I*)

dans laquelle :

- "*" signifie que l'atome de carbone ainsi marqué à la configuration absolue (+) ou (-) déterminée ;
- Am, m, $Ar_1$, A et T sont tels que définis pour les composés de formule (I) dans la revendication 1 ;

ainsi que ses sels avec des acides minéraux ou organiques.

**3.** Un composé selon l'une quelconque des revendications 1 ou 2, de formule (I) ou (I*) dans laquelle :

- Z est Z' et représente :
- un phényle non substitué ou substitué une ou plusieurs fois par un substituant choisi parmi : un atome d'halogène ; un trifluorométhyle ; un cyano ; un hydroxy ; un nitro ; un phényle non substitué ou substitué une ou plusieurs fois par un halogène, un trifluorométhyle, un $(C_1-C_4)$alkyle, un hydroxy, un $(C_1-C_4)$alcoxy, lesdits substituants étant identiques ou différents ; un amino non substitué ou substitué une ou deux fois par un $(C_1-C_4)$alkyle ; un benzylamino ; un carboxy ; un $(C_1-C_{10})$alkyle ; un $(C_3-C_8)$cycloalkyle non substitué ou substitué une ou plusieurs fois par un méthyle ; un $(C_1-C_{10})$alcoxy ; un $(C_3-C_8)$cycloalkyloxy non substitué ou substitué une ou plusieurs fois par un méthyle ; un mercapto ; un $(C_1-C_{10})$alkylthio ; un formyloxy ; un $(C_1-C_6)$alkylcarbonyloxy ; un formylamino ; un $(C_1-C_6)$alkylcarbonylamino ; un benzoylamino ; un $(C_1-C_4)$alcoxycarbonyle ; un $(C_3-C_7)$cycloalkyloxycarbonyle ; un $(C_3-C_7)$cycloalkylcarbonyl ; un carbamoyle non substitué ou substitué une ou deux fois par un $(C_1-C_4)$alkyle ; un uréido non substitué ou substitué une ou deux fois en position 3 par un $(C_1-C_4)$alkyle ou un $(C_3-C_7)$cycloalkyle ; un (pyrrolidin-1-yl)-carbonylamino, lesdits substituants étant identiques ou différents ;
- un naphtyle non substitué ou substitué une ou plusieurs fois par un halogène, un trifluorométhyle, un $(C_1-C_4)$alkyle, un hydroxy, un $(C_1-C_4)$alcoxy ;
- un pyridyle ; un thiényle ; un indolyle ; un quinolyle ; un benzothiényle ; un imidazolyle ;
- Am, m, $Ar_1$, A et T sont tels que définis pour un composé de formule (I) dans la revendication 1 ;

et ses sels avec des acides minéraux ou organiques.

**4.** Composé selon l'une des revendications 1 ou 2 de formule :

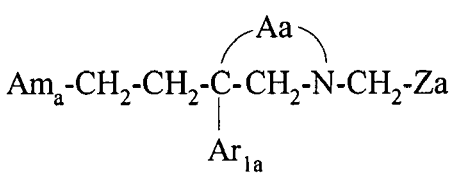

(Ia)

dans laquelle :

- Aa représente un radical bivalent choisi parmi : -O-CO- ; $-CH_2-O-CO-$ ; $-O-CH_2-CO-$ ; $-N(R_1)-CO-$ ou $-N(R_1)-CO-CO-$ ;

dans lesquels :

- $R_1$ représente un hydrogène ou un $(C_1-C_4)$alkyle ;
- $Am_a$ représente :

  * soit un groupe $Am_{2a}$ de formule :

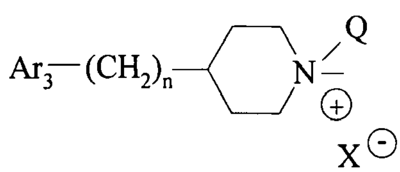

  * soit un groupe $Am_3$ de formule :

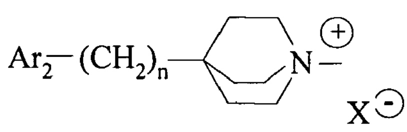

- n est 0 ou 1 ;
- Q est tel que défini pour un composé de formule (I) dans la revendication 1 et est en position axiale ;

- $X^{\ominus}$ représente un anion pharmaceutiquement acceptable ;
- $Ar_2$ et $Ar_3$ sont tels que définis pour un composé de formule (I) dans la revendication 1 ;
- $Ar_{1a}$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- Za représente un phényle non substitué ou substitué une ou plusieurs fois par un substituant choisi parmi : un atome d'halogène, un trifluorométhyle, un $(C_{1-}C_{10})$alkyle, un $(C_1-C_{10})$alcoxy, un hydroxy, lesdits substituants étant identiques ou différents ;

et ses sels avec des acides minéraux ou organiques.

**5.** Composé selon l'une des revendications 1 ou 2 de formule :

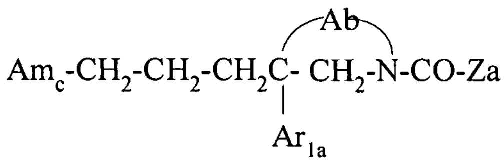

(Ie)

dans laquelle :

- Ab représente le radical bivalent $-O-CH_2-CH_2-$ ou $-N(R_1)-CH_2-CH_2-$ ou $-O-CH_2-$ ;
- $R_1$ représente un hydrogène ou un $(C_1-C_4)$alkyle ;
- $Am_c$ représente un groupe $Am_{1a}$ de formule:

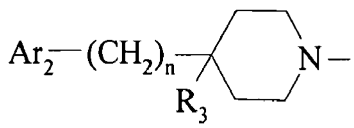

- n est 0 ou 1 ;
- $Ar_2$ et $R_3$ sont tels que définis pour un composé de formule (I) dans la revendication 1;
- $Ar_{1a}$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- Za représente un phényle non substitué ou substitué une ou plusieurs fois par un substituant choisi parmi : un atome d'halogène, un trifluorométhyle, un $(C_{1-}C_{10})$alkyle, un $(C_1-C_{10})$alcoxy, un hydroxy, lesdits substituants étant identiques ou différents ;

et ses sels avec des acides minéraux ou organiques.

**6.** Composé selon l'une des revendications 1 ou 2 de formule :

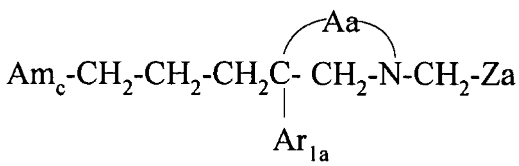

(If)

dans laquelle :

- Aa représente le radical bivalent $-O-CO-$, $-CH_2-O-CO-$, $-O-CH_2-CO-$, $-N(R_1)-CO-$ ou $-N(R_1)-CO-CO-$ ;

dans lesquels :

- $R_1$ représente un hydrogène ou un $(C_1-C_4)$alkyle ;
- $Am_c$ représente un groupe $Am_{1a}$ de formule :

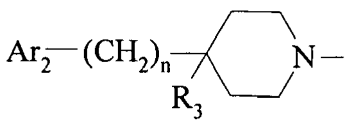

- n est 0 ou 1 ;
- $Ar_2$ et $R_3$ sont tels que définis pour un composé de formule (I) dans la revendication 1;
- $Ar_{1a}$ représente un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents ;
- Za représente un phényle non substitué ou substitué une ou plusieurs fois par un substituant choisi parmi : un atome d'halogène, un trifluorométhyle, un $(C_1-C_{10})$alkyle, un $(C_1-C_{10})$alcoxy, un hydroxy, lesdits substituants étant identiques ou différents ;

et ses sels avec des acides minéraux ou organiques.

**7.** Le 5-(3,4-difluorophényl)-5-[2-[4-phényl-1-azoniabicyclo[2.2.2]oct-1-yl]éthyl]-3-[3,5-bis(trifluorométhyl)benzyl]oxazolidin-2-one, avec un anion pharmaceutiquement acceptable, sous forme optiquement pure, préférentiellement sous forme de l'isomère (+).

**8.** Procédé pour la préparation des composés de formule (I) selon la revendication 1 et de leurs sels, **caractérisé en ce que** :

1) on traite un composé de formule :

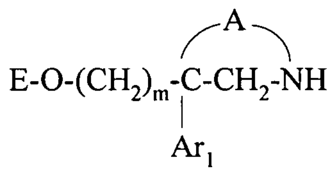

(II)

dans laquelle m, $Ar_1$ et A sont tels que définis pour un composé de formule (I) dans la revendication 1 et E représente l'hydrogène ou un groupe O-protecteur,

- soit avec un dérivé fonctionnel d'un acide de formule :

HOCO-B-Z (III)

dans laquelle B et Z sont tels que définis précédemment pour (I) dans la revendication 1, lorsqu'on doit préparer un composé de formule (I) où T est -COB-Z,
- soit avec un dérivé halogéné de formule :

$Hal-CH_2-Z$ (IV)

dans laquelle Z est tel que défini dans la revendication 1, et Hal représente un halogène, lorsqu'on doit préparer un composé de formule (I) où T est $-CH_2-Z$,
- soit avec un dérivé halogéné de formule :

$Hal-CH(C_6H_5)_2$ (V)

lorsqu'on doit préparer un composé de formule (I) où T est un groupe $-CH(C_6H_5)_2$,
- soit avec un dérivé halogéné de formule:

$Hal\text{-}C\text{-}(C_6H_5)_3$ (VI)

lorsqu'on doit préparer un composé de formule (I) où T est un groupe $-C(C_6H_5)_3$, pour obtenir un composé de formule :

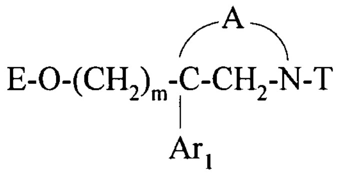

(VII) ;

dans laquelle E, m, $Ar_1$, A et T sont tels que définis ci-dessus ;
2) on élimine éventuellement le groupe O-protecteur par action d'un acide ou d'une base, pour obtenir l'alcool de formule :

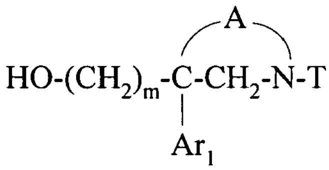

(VIII) ;

dans laquelle m, $Ar_1$, A et T sont tels que définis ci-dessus ;
3) on traite l'alcool (VIII) avec un composé de formule :

$Y\text{-}SO_2\text{-}Cl$ (IX)

dans laquelle Y représente un groupe méthyle, phényle, tolyle, trifluorométhyle, pour obtenir un composé de formule :

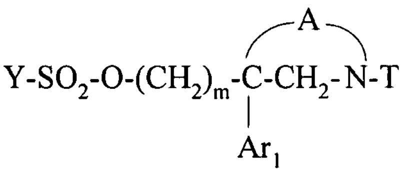

(X) ;

dans laquelle Y, m, $Ar_1$, A et T sont tels que définis ci-dessus ;
4) on fait réagir le composé (X) :

- soit avec une amine secondaire cyclique de formule :

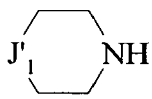

(XI)

dans laquelle $J'_1$ représente :

* soit un groupe

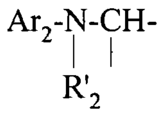

dans lequel $Ar_2$ est tel que défini pour (I) dans la revendication 1 et $R'_2$ représente soit $R_2$ tel que défini pour (I) dans la revendication 1 soit un précurseur de $R_2$ ;
* soit un groupe

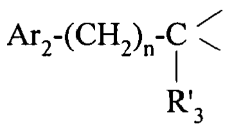

dans lequel $Ar_2$ et n sont tels que définis pour (I) dans la revendication 1 et $R'_3$ représente soit $R_3$ tel que défini pour (I) dans la revendication 1, soit un précurseur de $R_3$, étant entendu que lorsque $R'_3$ représente un hydroxyle ou un amino, ces groupes peuvent être protégés ;
* soit un groupe

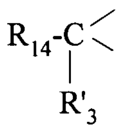

dans lequel $R_{14}$ est tel que défini pour (I) dans la revendication 1 et $R'_3$ est tel que défini ci-dessus ;

- soit avec une amine tertiaire de formule :

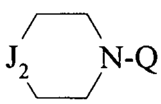 (XII)

dans laquelle $J_2$ et Q sont tels que définis pour (I) dans la revendication 1 ;
- soit avec une amine tertiaire cyclique de formule :

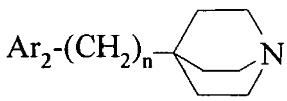 (XIII)

dans laquelle $Ar_2$ et n sont tels que définis pour (I) dans la revendication 1 ;
- soit avec un composé de formule :

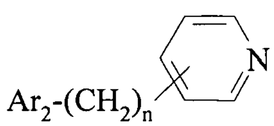 (XIV)

dans laquelle $Ar_2$ et n sont tels que définis pour (I) dans la revendication 1 ; et
5) - soit, lorsqu'on utilise une amine secondaire cyclique de formule (XI), et après déprotection éventuelle du groupe hydroxy ou du groupe amino représenté par $R'_3$ ou transformation éventuelle de $R'_2$ en $R_2$ ou de $R'_3$ en $R_3$, on transforme éventuellement le produit ainsi obtenu en un de ses sels ;

- soit, lorsqu'on utilise une amine tertiaire de formule (XII) ou une amine tertiaire cyclique de formule (XIII) ou un composé de formule (XIV), on isole le produit ainsi obtenu sous forme d'un sulfonate et éventuellement d'un sel d'acide sulfonique ou bien, éventuellement on échange l'anion et éventuellement le sel d'acide ainsi obtenu avec un autre anion et éventuellement un autre sel d'acide minéral ou organique pharmaceutiquement acceptables.

**9.** Procédé pour la préparation des composés de formule (I) selon la revendication 1 dans laquelle Am représente un groupe $Am_1$, et de ses sels, **caractérisé en ce que** : 1') on oxyde un composé de formule (VIII) tel que défini dans la revendication 9 pour obtenir un composé de formule :

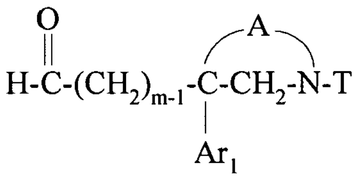

(XXXVIII)

dans laquelle m, $Ar_1$, A et T sont tels que définis pour un composé de formule (I) à la revendication 1,
2') on fait réagir le composé de formule (XXXVIII) avec un composé de formule (XI) tel que défini dans la revendication 9 en présence d'un acide, puis on réduit le sel d'imminium formé intermédiairement au moyen d'un agent réducteur ; et
3') après déprotection éventuelle des groupes hydroxyles ou des groupes aminés ou transformation éventuelle de $R'_2$ en $R_2$ ou de $R'_3$ en $R_3$, on transforme éventuellement le produit ainsi obtenu en l'un de ses sels.

**10.** Composé de formule :

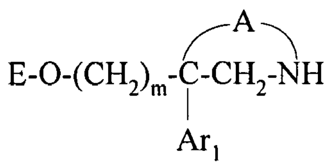

(II)

dans laquelle m, A et $Ar_1$ sont tels que définis pour (I) dans la revendication 1, et E représente l'hydrogène ou un groupe O-protecteur, à la condition que lorsque A est $-O-CH_2-CO-$, $-CH_2-O-CO-$, -O-CO-, $-O-CH_2-CH_2-$, $-N(R_1)-CH_2-CH_2-$ ou $-O-CH_2-$, $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, alors m n'est pas 2,
sous forme énantiomériquement pure ou sous forme racémique.

**11.** Composé de formule :

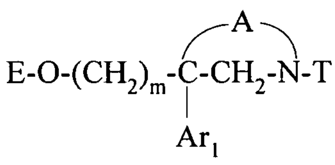

(VII)

dans laquelle m, A, $Ar_1$ et T sont tels que définis pour (I) dans la revendication 1, et E représente l'hydrogène ou un groupe O-protecteur à la condition que lorsque A est $-O-CH_2-CO-$, $-CH_2-O-CO-$, -O-CO-, $-O-CH_2-CH_2-$, $-N(R_1)-CH_2-CH_2$ ou $-O-CH_2-$, $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, T est -CO-Za ou $-CH_2$-Za, Za étant un phényle non substitué ou substitué une ou plusieurs fois par un substituant choisi parmi : un atome d'halogène, un trifluorométhyle, un $(C_1-C_{10})$alkyle, un $(C_1-C_{10})$alcoxy, un hydroxy, lesdits substituants étant identiques ou différents, alors m n'est pas 2, sous forme énantiomériquement pure ou sous forme racémique.

**12.** Composé de formule :

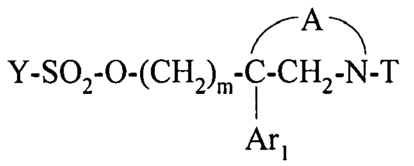

(X)

dans laquelle m, A, $Ar_1$ et T sont tels que définis pour (I) dans la revendication 1, et Y représente un groupe méthyle, phényle, tolyle ou trifluorométhyle, à la condition que lorsque A est $-O-CH_2-CO-$, $-CH_2-O-CO-$, $-O-CO-$, $-O-CH_2-CH_2-$, $-N(R_1)-CH_2-CH_2-$ ou $-O-CH_2-$, $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, T est $-CO-Za$ ou $-CH_2-Za$, Za étant un phényle non substitué ou substitué une ou plusieurs fois par un substituant choisi parmi : un atome d'halogène, un trifluorométhyle, un $(C_1-C_{10})$alkyle, un $(C_{1-}C_{10})$alcoxy, un hydroxy, lesdits substituants étant identiques ou différents, alors m n'est pas 2,
sous forme énantiomériquement pure ou sous forme racémique.

**13.** Composé de formule :

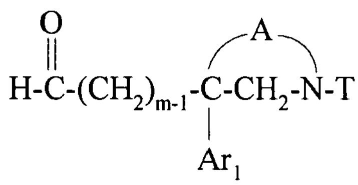

(XXXVIII)

dans laquelle m, $Ar_1$, A et T sont tels que définis pour (I) dans la revendication 1, à la condition que lorsque A est $-O-CH_2-CO-$, $-CH_2-O-CO-$, $-O-CO-$, $-O-CH_{2-}CH_2-$, $-N(R_1)-CH_2-CH_2-$ ou $-O-CH_2-$, $Ar_1$ est un phényle non substitué ou substitué une ou plusieurs fois par un ubstituant choisi parmi : un atome d'halogène, un hydroxy, un $(C_1-C_4)$ alcoxy, un $(C_1-C_4)$alkyle, un trifluorométhyle, lesdits substituants étant identiques ou différents, T est $-CO-Za$ ou $-CH_2-Za$, Za étant un phényle non substitué ou substitué une ou plusieurs fois par un substituant choisi parmi : un atome d'halogène, un trifluorométhyle, un $(C_1-C_{10})$alkyle, un $(C_1-C_{10})$alcoxy, un hydroxy, lesdits substituants étant identiques ou différents, alors m n'est pas 2,
sous forme énantiomériquement pure ou sous forme racémique.

**14.** Composé de formule :

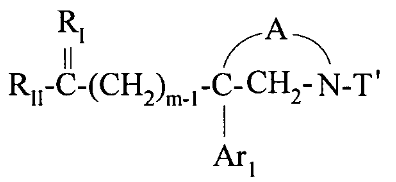

(XXXIX)

dans laquelle :

- m, $Ar_1$, A sont tels que définis pour un composé de formule (I) dans la revendication 1, à la condition que lorsque A est $-O-CH_2-CO-$, $-CH_2-O-CO-$, $-O-CO-$, $-O-CH_2-CH_2-$, $-N(R_1)-CH_2-CH_2-$ ou $-O-CH_2-$, $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, T' est $-CO-Za$ ou $-CH_2-Za$, Za étant un phényle non substitué ou substitué une ou plusieurs fois par un substituant choisi parmi : un atome d'halogène, un trifluorométhyle, un $(C_1-C_{10})$alkyle, un $(C_1-C_{10})$alcoxy, un hydroxy, lesdits substituants étant identiques ou différents, alors m n'est pas 2 ;
- $R_I$ représente deux atomes d'hydrogène et $R_{II}$ représente :
- soit un groupe -O-E dans lequel E représente un atome d'hydrogène ou un groupe O-protecteur,
- soit un groupe $-O-SO_2-Y$ dans lequel Y représente un groupe méthyle, phényle, tolyle ou trifluorométhyle ;

- ou bien $R_I$ représente un atome d'oxygène et $R_{II}$ représente un atome d'hydrogène ;
- T' représente T tel que défini pour un composé de formule (I), T' peut de plus représenter l'hydrogène lorsque à la fois $R_I$ représente 2 atomes d'hydrogène et $R_{II}$ représente un groupe -O-E ;

sous forme énantiomériquement pure ou sous forme de racémique.

**15.** Composé de formule :

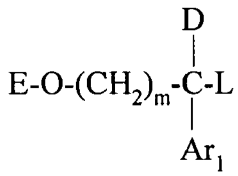

(XXXX)

dans laquelle :

- m et $Ar_1$ sont tels que définis pour un composé de formule (I) dans la revendication 1, à la condition que lorsque $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, alors m n'est pas 2 ;
- E représente un hydrogène ou un groupe O-protecteur ;
- L représente un groupe cyano ou un groupe amino méthyle ;
- D représente un groupe choisi parmi :

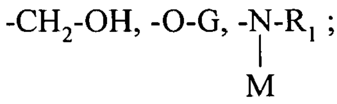

- G représente un hydrogène ou un groupe O-protecteur ;
- M représente un hydrogène ou un groupe N-protecteur ;
- $R_1$ est tel que défini pour un composé de formule (I) dans la revendication 1 ; sous forme énantiomériquement pure ou sous forme racémique lorsque L est un groupe aminométhyle.

**16.** Composé de formule :

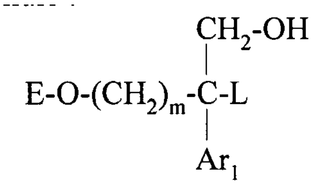

(XXXV)

dans laquelle :

- m et $Ar_1$ sont tels que définis pour (I) dans la revendication 1, à la condition que lorsque $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, alors m n'est pas 2 ;
- E représente un hydrogène ou un groupe O-protecteur ;
- L représente un groupe cyano ou un groupe aminométhyle, le composé étant sous forme énantiomériquement pure ou sous forme racémique lorsque L est un groupe aminométhyle.

**17.** Composé de formule :

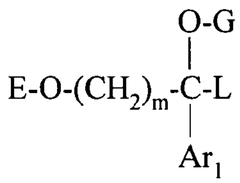

(XXXVI)

dans laquelle :

- m et $Ar_1$ sont tels que définis pour (I) dans la revendication 1, à la condition que lorsque $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, alors m n'est pas 2 ;
- E représente un hydrogène ou un groupe O-protecteur ;
- G représente un hydrogène ou un groupe O-protecteur ;
- L représente un groupe cyano ou un groupe aminométhyle, le composé étant sous forme énantiomériquement pure ou sous forme racémique lorsque L est un groupe aminométhyle.

**18.** Composé de formule:

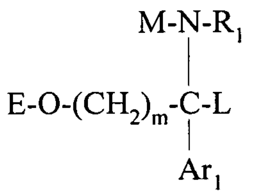

(XXXVII)

dans laquelle :

- m, $Ar_1$ et $R_1$ sont tels que définis pour (I) dans la revendication 1, à la condition que lorsque $Ar_1$ est un phényle non substitué ou substitué une ou plusieurs fois par un substituant choisi parmi : un atome d'halogène, un hydroxy, un $(C_1-C_4)$alcoxy, un $(C_1-C_4)$alkyle, un trifluorométhyle, lesdits substituants étant identiques ou différents, alors m n'est pas 2 ;
- E représente un hydrogène ou un groupe O-protecteur;
- M représente un hydrogène ou un groupe N-protecteur;
- L représente un groupe cyano ou un groupe aminométhyle, le composé étant sous forme énantiomériquement pure ou sous forme racémique lorsque L est un groupe aminométhyle.

**19.** Composition pharmaceutique contenant à titre de principe actif, un composé selon l'une quelconque des revendications 1 à 7, ou un de ses sels pharmaceutiquement acceptables.

**20.** Composition pharmaceutique selon la revendication 19, sous forme d'unité de dosage, dans laquelle le principe actif est mélangé à au moins un excipient pharmaceutique.

**21.** Composition pharmaceutique selon la revendication 20, contenant 0,5 à 1000 mg de principe actif.

**22.** Composition pharmaceutique selon la revendication 21, contenant 2,5 à 250 mg de principe actif.

Office européen des brevets

# RAPPORT DE RECHERCHE EUROPEENNE

Numéro de la demande
EP 06 00 5775

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,A | EP 0 512 901 A (ELF SANOFI) 11 novembre 1992 (1992-11-11) * revendications * | 1-22 | INV. C07D413/06 C07D401/06 C07D413/12 C07D265/30 C07D265/32 C07D453/02 C07D309/12 C07D263/38 C07D405/12 C07C219/22 C07C255/36 C07C233/18 C07C255/42 C07C271/22 C07C215/30 A61K31/445 |
| D,A | EP 0 591 040 A (ELF SANOFI) 6 avril 1994 (1994-04-06) * revendications * | 1-22 | |
| D,A | EP 0 515 240 A (ELF SANOFI) 25 novembre 1992 (1992-11-25) * revendications * | 1-22 | |
| | | | DOMAINES TECHNIQUES RECHERCHES (IPC) |
| | | | C07D C07C A61K |

Le présent rapport a été établi pour toutes les revendications

| Lieu de la recherche | Date d'achèvement de la recherche | Examinateur |
|---|---|---|
| Munich | 2 juin 2006 | Gregoire, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 06 00 5775

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-06-2006

| Document brevet cité au rapport de recherche | Date de publication | Membre(s) de la famille de brevet(s) | Date de publication |
|---|---|---|---|
| EP 0512901 A | 11-11-1992 | AT 181550 T | 15-07-1999 |
| | | AU 652046 B2 | 11-08-1994 |
| | | AU 1591692 A | 05-11-1992 |
| | | BR 9201656 A | 15-12-1992 |
| | | CA 2067877 A1 | 04-11-1992 |
| | | CS 9201329 A3 | 18-11-1992 |
| | | DE 69229460 D1 | 29-07-1999 |
| | | DE 69229460 T2 | 20-01-2000 |
| | | DK 512901 T3 | 06-12-1999 |
| | | ES 2137176 T3 | 16-12-1999 |
| | | FI 921951 A | 04-11-1992 |
| | | FR 2676055 A1 | 06-11-1992 |
| | | GR 3030755 T3 | 30-11-1999 |
| | | HK 1005138 A1 | 12-05-2000 |
| | | HU 61539 A2 | 28-01-1993 |
| | | IE 921364 A1 | 04-11-1992 |
| | | IL 101760 A | 18-02-1997 |
| | | JP 3242980 B2 | 25-12-2001 |
| | | JP 5186425 A | 27-07-1993 |
| | | KR 258373 B1 | 01-07-2000 |
| | | MX 9202027 A1 | 01-01-1993 |
| | | NO 921734 A | 04-11-1992 |
| | | NZ 242586 A | 26-10-1995 |
| | | RU 2083574 C1 | 10-07-1997 |
| | | US 5340822 A | 23-08-1994 |
| | | ZA 9203178 A | 27-01-1993 |
| EP 0591040 A | 06-04-1994 | AT 176469 T | 15-02-1999 |
| | | AU 674875 B2 | 16-01-1997 |
| | | AU 4870893 A | 14-04-1994 |
| | | BR 9303982 A | 25-10-1994 |
| | | CA 2107432 A1 | 31-03-1994 |
| | | CN 1088582 A | 29-06-1994 |
| | | CZ 9302035 A3 | 13-04-1994 |
| | | DE 69323375 D1 | 18-03-1999 |
| | | DE 69323375 T2 | 24-06-1999 |
| | | DK 591040 T3 | 20-09-1999 |
| | | ES 2130238 T3 | 01-07-1999 |
| | | FI 934273 A | 31-03-1994 |
| | | FR 2696178 A1 | 01-04-1994 |
| | | GR 3030000 T3 | 30-07-1999 |
| | | HU 65759 A2 | 28-07-1994 |
| | | HU 9500518 A3 | 30-10-1995 |
| | | JP 2857041 B2 | 10-02-1999 |
| | | JP 6211850 A | 02-08-1994 |
| | | JP 3243212 B2 | 07-01-2002 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 06 00 5775

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-06-2006

| Document brevet cité au rapport de recherche | Date de publication | Membre(s) de la famille de brevet(s) | Date de publication |
|---|---|---|---|
| EP 0591040 A | | JP 10175976 A | 30-06-1998 |
| | | MX 9306024 A1 | 31-03-1994 |
| | | NO 933481 A | 05-04-1994 |
| | | NZ 248821 A | 26-10-1995 |
| | | OA 9869 A | 15-08-1994 |
| | | RU 2120436 C1 | 20-10-1998 |
| | | SG 47807 A1 | 17-04-1998 |
| | | SI 9300513 A | 30-06-1994 |
| | | ZA 9307276 A | 25-04-1994 |
| EP 0515240 A | 25-11-1992 | AT 158574 T | 15-10-1997 |
| | | AU 657321 B2 | 09-03-1995 |
| | | AU 1591892 A | 05-11-1992 |
| | | BR 9201655 A | 15-12-1992 |
| | | CA 2067924 A1 | 04-11-1992 |
| | | CS 9201328 A3 | 18-11-1992 |
| | | DE 69222352 D1 | 30-10-1997 |
| | | DE 69222352 T2 | 09-04-1998 |
| | | DK 515240 T3 | 11-05-1998 |
| | | ES 2109987 T3 | 01-02-1998 |
| | | FI 921950 A | 04-11-1992 |
| | | FR 2676054 A1 | 06-11-1992 |
| | | GR 3025277 T3 | 27-02-1998 |
| | | HU 65273 A2 | 02-05-1994 |
| | | IE 921365 A1 | 04-11-1992 |
| | | IL 101762 A | 16-10-1996 |
| | | JP 3108719 B2 | 13-11-2000 |
| | | JP 5140103 A | 08-06-1993 |
| | | KR 244063 B1 | 02-03-2000 |
| | | MX 9202026 A1 | 01-11-1992 |
| | | NO 921733 A | 04-11-1992 |
| | | NZ 242584 A | 27-04-1995 |
| | | RU 2089547 C1 | 10-09-1997 |
| | | US 5411971 A | 02-05-1995 |
| | | ZA 9203176 A | 28-04-1993 |

EPO FORM P0460

Pour tout renseignement concernant cette annexe : voir Journal Officiel de l'Office européen des brevets, No.12/82